# EUROPEAN PATENT APPLICATION

(11) **EP 1 785 486 A2**
(43) Date of publication of application: **16.05.2007**
(21) Application number: 07000636.6
(22) Date of filing: 17.01.2007
(51) Int. Cl.: C12N 15/31, C07K 14/315, C07K 16/12, G01N 33/50, A61K 39/09, C12Q 1/68

(54) **Streptococcus pneumoniae proteins and nucleic acid molecules**

(30) Priority: 27.07.1998 GB 9816337; 19.03.1999 US 125164 P
(62) Divisional of application: 99934989.7
(71) Applicant: Sanofi Pasteur Limited, Toronto, Ontario M2R 3T4 (CA)
(72) Inventor: Gilbert, Christophe François Guy, 69622 Villeurbanne Cedex (FR); Hansbro, Philip Michael, Newcastle, NSW 2300 (AU)
(74) Representative: Hart, Deborah Mary

(57) **Abstract**

Novel protein antigens from *Streptococcus pneumoniae* are disclosed, together with nucleic acid sequences encoding them. Their use in vaccines and in screening methods is also described.

## Description

The present invention relates to proteins derived from *Streptococcus pneumoniae,* nucleic acid molecules encoding such proteins, the use of the nucleic acid and/or proteins as antigens/immunogens and in detection/diagnosis, as well as methods for screening the proteins/nucleic acid sequences as potential anti-microbial targets.

*Streptococcus pneumoniae,* commonly referred to as the pneumococcus, is an important pathogenic organism. The continuing significance of *Streptoccocus pneumoniae* infections in relation to human disease in developing and developed countries has been authoritatively reviewed (Fiber, G.R., Science, 265: 1385-1387 (1994)). That indicates that on a global scale this organism is believed to be the most common bacterial cause of acute respiratory infections, and is estimated to result in 1 million childhood deaths each year, mostly in developing countries (Stansfield, S.K., Pediatr. Infect. Dis., 6: 622 (1987)). In the USA it has been suggested (Breiman et al, Arch. Intern. Med., 150: 1401 (1990)) that the pneumococcus is still the most common cause of bacterial pneumonia, and that disease rates are particularly high in young children, in the elderly, and in patients with predisposing conditions such as asplenia, heart, lung and kidney disease, diabetes, alcoholism, or with immunosupressive disorders, especially AIDS. These groups are at higher risk of pneumococcal septicaemia and hence meningitis and therefore have a greater risk of dying from pneumococcal infection. The pneumococcus is also the leading cause of otitis media and sinusitis, which remain prevalent infections in children in developed countries, and which incur substantial costs.

The need for effective preventative strategies against pneumococcal infection is highlighted by the recent emergence of penicillin-resistant pneumococci. It has been reported that 6.6% of pneumoccal isolates in 13 US hospitals in 12 states were found to be resistant to penicillin and some isolates were also resistant to other antibiotics including third generation cyclosporins (Schappert, S.M., Vital and Health Statistics of the Centres for Disease Control/National Centre for Health Statistics, 214:1 (1992)). The rates of penicillin resistance can be higher (up to 20%) in some hospitals (Breiman et al, J. Am. Med. Assoc., 271: 1831 (1994)). Since the development of penicillin resistance among pneumococci is both recent and sudden, coming after decades during which penicillin remained an effective treatment, these findings are regarded as alarming.

For the reasons given above, there are therefore compelling grounds for considering improvements in the means of preventing, controlling, diagnosing or treating pneumococcal diseases.

Various approaches have been taken in order to provide vaccines for the prevention of pneumococcal infections. Difficulties arise for instance in view of the variety of serotypes (at least 90) based on the structure of the polysaccharide capsule surrounding the organism. Vaccines against individual serotypes are not effective against other serotypes and this means that vaccines must include polysaccharide antigens from a whole range of serotypes in order to be effective in a majority of cases. An additional problem arises because it ahs been found that the capsular polysaccharides (each of which determines the serotype and is the major protective antigen) when purified and used as a vaccine do not reliably induce protective antibody responses in children under two years of age, the age group which suffers the highest incidence of invasive pneumococcal infection and meningitis.

A modification of the approach using capsule antigens relies on conjugating the polysaccharide to a protein in order to derive an enhanced immune response, particularly by giving the response T-cell dependent character. This approach has been used in the development of a vaccine against *Haemophilus influenzae.* There are issues of cost concerning both the multi-polysaccharide vaccines and those based on conjugates.

A third approach is to look for other antigenic components which offer the potential to be vaccine candidates. In the present application we provide a group of proteins antigens which are secreted/exported proteins.

Thus, in a first aspect the present invention provides a *Streptococcus pneumoniae* protein or polypeptide having a sequence selected from those shown in table 2 herein.

A protein or polypeptide of the present invention may be provided in substantially pure form. For example, it may be provided in a form which is substantially free of other proteins.

In a preferred embodiment, a protein or polypeptide having an amino acid sequence as shown in Table 3 is provided.

The invention encompasses any protein coded for by a nucleic acid sequence as shown in Table 1 herein.

As discussed herein, the proteins and polypeptides of the invention are useful as antigenic material. Such material can be "antigenic" and/or "immunogenic". Generally, "antigenic" is taken to mean that the protein or polypeptide is capable of being used to raise antibodies or indeed is capable of inducing an antibody response in a subject. "Immunogenic" is taken to mean that the protein or polypeptide is capable of eliciting a protective immune response in a subject. Thus, in the latter case, the protein or polypeptide may be capable of not only generating an antibody response and in addition non-antibody based immune responses.

The skilled person will appreciate that homologues or derivatives of the proteins or polypeptides of the invention will also find use in the context of the present invention, ie as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

In the case of homologues and derivatives, the degree of identity with a protein or polypeptide as described herein is less important than that the homologue or derivative should retain its antigenicity or immunogenicity to streptoccocus pneumoniae. However, suitably, homologues or derivatives having at least 60% similarity (as discussed above) with the proteins or polypeptides described herein are provided.

Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

In an additional aspect of the invention there are provided antigenic fragments of the proteins or polypeptides of the invention, or of homologues or derivatives thereof.

For fragments of the proteins or polypeptides described herein, or of homologues or derivatives thereof, the situation is slightly different. It is well known that is possible to screen an antigenic protein or polypeptide to identify epitopic regions, ie those regions which are responsible for the protein or polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties.

Thus, what is important for homologues, derivatives and fragments is that they possess at least a degree of the antigenicity/immunogenicity of the protein or polypeptide from which they are derived.

Gene cloning techniques may be used to provide a protein of the invention in substantially pure form. These techniques are disclosed, for example, in J. Sambrook et al Molecular Cloning 2nd Edition, Cold Spring Harbor Laboratory Press (1989). Thus, in a fourth aspect, the present invention provides a nucleic acid molecule comprising or consisting of a sequence which is:
(i) any of the DNA sequences set out in Table 1 or their RNA equivalents;
(ii) a sequence which is complementary to any of the sequences of (i);
(iii) a sequence which codes for the same protein or polypeptide, as those sequences of (i) or (ii);
(iv) a sequence which is has substantial identity with any of those of (i), (ii) and (iii);
(v) a sequence which codes for a homologue, derivative or fragment of a protein as defined in Table 1.

In a fifth aspect the present invention provides a nucleic acid molecule comprising or consisting of a sequence which is:
(i) any of the DNA sequences set out in Table 4 or their RNA equivalents;
(ii) a sequence which is complementary to any of the sequences of (i);
(iii) a sequence which codes for the same protein or polypeptide, as those sequences of (i) or (ii);
(iv) a sequence which is has substantial identity with any of those of (i), (ii) and (iii);
(v)a sequence which codes for a homologue, derivative or fragment of a protein as defined in Table 4.

The nucleic acid molecules of the invention may include a plurality of such sequences, and/or fragments. The skilled person will appreciate that the present invention can include novel variants of those particular novel nucleic acid molecules which are exemplified herein. Such variants are encompassed by the present invention. These may occur in nature, for example because of strain variation. For example, additions, substitutions and/or deletions are included. In addition, and particularly when utilising microbial expression systems, one may wish to engineer the nucleic acid sequence by making use of known preferred codon usage in the particular organism being used for expression. Thus, synthetic or non-naturally occurring variants are also included within the scope of the invention.

The term "RNA equivalent" when used above indicates that a given RNA molecule has a sequence which is complementary to that of a given DNA molecule (allowing for the fact that in RNA "U" replaces "T" in the genetic code).

When comparing nucleic acid sequences for the purposes of determining the degree of homology or identity one can use programs such as BESTFIT and GAP (both from the Wisconsin Genetics Computer Group (GCG) software package) BESTFIT, for example, compares two sequences and produces an optimal alignment of the most similar segments. GAP enables sequences to be aligned along their whole length and finds the optimal alignment by inserting spaces in either sequence as appropriate. Suitably, in the context of the present invention compare when discussing identity of nucleic acid sequences, the comparison is made by alignment of the sequences along their whole length.

Preferably, sequences which have substantial identity have at least 50% sequence identity, desirably at least 75% sequence identity and more desirably at least 90 or at least 95 % sequence identity with said sequences. In some cases the sequence identity may be 99% or above.

Desirably, the term "substantial identity" indicates that said sequence has a greater degree of identity with any of the sequences described herein than with prior art nucleic acid sequences.

It should however be noted that where a nucleic acid sequence of the present invention codes for at least part of a novel gene product the present invention includes within its scope all possible sequence coding for the gene product or for a novel part thereof.

The nucleic acid molecule may be in isolated or recombinant form. It may be incorporated into a vector and the vector may be incorporated into a host. Such vectors and suitable hosts form yet further aspects of the present invention.

Therefore, for example, by using probes based upon the nucleic acid sequences provided herein, genes in *Streptococcus pneumoniae* can be identified. They can then be excised using restriction enzymes and cloned into a vector. The vector can be introduced into a suitable host for expression.

Nucleic acid molecules of the present invention may be obtained from *S.pneumoniae* by the use of appropriate probes complementary to part of the sequences of the nucleic acid molecules. Restriction enzymes or sonication techniques can be used to obtain appropriately sized fragments for probing.

Alternatively PCR techniques may be used to amplify a desired nucleic acid sequence. Thus the sequence data provided herein can be used to design two primers for use in PCR so that a desired sequence, including whole genes or fragments thereof, can be targeted and then amplified to a high degree. One primer will normally show a high degree of specificity for a first sequence located on one strand of a DNA molecule, and the other primer will normally show a high degree of specificity for a second sequence located on the complementary strand of the DNA sequence and being spaced from the complementary sequence to the first sequence.

Typically primers will be at least 15-25 nucleotides long.

As a further alternative chemical synthesis may be used. This may be automated. Relatively short sequences may be chemically synthesised and ligated together to provide a longer sequence.

In yet a further aspect the present invention provides an immunogenic/antigenic composition comprising one or more proteins or polypeptides selected from those whose sequences are shown in Tables 2-4, or homologues or derivatives thereof, and/or fragments of any of these. In preferred embodiments, the immunogenic/antigenic composition is a vaccine or is for use in a diagnostic assay.

In the case of vaccines suitable additional excipients, diluents, adjuvants or the like may be included. Numerous examples of these are well known in the art.

It is also possible to utilise the nucleic acid sequences shown in Table 1 in the preparation of so-called DNA vaccines. Thus, the invention also provides a vaccine composition comprising one or more nucleic acid sequences as defined herein. The use of such DNA vaccines is described in the art. See for instance, Donnelly et al, Ann. Rev. Immunol., 15:617-648 (1997).

As already discussed herein the proteins or polypeptides described herein, their homologues or derivatives, and/or fragments of any of these, can be used in methods of detecting/diagnosing *S.pneumoniae.* Such methods can be based on the detection of antibodies against such proteins which may be present in a subject. Therefore the present invention provides a method for the detection/diagnosis of *S.pneumoniae* which comprises the step of bringing into contact a sample to be tested with at least one protein, or homologue, derivative or fragment thereof, as described herein. Suitably, the sample is a biological sample, such as a tissue sample or a sample of blood or saliva obtained from a subject to be tested.

In an alternative approach, the proteins described herein, or homologues, derivatives and/or fragments thereof, can be used to raise antibodies, which in turn can be used to detect the antigens, and hence *S.pneumoniae.* Such antibodies form another aspect of the invention. Antibodies within the scope of the present invention may be monoclonal or polyclonal.

Polyclonal antibodies can be raised by stimulating their production in a suitable animal host (e.g. a mouse, rat, guinea pig, rabbit, sheep, goat or monkey) when a protein as described herein, or a homologue, derivative or fragment thereof, is injected into the animal. If desired, an adjuvant may be administered together with the protein. Well-known adjuvants include Freund's adjuvant (complete and incomplete) and aluminium hydroxide. The antibodies can then be purified by virtue of their binding to a protein as described herein.

Monoclonal antibodies can be produced from hybridomas. These can be formed by fusing myeloma cells and spleen cells which produce the desired antibody in order to form an immortal cell line. Thus the well-known Kohler & Milstein technique (Nature 256 (1975)) or subsequent variations upon this technique can be used.

Techniques for producing monoclonal and polyclonal antibodies that bind to a particular polypeptide/protein are now well developed in the art. They are discussed in standard immunology textbooks, for example in Roitt et al, Immunology second edition (1989), Churchill Livingstone, London.

In addition to whole antibodies, the present invention includes derivatives thereof which are capable of binding to proteins etc as described herein. Thus the present invention includes antibody fragments and synthetic constructs. Examples of antibody fragments and synthetic constructs are given by Dougall et al in Tibtech 12 372-379 (September 1994).

Antibody fragments include, for example, Fab, F(ab')₂ and Fv fragments. Fab fragments (These are discussed in Roitt *et al* [*supra*]). Fv fragments can be modified to produce a synthetic construct known as a single chain Fv (scFv) molecule. This includes a peptide linker covalently joining Vₕ and V, regions, which contributes to the stability of the molecule. Other synthetic constructs that can be used include CDR peptides. These are synthetic peptides comprising antigen-binding determinants. Peptide mimetics may also be used. These molecules are usually conformationally restricted organic rings that mimic the structure of a CDR loop and that include antigen-interactive side chains.

Synthetic constructs include chimaeric molecules. Thus, for example, humanised (or primatised) antibodies or derivatives thereof are within the scope of the present invention. An example of a humanised antibody is an antibody having human framework regions, but rodent hypervariable regions. Ways of producing chimaeric antibodies are discussed for example by Morrison et al in PNAS, 81, 6851-6855 (1984) and by Takeda et al in Nature. 314, 452-454 (1985).

Synthetic constructs also include molecules comprising an additional moiety that provides the molecule with some desirable property in addition to antigen binding. For example the moiety may be a label (e.g. a fluorescent or radioactive label). Alternatively, it may be a pharmaceutically active agent.

Antibodies, or derivatives thereof, find use in detection/diagnosis of *S.pneumoniae.* Thus, in another aspect the present invention provides a method for the detection/diagnosis of *S.pneumoniae* which comprises the step of bringing into contact a sample to be tested and antibodies capable of binding to one or more proteins described herein, or to homologues, derivatives and/or fragments thereof.

In addition, so-called "Affibodies" may be utilised. These are binding proteins selected from combinatorial libraries of an alpha-helical bacterial receptor domain (Nord *et al* ,) Thus, Small protein domains, capable of specific binding to different target proteins can be selected using combinatorial approaches.

It will also be clear that the nucleic acid sequences described herein may be used to detect/diagnose *S.pneumoniae.* Thus, in yet a further aspect, the present invention provides a method for the detection/diagnosis of *S.pneumoniae* which comprises the step of bringing into contact a sample to be tested with at least one nucleic acid sequence as described herein. Suitably, the sample is a biological sample, such as a tissue sample or a sample of blood or saliva obtained from a subject to be tested. Such samples may be pre-treated before being used in the methods of the invention. Trhus, for example, a sample may be treated to extract DNA. Then, DNA probes based on the nucleic acid sequences described herein (ie usually fragments of such sequences) may be used to detect nucleic acid from *S.pneumoniae.*

In additional aspects, the present invention provides:
(a) a method of vaccinating a subject against *S.pneumoniae* which comprises the step of administering to a subject a protein or polypeptide of the invention, or a derivative, homologue or fragment thereof, or an immunogenic composition of the invention;
(b) a method of vaccinating a subject against *S.pneumoniae* which comprises the step of administering to a subject a nucleic acid molecule as defined herein;
(c) a method for the prophylaxis or treatment of *S.pneumoniae* infection which comprises the step of administering to a subject a protein or polypeptide of the invention, or a derivative, homologue or fragment thereof, or an immunogenic composition of the invention;
(d) a method for the prophylaxis or treatment of *S.pneumoniae* infection which comprises the step of administering to a subject a nucleic acid molecule as defined herein;
(e) a kit for use in detecting/diagnosing *S. pneumoniae* infection comprising one or more proteins or polypeptides of the invention, or homologues, derivatives or fragments thereof, or an antigenic composition of the invention; and
(f) a kit for use in detecting/diagnosing *S.pneumoniae* infection comprising one or more nucleic acid molecules as defined herein.

Given that we have identified a group of important proteins, such proteins are potential targets for anti-microbial therapy. It is necessary, however, to determine whether each individual protein is essential for the organism's viability. Thus, the present invention also provides a method of determining whether a protein or polypeptide as described herein represents a potential anti-microbial target which comprises inactivating said protein and determining whether *S.pneumoniae* is still viable, *in vitro* or *in vivo.*

A suitable method for inactivating the protein is to effect selected gene knockouts, ie prevent expression of the protein and determine whether this results in a lethal change. Suitable methods for carrying out such gene knockouts are described in Li et al, P.N.A.S., 94:13251-13256 (1997).

In a final aspect the present invention provides the use of an agent capable of antagonising, inhibiting or otherwise interfering with the function or expression of a protein or polypeptide of the invention in the manufacture of a medicament for use in the treatment or prophylaxis of *S.pneumoniae* infection.

The invention will now be described with reference to the following examples, which should not be construed as in any way limiting the invention. The examples refer to the figures in which:

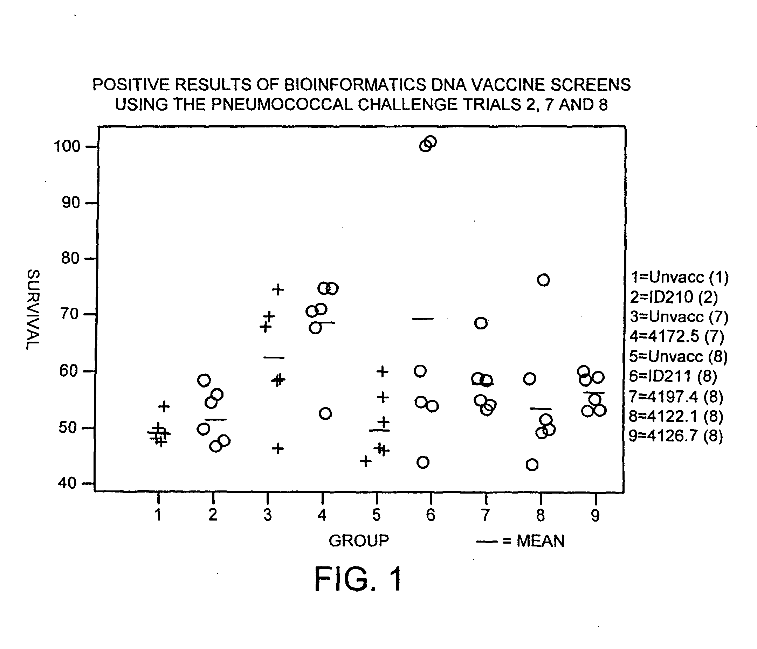

Figure 1: shows the results of various DNA vaccine trials; and

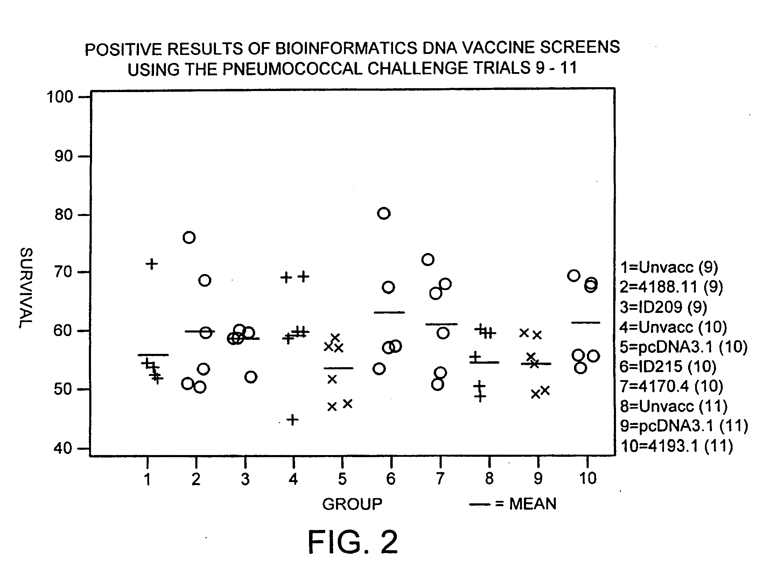

Figure 2: shows the results of further DNA vaccine trials.

### EXAMPLE 1

The Genome sequencing of *Streptococcus pneumoniae* type 4 is in progress at the

Institute for Genomic Research (TIGR, Rockville, MD, USA). Up to now, the whole sequence has not been completed or published. On 21^{st} November 1997, the TIGR centre released some DNA sequences as contigs which are not accurate reflections of the finished sequence. These contigs can be downloaded from their Webster (www@tigr.org). We downloaded these contigs and created a local database using the application GCGToBLAST (Wisconsin Package Version 9.1, Genetics Computer Group (GCG), Madison, USA). This database can be searched with the FastA and TfastA procedures (using the method of Pearson and Lipman (PNAS USA, 85:2444-2448 (1988)).

Using FastA and TfastA procedures, the local pneumococcus database was searched for putative leader sequence or anchor sequence features. Relevant sequences were used to interrogate for comparative novel sequences. These were:
(i) already described leader sequences of *Streptococcus pneumoniae* (from proteins NanA, NanB, LytA, PapA, pcpA, PsaA and PspA);
(ii) the leader sequence of Usp45, a secreted protein from *Lactococcus lactis*;
(iii) new hypothetical leader sequences derived from the searches in (i) and (ii);
(iv) the anchor motif LPxTG, a feature common to many Gram-positive bacteria surface proteins which are anchored by a mechanism involving the Sortase complex proteins.

Provided below is an example of this approach, with reference to the sequences derived from the database (see table 1).

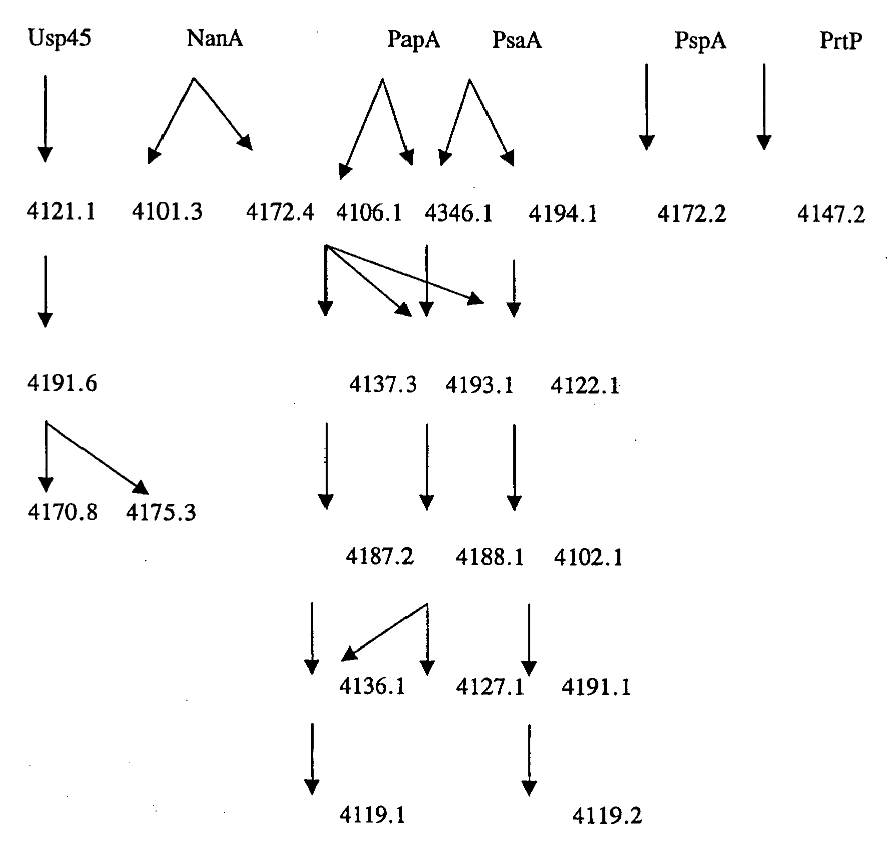

The protein leader sequences of different known exported proteins were used as a starting point for a search of the local pneumococcus database described above. The hypothetical proteins found with this search were then submitted to a Blast search in general databases such as EMBL, Swissprot etc. Proteins remaining unknown in the pneumococcus are kept and annotated. Then the search is performed again using the new potential protein leader sequence as a probe, using the TfastA procedure.

### Example 2: DNA vaccine trials

### pcDNA3.1+ as a DNA vaccine vector

### pcDNA3.1+

The vector chosen for use as a DNA vaccine vector was pcDNA3.1 (Invitrogen) (actually pcDNA3.1 +, the forward orientation was used in all cases but may be referred to as pcDNA3.1 here on). This vector has been widely and successfully employed as a host vector to test vaccine candidate genes to give protection against pathogens in the literature (Zhang, *et al.,* Kurar and Splitter, Anderson *et al.*). The vector was designed for high-level stable and non-replicative transient expression in mammalian cells. pcDNA3.1 contains the ColE1 origin of replication which allows convenient high-copy number replication and growth in *E. coli.* This in turn allows rapid and efficient cloning and testing of many genes. The pcDNA3.1 vector has a large number of cloning sites and also contains the gene encoding ampicillin resistance to aid in cloning selection and the human cytomegalovirus (CMV) immediate-early promoter/enhancer which permits efficient, high-level expression of the recombinant protein. The CMV promoter is a strong viral promoter in a wide range of cell types including both muscle and immune (antigen presenting) cells. This is important for optimal immune response as it remains unknown as to which cells types are most important in generating a protective response *in vivo.* A T7 promoter upstream of the multiple cloning site affords efficient expression of the modified insert of interest and which allows *in vitro* transcription of a cloned gene in the sense orientation.

Zhang, D., Yang, X., Berry, J. Shen, C., McClarty, G. and Brunham, R.C. (1997) "DNA vaccination with the major outer-membrane protein genes induces acquired immunity to Chlamydia trachomatis (mouse pneumonitis) infection". Infection and Immunity, 176, 1035-40.

Kurar, E. and Splitter, G.A. (1997) "Nucleic acid vaccination of Brucella abortus ribosomal L7/L12 gene elicits immune response". Vaccine, 15, 1851-57.

Anderson, R., Gao, X.-M., Papakonstantinopoulou, A., Roberts, M. and Dougan, G. (1996) "Immune response in mice following immunisation with DNA encoding fragment C of tetanus toxin". Infection and Immunity, 64, 3168-3173.

### Preparation of DNA vaccines

Oligonucleotide primers were designed for each individual gene of interest derived using the LEEP system. Each gene was examined thoroughly, and where possible, primers were designed such that they targeted that portion of the gene thought to encode only the mature portion of the gene protein. It was hoped that expressing those sequences that encode only the mature portion of a target gene protein, would facilitate its correct folding when expressed in mammalian cells. For example, in the majority of cases primers were designed such that putative N-terminal signal peptide sequences would not be included in the final amplification product to be cloned into the pcDNA3.1 expression vector. The signal peptide directs the polypeptide precursor to the cell membrane via the protein export pathway where it is normally cleaved off by signal peptidase **I** (or signal peptidase **II** if a lipoprotein). Hence the signal peptide does not make up any part of the mature protein whether it be displayed on the surface of the bacteria surface or secreted. Where a N-terminal leader peptide sequence was not immediately obvious, primers were designed to target the whole of the gene sequence for cloning and ultimately, expression in pcDNA3.1.

Having said that, however, other additional features of proteins may also affect the expression and presentation of a soluble protein. DNA sequences encoding such features in the genes encoding the proteins of interest were excluded during the design of oligonucleotides. These features included:
1. LPXTG cell wall anchoring motifs.
2. LXXC ipoprotein attachment sites.
3. Hydrophobic C-terminal domain.
4. Where no N-terminal signal peptide or LXXC was present the start codon was excluded.
5. Where no hydrophobic C-terminal domain or LPXTG motif was present the stop codon was removed.

Appropriate PCR primers were designed for each gene of interest and any and all of the regions encoding the above features was removed from the gene when designing these primers. The primers were designed with the appropriate enzyme restriction site followed by a conserved Kozak nucleotide sequence (in all cases) GCCACC was used. The Kozak sequence facilitates the recognition of initiator sequences by eukaryotic ribosomes) and an ATG start codon upstream of the insert of the gene of interest. For example the forward primer using a BamH1 site the primer would begin GCGGGATCCGCCACCATG followed by a small section of the 5' end of the gene of interest. The reverse primer was designed to be compatible with the forward primer and with a Not1 restriction site at the 5' end in all cases (this site is TTGCGGCCGC).

### PCR primers

The following PCR primers were designed and used to amplify the truncated genes of interest.

### ID210

Forward Primer 5' CGGATCCGCCACCATGTCTTCTAATGAATCTGCCGATG 3'
Reverse Primer 5' TTGCGGCCGCCTGTTTAGATTGGATATCTGTAAAGACTT 3'

### 4172.5

Forward Primer 5'
CGCGGATCCGCCACCATGGATTTTCCTTCAAATTTGGAGG 3'
Reverse Primer 5' TTGCGGCCGCACCGTACTGGCTGCTGACT 3'

### ID211

Forward Primer 5' CGGATCCGCCACCATGAGTGAGATCAAAATTATTAACGC 3'
Reverse Primer 5' TTGCGGCCGCCGTTCCATGGTTGACTCCT 3'

### 4197.4

Forward Primer 5' CGCGGATCCGCCACCATGTGGGACATATTGGTGGAAAC 3'
Reverse Primer 5' TTGCGGCCGCTTCACTTGAGCAAACTGAATCC 3'

### 4122.1

Forward Primer 5'
CGCGGATCCGCCACCATGTCACAAGAAAAAACAAAAAATGAA 3'
Reverse Primer 5' TTGCGGCCGCATCGACGTAGTCTCCGCC.3'

### 4126.7

Forward Primer 5'
CGCGGATCCGCCACCATGCTGGTTGGAACTTTCTACTATCAAT 3'
Reverse Primer 5' TTGCGGCCGCAACTTTCGTCCCTTTTTGG 3'

### 4188.11

Forward Primer 5' CGCGGATCCGCCACCATGGGCAATTCTGGCGGAA 3'
Reverse Primer 5' TTGCGGCCGCTTGTTTCATAGCTTTTTTGATTGTT 3'

### ID209

Forward Primer 5'
CGCGGATCCGCCACCATGCTATTGATACGAAATGCAGGG3'
Reverse Primer 5' TTGCGGCCGCAACATAATCTAGTAAATAAGCGTAGCC 3'

### ID215

Forward Primer 5' CGCGGATCCGCCACCATGACGGCGACGAATTTTC 3'
Reverse Primer 5' TTGCGGCCGCTTAATTCGTTTTTGAACTAGTTGCT 3'

### 4170.4

Forward Primer 5'
CGCGGATCCGCCACCATGGCTGTTTTTCTTCGCTATCATG 3'
Reverse Primer 5' TTGCGGCCGCTTTCTTCAACAAACCTTGTTCTTG 3'

### 4193.1

Forward Primer 5'
CGCGGATCCGCCACCATGGGTAACCGCTCTTCTCGTAAC 3'
Reverse Primer 5' TTGCGGCCGCGCTTCCATCAAGGATTTTAGC 3'

### Cloning

The insert along with the flanking features described above was amplified using PCR against a template of genomic DNA isolated from type 4 *S. pneumoniae* strain 11886 obtained from the National Collection of Type Cultures. The PCR product was cut with the appropriate restriction enzymes and cloned in to the multiple cloning site of pcDNA3.1 using conventional molecular biological techniques. Suitably mapped clones of the genes of interested were cultured and the plasmids isolated on a large scale (> 1.5 mg) using Plasmid Mega Kits (Qiagen). Successful cloning and maintenance of genes was confirmed by restriction mapping and sequencing ~ 700 base pairs through the 5' cloning junction of each large scale preparation of each construct.

### Strain validation

A strain of type 4 was used in cloning and challenge methods which is the strain from which the *S. pneumoniae* genome was sequenced. A freeze dried ampoule of a homogeneous laboratory strain of type 4 *S. pneumoniae* strain NCTC 11886 was obtained from the National Collection of Type Strains. The ampoule was opened and the cultured re suspended with 0.5 ml of tryptic soy broth (0.5% glucose, 5% blood). The suspension was subcultured into 10 ml tryptic soy broth (0.5% glucose, 5% blood) and incubated statically overnight at 37°C. This culture was streaked on to 5 % blood agar plates to check for contaminants and confirm viability and on to blood agar slopes and the rest of the culture was used to make 20% glycerol stocks. The slopes were sent to the Public Health Laboratory Service where the type 4 serotype was confirmed.

A glycerol stock of NCTC 11886 was streaked on a 5% blood agar plate and incubated overnight in a CO2 gas jar at 37°C. Fresh streaks were made and optochin sensitivity was confirmed.

### Pneumococcal challenge

A standard inoculum of type 4 *S. pneumoniae* was prepared and frozen down by passaging a culture of pneumococcus 1x through mice, harvesting from the blood of infected animals, and grown up to a predetermined viable count of around 10⁹ cfu/ml in broth before freezing down. The preparation is set out below as per the flow chart.

Streak pneumococcal culture and confirm identity
|
V
Grow over-night culture from 4-5 colonies on plate above
|
V
Animal passage pneumococcal culture
(i.p. injection of cardiac bleed to harvest)
|
V
Grow over-night culture from animal passaged pneumococcus

|
V
Grow day culture (to pre-determined optical density) from over-night of animal
passage and freeze down at -70°C - This is standard minimum
|
V
Thaw one aliquot of standard inoculum to viable count
|
V
Use standard inoculum to determine effective dose (called Virulence Testing)
|
V
All subsequent challenges - use standard inoculum to effective dose

An aliquot of standard inoculum was diluted 500x in PBS and used to inoculate the mice.

Mice were lightly anaesthetised using halothane and then a dose of 1.4 x 10⁵ cfu of pneumococcus was applied to the nose of each mouse. The uptake was facilitated by the normal breathing of the mouse, which was left to recover on its back.

### S. pneumoniae vaccine trials

Vaccine trials in mice were carried out by the administration of DNA to 6 week old CBA/ca mice (Harlan, UK). Mice to be vaccinated were divided into groups of six and each group was immunised with recombinant pcDNA3.1 + plasmid DNA containing a specific target-gene sequence of interest. A total of 100 µg of DNA in Dulbecco's PBS (Sigma) was injected intramuscularly into the tibialis anterior muscle of both legs (50 µl in each leg). A boost was carried using the same procedure 4 weeks later. For comparison, control groups were included in all vaccine trials. These control groups were either unvaccinated animals or those administered with non-recombinant pcDNA3.1 + DNA (sham vaccinated) only, using the same time course described above. 3 weeks after the second immunisation, all mice groups were challenged intra-nasally with a lethal dose of *S. pneumoniae* serotype 4 (strain NCTC 11886). The number of bacteria administered was monitored by plating serial dilutions of the inoculum on 5% blood agar plates. A problem with intranasal immunisations is that in some mice the inoculum bubbles out of the nostrils, this has been noted in results table and taken account of in calculations. A less obvious problem is that a certain amount of the inoculum for each mouse may be swallowed. It is assumed that this amount will be the same for each mouse and will average out over the course of innoculations. However, the sample sizes that have been used are small and this problem may have significant effects in some experiments. All mice remaining after the challenge were killed 3 or 4 days after infection. During the infection process, challenged mice were monitored for the development of symptoms associated with the onset of *S. pneumoniae* induced-disease. Typical symptoms in an appropriate order included piloerection, an increasingly hunched posture, discharge from eyes, increased lethargy and reluctance to move. The latter symptoms usually coincided with the development of a moribund state at which stage the mice were culled to prevent further suffering. These mice were deemed to be very close to death, and the time of culling was used to determine a survival time for statistical analysis. Where mice were found dead, the survival time was taken as the last time point when the mouse was monitored alive.

### Interpretation of Results

A positive result was taken as any DNA sequence that was cloned and used in challenge experiments as described above which gave protection against that challenge. Protection was taken as those DNA sequences that gave statistically significant protection (to a 95% confidence level (p<0.05)) and also those which were marginal or close to significant using Mann-Whitney or which show some protective features for example there were one or more outlying mice or because the time to the first death was prolonged. It is acceptable to allow marginal or non-significant results to be considered as potential positives when it is considered that the clarity of some of the results may be clouded by the problems associated with the administration of intranasal infections.

| **Results for vaccine trials 2, 7 and 8 (see figure 1)** | | | | | | | | | |
|---|---|---|---|---|---|---|---|---|---|
| **Mouse number** | **Mean survival times (hours)** | | | | | | | | |
| | **Unvacc control (2)** | **ID210 (2)** | **Unvacc control (7)** | **4172.5 (7)** | **Unvacc control (8)** | **ID211 (8)** | **4197.4 (8)** | **4122.1 (8)** | **4126.7 (8)** |
| 1 | 49.0 | 55.0 | 59.6 | 72.6 | 45.1 | 102.3T | 60.1 | 50.6 | 60.0 |
| 2 | 51.0 | 46.5 | 47.2 | 67.9 | 50.8 | 55.5 | 54.9 | 77.2 | 60.0 |
| 3 | 49.0 | 49.0 | 59.6 | 54.4 | 60.4 | 60.6* | 68.4 | 60.3 | 54.8 |
| 4 | 55.0 | 59.0 | 70.9 | 75.3 | 55.2 | 45.3 | 60.1 | 50.6 | 52.6 |
| 5 | 49.0 | 55.0 | 68.6* | 70.9 | 45.1 | 55.5 | 54.9 | 50.6* | 54.8 |
| 6 | 49.0 | 49.0 | 76.0 | 75.3 | 45.1 | 102.3T | 52.7 | 44.9 | 60 |
| **Mean** | **50.3** | **52.3** | **63.6** | **69.4** | **50.2** | **70.2** | **58.5** | **55.7** | **57.0** |
| **sd** | **2.4** | **4.8** | **10.3** | **7.9** | **6.4** | **25.3** | **5.7** | **11.6** | **3.4** |
| **p value 1** | **-** | **0.3333** | **-** | **0.2104** | **-** | **0.0215** | **0.0621** | **0.4038** | **0.0833** |

| | | | | | | | | | |
|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value 1 refers to significance tests compared to unvaccinated controls | | | | | | | | | |

### Statistical Analyses.

Trial 2 - The group vaccinated with ID210 also had a longer mean survival time than the unvaccinated controls but the results are not statistically significant.

Trial 7 - The group vaccinated with 4172.5 showed much greater survival times than unvaccinated controls although the differences were not statistically significant.

Trial 8 - The group vaccinated with ID211 survived significantly longer than unvaccinated controls. 4197.4, 4122.1 and 4126.7 vaccinated groups showed longer mean survival times than the unvaccinated group but the results were not statistically significant. The 4197.4 and 4126.7 groups also showed a prolonged time to the first death and the 4122.1 group showed 1 outlying result.

| **Results of pneumococcal challenge DNA vaccination trials 9-11 (see figure 2)** | | | | | | | | | | |
|---|---|---|---|---|---|---|---|---|---|---|
| **Mouse number** | **Mean survival times (hours)** | | | | | | | | | |
| | **Unvacc control (9)** | **4188.1 1 (9)** | **ID209 (9)** | **Unvacc control** (10) | **pcDNA3.1 + (10)** | **ID215 (10)** | **4170. 4** (10) | **Unvacc control** (11) | **pcDNA3.1 + (11)** | **4193.1 (11)** |
| 1 | (98.5)T | 69.4 | 60.2 | 68.4 | 58.6 | 79.2 | 68.1 | 60.0 | 53.2 | 54.8 |
| 2 | 53.4 | 53.7 | 60.2 | 59.0 | 58.6 | 54.2 | 58.6 | 50.0 | 50.4 | 54.8 |
| 3 | 53.4 | 51.2 | 60.2 | 59.0 | 50.8 | (103.2)*T | 50.9 | 60.0 | 55.4 | 68.7* |
| 4 | 53.4 | 75.0 | (98.0)*T | 45.1* | 58.6 | 58.8 | 72.1 | 55.0 | 60.6 | 54.8 |
| 5 | 70.8 | 51.2 | 60.2 | 68.4 | 46.5 | 68.3 | 68.1 | 60.0 | 50.4 | 68.7 |
| 6 | 53.4 | 61.2 | 52.9 | 59.0 | 48.9 | 58.8 | 54.0 | 50.0 | 60.6 | 68.7* |
| **Mean** | **56.9** | **60.3** | **58.8** | **59.8** | **53.6** | **63.9** | **62.0** | **55.8** | **55.1** | **61.7** |
| **Sd** | **7.8** | **10.0** | **3.3** | **8.5** | **5.6** | **10.0** | **8.7** | **5.0** | **4.6** | **7.6** |
| **p value 1** | **-** | **0.3894** | **0.2519** | **-** | **0.0307** | **< 30.0** | **< 39. 0** | **-** | **-** | **0.1837** |
| **p value 2** | **-** | **-** | **-** | **-** | **-** | **0.0168** | **0.031 6** | **-** | **-** | **0.0829** |

| | | | | | | | | | | |
|---|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value I refers to significance tests compared to unvaccinated controls p value 2 refers to significance tests compared to pcDNA3.1 + vaccinated controls | | | | | | | | | | |

### Statistical Analyses.

Trial 9 - Although not statistically significant the groups vaccinated with 4188.11 and ID209 did have noticeably higher mean survival times than unvaccinated controls.

Trial 10 - The unvaccinated control group survived for a significantly longer period than the pcDNA3.1 + vaccinated group.

The groups vaccinated with ID215 and 4170.4 showed statistically significant longer survival times compared to the sham vaccinated group (p=0.0168 and 0.0316) but not compared to the unvaccinated group.

Trial 11 - The group vaccinated with 4193.1 was the most promising and survived an average of 6.5 hours longer than the pcDNA3.1 + vaccinated group and 6 hours longer than the unvaccinated group although the results were not statistically significant.

Table 1

4101.1
ATGGAAGAGTTAGTGACCTTAGATTGTTTGTTTATTGACAGAACTAAGATTGAAGCCAATGCCAACAAGTATAGTT
TTGTGTGGAAGAAAACGACAGAGAAATTCTCCGCCAAACTTCAAGAACAGATACAGGTCTATTTCAAGAAGAAA
TCACTCCCCTTCTGATTAAATATGCCATGTTTGATAAGAAACAAAAGAGAGGGTATAAAGAGTCAGCTAAAAACT
TAGCGAATTGGCACTATAATGACAAGGAGGATAGCTACACACATCCTGATGGCTGGTATTATCGTTTTCACCATAC
CAAATATCAGAAAACACAGACAGACTTTCAACAAGAAATCAAGGTTTACTACGCCGACGAACCTGAATCAGCCCC
TCAAAAGGGACTGTATATGAACGAACGCTATCAAAACTTGAAAGCTAAAGAATGTCAGGCGCTTTTATCTCCCCA
AGGTAGACAGATTTTCGCTCAACGCAAGATTGATGTGGAACCTGTCTTTGGGCAGATAAAGGCTTCTTTGGGTTAC
AAGAGATGTAATCTGAGAGGGAAGCGTCAAGTGAGAATTGACATGGGATTGGTACTTATGGCCAATAACCTCCTA
AAATATAGTAAAATGAAATAA

4101.3
ATGGGGAAAGGCCATTGGAATCGGAAAAGAGTTTATAGCATTCGTAAGTTTGCTGTGGGAGCTTGCTCAGTAATG
ATTGGGACTTGTGCAGTTTTATTAGGAGGAAATATAGCTGGAGAATCTGTAGTTTATGCGGATGAAACACTTATTA
CTCATACTGCTGAGAAACCTAAAGAGGAAAAAATGATAGTAGAAGAAAAGGCTGATAAAGCTTTGGAAACTAAA
AATATAGTTGAAAGGACAGAACAAAGTGAACCTAGTTCAACTGAGGCTATTGCATCTGAGAAGAAAGAAGATGAA
GCCGTAACTCCAAAAGAGGAAAAAGTGTCTGCTAAACCGGAAGAAAAAGCTCCAAGGATAGAATCACAAGCTTC
AAATCAAGAAAAACCGCTCAAGGAAGATGCTAAAGCTGTAACAAATGAAGAAGTGAATCAAATGATTGAAGACA
GGAAAGTGGATTTTAATCAAAATTGGTACTTTAAACTCAATGCAAATTCTAAGGAAGCCATTAAACCTGATGCAG
ACGTATCTACGTGGAAAAAATTAGATTTACCGTATGACTGGAGTATCTTTAACGATTTCGATCATGAATCTCCTGC
ACAAAATGAAGGTGGACAGCTCAACGGTGGGGAAGCTTGGTATCGCAAGACTTTCAAACTAGATGAAAAAGACCT
CAAGAAAAATGTTCGCCTTACTTTTGATGGCGTCTACATGGATTCTCAAGTTTATGTCAATGGTCAGTTAGTGGGG
CATTATCCAAATGGTTATAACCAGTTCTCATATGATATCACCAAATACCTTCAAAAAGATGGTCGTGAGAATGTGA
TTGCTGTCCATGCAGTCAACAAACAGCCAAGTAGCCGTTGGTATTCAGGAAGTGGTATCTATCGTGATGTGACTTT
ACAAGTGACAGATAAGGTGCATGTTGAGAAAAATGGGACAACTATTTTAACACCAAAACTTGAAGAACAACAACA
TGGCAAGGTTGAAACTCATGTGACCAGCAAAATCGTCAATACGGACGACAAAGACCATGAACTTGTAGCCGAATA
TCAAATCGTTGAACGAGGTGGTCATGCTGTAACAGGCTTAGTTCGTACAGCGAGTCGTACCTTAAAAGCACATGA
ATCAACAAGCCTAGATGCGATTTTAGAAGTTGAAAGACCAAAACTCTGGACTGTTTTAAATGACAAACCTGCCTTG
TACGAATTGATTACGCGTGTTTACCGTGACGGTCAATTGGTTGATGCTAAGAAGGATTTGTTTGGTTACCGTTACT
ATCACTGGACTCCAAATGAAGGTTTCTCTTTGAATGGTGAACGTATTAAATTCCATGGAGTATCCTTGCACCACGA
CCATGGGGCGCTTGGAGCAGAAGAAAACTATAAAGCAGAATATCGCCGTCTCAAACAAATGAAGGAGATGGGAG
TTAACTCCATCCGTACAACCCACAACCCTGCTAGTGAGCAAACCTTGCAAATCGCAGCAGAACTAGGTTTACTCGT
TCAGGAAGAGGCCTTTGATACGTGGTATGGTGGCAAGAAACCTTATGACTATGGACGTTTCTTTGAAAAAGATGC
CACTCACCCAGAAGCTCGAAAAGGTGAAAAATGGTCTGATTTTGACCTACGTACCATGGTCGAAAGAGGCAAAAA
CAACCCTGCTATCTTCATGTGGTCAATTGGTAATGAAATAGGTGAAGCTAATGGTGATGCCCACTCTTTAGCAACT
GTTAAACGTTTGGTTAAGGTTATCAAGGATGTTGATAAGACTCGCTATGTTACCATGGGAGCAGATAAATTCCGTT
TCGGTAATGGTAGCGGAGGGCATGAGAAAATTGCTGATGAACTCGATGCTGTTGGATTTAACTATTCTGAAGATA
ATTACAAAGCCCTTAGAGCTAAGCATCCAAAATGGTTGATTTATGGATCAGAAACATCTTCAGCTACCCGTACACG
TGGAAGTTACTATCGCCCTGAACGTGAATTGAAACATAGCAATGGACCTGAGCGTAATTATGAACAGTCAGATTA
TGGAAATGATCGTGTGGGTTGGGGGAAAACAGCAACCGCTTCATGGACTTTTGACCGTGACAACGCTGGCTATGC
TGGACAGTTTATCTGGACAGGTACGGACTATATTGGTGAACCTACACCATGGCACAACCAAAATCAAACTCCTGTT
AAGAGCTCTTACTTTGGTATCGTAGATACAGCCGGCATTCCAAAACATGACTTCTATCTCTACCAAAGCCAATGGG
TTTCTGTTAAGAAGAAACCGATGGTACACCTTCTTCCTCACTGGAACTGGGAAAACAAAGAATTAGCATCCAAAG
TAGCTGACTCAGAAGGTAAGATTCCAGTTCGTGCTTATTCGAATGCTTCTAGTGTAGAATTGTTCTTGAATGGAAA
ATCTCTTGGTCTTAAGACTTTCAATAAAAAACAAACCAGCGATGGGCGGACTTACCAAGAAGGTGCAAATGCTAA
TGAACTTTATCTTGAATGGAAAGTTGCCTATCAACCAGGTACCTTGGAAGCAATTGCTCGTGATGAATCTGGCAAG
GAAATTGCTCGAGATAAGATTACGACTGCTGGTAAGCCAGCGGCAGTTCGTCTTATTAAGGAAGACCATGCGATT
GCAGCAGATGGAAAAGACTTGACTTACATCTACTATGAAATTGTTGACAGCCAGGGGAATGTGGTTCCAACTGCT
AATAATCTGGTTCGCTTCCAATTGCATGGCCAAGGTCAACTGGTCGGTGTAGATAACGGAGAACAAGCCAGCCGT
GAACGCTATAAGGCGCAAGCAGATGGTTCTTGGATTCGTAAAGCATTTAATGGTAAAGGTGTTGCCATTGTCAAAT
CAACTGAACAAGCAGGGAAATTCACCCTGACTGCCCACTCTGATCTCTTGAAATCGAACCAAGTCACTGTCTTTAC
TGGTAAGAAAGAAGGACAAGAGAAGACTGTTTTGGGGACAGAAGTGCCAAAAGTACAGACCATTATTGGAGAGG
CACCTGAAATGCCTACCACTGTTCCGTTTGTATACAGTGATGGTAGCCGTGCAGAACGTCCTGTAACCTGGTCTTC
AGTAGATGTGAGCAAGCCTGGTATTGTAACGGTGAAAGGTATGGCTGACGGACGAGAAGTAGAAGCTCGTGTAGA
AGTGATTGCTCTTAAATCAGAGCTACCAGTTGTGAAACGTATTGCTCCAAATACTGACTTGAATTCTGTAGACAAA
TCTGTTTCCTATGTTTTGATTGATGGAAGTGTTGAAGAGTATGAAGTGGACAAGTGGGAGATTGCCGAAGAAGATA
AAGCTAAGTTAGCAATTCCAGGTTCTCGTATTCAAGCGACCGGTTATTTAGAAGGTCAACCAATTCATGCAACCCT
TGTGGTAGAAGAAGGCAATCCTGCGGCACCTGCAGTACCAACTGTAACGGTTGGTGGTGAGGCAGTAACAGGTCT
TACTAGTCAAAAACCAATGCAATACCGCACTCTTGCTTATGGAGCTAAGTTGCCAGAAGTCACAGCAAGTGCTAA
AAATGCAGCTGTTACAGTTCTTCAAGCAAGCGCAGCAAACGGCATGCGTGCGAGCATCTTTATTCAGCCTAAAGA
TGGTGGCCCTCTTCAAACCTATGCAATTCAATTCCTTGAAGAAGCGCCAAAAATTGCTCACTTGAGCTTGCAAGTG
GAAAAAGCTGACAGTCTCAAAGAAGACCAAACTGTCAAATTGTCGGTTCGAGCTCACTATCAAGATGGAACGCAA

GCTGTATTACCAGCTGATAAAGTAACCTTCTCTACAAGTGGTGAAGGGGAAGTCGCAATTCGTAAAGGAATGCTT
GAGTTGCATAAGCCAGGAGCAGTCACTCTGAACGCTGAATATGAGGGAGCTAAAGACCAAGTTGAACTCACTATC
CAAGCCAATACTGAGAAGAAGATTGCGCAATCCATCCGTCCTGTAAATGTAGTGACAGATTTGCATCAGGAACCA
AGTCTTCCAGCAACAGTAACAGTTGAGTATGACAAAGGTTTCCCTAAAACTCATAAAGTCACTTGGCAAGCTATTC
CGAAAGAAAAACTAGACTCCTATCAAACATTTGAAGTACTAGGTAAAGTTGAAGGAATTGACCTTGAAGCGCGTG
CAAAAGTCTCTGTAGAAGGTATCGTTTCAGTTGAAGAAGTCAGTGTGACAACTCCAATCGCAGAAGCACCACAAT
TACCAGAAAGTGTTCGGACATATGATTCAAATGGTCACGTTTCATCAGCTAAGGTTGCATGGGATGCGATTCGTCC
AGAGCAATACGCTAAGGAAGGTGTCTTTACAGTTAATGGTCGCTTAGAAGGTACGCAATTAACAACTAAACTTCA
TGTTCGCGTATCTGCTCAAACTGAGCAAGGTGCAAACATTTCTGACCAATGGACCGGTTCAGAATTGCCACTTGCC
TTTGCTTCAGACTCAAATCCAAGCGACCCAGTTTCAAATGTTAATGACAAGCTCATTTCCTACAATAACCAACCAG
CCAATCGTTGGACAAACTGGAATCGTACTAATCCAGAAGCTTCAGTCGGTGTTCTGTTTGGAGATTCAGGTATCTT
GAGCAAACGCTCCGTTGATAATCTAAGTGTCGGATTCCATGAAGACCATGGAGTTGGTGTACCGAAGTCTTATGTG
ATTGAGTATTATGTTGGTAAGACTGTCCCAACAGCTCCTAAAAACCCTAGTTTTGTTGGTAATGAGGACCATGTCT
TTAATGATTCTGCCAACTGGAAACCAGTTACTAATCTAAAAGCCCCTGCTCAACTCAAGGCTGGAGAAATGAACC
ACTTTAGCTTTGATAAAGTTGAAACCTATGCTGTTCGTATTCGCATGGTTAAAGCAGATAACAAGCGTGGAACGTC
TATCACAGAGGTACAAATCTTTGCGAAACAAGTTGCGGCAGCCAAGCAAGGACAAACAAGAATCCAAGTTGACGG
CAAAGACTTAGCAAACTTCAACCCTGATTTGACAGACTACTACCTTGAGTCTGTAGATGGAAAAGTTCCGGCAGTC
ACAGCAAGTGTTAGCAACAATGGTCTCGCTACCGTCGTTCCAAGCGTTCGTGAAGGTGAGCCAGTTCGTGTCATCG
CGAAAGCTGAAAATGGCGACATCTTAGGAGAATACCGTCTGCACTTCACTAAGGATAAGAGCTTACTTTCTCATA
AACCAGTTGCTGCGGTTAAACAAGCTCGCTTGCTACAAGTAGGTCAAGCACTTGAATTGCCGACTAAGGTTCCAGT
TTACTTCACAGGTAAAGACGGCTACGAAACAAAAGACCTGACAGTTGAATGGAAGAAGTTCCAGCGGAAAATCT
GACAAAAGCAGGTCAATTTACTGTTCGAGGCCGTGTCCTTGGTAGTAACCTTGTTGCTGAGATCACTGTACGAGTG
ACAGACAAACTTGGTGAGACTCTTTCAGATAACCCTAACTATGATGAAAACAGTAACCAGGCCTTTGCTTCAGCA
ACCAATGATATTGACAAAAACTCTCATGACCGCGTTGACTATCTCAATGACGGAGATCATTCAGAAAATCGTCGTT
GGACAAACTGGTCACCAACACCATCTTCTAATCCAGAAGTATCAGCGGGTGTGATTTTCCGTGAAAATGGTAAGA
TTGTAGAACGGACTGTTACACAAGGAAAAGTTCAGTTCTTTGCAGATAGTGGTACGGATGCACCATCTAAACTCGT
TTTAGAACGCTATGTCGGTCCAGAGTTTGAAGTGCCAACCTACTATTCAAACTACCAAGCCTACGACGCAGACCAT
CCATTCAACAATCCAGAAAATTGGGAAGCTGTTCCTTATCGTGCGGATAAAGACATTGCAGCTGGTGATGAAATC
AACGTAACATTTAAAGCTATCAAAGCCAAAGCTATGAGATGGCGTATGGAGCGTAAAGCAGATAAGAGCGGTGTT
GCGATGATTGAGATGACCTTCCTTGCACCAAGTGAATTGCCTCAAGAAAGCACTCAATCAAAGATTCTTGTAGATG
GAAAAGAACTTGCTGATTTCGCTGAAAATCGTCAAGACTATCAAATTACCTATAAAGGTCAACGGCCAAAAGTCT
CAGTTGAAGAAAACAATCAAGTAGCTTCAACTGTGGTAGATAGTGGAGAAGATAGCTTTCCAGTACTTGTTCGCCT
CGTTTCAGAAAGTGGAAAACAAGTCAAGGAATACCGTATCCACTTGACTAAGGAA
AAACCAGTTTCTGAGAAGACAGTTGCTGCTGTACAAGAAGATCTTCCAAAAATCGAATTTGTTGAAAAAGATTTG
GCATACAAGACAGTTGAGAAAAAAGATTCAACACTGTATCTAGGTGAAACTCGTGTAGAACAAGAAGGAAAAGTT
GGAAAAGAACGTATCTTTACAGCGATTAATCCTGATGGAAGTAAGGAAGAAAAACTCCGTGAAGTGGTAGAAGTT
CCGACAGACCGCATCGTCTTGGTTGGAACCAAACCAGTAGCTCAAGAAGCTAAAAAACCACAAGTGTCAGAAAAA
GCAGATACAAAACCAATTGATTCAAGTGAAGCTAGTCAAACTAATAAAGCCCAGTTACCAAGTACAGGTAGTGCG
GCAAGCCAAGCAGCAGTAGCAGCAGGTTTAACTCTTCTAGGTTTGAGTGCAGGATTAGTAGTTACTAAAGGTAAA
AAAGAAGACTAG

4101.5
ATGGATGCAATCTTTGACCTAATCGGAAAGGTTTTCAATCCCATCTTAGAAATGGGTGGACCTGTCATCATGTTAA
TCATTTTGACAGTATTGGCTTTACTTTTTGGAGTGAAATTCTCCAAAGCGCTTGAAGGTGGTATCAAACTTGCCAT
CGCTCTTACAGGTATCGGTGCTATCATCGGTATGCTAAACACTGCTTTCTCAGCATCACTAGCAAAATTCGTTGAA
AACACTGGTATCCAATTGAGTATTACCGACGTTGGTTGGGCACCACTTGCTACAATCACTTGGGGTTCTGCTTGGA
CACTATACTTCTTGCTCATCATGTTGATTGTCAACATAGTGATGCTAGCTATGAAGAAAACAGATACACTTGATGT
CGATATCTTTGATATCTGGCACTTGTCTATCACAGGTCTCTTGATTAAATGGTATGCTGATAACAATGGTGTGAGT
CAAGGGGTTTCACTCTTTATTGCTACAGCAGCTATCGTCCTTGTCGGTGTGTTGAAAATTATCAACTCTGACTTGAT
GAAACCTACATTTGATGACCTTCTTAACGCCCCAAGTTCATCACCAATGACATCAACTCACATGAACTACATGATG
AACCCAGTTATCATGGTTTTGGATAAGATTTTTGAAAAATTCTTCCCAGGCCTTGATAAATATGACTTTGATGCTG
CTAAATTGAACAAGAAAATCGGTTTCTGGGGATCTAAATTCTTCATCGGTTTCATCCTTGGTATCGTTATCGGTATT
ATGGGAACTCCACATCCAATTGCAGGTGTTGCAGATGCAGATAAATGGCGTCTTGTTATCAAAGGATGGTTGTCTC
TTGGTTTGACTGCCGGTGTATCTTTGGAACTCTTCTCACTTATCGGTTCATGGTTCATCGCAGCCGTAGAACCACTA
TCACAAGGTATTACAAACGTTGCTACTAAACGTCTTCAAGGACGTAAATTCAATATCGGTCTTGACTGGCCATTCA
TCGCTGGTCGTGCTGAAATCTGGGCTTGTGCCAACGTACTTGCACCAATCATGTTGATTGAAGCAGTGCTTCTTTC
AAAAGTTGGAAATGGTATCTTGCCACTTGCAGGTATCATCGCTATGGGTGTTACTCCAGCTCTCTTGGTTGTAACT
CGTGGTAAATTGCTCCGTATGATTATCTTCGGAACACTCTTGTTGCCACTCTTCCTTCTTTCAGGTACACTTATTGC
ACCATTTGCAACAGAACTTGCTAAAGGTGTAGGTGCCTTCCCAGAAGGTGTGAGCCAAACTCAATTGATTACTCAC
TCTACTCTTGAAGGACCAATCGAAAAACTTCTTGGTTGGACAATTGGTAACACTACAACTGGTGATATCAAAGCAA
TCCTTGGTGCAGTAGTCTTCCTTGTATTCTATATCGGTATCTTTGCTTGGTACAGAAAACAAATGATCAAACGTAA
CGAAGAGTACGCAGCAAAAGCAAAATAA

4102.1

ATGAAGATTATGAAAAAAAAATATTGGACTTTAGCGATATTATTCTTTTGTTTGTTCAATAATTCTGTTACTGCTCA
AGAAATACCTAAAAATCTTGATGGCAATATAACTCACACTCAGACTAGCGAAAGTTTTTCTGAATCTGATGAAAA
ACAGGTTGACTATTCTAATAAAAATCAAGAAGAAGTAGACCAAAATAAATTTCGTATTCAAATCGATAAGACAGA
ATTATTTGTAACAACAGATAAACATTTAGAAAAAAACTGTTGTAAATTGGAACTTGAACCACAAATAAATAACGA
TATTGTTAACTCTGAAAGTAATAATTTACTAGGCGAAGATAATTTAGATAATAAAATTAAGGAAAATGTTTCTCAT
CTAGATAATAGAGGAGGAAATATAGAGCATGACAAAGATAACTTAGAATCGTCGATTGTAAGAAAATATGAATGG
GATATAGATAAAGTTACTGGTGGAGGCGAAAGTTATAAATTATATTCTAAAAGTAATTCTAAAGTTTCAATTGCTA
TTTTAGATTCAGGAGTCGATTTACAAAATACTGGATTACTGAAAAATCTTTCAAATCACTCAAAAAACTATGTCCC
CAATAAAGGATATTTAGGAAAAGAGGAGGGAGAGGAAGGAATAATATCAGATATTCAAGATAGATTAGGTCATG
GTACGGCTGTTGTAGCTCAAATTGTAGGGGATGACAATATTAATGGAGTAAATCCTCACGTTAATATTAACGTCTA
TAGAATATTTGGTAAGTCGTCAGCTAGTCCAGATTGGATTGTAAAAGCAATTTTTGATGCTGTAGATGATGGCAAT
GATATTATCAATCTTAGTACTGGACAATATTTAATGATTGATGGAGAATATGAGGACGGAACAAATGATTTTGAAA
CATTTTTGAAGTATAAAAAGGCTATTGATTACGCGAATCAAAAAGGAGTAATTATAGTAGCTGCATTAGGGAATG
ACTCCCTAAATGTATCAAATCAGTCAGATTTATTGAAACTTATTAGTTCACGCAAAAAAGTAAGAAAACCAGGATT
AGTAGTTGATGTTCCAAGTTATTTCTCATCTACAATTTCGGTCGGAGGCATAGATCGCTTAG
GTAATTTATCAGATTTTAGCAATAAAGGGGATTCTGATGCAATATATGCGCCTGCAGGCTCAACATTATCTCTTTC
AGAATTAGGACTTAATAACTTTATTAATGCAGAAAAATATAAAGAAGATTGGATTTTTTCGGCAACACTAGGAGG
ATATACGTATCTTTATGGAAACTCATTTGCTGCTCCTAAAGTTTCTGGTGCGATTGCAATGATTATTGATAAATACA
AATTAAAAGATCAGCCCTATAATTATATGTTTGTAAAAAAATTCTGGAAGAAACATTACCAGTAA

4106.1
ATGAAGAAAACATGGAAAGTGTTTTTAACGCTTGTAACAGCTCTTGTAGCTGTTGTGCTTGTGGCCTGTGGTCAAG
GAACTGCTTCTAAAGACAACAAAGAGGCAGAACTTAAGAAGGTTGACTTTATCCTAGACTGGACACCAAATACCA
ACCACACAGGGCTTTATGTTGCCAAGGAAAAAGGTTATTTCAAAGAAGCTGGAGTGGATGTTGATTTGAAATTGC
CACCAGAAGAAAGTTCTTCTGACTTGGTTATCAACGGAAAGGCACCATTTGCAGTGTATTTCCAAGACTACATGGC
TAAGAAATTGGAAAAAGGAGCAGGAATCACTGCCGTTGCAGCTATTGTTGAACACAATACATCAGGAATCATCTC
TCGTAAATCTGATAATGTAAGCAGTCCAAAAGACTTGGTTGGTAAGAAATATGGGACATGGAATGACCCAACTGA
ACTTGCTATGTTGAAAACCTTGGTAGAATCTCAAGGTGGAGACTTTGAGAAGGTTGAAAAAGTACCAAATAACGA
CTCAAACTCAATCACACCGATTGCCAATGGCGTCTTTGATACTGCTTGGATTTACTACGGTTGGGATGGTATCCTT
GCTAAATCTCAAGGTGTAGATGCTAACTTCATGTACTTGAAAGACTATGTCAAGGAGTTTGACTACTATTCACCAG
TTATCATCGCAAACAACGACTATCTGAAAGATAACAAAGAAGAAGCTCGCAAAGTCATCCAAGCCATCAAAAAAG
GCTACCAATATGCCATGGAACATCCAGAAGAAGCTGCAGATATTCTCATCAAGAATGCACCTGAACTCAAGGAAA
AACGTGACTTTGTCATCGAATCTCAAAAATACTTGTCAAAAGAATACGCAAGCGACAAGGAAAAATGGGGTCAAT
TTGACGCAGCTCGCTGGAATGCTTTCTACAAATGGGATAAAGAAAATGGTATCCTTAAAGAAGACTTGACAGACA
AAGGCTTCACCAACGAATTTGTGAAATAA

4106.4
ATGATAAAAAATCCTAAATTATTAACCAAGTCTTTTTTAAGAAGTTTTGCAATTCTAGGTGGTGTTGGTCTAGTCA
TTCATATAGCTATTTATTTGACCTTTCCTTTTTATTATATTCAACTGGAGGGGGAAAAGTTTAATGAGAGCGCAAG
AGTGTTTACGGAGTATTTAAAGACTAAGACATCTGATGAAATTCCAAGCTTACTCCAGTCTTATTCAAAGTCCTTG
ACCATATCTGCTCACCTTAAAAGAGATATTGTAGATAAGCGGCTCCCTCTTGTGCATGACTTGGATATTAAAGATG
GAAAGCTATCAAATTATATCGTGATGTTAGATATGTCTGTTAGTACAGCAGATGGTAAACAGGTAACCGTGCAATT
TGTTCACGGGGTGGATGTCTACAAAGAAGCAAAGAATATTTTGCTTTTGTATCTCCCATATACATTTTTGGTTACA
ATTGCTTTTTCCTTTGTTTTTTCTTATTTTTATACTAAACGCTTGCTCAATCCTCTTTTTTACATTTCAGAAGTGACT
AGTAAAATGCAAGATTTGGATGACAATATTCGTTTTGATGAAAGTAGGAAAGATGAAGTTGGTGAAGTTGGAAAA
CAGATTAATGGTATGTATGAGCACTTGTTGAAGGTTATTTATGAGTTGGAAAGTCGTAATGAGCAAATTGTAAAAT
TGCAAAATCAAAAGGTTTCCTTTGTCCGCGGAGCATCACATGAGTTGAAAACCCCTTTAGCCAGTCTTAGAATTAT
CCTAGAGAATATGCAGCATAATATTGGAGATTACAAAGATCATCCAAAATATATTGCAAAGAGTATAAATAAGAT
TGACCAGATGAGCCACTTATTAGAAGAAGTACTGGAGTCTTCTAAATTCCAAGAGTGGACAGAGTGTCGTGAGAC
CTTGACTGTTAAGCCAGTTTTAGTAGATATTTTATCACGTTATCAAGAATTAGCTCATTCAATAGGTGTTACAATTG
AAAATCAATTGACAGATGCTACCAGGGTCGTCATGAGTCTTAGGGCATTGGATAAGGTTTTGACAAACCTGATTA
GTAATGCAATTAAATATTCAGATAAAAATGGGCGTGTAATCATATCCGAGCAAGATGGCTATCTCTCTATCAAAA
ATACATGTGCGCCTCTAAGTGACCAAGAACTAGAACATTTATTTGATATATTCTATCATTCTCAAATCGTGACAGA
TAAGGATGAAAGTTCCGGTTTGGGTCTTTACATTGTGAATAATATTTTAGAAAGCTATCAAATGGATTATAGTTTT
CTCCCTTATGAACACGGTATGGAATTTAAGATTAGCTTGTAG

4106.6
ATGTATTTAGGAGATTTGATGGAGAAAGCCGAGTGTGGTCAATTTTCAATACTTTCCTTTCTATTACAAGAGTCTC
AGACGACCGTCAAGGCTGTAATGGAAGAAACAGGATTTTCAAAAGCAACCCTAACCAAATATGTCACCCTGCTCA
ATGACAAGGCTTTGGATAGTGGCTTAGAGCTGGCTATTCACTCAGAAGATGAAAATCTGCGTCTGTCTATCGGTGC
AGCTACCAAGGGGAGAGATATTCGGAGCTTGTTTTTGGAGAGTGCTGTTAAATACCAGATTTTGGTTTATCTTCTC
TACCACCAACAGTTTTTAGCCCATCAGCTGGCTCAAGAATTGGTGATTAGCGAGGCTACGCTTGGTCGTCACTTGG
CTGGTTTAAATCAGATTTTGTCAGAATTTGATTTATCCATCCAAAATGGCCGTTGGCGAGGTCCAGAGCATCAGAT
TCACTATTTCTATTTCTGTCTTTTCCGAAAGGTCTGGTCGAGTCAGGAATGGGAAGGTCACATGCAGAAACCAGAG
AGAAAACAGGAGATTGCCAATTTAGAGGAAATCTGCGGTGCAAGTTTGTCTGCGGGGCAGAAATTGGACTTGGTT

CTCTGGGCTCACATCAGTCAACAACGTCTTCGGGTCAATGCTTGTCAGTTTCAAGTCATAGAAGAGAAAATGCGA
GGGTATTTTGACAATATCTTTTATCTTCGTTTGCTGAGAAAGGTTCCGTCCTTTTTTGCTGGGCAACATATTCCACT
AGGAGTTGAGGATGGTGAGATGATGATATTCTTCTCTTTTCTCCTATCTCATCGCATTCTTCCTCTTCATACTATGG
AGTATATTCTTGGTTTTGGAGGGCAGTTGGCAGATTTACTGACGCAATTGATTCAAGAAATGAAGAAGGAGGAAC
TATTGGGGGATTATACAGAGGACCATGTCACCTATGAACTCAGTCAGCTTTGTGCTCAAGTCTATCTCTATAAGGG
CTATATTTTACAGGATCGCTACAAGTACCAGTTAGAGAATCGTCATCCATATTTACTGATGGAACATGATTTTAAA
GAGACAGCAGAGGAGATTTTTCATGCTCTACCTGCTTTTCAACAGGGGACAGATTTAGATAAGAAGATTCTCTGGG
AATGGCTCCAGTTAATCGAATATATGGCTGAAAACGGTGGCCAGCATATGCGGATTGGTCTGGATTTGACATCTGG
TTTTCTTGTCTTTTCAAGGATGGCAGCCATTTTGAAACGGTATTTGGAATACAATCGTTTTATTACCATTGAAGCTT
ATGACCCTAGTCGGCATTATGATTTGCTGGTTACCAATAACCCGATTCATAAGAAGGAACAGACACCAGTCTATTA
TTTAAAAAATGACTTGGATATGGAGGATTTGGTAGCGATTCGCCAGTTATTATTCACTTAA

4106.7
ATGGAATTTTCAAAGAAAACACGTGAATTGTCAATTAAAAAAATGCAGGAACGTACCCTGGACCTCTTGATTATC
GGTGGAGGAATCACAGGAGCTGGTGTAGCCTTGCAGGCGGCAGCTAGCGGTCTTGAGACTGGTTTGATTGAAATG
CAAGACTTTGCAGAAGGAACATCTAGTCGTTCAACAAAATTGGTTCACGGAGGACTTCGTTACCTCAAACAATTTG
ACGTAGAAGTGGTCTCAGATACGGTTTCTGAACGTGCAGTGGTTCAACAAATCGCTCCACACATTCCAAAATCAG
ATCCAATGCTCTTACCAGTTTACGATGAAGATGGAGCAACCTTTAGCCTCTTCCGTCTTAAAGTAGCCATGGACTT
GTACGACCTCTTGGCAGGTGTTAGCAACACACCAGCTGCGAACAAGGTTTTGAGCAAGGATCAAGTCTTGGAACG
CCAGCCAAACTTGAAGAAGGAAGGCTTGGTAGGAGGTGGAGTGTATCTTGACTTCCGTAACAACGATGCGCGTCT
CGTGATTGAAAACATCAAACGTGCCAACCAAGACGGTGCCCTCATTGCCAACCACGTGAAGGCAGAAGGCTTCCT
CTTTGACGAAAGTGGCAAGATTACAGGTGTTGTAGCTCGTGATCTCTTGACAGACCAAGTGTTTGAAATCAAGGCC
CGTCTGGTTATTAATACAACAGGTCCTTGGAGTGATAAAGTACGTAATTTGTCTAATAAGGGAACGCAATTCTCAC
AAATGCGCCCAACTAAGGGAGTTCACTTGGTAGTAGATTCAAGCAAAATCAAGGTTTCACAGCCAGTTTACTTCG
ACACAGGTTTGGGTGACGGTCGTATGGTCTTTGTTCTCCCACGTGAAAACAAGACTTACTTTGGTACAACTGATAC
AGACTACACAGGTGATTTGGAGCATCCAAAAGTAACTCAAGAAGATGTAGATTATCTACTTGGCATTGTCAACAA
CCGCTTCCCAGAATCCAACATCACCATTGATGATATCGAAAGCAGCTGGGCAGGTCTTCG
TCCATTGATTGCAGGGAACAGTGCCTCTGACTATAATGGTGGAAATAACGGTACCATCAGTGATGAAAGCTTTGA
CAACTTGATTGCGACTGTTGAATCTTATCTCTCCAAAGAAAAAACACGTGAAGATGTTGAGTCTGCTGTCAGCAAG
CTTGAAAGTAGCACATCTGAGAAACATTTGGATCCATCTGCAGTTTCTCGTGGGTCTAGCTTGGACCGTGATGACA
ATGGTCTCTTGACTCTTGCTGGTGGTAAAATCACAGACTACCGTAAGATGGCTGAAGGAGCTATGGAGCGCGTGG
TTGACATCCTCAAAGCAGAATTTGACCGTAGCTTTAAATTGATCAATTCTAAAACTTACCCTGTTTCAGGTGGAGA
ATTGAACCCAGCAAATGTGGATTCAGAAATCGAAGCCTTTGCGCAACTTGGAGTATCACGTGGTTTGGATAGCAA
GGAAGCTCACTATCTGGCAAATCTTTACGGTTCAAATGCACCGAAAGTCTTTGCACTTGCTCACAGCTTGGAACAA
GCGCCAGGACTCAGCTTGGCAGATACTTTGTCCCTTCACTATGCAATGCGCAATGAGTTGACTCTTAGCCCAGTTG
ACTTCCTTCTTCGTCGTACCAATCACATGCTCTTTATGCGTGATAGCTTGGATAGTATCGTTGAGCCAATTTTGGAT
GAAATGGGACGATTCTATGACTGGACAGAAGAAGAAAAAGCAACTTACCGTGCTGATGTCGAAGCAGCTCTCGCT
AACAACGATTTAGCAGAATTAAAAAATTAA

4106.8
ATGATGAATGAATTATTTGGAGAATTTCTAGGGACTTTAATCCTGATTCTTCTAGGAAATGGTGTTGTTGCAGGTG
TGGTTCTTCCTAAAACCAAGAGCAATAGCTCAGGTTGGATTGTGATTACTATGGGTTGGGGGATTGCAGTTGCGGT
TGCAGTCTTTGTATCTGGCAAGCTCAGTCCAGCTTATTTAAACCCAGCTGTGACCATCGGTGTGGCCTTAAAAGGT
GGTTTGCCTTGGGCTTCCGTTTTGCCTTATATCTTAGCCCAGTTCGCAGGGGCCATGCTGGGTCAGATTTTGGTTTG
GTTGCAATTCAAACCTCACTATGAGGCAGAAGAAAATGCAGGCAATATCCTGGCAACCTTCAGTACTGGACCAGC
CATCAAGGATACTGTATCAAACTTGATTAGCGAAATCCTTGGAACTTTTGTTTTGGTGTTGACAATCTTTGCTTTGG
GTCTTTACGACTTTCAGGCAGGTATCGGAACCTTTGCAGTGGGAACTTTGATTGTCGGTATCGGTCTATCACTAGG
TGGGACAACAGGTTATGCCTTGAACCCAGCTCGTGACCTTGGACCTCGTATCATGCACAGCATCTTGCCAATTCCA
AACAAGGGAGACGGAGACTGGTCTTACGCTTGGATTCCTGTTGTAGGCCCTGTTATCGGAGCAGCCTTGGCAGTG
CTTGTATTCTCACTTTTCTAG

4106.10
ATGAAAAGGACCTGGAGGAACTCATTCGTGACAAATCTTAATACACCTTTTATGATTGGCAATATTGAGATTCCCA
ATCGTACCGTTTTAGCGCCTATGGCTGGCGTGACCAACTCAGCCTTTCGTACTATCGCAAAGGAGCTCGGAGCTGG
ACTCGTTGTAATGGAAATGGTCTCTGACAAGGGAATCCAATACAACAACGAAAAAACCCTGCACATGCTTCATAT
CGATGAGGGCGAAAACCCTGTCTCTATCCAACTTTTTGGTAGCGATGAAGACAGCCTAGCACGCGCAGCAGAATT
CATCCAAGAAAACACCAAGACCGATATCGTCGATATCAACATGGGCTGCCCTGTCAACAAAATCGTGAAGAACGA
AGCTGGTGCTATGTGGCTCAAGGATCCAGACAAGATTTACTCCATCATCAACAAGGTCCAGTCTGTCCTTGATATC
CCACTTACTGTCAAAATGCGTACCGGCTGGGCGGACCCATCTCTTGCAGTAGAAAATGCTCTCGCTGCTGAAGCTG
CAGGTGTTTCTGCCCTCGCCATGCATGGCCGTACCCGTGAACAAATGTATACTGGCCACGCAGACCTTGAGACCCT
TTACAAGGTTGCCCAAGCTCTAACCAAGATTCCATTCATCGCCAACGGTGATATCCGTACTGTCCAAGAAGCCAA
GCAACGCATCGAAGAAGTTGGTGCTGACGCAGTCATGATTGGCCGAGCTGCCATGGGAAATCCTTACCTCTTCAA
CCAAATCAACCATTACTTTGAAACAGGAGAAATCCTACCTGATTTGACCTTTGAAGACAAGATGAAGATCGCCTA
CGAACACTTGAAACGATTGATTAACCTCAAAGGAGAAAACGTCGCAGTTCGTGAATTCCGCGGTCTCGCTCCTCA

CTATCTCCGTGGAACATCTGGCGCTGCCAAACTCCGTGGAGCCATTTCGCAAGCCAGCACCCTGGCAGAGATTGA
AACCCTCTTGCAATTGGAGAAGGCTTAA

4107.1
ATGACAAAGAAGAAAATTGAGCGTATTTCTGTAATACACCGAGAAAAGATTTTATGGCTCAAGTGGTATTTCATGC
GAGATAAAGAACAACCTAAGTATAGTGTCCTTGAGCGTAAAATGTTTGATGCTGCTAAAAATCAAGATATGCTAG
CTTATCAAAAATACGCAACTATCAAGCAGATAACAGATATTAGGGTACAAACAAGTGAGGCTGACATTTTAGAGG
CTGTAAAAGAGGTTTATGTGTACAATCACATGAATGTTATCGGAGCTTGTCAGCGGATATTATTTATCAGTCAATC
ACCAGCTTATGATAAGTTAAATAAGTGGTTTAATATCTATTCTGATTTGTATTTTAGCGTTGTACCCTTGCCCAAAA
TGGGGGTATATCATGAGATGGTAGGTATCTAG

4107.2
ATGAAAAATTCCAACGAGGCTGAGATGAAATTACTTTATACTGATATTCGGACTTCTTTGACAGAAATTCTAACAA
GAGAGGCAGAAGAGCTAGTTGCAGCTGGCAAGCGGGTCTTCTACATTGCCCCCAACTCTCTTTCTTTTGAAAAGGA
ACGCGCCGTGCTGGAATACTTGTCCCAGCAGGCTTCTTTTTCGATTACCGTCACGCGCTTTGCTCAAATGGCTCGC
TATCTGGTCTTGAATGATTTACCAGCTAAAACTACTCTTGATGATATCGGTCTTGGGTTGGCCTTTTACAAATGCCT
TGCCGAACTCGATCCCAAGGACTTGCGTGTTTATGGCGCTATTAAGCAGGATCCTCAATTGATCCAGCAGTTAATT
GAGCTTTACCATGAGATGACCAAATCTCAGATGAGTTTTTTGGACTTGGAGAATTTAACAGATGAGGATAAGAGG
GCGGATTTACTCTTGATTTTTGAGAAAGTAACAGCCTATCTTAATCAAGGTCAGTTAGCCCAGGAAAGTCAGTTGT
CCCATTTGATTGAGGCTATTGAGAATGACAAGGTAAGTAGTGATTTTAATCAAATCGCCTTGGTCATTGACGGCTT
TACTCGTTTTTCTGCTGAGGAAGAGCGGGTTGTGGACTTACTTCACGGCAAAGGTGTTGAGATTGTTATCGGGGCT
TATGCTAGTAAGAAAGCCTATACCAGTCCTTTTAGCGAGGGCAATCTCTACCAAGCCAGCGTAAAATTTCTCCATC
ATCTGGCTTCTAAATACCAAACGCCTGCTCAGGACTGTTCTCAAACTCATGAGAAGATGGATAGTTTTGACAAGGC
CTCTCGTTTGTTGGAGTCTTCTTATGACTTTTCAGAACTCGCTTTGGATGTCGATGAGAAAGACCGTGAAAATTTA
CAAATCTGGTCTTGTTTGACGCAAAAGGAGGAGTTGGAGCTAGTAGCCCGTAGTATTCGTCAGAAATTACATGAG
AACTCAGACCTGAGCTACAAGCATTTTCGTATTCTCTTGGGGGATGTAGCTTCTTACCAGTTATCTCTCAAAACCA
TTTTTGACCAGTATCAGATTCCTTTTTATCTTGGTAGAAGCGAAGCCATGGCTCATCATCCCTTGACTCAGTTTGTC
GAGTCTATTTTAGCTTTAAAACGTTACCGTTTTCGTCAGGAGGATTTGATTAATCTTCTTAGAACTGATTTGTATAC
TGACCTCAGTCAGTCTGATATTGATGCTTTTGAGCAATATATCCGCTATCTTGGTATCAATGGCTTGCCAGCCTTTC
AGCAAACCTTCACCAAATCCCACCATGGAAAATTTAATCTTGAGCGTTTGAATGTCCTCCGCCTGAGAATTTTAGC
ACCTCTTGAAACCCTCTTTGCCAGCCGAAAACAAAAGGCTGAAAAACTCCTACAAAAATGGAGTGTCTTTCTAAA
AGAAGGAGCTGTGACCAAGCAGTTACAAGATTTGACAACCACTTTGGAAGCTGTAGAACAGGAAAGACAAGCCG
AAGTTTGGAAGGCTTTCTGCCATGTTTTAGAACAATTTGCGACTGTTTTTGCTGGTTCACAGGTTAGTCTGGAAGA
CTTCCTAGCCTTGCTCCATTCTGGAATGAGTTTGTCCCAATACCGTACCATTCCAGCAACAGTGGACACTGTTCTG
GTGCAGAGTTACGATTTGATTGCACCATTGACTGCTGACTTTGTCTATGCTATTGGACTAACTCAGGACAATTTAC
CAAAAATTTCTCAAAACACCAGTCTTCTGACAGATGAAGAAAGGCAAAACCTAAACCAAGCGACCGAAGAAGGC
GTTCAATTACTGATTGCCAGCAGTGAAAATCTCAAGAAAAATCGCTACACTATGCTTTCCTTGGTCAATTCTGCTC
GTAAGCAGTTGTTCTTGTCGGCTCCAAGCCTTTTTAACGAAAGTGAAAGTAAGGAATCTGCCTATCTTCAAGAGTT
GATCCATTTTGGATTTAGGCGGAGAGAGAAGAGGATGAATCACAAAGGACTGTCTAAGGAGGATATGGGGTCCTA
TCACAGTCTTTTGTCTAGTCTGGTTGCCTATCACCAGCAGGGTGAGATGAGCGATACTGAGCAAGATTTGACTTTT
GTCAAGGTTCTGTCGCGTGTCATAGGTAAAAAACTAGATCAGCAAGGTCTGGAAAATCCAGCTATCCCAACCAGT
CCAAGCAGCAAGACCTTAGCCAAGGACACCTTGCAAGCTCT
CTATCCTGCCAAACAGGAGTTTTACCTGTCTACGTCGGGTTTGACAGAGTTTTATCGCAATGAATACAGTTATTTC
CTACGCTACGTTTTAGGCTTGCAGGAGGAATTACGTTTGCATCCTGATGCCCGTAGTCACGGGAATTTCTTGCATC
GTATCTTTGAACGCGCCTTACAGTTGCCTAATGAAGATTCCTTTGACCAACGTCTAGAACAAGCTATTCAAGAAAC
CAGTCAAGAACGCGAATTTGAAGCTATTTATCAAGAAAGTTTGGAAGCCCAGTTTACCAAGGAAGTTTTGCTTGAT
GTTGCACGGACAACTGGACATATTCTCCGACACAATCCAGCCATCGAAACCATCAAAGAAGAAGCAAATTTTGGT
GGAAAAGACCAAGCCTTTATTCAATTAGACAATGGACGCAGTGTCTTTGTACGAGGCAAGGTGGACCGGATTGAC
CGTTTGAAAGCTAATGGAGCGATAGGAGTAGTAGACTACAAATCCAGTCTGACTCAGTTCCAGTTTCCTCATTTCT
TTAATGGGCTCAATTCTCAGTTACCAACCTATCTTGCTGCCCTAAAAAGAGAAGGGGAGCAGAACTTTTTCGGCGC
CATGTACTTGGAAATGGCTGAACCTGTCCAATCTCTGATGGCGGTAAAAAGTCTGGCAGGAGCAGTGGTAGAAGC
CAGCAAATCTATGAAATACCAAGGGCTCTTCTTGGAAAAAGAAAGCAGTTATTTAGGCGAATTTTATAACAAAAA
CAAGGCTAATCAACTGACAGATGAGGAATTTCAGCTCCTACTGGACTACAATGCCTATCTTTACAAGAAAGCTGCT
GAGAAGATTTTAGCAGGCCGGTTCGCCATCAATCCTTATACTGAAAATGGCAGAAGCATTGCCCCATACGTCCAG
CAACATCAGGCTATTACAGGCTTTGAAGCCAATTACCATCTGGGCCAAGCCCGTTTCCTAGAAAAGTTGGACCTAG
CTGATGGCAAGCGTCTGGTCGGAGAAAAACTCAAGCAAGCTTGGCTTGAAAAAATAA
GAGAGGAGTTGAATCGATGA

4107.3
ATGAAGCTTATTCCCTTTTTAAGTGAGGAGGAGATTCAAAAACTGCAAGAAGCAGAAGCAAATTCGAGCAAGGAA
CAGAAGAAAACTGCCGAGCAAATCGAAGCTATCTACACTTCTGCCCAGAATATCCTGGTCTCAGCATCGGCTGGT
TCTGGAAAGACCTTTGTCATGGCAGAGCGCATTCTGGACCAATTGGCGCGTGGTGTCGAAATTTCTCAACTCTTTA

TCTCAACCTTTACCGTCAAGGCTGCAACTGAACTTAAAGAACGTTTAGAGAAAAAAATCAGCAAGAAAATCCAAG
AAACAGATGATGTCGACCTCAAACAACACTTGGGTCGCCAGTTGGCAGACCTACCCAACGCTGCCATTGGAACCA
TGGATTCTTTCACACAAAAATTCCTTGGCAAACATGGTTATCTGCTTGATATTGCACCTAATTTCCGTATTTTACAA
AACCAAAGCGAGCAACTTATTCTCGAAAACGAAGTCTTTCATGAGGTCTTTGAAGCGCATTACCAAGGTAAACAG
AAAGAGACCTTTAGTCATTTGCTGAAAAACTTTGCTGGGCGTGGCAAGGACGAACGGGGTCTGCGCCAGCAGGTC
TATAAAATCTATGACTTCCTCCAATCCACCAGTAATCCTCAAAAGTGGCTGAGTGAATCTTTCCTCAAAGGATTTG
AGAAAGCTGATTTTACCAGTGAAAAAGAAAAACTGACCGAGCAAATCAAACAAGCCCTTTGGGATTTGGAAAGCT
TTTTCCGTTACCATCTGGATAACGATGCCAAGGAGTTTGCAAAGGCTGCCTATTTAGAAAATGTTCAGTTAATTCT
GGATGAAATTGGCTCCCTAAATCAGGAGTCCGATAGTCAGGCTTATCAGGCAGTGCTTGCGCGTGTTGTCGCCATC
TCTAAGGAGAAAAACGGTCGAGCTCTGACTAATGCCAGCCGTAAGGCTGATTTGAAGCCCCTGGCTGATGCCTAC
AACGAAGAGAGAAAGACCCAGTTTGCTAAACTAGGACAATTATCAGACCAGATAGCGAT
TCTCGACTATCAAGAACGTTATCATGGAGACACTTGGAAACTAGCTAAAACCTTCCAATCTTTCATGAGCGATTTT
GTAGAGGCTTATCGTCAGAGAAAACGACAGGAAAATGCCTTCGAATTCGCTGATATCAGCCATTACACCATTGAG
ATTTTAGAGAATTTCCCACAAGTTCGTGAGTCTTATCAGGAGCGCTTCCATGAAGTCATGGTCGATGAGTATCAGG
ATACCAACCATATTCAAGAACGGATGCTGGAATTGTTGTCTAATGGCCACAATCGCTTTATGGTGGGAGATATCAA
GCAATCCATCTATCGTTTCCGTCAGGCAGACCCGCAGATTTTCAATGAGAAATTCCAACGCTATGCGCAAAATCCC
CAAGAAGGCAGGCTCATTATCCTCAAGGAAAATTTCCGTAGTAGTTCAGAAGTGCTGTCAGCAACCAATGATGTC
TTTGAACGTCTCATGGACCAAGAGGTCGGCGAAATCAACTATGATAACAAGCACCAGCTTGTTTTTGCCAATACCA
AACTGACTCCCAATCCAGACAACAAGGCAGCATTTCTCCTCTACGACAAGGACGATACAGGTGAGGAAGAAGAGA
GTCAAACAGAAACGAAACTAACAGGCGAAATGCGCTTAGTTATCAAGGAGATTCTGAAACTTCATCAAGAAAAAG
GTGTTGCCTTTAAGGAAATTGCCCTTCTGACCTCCAGCCGCAGTCGTAATGACCAGATTCTCCTCGCCCTGTCTGA
GTACGGAATTCCTGTCAAAACTGACGGAGAGCAAAACAATTATCTCCAATCCCTAGAAGTGCAAGTCATGCTAGA
CACTCTTCGTGTCATTCACAATCCCCTGCAAGACTACGCCTTGGTTGCCCTTATGAAGTCTCCAATGTTTGGTTTTG
ATGAGGATGAGCTAGCACGTTTGTCCCTTCAGAAAGCAGAGGATAAAGTCCACGAAAATCTCTATGAGAAACTGG
TCAATGCACAAAAAAATGGCAAGTAGTCAAAAAGGCTTGATTCACACAGCTCTAGCTG
AAAAACTAAAGCAATTCATGGATATCCTAGCTTCTTGGCGCTTGTATGCCAAAACCCACTCTCTCTATGACTTGAT
TTGGAAGATTTACAACGACCGTTTTTATTATGACTATGTTGGGGCTTTGCCGAATGGTCCTGCTAGGCAGGCCAAT
CTCTATGCCCTAGCACTGCGTGCTGATCAATTTGAAAAGAGCAATTTCAAAGGTTTGTCGCGTTTTATTCGTATGA
TTGACCAAGTCTTAGAAGCCCAGCACGATTTGGCAAGCGTGGCCGTCGCACCGCCAAAAGATGCAGTAGAGCTCA
TGACCATCCACAAGAGTAAAGGGCTGGAGTTTCCTTACGTCTTTATCCTCAATATGGATCAAGATTTCAACAAGCA
AGACTCTATGTCAGAAGTCATTCTCAGTCGTCAGAATGGTCTTGGTGTCAAATATATTGCCAAGATGGAGACAGGG
GCAGTAGAAGACCACTATCCTAAAACCATCAAACTCTCCATTCCTAGTCTGACCTATAGGCAGAACGAAGAGGAA
TTACAGCTAGCAAGCTATTCTGAGCAGATGCGTTTGCTGTATGTTGCTATGACGCGGGCTGAGAAAAAGCTCTATC
TTGTCGGCAAGGGTTCTCGTGAAAAGCTGGAATCCAAGGAATACCCAGCAGCCAAAAATGGGAAACTAAATAGCA
ATACTAGACTGCAAGCACGGAATTTCCAAGATTGGCTTTGGGCTATCAGTAAAGTGTTTACTAAGGACAAGCTCA
ACTTTAGTTATCGTTTTATTGGCGAAGATCAGTTGACCAGAGAAGCTATCGGAGAGTTGGAAACCAAGAGTCCTCT
CCAAGATAGCTCCCAAGCAGACAATCGTCAGTCAGATACCATCAAAGAAGCTCTGGAAATGCTGAAGGAGGTGGA
AGTTTATAATACTCTTCACCGCGCAGCTATTGAACTTCCTAGTGTTCAAACCCCAAGTCAAATCAAGAAATTCTAC
GAACCAGTTATGGATATGGAAGGTGTCGAGATTGCTGGTCAAGGTCAGTCAGTAGGCAAGAAAATCAGCTTCGAT
TTGCCAGATTTTTCAACCAAAGAAAAGGTAACTGGAGCTGAGATTGGTAGTGCTACTCACGAACTCATGCAGAGA
ATTGACCTCAGCCAGCAACTAACCCTTGCTAGCCTAACAGAAACACTCAAACAAGTTCAAACTAGCCAAGCTGTC
AGAGACAAGATCAATCTTGATAAAATTCTTGCTTTCTTTGACACAGTACTCGGTCAGGAAATTCTTGCTAATACCG
ACCATCTTTATCGCGAGCAACCTTTCTCCATGCTCAAACGAGACCAAAAGAGTCAGGAAGACTTTGTTGTCCGTGG
TATCCTTGATGGCTATCTGCTTTACGAAAACAAAATTGTTCTGTTCGACTACAAGACAGACCGCTATGATGAACCA
AGTCAACTCGTAGACCGCTATCGTGGTCAGTTAGCTCTATACGAAGAGGCTTTATCACGAGCCTATTCGATTGAAA
ATATTGAAAAATACTTGATTTTACTCGGTAAAGACGAGGTTCAAGTTGTAAAAGTATAA

4109.1
ATGGAACTTGCTCGCCATGCTGAAAGCTTGGGAGTAGATGCTATTGCAACGATTCCACCAATTTATTTCCGCTTGC
CAGAATACTCAGTTGCCAAATACTGGAACGATATCAGTTCTGCAGCTCCAAACACAGACTACGTGATTTACAACA
TTCCTCAATTGGCAGGGGTTGCTTTGACTCCAAGCCTTTACACAGAAATGTTGAAAAATCCTCGTGTTATCGGTGT
GAAGAACTCTTCTATGCCAGTTCAAGATATCCAAACCTTTGTCAGCCTTGGTGGAGAAGACCATATCGTCTTTAAT
GGTCCTGATGAGCAGTTCCTAGGAGGACGCCTCATGGGGGCTAGGGCTGGTATCGGTGGTACTTATGGTGCTATGC
CAGAACTCTTCTTGAAACTCAATCAGTTGATTGCGGATAAGGACCTAGAAACAGCGCGTGAATTGCAGTATGCTAT
CAACGCAATCATTGGTAAACTCACTTCTGCTCATGGAAATATGTACGGTGTCATCAAAGAAGTCTTGAAAATCAAT
GAAGGCTTGAATATTGGATCTGTTCGTTCACCATTGACACCAGTGACTGAAGAAGATCGTCCAGTTGTAGAAGCG
GCTGCTGCCTTGATTCGTGAAACCAAGGAGCGCTTCCTCTAA

4110.2
ATGTATAAGACAAAGTGTTTACGAGAGAAGTTAGTATTATTTTTAAAAATTTTCTTCCCAATCCTGATCTACCAAT
TTGCCAATTATTCTGCCTCTTTTGTTGATACTGCAATGACAGGTCAATACAACACTATGGACTTGGCTGGTGTATCT
ATGGCAACCAGTATCTGGAATCCTTTCTTTACATTTCTAACAGGGATTGTGTCAGCCTTGGTGCCTATCATTGGTCA
CCATCTTGGTCGAGGCAAAAAGGAAGAAGTTGCGTCTGATTTTTACCAATTTATTTATTTGGCCTTGGGCCTATCT
GTGGTCTTGCTGGGGATGGTACTTTTCTTGGCACCAATAATCTTGAATCATATTGGGTTAGAAGCAGCAGTAGCGG
CAGTAGCGGTTCGCTATCTTTGGTTTTTATCTATCGGGATTATCCCCTTGTTGCTCTTTAGCGTCATTCGTTCCTTGC

TGGATTCGCTGGGCTTGACCAAACTGTCCATGTACCTCATGCTTTTGTTACTCCCTCTCAATAGCGGATTTAACTAT
CTCTTGATTTACGGTGCCTTTGGTGTTCCAGAACTGGGAGGGGCTGGTGCTGGTTTAGGAACATCCTTGGCCTACT
GGGTCTTGCTTGGGATTTCTGTTCTGGTTTTATTTAAACAGGAGAAGCTCAAAGCCTTACACCTTGAGAAACGAAT
TCCACTTAATATGGATAAAATTAAGGAAGGAGTTCGTTTAGGTCTGCCTATTGGGGGAACTGTCTTCGCGGAAGTG
GCTATCTTTTCAGTGGTTGGCTTGATTATGGCTAAGTTTTCGCCCTTGATTATAGCTAGTCACCAGTCAGCTATGAA
CTTTTCAAGTCTTATGTACGCCTTTCCTATGAGTATCTCATCGGCTATGGCTATTGTCGTTTCCTATGAAGTGGGAG
CCAAGCGATTTGATGATGCGAAAACCTATATTGGTCTAGGAAGATGGACTGCCCTCATTTTTGCGGCCTTCACCTT
AACCTTCCTTTACATTTTTAGGGGAAATGTGGCCAGTCTTTATGGTAACGACCCAAAATTTATCGATTTGACAGTG
CGTTTTTTAACTTATAGTCTTTTCTTCCAGTTAGCAGATACCTTTGCGGCGCCGCTTCAGGGAATTTTGCGGGGGTA
TAAGGATACAGTTATTCCTTTTTACCTTGGTTTGCTTGGTTATTGGGGCGTAGCAATCCCTGTGTACGCTATTTGA

4112.2
ATGAGTACTTTAGCAAAAATAGAAGCGCTCTTGTTTGTAGCGGGTGAAGATGGGATTCGGGTCCGCCAGTTAGCT
GAACTCCTCTCTCTGCCACCGACAGGCATCCAGCAAAGTTTAGGAAAATTAGCCCAGAAGTATGAAAAGGACCCA
GATTCCAGTTTGGCTTTGATTGAGACAAGTGGTGCTTATAGATTGGTGACCAAGCCTCAATTTGCAGAGATTTTGA
AGGAATACTCTAAGGCGCCTATCAACCAGAGCTTGTCTCGGGCTGCCCTTGAGACCTTGTCCATTATTGCCTACAA
ACAGCCGATTACGCGGATAGAAATTGATGCCATCCGTGGAGTTAACTCGAGTGGAGCCTTGGCAAAGTTGCAGGC
TTTTGACCTGATAAAGGAAGACGGGAAAAAGGAAGTATTGGGGCGCCCCAACCTCTATGTGACTACGGATTATTT
CCTAGATTACATGGGGATAAACCATTTAGAAGAATTACCAGTGATTGATGAGCTTGAGATTCAAGCCCAAGAAAG
CCAATTATTTGGTGAAAGGATAGAAGAAGATGAGAATCAATAA

4113.1
ATGGATACGATGATTAGTAGATTTTTTCGCCATTTATTTGAAGCCTTAAAAAGTTTGAAACGAAATGGTTGGATGA
CAGTAGCTGCTGTCAGTTCAGTCATGATTACTTTGACCTTGGTGGCAATATTTGCATCTGTTATTTTCAATACAGCG
AAACTAGCTACAGATATTGAAAATAATGTCCGTGTAGTAGTTTATATCCGAAAGGATGTGGAAGATAATAGTCAG
ACAATTGAAAAAGAAGGTCAAACTGTTACAAATAATGACTACCACAAGGTATATGATTCTTTGAAGAACATGTCT
ACGGTTAAAAGTGTTACCTTTTCAAGTAAAGAAGAACAATATGAAAAATTAACCGAGATAATGGGAGATAACTGG
AAAATCTTTGAAGGAGATGCCAATCCTCTCTATGATGCCTATATTGTAGAGGCAAACACTCCAAATGATGTAAAA
ACTATAGCCGAAGATGCTAAAAAAATTGAAGGTGTCTCTGAGGTTCAAGATGGCGGTGCCAATACAGAAAGACTC
TTCAAGTTAGCTTCATTTATCCGTGTTTGGGGACTAGGGATTGCTGCTTTGTTAATTTTTATCGCAGTTTTCTTGAT
TTCAAATACCATTCGTATTACCATTATTTCCCGCAGTCGCGAAATTCAAATCATGCGCTTGGTCGGAGCTAAAAAC
AGTTATATCCGTGGACCGTTCTTGTTAGAAGGAGCCTTTATCGGTTTATTGGGAGCTATCGCACCATCTGTTTTGGT
CTTTATTGTTTATCAAATTGTTTACCAATCTGTCAACAAATCGTTGGTAGGGCAAAATCTATCCATGATTAGTCCA
GATTTATTTAGTCCGTTGATGATTGCCCTACTATTTGTGATTGGGGTTTTCATTGGTTCATTGGGATCAGGAATATC
CATGCGCCGATTCTTGAAGATTTAG

4117.1
ATGAAGAAAGTAAGATTTATTTTTTTAGCTCTGCTATTTTTCTTAGCTAGTCCAGAGGGTGCAATGGCTAGTGATG
GTACTTGGCAAGGAAAACAGTATCTGAAAGAAGATGGCAGTCAAGCAGCAAATGAGTGGGTTTTTGATACTCATT
ATCAATCTTGGTTCTATATAAAAGCAGATGCTAACTATGCTGAAAATGAATGGCTAAAGCAAGGTGACGACTATTT
TTACCTCAAATCTGGTGGCTATATGGCCAAATCAGAATGGGTAGAAGACAAGGGAGCCTTTTATTATCTTGACCAA
GATGGAAAGATGAAAAGAAATGCTTGGGTAGGAACTTCCTATGTTGGTGCAACAGGTGCCAAAGTAATAGAAGAC
TGGGTCTATGATTCTCAATACGATGCTTGGTTTTATATCAAAGCAGATGGACAGCACGCAGAGAAAGAATGGCTC
CAAATTAAAGGGAAGGACTATTATTTCAAATCCGGTGGTTATCTACTGACAAGTCAGTGGATTAATCAAGCTTATG
TGAATGCTAGTGGTGCCAAAGTACAGCAAGGTTGGCTTTTTGACAAACAATACCAATCTTGGTTTTACATCAAAGA
AAATGGAAACTATGCTGATAAAGAATGGATTTTCGAGAATGGTCACTATTATTATCTAAAATCCGGTGGCTACATG
GCAGCCAATGAATGGATTTGGGATAAGGAATCTTGGTTTTATCTCAAATTTGATGGGAAAATGGCTGAAAAAGAA
TGGGTCTACGATTCTCATAGTCAAGCTTGGTACTACTTCAAATCCGGTGGTTACATGACAGCCAATGAATGGATTT
GGGATAAGGAATCTTGGTTTTATCTCAAATCTGATGGGAAAATAGCTGAAAAAGAATGGGTCTACGATTCTCATA
GTCAAGCTTGGTACTACTTCAAATCCGGTGGTTACATGACAGCCAATGAATGGATTTGGGATAAGGAATCTTGGTT
TTACCTCAAATCTGATGGGAAAATAGCTGAAAAAGAATGGGTCTACGATTCTCATAGTCAAGCTTGGTACTACTTC
AAATCTGGTGGCTACATGGCGAAAAATGAGACAGTAGATGGTTATCAGCTTGGAAGCGATGGTAAATGGCTTGGA
GGAAAAACTACAAATGAAAATGCTGCTTACTATCAAGTAGTGCCTGTTACAGCCAATGTTTATGATTCAGATGGTG
AAAAGCTTTCCTATATATCGCAAGGTAGTGTCGTATGGCTAGATAAGGATAGAAAAAGTGATGACAAGCGCTTGG
CTATTACTATTTCTGGTTTGTCAGGCTATATGAAAACAGAAGATTTACAAGCGCTAGATGCTAGTAAGGACTTTAT
CCCTTATTATGAGAGTGATGGCCACCGTTTTTATCACTATGTGGCTCAGAATGCTAGTATCCCAGTAGCTTCTCAT
CTTTCTGATATGGAAGTAGGCAAGAAATATTATTCGGCAGATGGCCTGCATTTTGATGGTTTTAAGCTTGAGAATC
CCTTCCTTTTCAAAGATTTAACAGAGGCTACAAACTACAGTGCTGAAGAATTGGATAAGGTATTTAGTTTGCTAAA
CATTAACAATAGCCTTTTGGAGAACAAGGGCGCTACTTTTAAGGAAGCCGAAGAACATTACCATATCAATGCTCTT
TATCTCCTTGCCCATAGTGCCCTAGAAAGTAACTGGGGAAGAAGTAAAATTGCCAAAGATAAGAATAATTTCTTTG
GCATTACAGCCTATGATACGACCCCTTACCTTTCTGCTAAGACATTTGATGATGTGGATAAGGGAATTTTAGGTGC
AACCAAGTGGATTAAGGAAAATTATATCGATAGGGGAAGAACTTTCCTTGGAAACAAGGCTTCTGGTATGAATGT
GGAATATGCTTCAGACCCTTATTGGGGCGAAAAAATTGCTAGTGTGATGATGAAAATCAATGAGAAGCTAGGTGG
CAAAGATTAG

4119.2
ATGAAAAAAGTATTACAAAAATATTGGGCATGGGCTTTTGTGGTCATCCCCCTCTTGTTACAAGCAATTTTCTTCT
ATGTGCCGATGTTTCAAGGAGCCTTTTACAGTTTTACCAACTGGACAGGATTGACTTATAACTACAAATTTGTTGG
CTTAAACAACTTTAAGCTCCTCTTCATGGATCCAAAATTCATGAATGCGATTGGCTTTACCGCAATCATTGCGATT
GCCATGGTGGTTGGTGAGATTGCACTCGGGATCTTCATTGCGCGTGTCTTGAATTCTAAAATCAAAGGCCAAACCT
TCTTCCGTGCTTGGTTCTTCTTCCCAGCTGTTTTATCTGGTTTGACAGTGGCTTTGATCTTCAAGCAAGTCTTCAAC
TACGGTCTTCCAGCGATTGGAAATGCCCTTCATATTGAATTTTTCCAAACCAGTCTTTTAGGGACTAAGTGGGGAG
CAATCTTTGCGGCTGTCTTTGTCCTTCTTTGGCAAGGGGTGGCTATGCCCATCATCATCTTCCTAGCTGGTTTGCAA
TCTATTCCAACTGAGATTACAGAGGCAGCAAGGATTGATGGTGCGACTAGCAAGCAAGTTTTCTGGAACATTGAA
TTGCCTTACTTGCTACCAAGTGTCTCTATGGTCTTTATCCTAGCCCTAAAAGGTGGGCTGACTGCCTTTGACCAAGT
CTTTGCCATGACCGGTGGTGGTCCAAACAATGCCACAACCTCACTTGGGCTCTTGGTTTATAACTATGCCTTTAAA
AACAACCAATTCGGTTATGCCAATGCCATTGCCGTAATCTTGTTCTTCTTAATTGTAGTGATTTCGATCATCCAATT
GAGAGTATCTAAGAAATTTGAAATTTAA

4119.3
ATGATGAAACAAGATGAAAGAAAAGCCCTGATTGGCAAATACATTCTATTGATTCTAGGATCGGTTCTGATTTTAG
TGCCGCTCCTTGCTACCCTCTTTAGTTCCTTCAAACCCACTAAGGATATTGTAGATAATTTCTTTGGCTTTCCAACC
AACTTCACATGGGACAACTTTAGCCGTCTCTTAGCTGATGGGATTGGAGGCTATTATTGGAACTCTGTCGTCATCA
CTGTCTTGTCTTTACTTGCAGTAATGATCTTTATCCCTATGGCAGCCTACTCCATCGCTCGCAATATGAGTAAAAG
AAAAGCCTTTACCATCATGTATACCCTCTTAATCCTCGGAATCTTCGTACCTTTCCAAGTCATCATGATTCCGATTA
CGGTTATGATGAGTAAACTCGGTTTGGCTAATACCTTTGGTTTGATCTTGCTCTACTTGACCTATGCGATTCCACAG
ACCCTCTTTCTCTATGTTGGCTATATCAAAATCTCGATTCCAGAAAGTCTGGATGAAGCAGCAGAGATCGATGGGG
CTAATCAATTTACAACCTATTTCCGCATCATCTTCCCAATGATGAAACCGATGCATGCGACAACCATGATCATCAA
TGCCCTTTGGTTCTGGAATGACTTCATGTTGCCACTCCTTGTCTTGAACCGGGATTCCAAAATGTGGACTCTGCCTT
TGTTCCAATACAACTACGCAGGCCAATATTTCAACGACTACGGACCAAGCTTTGCCTCTTACGTGGTCGGCATTAT
CAGTATCACCATTGTCTATCTCTTCTTCCAACGCCATATCATTTCAGGAATGAGCAACGGGGCAGTGAAGTAA

4119.4
ATGAAAAGTATTCTTCAGAAAATGGGGGAGCATCCGATGCTGCTTCTTTTTCTTAGCTATAGTACTGTTATATCCA
TTCTTGCACAAAATTGGATGGGTCTTGTGGCTTCAGTAGGAATGTTTCTATTTACTATTTTCTTTTTGCACTATCAG
TCGATTTTATCCCATAAATTCTTTCGATTGATTTTGCAGTTTGTCTTGTTTGGTAGTGTCTTGTCAGCTGCTTTTGCC
AGTTTAGAACATTTCCAAATTGTGAAGAAATTTAACTATGCTTTTCTTTCACCCAATATGCAGGTGTGGCATCAGA
ACCGGGCAGAAGTGACCTTCTTTAATCCTAATTATTATGGAATTATTTGTTGTTTCTGTATTATGATTGCTTTCTAT
CTGTTTACAACGACCAAGTTGAATTGGTTGAAAGTATTCTGTGTGATTGCAGGCTTTGTTAATCTCTTTGGTTTGAA
CTTTACTCAAAATCGAACTGCCTTTCCTGCTATTATCGCTGGAGCAATTATCTATCTCTTTACGACTATTAAAAACT
GGAAGGCCTTTTGGCTTAGTATTGGGGTCTTCGCGATTGGTTTGAGTTTCCTCTTTTCTAGTGATTTGGGAGTTCGA
ATGGGTACTTTAGACTCTTCTATGGAAGAACGCATTTCTATCTGGGATGCTGGGATGGCCTTGTTTAAGCAAAATC
CTTTTTGGGGTGAAGGGCCATTGACCTATATGAACTCTTATCCTCGGATACATGCTCCTTATCATGAACATGCCCA
CAGTCTTTATATTGATACGATTCTGAGTTACGGAATTGTGGGGACTATTTTATTAGTTTTGTCTTCTGTTGCTCCTG
TTCGCTTGATGATGGATATGAGTCAGGAGTCGGGGAAACGTCCGATTATCGGCCTTTATCTATCTTTCCTTACAGT
GGTTGCTGTGCACGGAATTTTTGACTTGGCTCTCTTCTGGATTCAGTCAGGCTTTATTTTCTTGCTAGTTATGTGCA
GTATTCCATTGGAGCATCGAATGTTGGTATCGGACATGACGGATTAA

4120.1
ATGTCAAAGATGGATGTTCAGAAAATCATTGCACCGATGATGAAGTTTGTGAATATGCGTGGCATTATAGCTCTAA
AAGATGGGATGTTAGCAATTTTGCCATTGACAGTAGTTGGTAGTTTGTTCTTGATTATGGGACAATTGCCGTTCGA
AGGATTAAATAAGAGCATTGCTAGTGTTTTTGGAGCTAATTGGACAGAGCCGTTTATGCAAGTATATTCAGGAACT
TTTGCTATTATGGGTCTAATTTCTTGTTTTTCAATTGCCTATTCTTATGCTAAGAATAGCGGAGTAGAGGCTTTACC
AGCTGGAGTTCTATCTGTATCTGCATTCTTTATTTTGCTAAGATCATCTTATATCCCTAAACAAGGTGAGGCGATTG
GGACGCTATTAGTAAAGTTTGGTTTGGAGGCCAAGGAATTATCGGTGCTATCATTATAGGTTTGGTAGTAGGAAG
TATTTATACCTTCTTTATAAAGAGAAAAATTGTTATTAAGATGCCAGAACAAGTTCCACAAGCTATTGCCAAACAG
TTTGAAGCAATGATTCCAGCATTTGTAATTTTCTTATCTTCTATGATTGTATATATTTTAGCGAAGTCATTGACTAA
TGGCGGAACATTCATAGAAATGATTTATTCTGCTATTCAAGTTCCGTTGCAAGGTTTAACTGGATCTTTGTATGGT
GCTATTGGAATTGCATTCTTTATATCATTTTTGTGGTGGTTTGGTGTTCATGGGCAATCGGTAGTAAATGGAGTAGT
GACAGCTCTGCTTTTATCTAATCTTGATGCTAATAAAGCTATGTTAGCCTCTGCTAATCTATCATTAGAAAATGGT
GCACATATTGTTACTCAACAATTTTTAGATTCATTTTTAATTCTATCAGGTTCAGGGATTACGTTTGGTCTTGTAGT
TGCCATGCTTTTTGCAGCAAAATCAAAACAATACCAAGCCTTAGGAAAAGTTGCAGCTTTTCCAGCAATATTTAAC
GTAAATGAGCCAGTTGTATTTGGATTTCCGATTGTCATGAATCCAGTTATGTTTGTACCTTTCATTCTTGTTCCTGT
ACTTGCAGCTGTGATAGTATATGGAGCTATTGCAACAGGTTTCATGCAGCCATTCTCAGGGGTAACATTGCCTTGG
AGTACACCAGCTATTTTATCAGGATTTTTGGTGGGTGGATGGCAAGGAGTTATTACTCAGCTGGTGATATTAGCGA
TGTCTACATTGGTTTATTTTCCATTCTTTAAAGTACAGGATCGTTTAGCTTACCAAAATGAAATCAAACAATCTTAG

4121.2
ATGAAGAAAAAGGACTTAGTAGACCAACTAGTCTCAGAGATCGAGACGGGGAAAGTCAGGACACTGGGAATATA
CGGTCATGGAGCTTCAGGTAAATCAACCTTTGCACAGGAATTGTACCAAGCTTTAGATTCTACTACAGTAAATTTG

CTAGAGACAGATCCTTATATCACCTCAGGACGCCATCTGGTAGTACCCAAGGACGCGCCGAATCAAAAGGTGACA
GCCAGTCTGCCAGTGGCGCATGAACTGGAGAGTTTGCAGAGAGATATCCTTgCTTGCAGGCGGGTATGGATGTCTT
GA

4122.1
ATGAAGAAAAGATACCTAGTCTTGACAGCTTTGCTAGCCTTGAGTCTAGCAGCTTGTTCACAAGAAAAAACAAAA
AATGAAGATGGAGAAACTAAGACAGAACAGACAGCCAAAGCTGATGGAACAGTCGGTAGTAAGTCTCAAGGAGC
TGCCCAGAAGAAAGCAGAAGTGGTCAATAAAGGTGATTACTACAGCATTCAAGGGAAATACGATGAAATCATCGT
AGCCAACAAACACTATCCATTGTCTAAAGACTATAATCCAGGGGAAAATCCAACAGCCAAGGCAGAGTTGGTCAA
ACTCATCAAAGCGATGCAAGAGGCAGGTTTCCCTATTAGTGATCATTACAGTGGTTTTAGAAGTTATGAAACTCAG
ACCAAGCTCTATCAAGATTATGTCAACCAAGATGGAAAGGCAGCAGCTGACCGTTACTCTGCCCGTCCTGGCTAT
AGCGAACACCAGACAGGCTTGGCCTTTGATGTGATTGGGACTGATGGTGATTTGGTGACAGAAGAAAAAGCAGCC
CAATGGCTCTTGGATCATGCAGCTGATTATGGCTTTGTTGTCCGTTATCTCAAAGGCAAGGAAAAGGAAACAGGCT
ATATGGCTGAAGAATGGCACCTGCGTTATGTAGGAAAAGAAGCTAAAGAAATTGCTGCAAGTGGTCTCAGTTTGG
AAGAATACTATGGCTTTGAAGGCGGAGACTACGTCGATTAA

4125.6
ATGCGTAAATTCTTAATTATTTTGTTGCTACCAAGTTTTTTGACCATTTCAAAAGTCGTTAGCACAGAAAAAGAAG
TCGTCTATACTTCGAAAGAAATTTATTACCTTTCACAATCTGACTTTGGTATTTATTTTAGAGAAAAATTAAGTTCT
CCCATGGTTTATGGAGAGGTTCCTGTTTATGCGAATGAAGATTTAGTAGTGGAATCTGGGAAATTGACTCCCAAAA
CAAGTTTTCAAATAACCGAGTGGCGCTTAAATAAACAAGGAATTCCAGTATTTAAGCTATCAAATCATCAATTTAT
AGCTGCGGACAAACGATTTTTATATGATCAATCAGAGGTAACTCCAACAATAAAAAAAGTATGGTTAGAATCTGA
CTTTAAACTGTACAATAGTCCTTATGATTTAAAAGAAGTGAAATCATCCTTATCAGCTTATTCGCAAGTATCAATC
GACAAGACCATGTTTGTAGAAGGAAGAGAATTTCTACATATTGATCAGGCTGGATGGGTAGCTAAAGAATCAACT
TCTGAAGAAGATAATCGGATGAGTAAAGTTCAAGAAATGTTATCTGAAAAATATCAGAAAGATTCTTTCTCTATTT
ATGTTAAGCAACTGACTACTGGAAAAGAAGCTGGTATCAATCAAGATGAAAAGATGTATGCAGCCAGCGTTTTGA
AACTCTCTTATCTCTATTATACGCAAGAAAAAATAAATGAGGGTCTTTATCAGTTAGATACGACTGTAAAATACGT
ATCTGCAGTCAATGATTTTCCAGGTTCTTATAAACCAGAGGGAAGTGGTAGTCTTCCTAAAAAAGAAGATAATAA
AGAATATTCTTTAAAGGATTTAATTACGAAAGTATCAAAAGAATCTGATAATGTAGCTCATAATCTATTGGGATAT
TACATTTCAAACCAATCTGATGCCACATTCAAATCCAAGATGTCTGCCATTATGGGAGATGATTGGGATCCAAAAG
AAAAATTGATTTCTTCTAAGATGGCCGGGAAGTTTATGGAAGCTATTTATAATCAAAATGGATTTGTGCTAGAGTC
TTTGACTAAAACAGATTTTGATAGTCAGCGAATTGCCAAAGGTGTTTCTGTTAAAGTAG
CTCATAAAATTGGAGATGCGGATGAATTTAAGCATGATACGGGTGTTGTCTATGCAGATTCTCCATTTATTCTTTCT
ATTTTCACTAAGAATTCTGATTATGATACGATTTCTAAGATAGCCAAGGATGTTTATGAGGTTCTAAAATGA

4125.7
ATGAAAAAACAAAATAATGGTTTAATTAAAAATCCTTTTCTATGGTTATTATTTATCTTTTTCCTTGTGACAGGATT
CCAGTATTTCTATTCTGGGAATAACTCAGGAGGAAGTCAGCAAATCAACTATACTGAGTTGGTACAAGAAATTAC
CGATGGTAATGTAAAAGAATTAACTTACCAACCAAATGGTAGTGTTATCGAAGTTTCTGGTGTCTATAAAAATCCT
AAAACAAGTAAAGAAGAAACAGGTATTCAGTTTTTCACGCCATCTGTTACTAAGGTAGAGAAATTTACCAGCACT
ATTCTTCCTGCAGATACTACCGTATCAGAATTGCAAAAACTTGCTACTGACCATAAAGCAGAAGTAACTGTTAAGC
ATGAAAGTTCAAGTGGTATATGGATTAATCTACTCGTATCCATTGTGCCATTTGGAATTCTATTCTTCTTCCTATTC
TCTATGATGGGAAATATGGGAGGAGGCAATGGCCGTAATCCAATGAGTTTTGGACGTAGTAAGGCTAAAGCAGCA
AATAAAGAAGATATTAAAGTAAGATTTTCAGATGTTGCTGGAGCTGAGGAAGAAAAACAAGAACTAGTTGAAGTT
GTTGAGTTCTTAAAAGATCCAAAACGATTCACAAAACTTGGAGCCCGTATTCCAGCAGGTGTTCTTTTGGAGGGAC
CTCCGGGGACAGGTAAAACTTTGCTTGCTAAGGCAGTCGCTGGAGAAGCAGGTGTTCCATTCTTTAGTATCTCAGG
TTCTGACTTTGTAGAAATGTTTGTCGGAGTTGGAGCTAGTCGTGTTCGCTCTCTTTTTGAGGATGCCAAAAAAGCA
GCACCAGCTATCATCTTTATCGATGAAATTGATGCTGTTGGACGTCAACGTGGAGTCGGTCTCGGCGGAGGTAATG
ACGAACGTGAACAAACCTTGAACCAACTTTTGATTGAGATGGATGGTTTTGAGGGAAATGAAGGGATTATCGTCA
TCGCTGCGACAAACCGTTCAGATGTACTTGACCCTGCCCTTTTGCGTCCAGGACGTTTTGATAGAAAAGTATTGGT
TGGTCGTCCTGATGTTAAAGGTCGTGAAGCAATCTTGAAAGTTCACGCTAAGAATAAGC
CTTTAGCAGAAGATGTTGATTTGAAATTAGTGGCTCAACAAACTCCAGGCTTTGTTGGTGCTGATTTAGAGAATGT
CTTGAATGAAGCAGCTTTAGTTGCTGCTCGTCGCAATAAATCGATAATTGATGCTTCAGATATTGATGAAGCAGAA
GATAGAGTTATTGCTGGACCTTCTAAGAAAGATAAGACAGTTTCACAAAAAGAACGAGAATTGGTTGCTTACCAT
GAGGCAGGACATACCATTGTTGGTCTAGTCTTGTCGAATGCTCGCGTTGTCCATAAGGTTACAATTGTACCACGCG
GCCGTGCAGGCGGATACATGATTGCACTTCCTAAAGAGGATCAAATGCTTCTATCTAAAGAAGATATGAAAGAGC
AATTGGCTGGCTTAATGGGTGGACGTGTAGCTGAAGAAATTATCTTTAATGTCCAAACCACAGGAGCTTCAAACG
ACTTTGAACAAGCGACACAAATGGCACGTGCAATGGTTACAGAGTACGGTATGAGTGAAAAACTTGGCCCAGTAC
AATATGAAGGAAACCATGCTATGCTTGGTGCACAGAGTCCTCAAAAATCAATTTCAGAACAAACAGCTTATGAAA
TTGATGAAGAGGTTCGTTCATTATTAAATGAGGCACGAAATAAAGCTGCTGAAATTATTCAGTCAAATCGTGAAAC
TCACAAGTTAATTGCAGAAGCATTATTGAAATACGAAACATTGGATAGTACACAAATTAAAGCTCTTTACGAAAC
AGGAAAGATGCCTGAAGCAGTAGAAGAGGAATCTCATGCACTATCCTATGATGAAGTAAAGTCAAAAATGAATGA
CGAAAAATAA

4125.10
ATGAGGGAACCAGATTTTTTAAATCATTTTCTCAAGAAGGGATATTTCAAAAAGCATGCTAAGGCGGTTCTAGCTC
TTTCTGGTGGATTAGATTCCATGTTTCTATTTAAGGTATTGTCTACTTATCAAAAAGAGTTAGAGATTGAATTGATT
CTAGCTCATGTGAATCATAAGCAGAGAATTGAATCAGATTGGGAAGAAAAGGAATTAAGGAAGTTGGCTGCTGAA
GCAGAGCTTCCTATTTATATCAGCAATTTTTCAGGAGAATTTTCAGAAGCGCGTGCACGAAATTTTCGTTATGATT
TTTTTCAAGAGGTCATGAAAAAGACAGGTGCGACAGCTTTAGTCACTGCCCACCATGCTGATGATCAGGTGGAAA
CGATTTTTATGCGCTTGATTCGAGGAACTCGCTTGCGCTATCTATCAGGAATTAAGGAGAAGCAAGTAGTCGGAGA
GATAGAAATCATTCGTCCCTTCTTGCATTTTCAGAAAAAAGACTTTCCATCAATTTTTCACTTTGAAGATACATCA
AATCAGGAGAATCATTATTTTCGAAATCGTATTCGAAATTCTTACTTACCAGAATTGGAAAAAGAAAATCCTCGAT
TTAGGGATGCAATCTTAGGCATTGGCAATGAAATTTTAGATTATGATTTGGCAATAGCTGAATTATCTAACAATAT
TAATGTGGAAGATTTACAGCAGTTATTTCTTACTCTGAGTCTACACAAAGAGTTTTACTTCAAACTTATCTGAATC
GTTTTCCAGATTTGAATCTTACAAAAGCTCAGTTTGCTGAAGTTCAGCAGATTTTAAAATCTAAAAGCCAGTATCG
TCATCCGATTAAAAATGGCTATGAATTGATAAAAGAGTACCAACAGTTTCAGATTTGTAAAATCAGTCCGCAGGCT
GATGAAAAGGAAGATGAACTTGTGTTACACTATCAAAATCAGGTAGCTTATCAAGGATATTTATTTTCTTTTGGAC
TTCCATTAGAAGGTGAATTAATTCAACAAATACCTGTTTCACGTGAAACATCCATACACATTCGTCATCGAAAAAC
AGGAGATGTTTTGATTAAAAATGGGCATAGAAAAAAACTCAGACGTTTATTTATTG
ATTTGAAAATCCCTATGGAAAAGAGAAACTCTGCTCTTATTATTGAGCAATTTGGTGAAATTGTCTCAATTTTGGG
AATTGCGACCAATAATTTGAGTAAAAAAACGAAAAATGATATAATGAACACTGTACTTTATATAGAAAAAATAGA
TAGGTAA

4126.1
ATGAAGCGTTCTTCTCTTTTAGTTAGAATGGTTATTTCCATCTTTCTGGTCTTTCTCATTCTCCTAGCTCTGGTTGGA
ACTTTCTACTATCAATCAAGTTCTTCAGCCATTGAGGCCACCATTGAGGGCAACAGCCAAACGACCATCAGCCAG
ACTAGCCACTTTATTCAGTCTTATATCAAAAAACTAGAAACCACCTCGACTGGTTTGACCCAGCAGACGGATGTTC
TGGCCTATGCTGAGAATCCCAGTCAAGACAAGGTCGAGGGAATCCGAGATTTGTTTTTGACCATCTTGAAGTCAGA
TAAGGACTTGAAAACTGTTGTGCTGGTGACCAAATCTGGTCAGGTCATTTCTACAGATGACAGTGTGCAGATGAA
AACTTCCTCTGATATGATGGCTGAGGATTGGTACCAAAAGGCCATTCATCAGGGAGCTATGCCTGTTTTGACTCCA
GCTCGTAAATCAGATAGTCAGTGGGTCATTTCTGTCACTCAAGAACTTGTTGATGCAAAGGGAGCCAATCTTGGTG
TGCTTCGTTTGGATATTTCTTATGAAACTCTGGAAGCCTATCTCAATCAACTCCAGTTGGGGCAGCAGGGCTTTGC
CTTCATTATCAATGAAAACCATGAATTTGTCTACCATCCTCAACACACAGTTTATAGTTCGTCTAGCAAAATGGAG
GCTATGAAACCCTACATCGATACAGGTCAGGGTTATACTCCTGGTCACAAATCCTACGTCAGTCAAGAGAAGATT
GCAGGAACTGATTGGACGGTGCTTGGCGTGTCATCATTGGAAAAGTTAGACCAGGTTCGGAGTCAGCTCTTGTGG
ACCTTGCTTGGGGCCAGTGTCACATCTCTTCTTGTCTGTCTCTGCTTAGTGTGGTTCAGTCTTAAACGCTGGATTGC
TCCTTTGAAGGATTTGAGAGAAACCATGTTGGAAATTGCTTCTGGTGCTCAAAATCTTCGTGCCAAGGAAGTTGGT
GCCTATGAACTGAGAGAAGTAACTCGCCAATTTAATGCTATGTTGGATCAGATTGATCAGTTGATGGTAGCTATTC
GTAGCCAGGAAGAAACGACCCGTCAGTACCAACTTCAAGCCCTTTCGAGCCAGATTAATCCACATTTCCTCTATAA
CACTTTGGACACCATCATCTGGATGGCTGAATTTCATGATAGTCAGCGAGTGGTGCAGGTGACCAAGTCCTTGGCA
ACCTATTTCCGCTTGGCGCTCAATCAAGGCAAGGACTTGATTTGTCTCTCTGACGAAATCAATCATGTCCGCCAGT
ATCTCTTTATCCAGAAACAACGCTATGGAGATAAGCTGGAATACGAAATTAATGAAAATGTTGCCTTTGATAATTT
AGTCTTACCCAAGCTGGTCCTACAACCCCTTGTAGAAAATGCTCTTTACCATGGCATTAAGGAAAAGGAAGGTCA
GGGCCATATTAAACTTTCTGTCCAGAAACAGGATTCGGGATTGGTCATCCGTATTGAGGATGATGGCGTTGGCTTC
CAAGATGCTGGTGATAGTAGTCAAAGTCAACTCAAACGTGGGGGAGTTGGTCTTCAAAATGTCGATCAACGGCTC
AAACTTCATTTTGGAGCCAATTACCATATGAAGATTGATTCTAGACCCCAAAAAGGGACGAAAGTTGAAATATAT
ATAAATAGAATAGAAACTAGCTAA

4126.7
ATGAAGCGTTCTTCTCTTTTAGTTAGAATGGTTATTTCCATCTTTCTGGTCTTTCTCATTCTCCTAGCTCTGGTTGGA
ACTTTCTACTATCAATCAAGTTCTTCAGCCATTGAGGCCACCATTGAGGGCAACAGCCAAACGACCATCAGCCAG
ACTAGCCACTTTATTCAGTCTTATATCAAAAAACTAGAAACCACCTCGACTGGTTTGACCCAGCAGACGGATGTTC
TGGCCTATGCTGAGAATCCCAGTCAAGACAAGGTCGAGGGAATCCGAGATTTGTTTTTGACCATCTTGAAGTCAGA
TAAGGACTTGAAAACTGTTGTGCTGGTGACCAAATCTGGTCAGGTCATTTCTACAGATGACAGTGTGCAGATGAA
AACTTCCTCTGATATGATGGCTGAGGATTGGTACCAAAAGGCCATTCATCAGGGAGCTATGCCTGTTTTGACTCCA
GCTCGTAAATCAGATAGTCAGTGGGTCATTTCTGTCACTCAAGAACTTGTTGATGCAAAGGGAGCCAATCTTGGTG
TGCTTCGTTTGGATATTTCTTATGAAACTCTGGAAGCCTATCTCAATCAACTCCAGTTGGGGCAGCAGGGCTTTGC
CTTCATTATCAATGAAAACCATGAATTTGTCTACCATCCTCAACACACAGTTTATAGTTCGTCTAGCAAAATGGAG
GCTATGAAACCCTACATCGATACAGGTCAGGGTTATACTCCTGGTCACAAATCCTACGTCAGTCAAGAGAAGATT
GCAGGAACTGATTGGACGGTGCTTGGCGTGTCATCATTGGAAAAGTTAGACCAGGTTCGGAGTCAGCTCTTGTGG
ACCTTGCTTGGGGCCAGTGTCACATCTCTTCTTGTCTGTCTCTGCTTAGTGTGGTTCAGTCTTAAACGCTGGATTGC
TCCTTTGAAGGATTTGAGAGAAACCATGTTGGAAATTGCTTCTGGTGCTCAAAATCTTCGTGCCAAGGAAGTTGGT
GCCTATGAACTGAGAGAAGTAACTCGCCAATTTAATGCTATGTTGGATCAGATTGATCAGTTGATGGTAGCTATTC
GTAGCCAGGAAGAAACGACCCGTCAGTACCAACTTCAAGCCCTTTCGAGCCAGATTAATCCACATTTCCTCTATAA
CACTTTGGACACCATCATCTGGATGGCTGAATTTCATGATAGTCAGCGAGTGGTGCAGGTGACCAAGTCCTTGGCA
ACCTATTTCCGCTTGGCGCTCAATCAAGGCAAGGACTTGATTTGTCTCTCTGACGAAATCAATCATGTCCGCCAGT
ATCTCTTTATCCAGAAACAACGCTATGGAGATAAGCTGGAATACGAAATTAATGAAAATGTTGCCTTTGATAATTT

AGTCTTACCCAAGCTGGTCCTACAACCCCTTGTAGAAAATGCTCTTTACCATGGCATTAAGGAAAAGGAAGGTCA
GGGCCATATTAAACTTTCTGTCCAGAAACAGGATTCGGGATTGGTCATCCGTATTGAGGATGATGGCGTTGGCTTC
CAAGATGCTGGTGATAGTAGTCAAAGTCAACTCAAACGTGGGGGAGTTGGTCTTCAAAATGTCGATCAACGGCTC
AAACTTCATTTTGGAGCCAATTACCATATGAAGATTGATTCTAGACCCCAAAAAGGGACGAAAGTTGAAATATAT
ATAAATAGAATAGAAACTAGCTAA

4127.4
ATGTTTTTTAAATTATTAAGAGAAGCTCTTAAAGTCAAGCAGGTTCGATCAAAAATTTTATTTACAATTTTTATCGT
TTTGGTCTTTCGTATCGGAACTAGCATTACAGTTCCTGGTGTGAATGCCAATAGCTTGAATGCTTTAAGTGGATTAT
CCTTCTTAAACATGTTGAGCTTGGTGTCGGGGAATGCCCTAAAAAACTTTTCGATTTTTGCCCTAGGAGTTAGTCC
CTATATCACCGCTTCTATTGTTGTCCAACTCTTGCAAATGGATATTTTACCCAAGTTTGTAGAGTGGGGTAAACAA
GGGGAAGTAGGTCGAAGAAAATTGAATCAAGCTACTCGTTATATTGCTCTAGTTCTCGCTTTTGTGCAATCTATCG
GGATTACAGCTGGTTTTAATACCTTGGCTGGAGCTCAATTGATTAAAACTGCTTTAACTCCACAAGTTTTTCTGAC
GATTGGTATCATCTTAACAGCTGGTAGTATGATTGTCACTTGGTTGGGTGAGCAAATTACAGATAAGGGATACGGA
AACGGTGTTTCCATGATTATCTTTGCCGGGATTGTTTCCTCAATTCCAGAGATGATTCAGGGCATCTATGTGGACT
ACTTTGTGAACGTCCCAAGTAGCCGTATCACTTCATCTATCATTTTCGTAATCATTTTGATTATTACTGTATTGTTG
ATTATTTACTTTACAACTTATGTTCAACAAGCAGAATACAAAATTCCAATCCAATATACTAAGGTTGCACAAGGTG
CTCCATCTAGCTCTTACCTTCCGTTAAAAGTAAACCCTGCTGGAGTTATCCCTGTTATCTTTGCCAGTTCGATTACT
GCAGCGCCTGCGGCTATTCTTCAGTTTTTGAGTGCCACAGGTCATGATTGGGCTTGGGTAAGGGTAGCACAAGAGA
TGTTGGCAACTACTTCTCCAACTGGTATTGCCATGTATGCTTTGTTGATTATTCTCTTTACATTCTTCTATACGTTTG
TACAGATTAATCCTGAAAAAGCAGCAGAGACCTACAAAAGAGTGGTGCCTATATCCATGGAGTTCGTCCTGGTAA
AGGTACAGAAGAATATATGTCTAAACTTCTTCGTCGTCTTGCAACTGTTGGTTCCCTCTTCCTTGGTGTGA

4127.5
ATGGATATTAGACAAGTTACTGAAACCATCGCCATGATTGAGGAGCAAAACTTCGATATTAGAACCATTACCATG
GGGATTTCTCTTTTGGACTGTATCGATCCAGATATCAATCGTGCTGCGGAGAAAATCTATCAAAAAATTACGACAA
AGGCGGCTAATTTAGTAGCTGTTGGTGATGAAATTGCGGCTGAGTTGGGAATTCCTATCGTTAATAAGCGTGTATC
GGTGACACCTATTTCTCTGATTGGGGCAGCGACAGATGCGACGGACTACGTGGTTCTGGCAAAAGCGCTTGATAA
GGCTGCGAAAGAGATTGGTGTGGACTTTATTGGTGGTTTTTCTGCCTTAGTACAAAAAGGTTATCAAAAGGGAGAT
GAGATTCTCATCAATTCCATTCCTCGCGCTTTGGCTGAGACGGATAAGGTCTGCTCGTCAGTCAATATCGGCTCAA
CCAAGTCTGGTATTAATATGACGGCTGTGGCAGATATGGGACGAATTATCAAGGAAACAGCAAATCTTTCAGATA
TGGGAGTGGCCAAGTTGGTTGTATTCGCTAATGCTGTTGAGGACAATCCATTTATGGCGGGTGCCTTTCATGGTGT
TGGGGAAGCAGATGTTATCATCAATGTCGGAGTTTCTGGTCCTGGTGTTGTGAAACGTGCTTTGGAAAAAGTTCGT
GGACAGAGCTTTGATGTAGTAGCCGAAACAGTTAAGAAAACTGCCTTTAAAATCACTCGTATCGGTCAATTGGTTG
GTCAAATGGCCAGTGAGAGACTGGGTGTGGAGTTTGGTATTGTGGACTTGAGTTTGGCACCAACCCCTGCGGTTGG
AGACTCTGTGGCACGTGTCCTTGAGGAAATGGGGCTAGAAACAGTTGGCACGCATGGAACGACGGCTGCCTTGGC
CCTCTTGAACGACCAAGTTAAAAAGGGTGGAGTGATGGCCTGCAACCAAGTCGGTGGTTTATCTGGTGCCTTTATC
CCTGTTTCTGAGGATGAAGGAATGATTGCTGCAGTGCAAAATGGCTCTCTTAATTTAGAAAAACTAGAAGCTATGA
CGGCTATCTGTTCTGTTGGATTGGATATGATTGCCATCCCAGAAGATACGCCTGCTGAAACTATTGCGGCTATGAT
TGCGGATGAAGCAGCAATCGGTGTTATCAACATGAAAACAACAGCTGTTCGTATCATTCCCAAAGGAAAAGAAGG
CGATATGATTGAGTTTGGTGGTCTATTAGGAACTGCACCCGTTATGAAGGTTAATGGGGCTTCGTCTGTCGACTTC
ATCTCTCGCGGTGGACAAATCCCAGCACCAATTCATAGTTTTAAAAATTAA

4128.1
ATGACACAGATTATTGATGGGAAAGCTTTAGCGGCCAAATTGCAGGGGCAGTTGGCTGAAAAGACTGCAAAATTA
AAGGAAGAAACAGGTCTAGTGCCTGGTTTGGTAGTGATTTTGGTTGGGGACAATCCAGCCAGCCAAGTCTACGTT
CGCAACAAGGAGAGGTCAGCCCTTGCGGCTGGTTTCCGTAGCGAAGTAGTACGGGTTCCAGAGACCATTACTCAA
GAGGAATTGTTAGACCTGATTGCTAAATACAATCAGGATCCAGCTTGGCATGGGATTTTGGTTCAGTTGCCATTAC
CAAAACACATTGATGAAGAGGCGGTTCTATTGGCTATTGACCCAGAAAAGGATGTGGATGGTTTCCATCCTCTAA
ACATGGGGCGTCTTTGGTCTGGTCATCCAGTCATGATTCCTTCGACACCGGCAGGAATTATGGAAATGTTCCATGA
ATATGGGATTGACTTGGAAGGTAAAAATGCAGTCGTCATCGGTCGATCCAATATTGTCGGAAAACCTATGGCCCA
GCTTCTTTTGGCAAAGAATGCAACAGTAACCTTGACTCACTCACGTACTCATAATCTTTCCAAGGTGGCTGCAAAA
GCAGATATTCTGGTTGTTGCAATCGGTCGTGCCAAGTTTGTGACTGCTGACTTTGTCAAACCAGGTGCGGTAGTCA
TTGACGTTGGGATGAACCGCGATGAAAATGGTAAGCTCTGTGGGGATGTTGATTATGAGGCGGTTGCCCCACTTGC
TAGCCACATTACGCCAGTCCCTGGAGGTGTCGGTCCTATGACCATTACTATGCTGATGGAGCAAACCTATCAGGCA
GCACTTAGGACATTGGATAGAAAATAA

4128.2
ATGTCTAAATTTAATCGTATTCATTTGGTGGTACTGGATTCTGTAGGAATCGGTGCAGCACCAGATGCTAATAACT
TTGTCAATGCAGGGGTTCCAGATGGAGCTTCTGACACACTGGGACACATTTCAAAAACAGTTGGTTTGAATGTCCC
AAACATGGCTAAAATAGGTCTTGGAAATATTCCTCGTGAAACTCCTCTTAAGACTGTAGCAGCTGAAAGCAATCC
AACTGGATATGCAACAAAATTAGAGGAAGTATCTCTTGGTAAGGATACTATGACTGGACACTGGGAAATCATGGG
ACTCAACATTACTGAGCCTTTCGATACTTTCTGGAACGGATTCCCAGAAGAAATCCTGACAAAAATCGAAGAATTC
TCAGGACGCAAGGTTATTCGTGAAGCCAACAAACCTTATTCAGGAACGGCTGTTATCTATGATTTTGGACCACGTC

AGATGGAAACTGGAGAGTTGATTATCTATACTTCAGCTGACCCTGTTTTGCAGATTGCTGCCCACGAAGACATTAT
TCCTTTGGATGAATTGTACCGTATCTGTGAATACGCTCGTTCGATTACCCTTGAGCGTCCTGCCCTTCTTGGTCGCA
TCATTGCTCGCCCTTATGTAGGTGAACCAGGTAACTTCACTCGTACGGCAAACCGTCGTGACTTGGCTGTATCTCC
ATTTTTCCCAACTGTTTTGGATAAATTGAATGAGGCTGGTATCGATACTTATGCTGTGGGTAAAATCAACGATATC
TTTAACGGTGCTGGTATCAACCATGACATGGGTCACAACAAGTCAAATAGTCATGGAATTGATACACTATTGAAG
ACTATGGGACTTGCTGAGTTTGAAAAAGGATTCTCATTCACAAACCTAGTTGACTTTGATGCCCTTTACGGCCATC
GTCGTAATGCTCACGGTTACCGTGATTGCTTGCATGAGTTTGATGAACGCTTACCTGAAATTATCGCAGCTATGAG
AGAGAATGACCTTCTCTTGATTACTGCGGACCATGGAAATGACCCAACGTATGCAGGAACGGATCACACTCGGGA
ATATATTCCATTGTTGGCCTATAGCCCTGCCTTTAAAGGAAATGGTCTCATTCCAGTAGGACATTTTGCAGATATTT
CAGCGACTGTTGCCGATAACTTTGGTGTGGAAACTGCTATGATTGGGGAAAGTTTCTTAGATAAATTGGTATAA

4129.2
ATGTTTATTTCCATCAGTGCTGGAATTGTGACATTTTTACTAACTTTAGTAGAAATTCCGGCCTTTATCCAATTTTA
TAGAAAGGCGCAAATTACAGGCCAGCAGATGCATGAGGATGTCAAACAGCATCAGGCAAAAGCTGGGACTCCTA
CAATGGGAGGTTTGGTTTTCTTGATTACTTCTGTTTTGGTTGCTTTCTTTTTCGCCCTATTTAGTAGCCAATTCAGCA
ATAATGTGGGAATGATTTTGTTCATCTTGGTCTTGTATGGCTTGGTCGGATTTTTAGATGACTTTCTCAAGGTCTTT
CGTAAAATCAATGAGGGGCTTAATCCTAAGCAAAAATTAGCTCTTCAGCTTCTAGGTGGAGTTATCTTCTATCTTT
TCTATGAGCGCGGTGGCGATATCCTGTCTGTCTTTGGTTATCCAGTTCATTTGGGATTTTTCTATATTTTCTTCGCT
CTTTTCTGGCTAGTCGGTTTTTCAAACGCAGTAAACTTGACAGACGGTGTTGACGGTTTAGCTAGTATTTCCGTTGT
GATTAGTTTGTCTGCCTATGGAGTTATTGCCTATGTGCAAGGTCAGATGGATATTCTTCTAGTGATTCTTGCCATGA
TTGGTGGTTTGCTCGGTTTCTTCATCTTTAACCATAAGCCTGCCAAGGTCTTTATGGGTGATGTGGGAAGTTTGGCC
CTAGGTGGGATGCTGGCAGCTATCTCTATGGCTCTCCACCAAGAATGGACTCTCTTGATTATCGGAATTGTGTATG
TTTTTGAAACAACTTCTGTTATGATGCAAGTCAGTTATTTCAAACTGACAGGTGGTAAACGTATTTTCCGTATGAC
GCCTGTACATCACCATTTTGAGCTTGGGGGATTGTCTGGTAAAGGAAATCCTTGGAGCGAGTGGAAGGTTGACTTC
TTCTTTTGGGGAGTGGGACTTCTAGCAAGTCTCCTGACCCTAGCAATTTTATATTTGATGTAA

4133.1
TTGTTTAAGAAAAATAAAGACATTCTTAATATTGCATTGCCAGCTATGGGTGAAAACTTTTTGCAGATGCTAATGG
GAATGGTGGACAGTTATTTGGTTGCTCATTTAGGATTGATAGCTATTTCAGGGGTTTCAGTAGCTGGTAATATTAT
CACCATTTATCAGGCGATTTTCATCGCTCTGGGAGCTGCTATTTCCAGTGTTATTTCAAAAAGCATAGGGCAGAAA
GACCAGTCGAAGTTGGCCTATCATGTGACTGAGGCGTTGAAGATTACCTTACTATTAAGTTTCCTTTTAGGATTTTT
GTCCATCTTCGCTGGGAAAGAGATGATAGGACTTTTGGGGACGGAGAGGGATGTAGCTGAGAGTGGTGGACTGTA
TCTATCTTTGGTAGGCGGATCGATTGTTCTCTTAGGTTTAATGACTAGTCTAGGAGCCTTGATTCGTGCAACGCAT
AATCCACGTCTGCCTCTCTATGTTAGTTTTTTATCCAATGCCTTGAATATTCTTTTTTCAAGTCTAGCTATTTTTGTT
CTGGATATGGGGATAGCTGGTGTTGCTTGGGGGACAATTGTGTCTCGTTTGGTTGGTCTTGTGATTTTGTGGTCAC
AATTAAAACTGCCTTATGGGAAGCCAACTTTTGGTTTAGATAAGGAACTGTTGACCTTGGCTTTACCAGCAGCTGG
AGAGCGACTTATGATGAGGGCTGGAGATGTAGTGATCATTGCCTTGGTCGTTTCTTTTGGGACGGAGGCAGTTGCT
GGGAATGCAATCGGAGAAGTCTTGACCCAGTTTAACTATATGCCTGCCTTTGGCGTCGCTACGGCAACGGTCATGC
TGTTGGCCCGAGCAGTTGGAGAGGATGATTGGAAAAGAGTTGCTAGTTTGAGTAAACAAACCTTTTGGCTTTCTCT
GTTCCTCATGTTGCCCCTGTCCTTTAGTATATATGTCTTGGGTGTACCATTAACTCATCTCTATACGACTGATTCTC
TAGCGGTGGAGGCTAGTGTTCTAGTGACACTGTTTTCACTACTTGGGACCCCTATGACGACAGGAACAGTCATCTA
TACGGCAGTCTGGCAGGGATTAGGAAATGCACGCCTCCCTTTTTATGCGACAAGTATAGGAATGTGGTGTATCCGC
ATTGGGACAGGATATCTGATGGGGATTGTGCTTGGTTGGGGCTTGCCTGGTATTTGGGCAGGGTCTCTCTTGGATA
ATGGTTTTCGCTGGTTATTTCTACGCTATCGTTACCAGCGCTATATGAGCTTGAAAGGATAG

4135.2
ATGCAAACTCAAGAAAAACACTCGCAAGCAGCCGTTCTTGGCTTGCAGCACTTACTAGCCATGTACTCAGGATCT
ATCCTGGTTCCCATCATGATTGCGACAGCCCTTGGCTATTCAGCTGAGCAGTTGACCTACCTGATTTCTACAGATA
TCTTCATGTGTGGGGTGGCAACCTTCCTCCAACTCCAACTCAACAAATACTTTGGGATTGGACTCCCAGTCGTTCT
TGGAGTTGCATTCCAGTCGGTCGCTCCCTTGATTATGATTGGGCAAAGCCATGGTAGTGGCGCTATGTTTGGTGCC
CTTATCGCATCTGGGATTTACGTGGTTCTTGTTTCAGGCATCTTCTCAAAAGTAGCCAATCTCTTCCCATCTATCGT
AACAGGATCTGTTATTACCACGATTGGTTTAACCTTGATCCCTGTCGCTATTGGAAATATGGGAAATAACGTTCCA
GAGCCAACTGGTCAAAGTCTCTTGCTTGCAGCTATTACTGTTCTGATTATCCTCTTGATCAACATCTTTACCAAAG
GATTTATCAAGTCTATCTCTATTTTGATTGGTCTGGTTGTTGGAACTGCCATTGCTGCTACTATGGGCTTGGTGGAC
TTCTCTCCTGTTGCGGTAGCTCCACTTGTCCATGTCCCAACTCCACTCTACTTTGGGATGCCAACCTTTGAAATCTC
ATCTATTGTCATGATGTGTATCATCGCAACGGTGTCTATGGTTGAGTCAACTGGTGTTTATCTGGCCTTGTCTGATA
TCACAAAGGATCCAATCGACAGCACGCGCCTTCGCAACGGATACCGCGCAGAAGGTTTGGCCGTACTTCTCGGAG
GAATCTTTAACACCTTCCCTTACACCGGATTTTCACAAAACGTTGGTTTGGTTAAATTGTCAGGCATCAAAAAACG
CCTGCCAATCTACTACGCAGCTGGTTTCCTGGTTCTCCTTGGACTGCTTCCTAAGTTTGGCGCCCTTGCCCAAATCA
TTCCAAGCTCCGTCCTCGGTGGTGCCATGCTGGTAATGTTTGGTTTTGTATCAATTCAAGGGATGCAAATCCTCGC
CCGTGTTGACTTTGCTAACAATGAACACAACTTCCTTATCGCAGCTGTTTCAATCGCTGCAGGTGTCGGTCTCAAC
AACAGTAATCTCTTTGTCAGCATGCCGACAGCCTTCCAAATGTTCTTCTCAAACGGAATCGTCGTAGCCAGCCTAC
TCGCTATTGTCCTCAATGCCGTATTAAATCATAAAAAGAAATAA

4136.2

ATGAAAGATAGAATAAAAGAATATTTACAAGACAAGGGAAAGGTGACTGTTAATGATTTGGCTCAGGCTTTGGGA
AAAGACAGTTCCAAGGATTTTCGTGAGTTGATTAAAACCTTGTCCTTAATGGAAAGAAAGCACCAAATTCGTTTTG
AAGAAGATGGTAGTCTGACATTAGAAATTAAGAAAAAACATGAGATTACCCTCAAGGGGATTTTTCATGCCCATA
AAAATGGCTTTGGCTTTGTTAGTCTGGAAGGCGAGGAGGACGACCTTTTTGTAGGGAAAAATGATGTCAACTATGC
TATTGATGGTGATACCGTCGAGGTAGTGATTAAGAAAGTCGCTGACCGCAATAAGGGAACAGCAGCAGAAGCCAA
AATTATTGATATCCTAGAACACAGTTTGACAACAGTTGTCGGGCAAATCGTTCTGGATCAGGAAAAACCTAAGTAT
GCTGGCTATATTCGTTCAAAAAATCAGAAAATCAGTCAACCGATTTATGTTAAGAAACCAGCCCTAAAATTAGAA
GGAACAGAAGTTCTCAAAGTCTTTATCGATAAATACCCAAGCAAGAAACATGATTTCTTTGTCGCGAGTGTTCTCG
ATGTAGTGGGACACTCAACGGATGTCGGAATTGATGTTCTTGAGGTCTTGGAATCAATGGACATTGTATCCGAGTT
TCCAGAAGCTGTTGTTAAGGAAGCAGAAAGTGTGCCTGATGCTCCGTCTCAAAAGGATATGGAAGGTCGTCTGGA
TCTAAGAGATGAAATTACCTTTACCATTGACGGTGCGGATGCCAAGGACTTGGACGATGCAGTGCATATCAAGGC
TCTGAAAAATGGCAATCTGGAGTTTGGGGTTCACATCGCAGATGTTTCTTATTATGTGACCGAGGGGTCTGCCCTT
GACAAGGAAGCCCTTAACCGTGCGACTTCTGTTTACGTGACAGACCGAGTGGTGCCAATGCTTCCAGAACGACTA
TCAAATGGCATCTGCTCTCTCAATCCCCAAGTTGACCGCCTGACCCAGTCTGCTATTAT
GGAGATTGATAAACATGGTCGTGTGGTCAACTATACCATTACACAAACAGTTATCAAGACCAGTTTTCGTATGACC
TATAGCGATGTCAATGATATCCTAGCTGGCGATGAAGAAAAGAGAAAAGAATATCATAAAATTGTATCAAGTATC
GAACTCATGGCCAAGCTTCATGAAACTTTAGAAAACATGCGTGTGAAACGTGGAGCTCTCAATTTTGATACCAATG
AAGCGAAGATTTTAGTGGATAAACAAGGTAAGCCTGTTGATATCGTTCTTCGGCAGCGTGGTATTGCCGAGCGGA
TGATTGAGTCTTTTATGTTGATGGCTAATGAAACAGTTGCCGAACATTTCAGCAAGTTGGATTTGCCTTTTATCTAT
CGAATTCACGAGGAGCCTAAGGCTGAAAAGGTTCAGAAGTTTATTGATTATGCTTCGAGTTTTGGCTTGCGCATTT
ATGGAACTGCCAGTGAGATTAGTCAGGAGGCACTTCAAGACATCATGCGTGCTGTTGAGGGAGAACCTTATGCAG
ATGTATTGTCCATGATGCTTCTTCGCTCTATGCAGCAGGCTCGTTATTCGGAGCACAATCACGGCCACTATGGACT
AGCTGCTGACTATTATACTCACTTTACCAGTCCAATTCGTCGTTATCCAGACCTTCTTGTTCACCGTATGATTCGGG
ATTACGGCCGTTCTAAGGAAATAGCAGAGCATTTTGAACAAGTGATTCCAGAGATTGCGACCCAGTCTTCCAACC
GTGAACGTCGTGCCATAGAAGCTGAGCGTGAAGTCGAAGCCATGAAAAAGGCTGAGTATATGGAAGAATACGTGG
GTGAAGAGTATGATGCAGTTGTATCAAGTATTGTCAAATTCGGTCTCTTTGTCGAATTGCCAAACACAGTTGAAGG
CTTGATTCACATCACTAATCTGCCTGAATTTTATCATTTCAATGAGCGTGATTTGACTCTTCGTGGAGAAAAATCA
GGTATCACTTTCCGAGTGGGTCAGCAGATCCGTATCCGTGTTGAAAGAGCGGATAAAATGACTGGAGAGATTGAT
TTTTCATTCGTACCTAGTGAGTTTGATGTGATTGAAAAAGGCTTGAAACAGTCTAGTCGT
AGTGGCAGAGGGCGTGATTCAAATCGTCGTTCGGATAAGAAGGAAGACAAGAGAAAATCAGGACGCTCAAATGA
TAAGCGTAAGCATTCACAAAAAGACAAGAAGAAAAAAGGAAAGAAACCTTTTTACAAGGAAGTAGCTAAGAAAG
GAGCCAAGCATGGCAAAGGGCGAGGGAAAGGTCGTCGCACAAAATAA

4137.2
ATGGGCACAACAGGATTTACAATAATTGACTTAATTATCTTGATTGTTTATTTACTTGCGGTGTTGGTTGCAGGTAT
CTATTTCTCTAAAAAAGAGATGAAGGAAAAGAGTTCTTTAAAGGAGATGGTTCGGTTCCTTGGTATGTTACTTCG
GTATCCATTTTTGCCACAATGCTCAGTCCGATTTCCTTCTTGGGACTCGCTGGTAGCTCTTATGCAGGTAGCTGGAT
TTTATGGTTTGCTCAATTAGGGATGGTAGTAGCTATTCCACTGACAATTCGTTTTATCTTACCTATCTTTGCACGGA
TAGACATCGATACGGCATATGATTACTTGGATAAACGTTTTAATTCTAAAGCACTTCGTATTATTTCAGCACTCTT
GTTTATTATTTATCAATTGGGACGTATGTCTATCATTATGTACCTCCCATCAGCTGGTTTATCAGTATTGACAGGAA
TTGACATCAATATTTTGATTATTTTGATGGGTGTAGTTGCAATTGTTTATTCTTATACTGGTGGTCTAAAATCCGTA
TTATGGACAGACTTTATTCAAGGTGTGATTCTGATTAGTGGTGTCGTTTTAGCTTTATTTGTACTGATTGCTAATAT
TAAAGGTGGCTTTGGTGCAGTAGCAGAAACATTAGCAAACGGGAAATTCCTTGCTGCAAATGAAAAACTTTTCGA
TCCTAACTTGCTTTCAAACTCCATCTTTTTAATTGTGATGGGTTCAGGCTTTACAATCTTGTCTTCCTATGCTTCATC
TCAAGATTTGGTTCAACGTTTTACTACAACACAAAATATTAAGAAACTTAATAAGATGTTGTTCACAAACGGTGTT
TTGTCACTTGCAACTGCAACAGTCTTTTACTTGATTGGTACAGGCTTGTACGTATTCTATCAAGTACAAAATGCAG
ATAGTGCAGCTAGCAATATCCCTCAAGACCAAATCTTTATGTACTTTATTGCATACCAGTTACCAGTAGGTATCAC
AGGTTTGATCTTGGCAGCGATTTATGCAGCATCTCAATCAACTATTTCAACAGGTTTGAACTCTGTTGCAACTTCA
TGGACATTGGATATTCAAGATGTCATTTCTAAAAATATGTCAGACAATCGTCGTACGAAAATTGCACAATTCGTAT
CTCTAGCAGTAGGTTTATTCTCAATTGGTGTTTCCATTGTCATGGCTCACTCAGATATTAAATCTGCATACGAATGG
TTCAATAGTTTCATGGGACTTGTACTTGGTCTACTTGGTGGTGTATTTATTCTTGGATTTGTTTCTAAAAAAGCAAA
TAAACAAGGTGCTTATGCAGCGCTGATTGTATCAACCATCGTCATGGTATTTATTAAATACTTCCTTCCTCCAACA
GCTGTTAGCTACTGGGCATATTCATTGATTTCAATCTCTGTATCAGTAGTTTCAGGTTATATTGTATCTGTTCTTAC
TGGAAATAAAGTATCTGCACCTAAATATACAACGATTCATGATATTACAGAAATTAAAGCGGATTCAAGTTGGGA
AGTTCGTCACTAA

4138.1
ATGAAATTTAGTAAAAAATATATAGCAGCTGGATCAGCTGTTATCGTATCCTTGAGTCTATGTGCCTATGCACTAA
ACCAGCATCGTTCGCAGGAAAATAAGGACAATAATCGTGTCTCTTATGTGGATGGCAGCCAGTCAAGTCAGAAAA
GTGAAAACTTGACACCAGACCAGGTTAGCCAGAAAGAAGGAATTCAGGCTGAGCAAATTGTAATCAAAATTACAG
ATCAGGGCTATGTAACGTCACACGGTGACCACTATCATTACTATAATGGGAAAGTTCCTTATGATGCCCTCTTTAG
TGAAGAACTCTTGATGAAGGATCCAAACTATCAACTTAAAGACGCTGATATTGTCAATGAAGTCAAGGGTGGTTA
TATCATCAAGGTCGATGGAAAATATTATGTCTACCTGAAAGATGCAGCTCATGCTGATAATGTTCGAACTAAAGAT
GAAATCAATCGTCAAAAACAAGAACATGTCAAAGATAATGAGAAGGTTAACTCTAATGTTGCTGTAGCAAGGTCT
CAGGGACGATATACGACAAATGATGGTTATGTCTTTAATCCAGCTGATATTATCGAAGATACGGGTAATGCTTATA

TCGTTCCTCATGGAGGTCACTATCACTACATTCCCAAAAGCGATTTATCTGCTAGTGAATTAGCAGCAGCTAAAGC
ACATCTGGCTGGAAAAAATATGCAACCGAGTCAGTTAAGCTATTCTTCAACAGCTAGTGACAATAACACGCAATC
TGTAGCAAAAGGATCAACTAGCAAGCCAGCAAATAAATCTGAAAATCTCCAGAGTCTTTTGAAGGAACTCTATGA
TTCACCTAGCGCCCAACGTTACAGTGAATCAGATGGCCTGGTCTTTGACCCTGCTAAGATTATCAGTCGTACACCA
AATGGAGTTGCGATTCCGCATGGCGACCATTACCACTTTATTCCTTACAGCAAGCTTTCTGCCTTAGAAGAAAAGA
TTGCCAGAATGGTGCCTATCAGTGGAACTGGTTCTACAGTTTCTACAAATGCAAAACCTAATGAAGTAGTGTCTAG
TCTAGGCAGTCTTTCAAGCAATCCTTCTTCTTTAACGACAAGTAAGGAGCTCTCTTCAGCATCTGATGGTTATATTT
TTAATCCAAAAGATATCGTTGAAGAAACGGCTACAGCTTATATTGTAAGACATGGTGATCATTTCCATTACATTCC
AAAATCAAATCAAATTGGGCAACCGACTCTTCCAAACAATAGTCTAGCAACACCTTCTCCATCTCTTCCAATCAAT
CCAGGAACTTCACATGAGAAACATGAAGAAGATGGATACGGATTTGATGCTAATCGTATTATCGCTGAAGATGAA
TCAGGTTTTGTCATGAGTCACGGAGACCACAATCATTATTTCTTCAAGAAGGACTTGACAGAAGAGCAAATTAAG
GTGCGCAAAAACATTTAG

4139.1
ATGAAAAAAAGAGCAATAGTGGCAGTCATTGTACTGCTTTTGATTGGGCTGGATCAGTTGGTCAAATCCTATATCG
TCCAGCAGATTCCACTGGGTGAAGTGCGCTCCTGGATCCCCAATTTCGTTAGCTTGACCTACCTGCAAAATCGAGG
TGCAGCCTTTTCTATCTTACAAGATCAGCAGCTGTTATTCGCTGTCATTACTCTGGTTGTCGTGATAGGTGCCATTT
GGTATTTACATAAACACATGGAGGACTCATTCTGGATGGTCTTGGGTTTGACTCTAATAATCGCGGGTGGTCTTGG
AAACTTTATTGACAGGGTCAGTCAGGGCTTTGTTGTGGATATGTTCCACCTTGACTTTATCAACTTTGCAATTTTCA
ATGTGGCAGATAGCTATCTGACGGTTGGAGTGATTATTTTATTGATTGCAATGCTAAAAGAGGAAATAAATGGAA
ATTAA

4139.5
ATGAATACAAATCTTGCAAGTTTTATCGTTGGACTGATCATCGATGAAAACGACCGTTTTTACTTTGTGCAAAAGG
ATGGTCAAACCTATGCTCTTGCTAAGGAAGAAGGCCAACATACAGTAGGGGATACGGTCAAAGGTTTTGCATACA
CGGATATGAAGCAAAAACTCCGCCTGACAACCTTAGAAGTGACTGCCACTCAGGACCAATTTGGTTGGGGACGTG
TCACAGAGGTTCGTAAGGACTTGGGTGTCTTTGTGGATACAGGCCTTCCTGACAAGGAAATCGTTGTGTCACTCGA
TATTCTCCCTGAGCTCAAGGAACTCTGGCCTAAGAAGGGCGACCAACTCTACATCCGTCTTGAAGTGGATAAGAA
AGACCGTATCTGGGGCCTCTTGGCTTATCAAGAAGACTTCCAACGTCTTGCTCGTCCTGCCTACAACAACATGCAG
AACCAAAACTGGCCAGCCATTGTTTACCGTCTCAAGCTGTCAGGAACTTTTGTTTACCTACCAGAAAATAATATGC
TTGGTTTTATTCATCCTAGCGAGCGTTACGCAGAGCCACGTTTGGGGCAAGTATTAGATGCGCGCGTTATTGGTTT
CCGTGAAGTGGACCGCACTCTGAACCTCTCCCTCAAACCACGCTCCTTTGAAATGTTGGAAAACGATGCTCAGATG
ATTTTGACTTATTTGGAAAGCAATGGCGGTTTCATGACCTTAAATGACAAGTCATCTCCAGACGACATCAAGGCAA
CCTTTGGCATTTCTAAAGGTCAGTTCAAGAAAGCTTTAGGTGGTCTTATGAAGGCTGGTAAAATCAAGCAGGACCA
GTTTGGGACAGAGTTGATTTAG

4139.8
ATGAAAGATGTTAGTCTATTTTTATTGAAAAAAGTTTTCAAAAGCCGCTTAAACTGGATTGTCTTAGCTTTATTTGT
ATCTGTACTCGGTGTTACCTTTTATTTAAATAGTCAGACTGCAAACTCACACAGCTTGGAGAGCAGGTTGGAAAGT
CGCATTGCAGCCAACGAGAGGGCTATCAATGAAAATGAAGAGAAACTCTCCCAAATGTCTGATACCAGCTCGGAG
GAATACCAGTTTGCTAAAAATAATTTAGACGTGCAAAAAAATCTTTTGACGCGAAAGACAGAAATTCTGACTTTAT
TAAAAGAAGGGCGCTGGAAAGAAGCCTACTATTTGCAGTGGCAAGATGAAGAGAAGAATTATGAATTTGTATCAA
ATGACCCGACTGCTAGCCCTGGCTTAAAAATGGGGGTTGACCGCGAACGGAAGATTTACCAAGCCCTGTATCCCT
TGAACATAAAAGCACATACTTTGGAGTTTCCGACCCACGGGATTGATCAGATTGTCTGGATTTTAGAGGTTATCAT
CCCAAGTTTGTTTGTGGTTGCTATTATTTTTATGCTAACACAACTATTTGCAGAAAGATATCAAAATCATCTGGAC
ACAGCTCACTTATATCCTGTTTCAAAAGTGACATTTGCAATATCCTCTCTTGGAGTTGGAGTGGGATATGTAACTG
TGCTGTTTATCGGAATCTGTGGCTTTTCTTTTCTAGTGGGAAGTCTGATAAGTGGTTTTGGACAGTTAGATTATCCC
TACCCAATTTATAGCTTAGTGAATCAAGAAGTAACTATTGGGAAAATACAAGATGTATTATTTCCTGGCTTGCTCT
TAGCTTTCTTAGCCTTTATCGTCATTGTGGAAGTTGTGTACTTGATTGCTTACTTTTTCAAGCAAAAAATGCCTGTC
CTCTTTCTTTCACTCATTGGGATTGTTGGCTTATTGTTTGGTATCCAAACCATTCAGCCTCTTCAAAGGATTGCACA
TCTGATTCCCTTTACTTACTTGCGTTCAGTGGAGATTTTATCTGGAAGATTACCTAAGCAGATTGATAATGTCGATC
TAAATTGGAGCATGGGAATGGTCTTACTTCCTTGCCTGATTATCTTTTTGCTATTGGGAATTCTATTTATTGAAAGA
TGGGGAAGTTCACAGAAAAAAGAATTTTTTAATAGATTCTAG

4141.1
ATGATGAAGTTCATATTGGATATTGTTAGTACACCAGCTATTTTAGTAGCTTTAATTGCAATCTTAGGATTAGTTCT
TCAGAAGAAGAAATTACCTGATATTATTAAAGGTGGAATTAAGACCTTTGTTGGTTTCTTAGTTGTATCTGGTGGT
GCAGGAATTGTACAAAATTCTTTAAATCCATTTGGTACCATGTTTGAGCATGCTTTTCATTTATCTGGCGTTGTGCC
GAATAATGAAGCAATTGTAGCTGTAGCTTTAACAACATATGGCTCAGCTACTGCAATGATTATGTTTGCAGGCATG
GTGTTCAATATCTTAATCGCTCGTTTTACTCGATTTAAATATATTTTTTTAACAGGGCACCACACTCTATATATGGC
ATGTATGATTGCGGTCATTTTATCAGTTGCTGGCTTTACTAGCTTGCCTCTCATCTTACTAGGAGGATTAGCACTCG
GTATTATTATGAGTATTTCCCCAGCATTTGTGCAAAAATATATGGTTCAATTAACTGGAAATGACAAGGTAGCTTT
AGGTCATTTCAGTTCTTTGGGATATTGGTTGAGTGGTTTTACTGGTAGCCTTATCGGTGACAAATCAAAATCAACA
GAGGACATTAAATTTCCAAAGAGTTTAGCTTTTTTACGTGATAGTACTGTTAGTATTACTTTATCCATGGCAGTTAT
TTACATTATTGTAGCTATCTTTGCAGGGTCAGAATATATAGAAAAAGAAATCAGTAGTGGTACAAGTGGTCTAGTT

TATGCTTTACAATTAGCAGGTCAATTTGCAGCAGGGGTATTTGTTATTTTAGCAGGTGTTCGCCTTATTTTGGGCGA
AATTGTTCCAGCCTTTAAAGGTATTTCAGAGCGTCTTGTACCTAATTCAAAACCTGCTTTGGATTGTCCGATTGTTT
ATACTTATGCACCCAATGCAGTTCTAATTGGATTTATCTCTAGTTTTGTTGGTGGTTTAGTAAGTATGGTAATTATG
ATTGCTTCAGGAACGGTTGTTATCTTACCAGGTGTTGTGCCTCATTTCTTCTGTGGAGCGACTGCAGGTGTCATTGG
GAATGCATCTGGTGGTGTTCGTGGAGCCACTATTGGAGCATTTTTACAAGGTATTTTAATCAGTTTTCTTCCAGTCT
TTTTAATGCCAGTTTTGGGAGGACTTGGTTTCCAAGGATCAACTTTCTCAGATGCAGATTTTGGTCTATCAGGAATT
ATTTTAGGAATGTTAAATCAATTTGGCTCACAAGCAGGCATTGTGATTGGTCTTGTTCTTATTCTAGCAGTTATGTT
TGGAGTATCCTTTATTAAAAAGCCATCTGCAACGGAGGAATAA

4142.3
ATGATTAAAACATTTCTCTCTGCCCTTTCGGTCATTCTCTTTTCTATCCCTATCATAACTTATTCTTTTTTCCCATCT
TCTAATCTTAACATTTGGCTATCTACCCAACCTATCTTGGCACAGATTTATGCCTTCCCCTTAGCTACTGCAACTAT
GGCTGCTATTTTAAGTTTCTTATTTTTTTTCCTATCTTTTTACAAGAAAAATAAACAAATACGGTTTTACTCTGGCA
TTTTGCTCTTACTATCGCTCATATTACTATTATTCGGAACAGATAAAACCCTTTCTTCTGCATCAAATAAGACTAAA
ACCTTAAAATTAGTAACTTGGAACGTCGCTAATCAAATAGAAGCACAACATATTGAGCGAATTTTTAGCCATTTTG
ACGCCGATATGGCTATATTCCCTGAACTAGCTACCAATATCAGAGGTGAGCAAGAAAACCAGAGAATCAAACTAT
TGTTTCATCAAGTTGGACTTTCTATGGCCAACTATGATATTTTCACTTCTCCACCTACCAATAGTGGAATAGCTCCT
GTGACTGTGATTGTCAAGAAAAGTTATGGTTTCTATACAGAAGCTAAAACTTTTCATACAACACGGTTCGGACAA
TTGTATTACATTCGAGAAAACAAAATATACCAGATATCATTGCCTTGCATACTGCGCCTCCTCTGCCAGGTTTAAT
GGAAATCTGGAAGCAAGACTTAAACATCATTCATAATCAATTGGCTTCAAAATATCCAAAGGCTATTATTGCAGGT
GATTTTAATGCAACTATGCGTCATGGAGCACTTGCAAAAATAAGCTCTCATAGGGACGCATTAAATGCACTGCCA
CCTTTTGAAAGAGGAACTTGGAATAGCCAAAGTCCAAAACTTTTTAATGCAACAATAGATCATATTTTATTGCCTA
AAAACCACTACTATGTTAAAGATTTAGACATTGTAAGTTTTCAAAACTCTGATCATAGATGTATTTTTACAGAAAT
CACATTTTAA

4142.4
ATGAATCCAATCCAAAGATCTTGGGCTTATGTCAGCAGAAAGCGACTGAGAAGTTTTATTTTATTTCTGATTTTAT
TGGTCTTATTGGCCGGAATTTCAGCCTGTTTGACTCTGATGAAGTCCAACAAAACAGTAGAAAGCAATCTTTATAA
ATCACTCAATACATCTTTTTCTATTAAGAAGATAGAGAATGGTCAGACATTCAAGTTGTCAGACCTAGCATCTGTA
AGCAAGATTAAGGGGCTGGAAAATGTCTCTCCTGAACTTGAGACGGTCGCAAAACTAAAAGACAAGGAAGCAGTG
ACTGGCGAGCAGAGCGTGGAGCGTGATGATTTATCAGCTGCAGACAATAACTTGGTTAGCTTAACGGCTCTTGAG
GATTCATCCAAGGATGTAACCTTTACCAGTTCGGCTTTCAATCTAAAAGAAGGGCGACACCTTCAAAAAGGGGAT
TCCAAGAAAATCCTTATCCACGAAGAATTGGCTAAGAAGAACGGTCTTTCGCTTCATGACAAGATTGGCTTGGATG
CTGGTCAGTCTGAATCTGGAAAAGGACAAACAGTAGAGTTTGAGATTATCGGCATCTTTTCTGGTAAAAAACAAG
AGAAATTCACAGGCTTGTCTTCTGACTTCAGTGAAAATCAAGTCTTTACAGACTATGAAAGTAGCCAAACCCTTTT
GGGCAATAGTGAAGCTCAAGTCAGTGCAGCACGCTTCTATGTAGAAAATCCTAAGGAAATGGACGGACTCATGAA
GCAGGTAGAAAACTTGGCCTTGGAAAATCAAGGCTACCAAGTCGAAAAGGAAAACAAGGCTTTTGAACAAATCAA
AGACTCAGTTGCAACTTTCCAAACCTTCCTGACCATCTTCCTTTATGGGATGTTGATAGCAGGAGCTGGAGCCTTA
ATTCTGGTTTTGTCTCTCTGGTTGAGAGAACGGGTCTATGAAGTGGGGATTTTACTTGCACTTGGAAAAGGCAAGA
GCTCGATCTTCCTACAATTCTGTTTAGAGGTAGTTTTGGTATCTCTTGGAGCTTTGCTTCCAGCATTTGTTGCAGGA
AACGCAATCACAACTTACCTACTCCAAACTCTACTAGCAAGTGGAGATCAGGCAAGCTTACAAGATACACTAGCC
AAAGCAAGCAGTTTATCAACTAGCATCTTATCTTTTGCAGAATCCTATGTTTTTCTAGTTCTGCTTAGTTGCTTATC
TGTAGCCCTTTGTTTCCTATTCTTATTTAGAAAATCACCGAAAGAAATTTTATCATCTATTAGTTAA

4142.5
ATGTTACACAACGCATTTGCCTATGTTACAAGGAAGTTTTTCAAATCGATTGTCATCTTCCTGATTATTCTCCTCAT
GGCGAGCTTGAGTTTGGTCGGCTTGTCAATCAAGGGAGCTACTGCCAAGGCTTCTCAGGAGACCTTTAAAAATATC
ACCAATAGCTTCTCCATGCAAATCAATCGTCGCGTCAACCAAGGAACGCCTCGTGGTGCTGGGAATATCAAGGGT
GAAGACATCAAAAAAATCACCGAAAACAAGGCCATTGAGTCTTATGTCAAACGTATCAACGCTATCGGAGATTTG
ACTGGATATGACCTGATTGAAACGCCAGAAACCAAGAAGAATCTCACTGCTGATCGTGCCAAGCGTTTTGGAAGT
AGCTTGATGATTACAGGTGTCAATGACTCCTCTAAAGAAGACAAGTTTGTCTCTGGTTCTTATAAACTAGTCGAAG
GAGAGCACTTAACCAACGACGACAAGGATAAAATCCTCTTGCACAAGGACTTGGCAGCCAAACACGGCTGGAAA
GTAGGGGACAAGGTTAAACTGGACTCTAATATCTACGATGCAGATAATGAAAAAGGAGCCAAGGAAACAGTTGA
AGTGACAATCAAGGGACTCTTTGATGGTCATAATAAGTCAGCAGTAACCTACTCACAAGAACTTTACGAAAACAC
AGCTATTACAGACATTCACACTGCTGCAAAACTTTATGGATACACAGAAGACACAGCCATTTATGGGGACGCAAC
CTTCTTTGTAACAGCAGACAAGAACTTGGATGATGTTATGAAAGAGTTGAATGGCATCAGTGGTATCAACTGGAA
GAGCTACACACTCGTCAAGAGCTCCTCTAACTACCCAGCTCTTGAGCAATCTATCTCTGGTATGTACAAGATGGCC
AACCTCCTCTTCTGGGGTAGCTTGAGCTTCTCAGTTCTCCTCCTTGCCCTCTTGCTCAGCCTTTGGATCAACGCCCG
TCGCAAGGAAGTGGGAATTCTCCTCTCTATCGGCCTCAAGCAGGCAAGTATCTTGGGTCA
ATTCATCACCGAATCTATCTTGATTGCTATCCCTGCTCTAGTTTCTGCTTACTTCCTAGCTAATTACACTGCCCGTG
CAATTGGAAACACTGTCCTTGCCAATGTGACTTCAGGTGTTGCCAAACAGGCTAGTAAGGCGGCTCAAGCCTCTA
ACCTTGGTGGTGGTGCAGAAGTAGATGGCTTTAGCAAGACCTTGTCGAGCCTAGACATTTCCATTCAGACATCAGA
CTTTATCATCATTTTTGTCCTTGCCTTGGTTCTAGTGGTTCTCGTTATGGCGCTTGCTTCAAGCAATCTCCTTAGAA
AACAACCAAAAGAGCTCTTGCTGGATGGTGAATAA

4144.1
ATGTCACAGGATAAACAAATGAAAGCTGTTTCTCCCCTTCTGCAGCGAGTTATCAATATCTCATCGATTGTCGGTG
GGGTTGGGAGTTTGATTTTCTGTATTTGGGCTTATCAGGCTGGGATTTTACAATCCAAGGAAACCCTCTCTGCCTTT
ATCCAGCAGGCAGGCATCTGGGGTCCACCTCTCTTTATCTTTTTACAGATTTTACAGACTGTCGTCCCTATCATTCC
AGGGGCCTTGACCTCGGTGGCTGGGGTCTTTATCTACGGGCACATCATCGGGACTATCTACAACTATATCGGCATC
GTGATTGGCTGTGCCATTATCTTTTATCTAGTGCGCCTATACGGAGCTGCCTTTGTCCAGTCTGTCGTCAGCAAGC
GCACCTACGACAAGTACATCGACTGGCTAGATAAGGGCAATCGTTTTGACCGCTTCTTTATTTTTATGATGATTTG
GCCCATTAGCCCAGCTGACTTTCTCTGTATGCTGGCTGCCCTGACCAAGATGAGCTTCAAGCGCTACATGACCATC
ATCATTCTGACCAAACCCTTTACCCTCGTGGTTTATACCTACGGTCTGACCTATATTATTGACTTTTTCTGGCAAAT
GCTTTGA

4144.2
ATGAGAAATATGTGGGTTGTAATCAAGGAAACCTATCTTCGACATGTCGAGTCATGGAGTTTCTTCTTTATGGTGA
TTTCGCCGTTCCTCTTTTTAGGAATCTCTGTAGGAATTGGGCATCTCCAAGGTTCTTCTATGGCTAAAAATAATAAA
GTGGCAGTAGTGACAACAGTGCCATCTGTAGCAGAAGGACTGAAGAATGTAAATGGTGTTAACTTCGACTATAAA
GACGAAGCAAGTGCCAAAGAAGCAATTAAAGAAGAAAAATTAAAAGGTTATTTGACCATTGATCAAGAAGATAGT
GTTCTAAAGGCAGTTTATCATGGCGAAACATCGCTTGAAAATGGAATTAAATTTGAGGTTACAGGTACACTCAATG
AACTGCAAAATCAGCTTAATCGTTCAACTGCTTCCTTGTCTCAAGAGCAGGAAAAACGCTTAGCGCAGACAATTC
AATTCACAGAAAAAGATTGATGAAGCCAAGGAAAATAAAAAGTTTATTCAAACAATTGCAGCAGGTGCCTTAGGAT
TCTTTCTTTATATGATTCTGATTACCTATGCGGGTGTAACAGCTCAGGAAGTTGCCAGTGAAAAAGGCACCAAAAT
TATGGAAGTCGTTTTTTCTAGCATAAGGGCAAGTCACTATTTCTATGCGCGGATGATGGCTCTGTTTCTAGTGATTT
TAACGCATATTGGGATCTATGTTGTAGGTGGTCTGGCTGCCGTTTTGCTCTTTAAAGATTTGCCATTCTTGGCTCAG
TCTGGTATTTTGGATCACTTGGGAGATGCTATCTCACTGAATACCTTGCTCTTTATTTTGATCAGTCTTTTCATGTA
CGTAGTCTTGGCAGCCTTCCTAGGATCTATGGTTTCTCGTCCTGAGGACTCAGGGAAAGCCTTGTCGCCTTTGATG
ATTTTGATTATGGGTGGTTTTTTTGGAGTGACAGCTCTAGGTGCAGCTGGTGACAATCTCCTCTTGAAGATTGGTTC
TTATATTCCCTTTATTTCGACCTTCTTTATGCCGTTTCGAACGATTAATGACTATGCGGGGGGAGCAGAAGCATGG
ATTTCACTTGCTATTACAGTGATTTTTGCGGTGGTAGCAACAGGATTTATCGGACGCATGTATGCTAGTCTCGTTCT
TCAAACGGATGATTTAGGGATTTGGAAAACCTTTAAACGTGCCTTATCTTATAAATAG

4144.3
ATGACAGAAACCATTAAATTGATGAAGGCTCATACTTCAGTGCGCAGGTTTAAAGAGCAAGAAATTCCCCAAGTA
GACTTAAATGAGATTTTGACAGCAGCCCAGATGGCATCATCTTGGAAGAATTTCCAATCCTACTCTGTGATTGTGG
TACGAAGTCAAGAGAAGAAAGATGCCTTGTATGAATTGGTACCTCAAGAAGCCATTCGCCAGTCTGCTGTTTTCCT
TCTCTTTGTCGGAGATTTGAACCGAGCAGAAAAGGGAGCCCGACTTCATACCGACACCTTCCAACCCCAAGGTGT
GGAAGGTCTCTTGATTAGTTCGGTCGATGCAGCTCTTGCTGGACAAAACGCCTTGTTGGCAGCTGAAAGCTTGGGC
TATGGTGGTGTGATTATCGGTTTGGTTCGATACAAGTCTGAAGAAGTGGCAGAGCTCTTTAACCTACCTGACTACA
CCTATTCTGTCTTTGGGATGGCACTGGGTGTGCCAAATCAACATCATGATATGAAACCGAGACTGCCACTAGAGA
ATGTTGTCTTTGAGGAAGAATACCAAGAACAGTCAACTGAGGCAATCCAAGCTTATGACCGTGTTCAGGCTGACT
ATGCTGGGGCGCGTGCGACCACAAGCTGGAGTCAGCGCCTAGCAGAACAGTTTGGTCAAGCTGAACCAAGCTCAA
CTAGAAAAAATCTTGAACAGAAGAAATTATTGTAG

4146.1
ATGTTAAAACTTATTGCTATTGTTGGAACAAATTCAAAACGTTCTACAAACCGTCAATTGCTTCAATACATGCAAA
AACACTTTACTGACAAAGCTGAAATTGAACTTGTTGAAATCAAGGCCATTCCTGTCTTCAACAAACCAGCTGACAA
GCAAGTACCTGCTGAAATATTGGAAATTGCTGCTAAAATCGAAGAGGCAGATGGCGTTATTATCGGTACTCCTGA
GTATGATCACTCTATTCCAGCTGTTTTGATGAGCGCTCTTGCTTGGTTGTCTTATGGTATTTACCCACTTTTGAACA
AACCAATCATGATTACAGGTGCTTCTTACGGTACGCTTGGTTCATCTCGTGCCCAATTGCAACTTCGTCAAATCTT
GAATGCTCCTGAAATCAAGGCAAATGTTCTTCCAGATGAATTCTTGCTCTCACACTCTCTTCAAGCATTTAACCCA
AGTGGCGACTTGGTTGACCTTGATGTTATCAAGAAATTGGATGCCATCTTTGATGACTTCCGTATCTTTGTAAAAA
TCACAGAAAAATTACGTAATGCACAAGAATTACTTCGCAAAGATGCTGAAGACTTTGACTGGGAAAATTTGTAA

4146.2
ATGAATACCTATCAATTAAATAATGGAGTAGAAATTCCAGTATTGGGATTTGGAACTTTTAAGGCTAAGGATGGA
GAAGAAGCCTATCGTGCAGTGTTAGAAGCCTTGAAGGCTGGTTATCGTCATATTGATACGGCGGCGATTTATCAGA
ATGAAGAAAGTGTTGGTCAAGCAATCAAAGATAGCGGAGTTCCACGTGAAGAAATGTTCGTAACTACCAAGCTTT
GGAATAGTCAGCAAACCTATGAGCAAACTCGTCAAGCTTTGGAAAAATCTATAGAAAAACTGGGCTTGGATTATT
TGGATTTGTATTTGATTCATTGGCCGAACCCAAAACCGCTCAGAGAAAATGACGCATGGAAAACTCGCAATGCGG
AAGTTTGGAGAGCGATGGAAGACCTCTATCAAGAAGGGAAAATCCGTGCTATCGGCGTTAGCAATTTTCTTCCCC
ATCATTTGGATGCCTTGCTTGAAACTGCAACTATCGTTCCTGCGGTCAATCAAGTTCGCTTGGCGCCAGGTGTGTA
TCAAGATCAAGTCGTAGCTTACTGTCGTGAAAAGGGAATTTTATTGGAAGCTTGGGGGCCTTTTGGACAAGGAGA
ACTGTTTGATAGCAAGCAAGTCCAAGAAATAGCAGCAAATCACGGAAAATCGGTTGCTCAGATAGCCTTGGCCTG
GAGCTTGGCAGAAGGATTTTTACCACTTCCAAAATCTGTCACAACCTCTCGTATTCAAGCTAATCTTGATTGCTTT
GGAATTGAACTGAGTCATGAGGAGAGAGAAACCTTAAAAACGATTGCTGTTCAATCGGGTGCTCCACGAGTTGAT
GATGTGGATTTCTAG

4147.1
ATGAGGTGCAAAATGCTTGATCCAATTGCTATTCAACTAGGACCCCTAGCCATTCGTTGGTATGCCTTATGTATTG
TGACAGGCTTGATTCTTGCGGTTTATTTGACCATGAAAGAAGCACCTAGAAAGAAGATCATACCAGACGATATTTT
AGATTTTATCTTAGTAGCCTTTCCCTTGGCTATTTTAGGAGCTCGTCTCTACTATGTTATTTTCCGATTTGATTACTA
TAGTCAGAATTTAGGAGAGATTTTTGCCATTTGGAATGGTGGTTTGGCCATTTACGGTGGTTTGATAACTGGGGCT
CTTGTGCTCTATATCTTTGCTGACCGTAAACTCATCAATACTTGGGATTTTCTAGATATTGCGGCGCCTAGCGTTAT
GATTGCTCAAAGTTTGGGGCGTTGGGGTAATTTCTTTAACCAAGAAGCTTATGGTGCAACAGTGGATAATCTGGAT
TATCTACCTGGCTTTATCCGTGACCAGATGTATATTGAGGGGAGCTACCGTCAACCGACTTTCCTTTATGAGTCTC
TATGGAATCTGCTTGGCTTTGCCTTGATTCTGATTTTAGACGGAAATGGAAGAGTCTCAGACGAGGTCATATCAC
GGCCTTTTACTTGATTTGGTATGGTTTCGGTCGTATGGTTATCGAAGGTATGCGAACAGATAGTCTCATGTTCTTCG
GCTTTCGAGTGTCCCAATGGCTGTCAGTTGTCCTTATCGGTCTCGGTATAATGATCGTTATTTATCAAAATCGAAA
GAAGGCCCCTTACTATATTACAGAGGAGGAAAACTAA

4147.2
ATGGGTAAATTATCCTCAATCCTTTTAGGAACCGTTTCAGGTGCAGCTCTTGCCTTGTTTTTAACAAGTGATAAGG
GCAAACAAGTTTGCAGTCAGGCTCAAGATTTTCTAGATGATTTGAGAGAAGATCCGGAGTATGCCAAGGAGCAAG
TCTGTGAAAAACTGACAGAAGTTAAGGAGCAGGCTACAGATTTTGTTCTGAAAACAAAAGAACAGGTTGAGTCAG
GTGAAATCACTGTGGACAGTATACTTGCTCAAACTAAATCCTATGCTTTTCAAGCGACAGAAGCATCAAAAAATC
AATTAAATAATCTCAAGGAGCAATGGCAAGAAAAAGCCGAAGCTCTTGATGACTCAGAAGAGATTGTGATTGATA
TAACAGAAGAATAA

4147.3
ATGAAAACTAAATTGATCTTTTGGGGCTCTATGCTCTTTCTCCTCTCCCTCTCCATCCTTCTGACCATTTATCTGGC
TTGGATTTTCTATCCTATGGAGATTCAGTGGCTAAACTTAACGAATCGAGTCTATCTAAAACCAGAAACCATTCAA
TACAATTTTCATATCTTGATGAATTATCTGACCAATCCTTTTAGTCAGGTCTTACAGATGCCTGATTTTCGTTCGTC
AGCAGCTGGTCTGCACCATTTCGCAGTGGTCAAGAATCTCTTTCATTTGGTTCAGCTAGTAGCTCTAGTGACACTG
CCAAGTTTCTATGTCTTTGTCAATAGGATTGTGAAAAAGGACTTTTTGTCTCTTTATCGAAAAAGTCTCCTGGCTCT
AGTAGTCTTACCTGTGATGATTGGACTTGGGGGAGTTTTGATTGGTTTTGACCAATTCTTTACTCTTTTCCATCAAA
TTCTCTTTGTGGGAGATGATACCTGGCTTTTTGATCCAGCCAAGGATCCTGTTATTATGATTTTGCCAGAGACCTTC
TTTCTTCATGCCTTCCTCCTCTTTTTTGCCCTCTATGAAAACTTCTTTGGCTATCTGTATCTGAAAAGTCGTAGGAA
GTGA

4149.1
ATGACTTATCATTTTACTGAAGAATACGATATTATTGTAATTGGTGCGGGACACGCTGGGGTTGAGGCTTCCTTGG
CCGCTAGCCGTATGGGCTGTAAGGTCCTGCTTGCGACCATCAATATTGAAATGCTGGCTTTCATGCCTTGTAATCC
CTCTATCGGTGGTTCTGCCAAGGGGATTGTCGTGCGTGAAGTCGATGCCCTCGGTGGCGAGATGGCCAAAACCATT
GACAAGACTTACATCCAGATGAAGATGCTAAACACAGGGAAGGGGCCAGCTGTCCGTGCCCTTCGTGCGCAGGCT
GACAAGGAACTTTACTCTAAGGAGATGCGCAAGACGGTTGAAAACCAAGAAAATCTGACCCTTCGTCAAACCATG
ATTGATGAGATTTTGGTGGAAGATGGCAAGGTTGTCGGTGTGCGTACAGCCACCCATCAAGAATATGCTGCTAAG
GCTGTTATTGTGACGACAGGGACTGCTCTCCGTGGGGAAATTATCATCGGAGACCTCAAGTACTCATCAGGTCCTA
ACCACAGCTTGGCTTCTATTAACCTAGCTGACAATCTCAAGGAACTGGGTCTCGAAATCGGTCGTTTCAAGACAGG
AACCCCTCCACGTGTCAAGGCTTCTTCTATCAATTACGATGTGACAGAAATTCAGCCAGGAGACGAAGTGCCTAAT
CATTTCTCATACACTTCACGTGATGAGGATTATGTCAAGGACCAAGTACCATGCTGGTTGACCTATACCAATGGTA
CCAGTCATGAGATTATCCAAAACAACCTCCACCGTGCGCCTATGTTTACAGGTGTGGTCAAGGGAGTGGGGCCTC
GTTACTGTCCGTCGATTGAAGACAAGATTGTGCGCTTTGCGGACAAGGAACGTCACCAACTCTTCCTTGAGCCAGA
AGGGCGCAATACTGAGGAAGTCTATGTGCAAGGACTTTCAACCAGTCTGCCTGAGGATGTCCAGCGTGACTTGGT
GCATTCCATCAAAGGTTTGGAAAATGCAGAGATGATGCGGACAGGTTATGCTATTGA
GTATGATATGGTCTTGCCTCATCAGTTGCGTGCGACTTTGGAAACCAAGAAAATCTCAGGTCTCTTCACTGCTGGT
CAGACAAATGGAACATCAGGTTACGAAGAGGCAGCAGGCCAAGGGATTATCGCGGGTATCAATGCGGCTCTGAA
AATCCAAGGCAAGCCTGAATTGATTTTGAAGCGCAGTGATGGTTATATCGGGGTGATGATCGACGACTTGGTGAC
CAAGGGAACCATTGAACCCTACCGTCTCTTGACCAGTCGTGCTGAATACCGTCTCATTCTTCGTCATGACAATGCT
GATATGCGCTTGACTGAGATGGGACGCGAGATTGGCCTTGTGGACGATGAACGCTGGGCTCGTTTTGAAATCAAG
AAAAATCAATTTGATAATGAGATGAAGCGCCTAGACAGTATCAAACTCAAGCCAGTCAAGGAAACCAATGCCAAG
GTTGAGGAGATGGGCTTCAAACCCTTGACCGATGCAGTGACAGCCAAGGAATTCCTTCGCCGTCCAGAAGTTTCTT
ACCAAGATGTGGTGGCCTTCATCGGACCAGCTGCAGAAGACTTGGATGACAAGATTATCGAATTGATTGAAACAG
AAATCAAGTATGAAGGCTATATTTCCAAAGCCATGGACCAGGTTGCCAAGATGAAACGCATGGAAGAAAAACGCA
TTCCGGCCAATATCGACTGGGATGACATTGATTCTATCGCAACCGAAGCCCGTCAGAAGTTCAAACTCATCAATCC
AGAAACCATCGGCCAAGCCAGCCGTATTTCGGGAGTAAACCCAGCAGATATTTCTATTTTGATGGTGTATCTGGAA
GGTAAAAAATCGTAGTATTTCTAAAACTCTTCAAAAATCAAAATGA

4149.2
ATGAAAGTATTAGCTTTTGATACGTCCAGCAAGGCTCTTTCTCTGGCTATTTTAGAGGATAAGCAGGTTCTTGCCG
AGACGACGATTAATATTAAGAAAAATCACAGTATTACTCTTATGCCTGCCATCGATTTTTTGATGGCAAGTTTGGA

TTGGACACCCAAGGATTTGGACCGAATCGTGGTAGCTGAAGGGCCGGGTAGCTATACAGGCTTGCGAATTGCGGT
AGCAACTGCTAAGACCTTAGCTCACACCCTGAACATCGAGTTGGTTGGTATGTCGAGTCTCTTGGCTCTGGTGCCC
CATCAACAAGAAGGTTTGTTTGTCCCCTTGATGGATGCGCGTCGCAATAATGTTTATGCAGGATTTTATGAAAATG
CCAAACCTGTCATGGCAGAAGCGCACCTATCTTTTGAAGAGGTGCTAGAAAAAGTCAAGGGTACTAGTCAGGTAA
CCTTTGTCGGAGAAGTTGGCCCCTTTGTTGAGCAGATTCAAAAACACTTGCCAAGGACTGATTACAAAGAAACATT
GCCCAATGCAGCTAATCTAGCTCTTTTGGCCTGGGACAAGGAAGCAGACTCCTTGCATGATTTTGTGCCGAATTAC
CTCAAACGAGTCGAGGCTGAGGAAAACTGGCTCAAGAACCATACCGAGTCTGGCGAGTCTTACATTAAACGCCTA
TGA

4149.3
ATGATAGAAATCAAGCGAATTCAACAACAGCCTGACCTAGCTCAAGCCATCTACGCTGTTATGGCAGCTGTTTACC
TAGTCAGTCCTTGGACTCTGGAGCAAATCCAAGCAGATCTGTCCCAAGACCAGACTTGGTATGCATTGGCTTATGA
TGGGGCAGAAGTGATTGGATTTCTAGCTGTGCAGGAGAATCTTTTTGAAGCAGAAGTCCTGCAAATCGCTGTCAA
AGGAGCTTATCAGGGTCAGGGGATTGCGTCagCCTTGTTTGCTCAATTGCCGACAGACAAGGAAATTTTCCTCGAA
GTCAGACAGTCAAATCAACGAGCGCAAGCATTTTACAAGAAAGAAAAGATGACAGTTATCGCTGAGCGAAAGGC
CTACTACCATGACCCAGTCGAGGACGCCATTATCATGAAGAGAGAAATAGATGAAGGATAG

4152.2
ATGACAAAACAAGTCTTATTAGTGGATGATGAAGAACACATTCTGAAATTGCTTGACTACCATTTAAGTAAGGAA
GGCTTTTCTACTCAATTGGTGACAAATGGACGGAAGGCCTTAGCTTTGGCAGAAACAGAACCCTTTGATTTTATCT
TGCTTGATATCATGTTACCACAATTAGATGGCATGGAAGTTTGTAAGCGGCTGAGAGCCAAAGGCGTCAAAACTC
CAATTATGATGGTTTCTGCGAAAAGTGATGAATTTGATAAGGTTTTGGCCTTGGAATTAGGGGCTGATGACTACCT
GACCAAGCCTTTTAGCCCTAGAGAATTGCTGGCGCGTGTCAAGGCTGTCCTCAGGCGAACTAAAGGAGAACAAGA
AGGAGATGATTCAGATAATATCGCTGACGATTCTTGGCTATTTGGACCTTGAAAGTATACCCTGAGCGTCATGAA
GTCTACAAGGCGAATAAGTTACTGAGTTTGACCCCAAAAGAATTTGAAAGCGATAAAAATCCGTTTTTTGAAGTTT
TCAAAGTTTCGAAAGTAACCGCCCAATAA

4154.1
ATGACTACTTTTAAAGATGGATTTTTATGGGGTGGTGCTGTTGCTGCTCATCAACTTGAAGGTGGATGGCAAGAAG
GTGGCAAGGGAATTAGTGTTGCTGATGTTATGACTGCTGGTCGTCATGGAGTAGCTCGTGAAATTACTTTGGGAGT
TTTAGAGGGTAAATATTATCCAAATCATGAGGCGATAGATTTTTATCACCGTTATAAAGAAGATATAGCACTTTTT
GCTGAAATGGGATTCAAGTGCTTCCGTACCTCTATTGCATGGACACGTATCTTTCCAAAAGGTGATGAGTTAGAGC
CGAATGAAGAAGGATTACAGTTTTATGATAATCTTTTTGATGAATGCTTAAAGAATGGTATTGAACCTGTCATCAC
TCTATCTCATTTTGAAATGCCTTATCACTTAGTGACCGAATATGGTGGTTGGAAAAATAGGAAATTGATTGATTTC
TTTGCTCGTTTTGCAGAAGTCGTATTTAAACGTTACAAAGATAAGGTTAAATATTGGATGACTTTCAATGAAATCA
ATAATCAAGCGAATTATCAGGAAGATTTTGCACCATTTACTAACTCAGGTATTGTATATGAGGAAGGTGATAATAG
AGAAGCAATTATGTATCAAGCAGCACATTACGAATTAGTTGCTTCTGCACGAGCTGTAAAAATTGGTCATGAGATT
AATCCAGATTTTCAAATAGGTTGTATGATTGCGATGTGTCCAATTTATCCAGTTACTTGCAATCCTAAGGATATCTT
AATGGCAATGAAAGCTATGCAGAAGCGTTATTATTTTGCTGATGTGCATGTTTTAGGTAAATATCCTGAGCATATT
TTCAAGTATTGGGAACGAAAAGGTATTTCAGTTGATTTTACTGCCCAGGATAAAGAAGATTTACTTGGTGGGACTG
TAGATTACATTGGTTTCAGTTACTATATGTCCTTTGCTATCGACTCTCATCGTGAAAATAATCCTTATTTTGATTAT
CTTGAAACAGAAGATTTAGTGAAAAATAATTATGTTAAGGCTTCTGAATGGGAGTGGCAAATTGATCCAGAAGGT
TTGCGTTATGCGTTAAATTGGTTTACAGACCACTATCACTTACCACTCTTTATTGTTGAAAATGGTTTTGGAGCTAT
AGATCAAGTTGCAGCAGATGGTATGGTACATGATGATTATAGAATTGAATATCTAGGTGCCCATATTCGTGAAATG
AAAAAGGCTGTAGTTGAAGATGGTGTTGATTTAATGGGTTATACTCCATGGGGATGTATTGATTTGGTTTCAGCTG
GTACCGGTGAAATGCGGAAACGTTATGGCTTTATTTATGTAGATAAAGATGATAATGGGAAGGGAAGTTATAATC
GTTCCCCGAAAAAATCTTTTGGCTGGTATAAGGAAGTTATTTCATCTAACGGTGAATCAGTAGAATAG

4154.2
ATGGATCAACAAAACGGGTTGTTTGGTTTTCTTGAAAACCATGTTATGGGACCAATGGGCAAACTTGCTCAGTTTA
AAGTAGTACGTGCTATCACGGCTGCAGGTATGGCTGCTGTACCATTTACTATTGTAGGATCAATGTTTTTGGTATT
CAGTATTTTGCCACAAGCTTTCTCATTTTGGCCAATTGTGGCAGATATTTTCTCTGCTTCATTTGATAAATTCACAT
CACTTTACATGGTTGCAAACTATGCGACTATGGGTTCTCTATCTCTTTATTTCGTTCTATCACTTGCATATGAATTG
ACAAAAAATTTATGCAGAGGAAGAAGAACTCAATATGAATCCTCTTAATGGTGCCTTGCTTGCCTTGATGGCTTTTG
TCATGACAGTACCGCAAATCATTTTTGATGGTGGAATGATGAAGACTGTGACAAGTCTAAAAGAAGGTGCAGTAA
TTGCAGATGGATGGGCAATGGGAAATGTAGTCGCACGTTTTGGGACAACAGGGATTTTTACCGCAATCATTATGG
CAATTGTGACTGTTCTTATTTATCGTATGTGTGTTAAACATAATTGGGTTATTAAAATGCCTGAAGCTGTTCCAGAA
GGAGTTTCTCGTGGATTTACCGCTTTGGTTCCGGGATTTGTTGTTGCATTTGTTGTTATCTTTATCAACGGTCTTCTT
GTAGCAATGGGAACAGATATTTTTAAAGTCATTGCAATTCCATTTGGTTTTGTATCCAATCTGACTAATTCGTGGA
TTGGTTTAATGATTATTTATCTATTGACTCAACTACTTTGGATTGTAGGTATCCACGGTGCGAACATTGTTTTTGCA
TTTGTTAGTCCAATTGCTCTTGCTAACATGGCTGAAAATGCTGCTGGCGGGCACTTCGCTGTTGCAGGTGAATTTT
CTAATATGTTTGTAATTGCAGGTGGTTCTGGTGCAACTTTAGGACTATGTTTATATATTGCTTTTGCCTCTAAATCT
GAACAGCTTAAAGCAATAGGACGAGCATCTGTAGTTCCAGCCTTATTTAATATTAATGAACCATTAATTTTTGGAT
TACCTATTATCTATAATCCAGCCTTGGCTATACCATTTATTTTAGCACCAATGGTTACTGCTACTATTTATTACGTA
GCGAATTCTCTAAACTTTATTAAGCCAATTATCGCACAGGTTCCATGGCCAACTCCAGTAGGGATTGGAGCTTTCT

TAGGGACAGCAGATCTTCGAGCTGTATTAGTTGCTCTAGTATGTGCATTTGCAGCATTCCTAGTCTATCTTCCATTC
ATCCGTGTATATGATCAAAAATTGGTGAAAGAAGAGCAAGGTATCTAA

4155.1
ATGAAAAAATTTTATGTAAGTCCAATTTTTCCTATTCTAGTAGGATTGATTGCGTTTGGAGTCTTATCCACTTTCAT
TATTTTTGTTAATAATAATCTGTTGACGGTTTTAATTTTGTTTCTTTTTGTAGGAGGCTATGTTTTTTATTTAAGAA
ACTGAGAGTGCATTATACAAGGAGTGATGTAGAACAGATACAGTATGTAAACCACCAAGCGGAAGAAAGTTTGAC
AGCTCTATTGGAACAGATGCCTGTAGGTGTTATGAAATTGAATTTATCTTCTGGAGAGGTTGAGTGGTTTAATCCC
TATGCTGAATTGATTTTGACCAAGGAAGATGGTGATTTTGATTTAGAAGCTGTTCAAACGATTATCAAGGCTTCAG
TAGGAAATCCGTCTACTTATGCCAAGCTTGGTGAGAAGCGTTATGCTGTTCATATGGATGCTTCTTCCGGTGTTTT
GTATTTTGTAGATGTATCCAGGGAACAAGCCATAACAGATGAATTGGTAACAAGTAGACCAGTGATTGGGATTGT
CTCTGTGGATAATTATGATGATTTGGAGGATGAAACTTCTGAGTCAGATATTAGTCAAATCAATAGTTTTGTAGCT
AATTTTATATCAGAGTTTTCAGAAAAACACATGATGTTTTCTCGTCGGGTAAGTATGGATCGATTTTATCTATTTAC
TGACTACACGGTGCTTGAGGGCTTGATGAATGATAAATTTTCTGTTATTGATGCTTTCAGAGAAGAGTCGAAACAG
AGACAGTTGCCCTTGACCTTAAGTATGGGATTTTCTTATGGCGATGGAAATCATGATGAGATAGGGAAAGTTGCTT
TGCTCAATTTGAACTTGGCTGAAGTACGTGGTGGCGACCAGGTGGTTGTTAAGGAAAACGACGAAACGAAAAATC
CAGTTTATTTTGGTGGTGGGTCTGCTGCTTCAATCAAGCGTACACGGACTCGTACGCGCGCTATGATGACAGCTAT
TTCAGATAAGATTCGGAGTGTAGATCAGGTTTTTGTAGTCGGTCACAAAAATTTAGACATGGATGCTTTGGGCTCT
GCTGTAGGTATGCAGTTGTTCGCCAGCAATGTGATTGAAAATAGCTATGCTCTTTATGATGAAGAACAAATGTCTC
CAGATATTGAACGAGCTGTTTCATTCATAGAAAAAGAAGGAGTTACGAAGTTGTTGTCTGTTAAGGATGCAATGG
GGATGGTGACCAATCGTTCTTTGTTGATTCTTGTAGACCATTCAAAGACAGCCTTAACATTATCAAAAGAATTTTA
TGATTTATTTACCCAAACCATTGTTATTGACCACCATAGAAGGGATCAGGATTTTCCAGATAATGCGGTTATTACT
TATATCGAAAGTGGTGCAAGTAGTGCCAGTGAGTTGGTAACGGAATTGATTCAGTTCCAGAATTCTAAGAAAAAT
CGTTTGAGTCGTATGCAAGCAAGTGTCTTGATGGCTGGTATGATGTTGGATACTAAAAATTTCACCTCGCGAGTAA
CTAGTCGGACATTTGATGTTGCTAGCTATCTCAGAACGCGCGGAAGTGATAGTATTGCTATCCAGGAAATCGCTGC
GACAGATTTTGAAGAATATCGTGAGGTCAATGAACTTATTTTACAGGGGCGTAAATTAGGTTCAGATGTACTAATA
GCAGAGGCTAAGGACATGAAATGCTATGATACAGTTGTTATTAGTAAGGCAGCAGATGCCATGTTAGCCATGTCA
GGTATTGAAGCGAGTTTTGTTCTTGCGAAGAATACACAAGGATTTATCTCTATCTCAGCTCGAAGTCGTAGTAAAC
TGAATGTACAACGGATTATGGAAGAGTTAGGCGGTGGAGGCCACTTTAATTTGGCAGCAGCTCAAATTAAAGATG
TAACCTTGTCAGAAGCAGGTGAAAAACTGACAGAAATTGTATTAAATGAAATGAAGGAAAAGGAGAAAGAAGAA
TGA

4156.1
ATGAAAGAGAAAAATATGTGGAAAGAATTGTTGAATCGTGCAGGCTGGATTTTGGTCTTTTTACTTGCCGTCCTTT
TATATCAGGTTCCCCTAGTGGTTACCTCTATTTTGACTTTAAAAGAAGTAGCCCTGCTACAGTCAGGGCTGATAGT
TGCTGGCCTTTCAATTGTGGTTCTGGCTCTATTTATTATGGGAGCTCGTAAAACCAAGTTAGCTAGTTTTAATTTTT
CTTTTTTTAGAGCTAAAGATTTGGCACGTTTGGGCTTGAGTTATCTAGTTATTGTCGGGTCAAATATACTTGGTTCC
ATTTTATTGCAACTGTCAAATGAGACGACAACAGCTAACCAGTCTCAGATTAATGATATGGTTCAAAATAGTTCGT
TGATTTCCAGTTTCTTCTTGCTAGCCTTGCTTGCTCCGATTTGTGAGGAAATCTTGTGTCGTGGGATTGTTCCTAAA
AAGATTTTCCGAGGCAAGGAGAACTTGGGATTTGTAGTCGGTACGATTGTGTTTGCTTTATTGCATCAACCAAGTA
ATTTACCTTCTTTATTGATTTATGGAGGTATGTCGACAGTTCTATCTTGGACAGCCTACAAGACCCAACGTTTGGA
AATGTCGATCTTGCTTCACATGATTGTTAATGGGATTGCTTTCTGTTTGTTGGCTCTTGTGGTGATTATGAGTCGGA
CATTAGGAATTTCTGTTTAAATGAAAGAGAAAAATATGTGGAAAGAATTGTTGAATCGTGCAGGCTGGATTTTGGT
CTTTTTACTTGCCGTCCTTTTATATCAGGTTCCCCTAGTGGTTACCTCTATTTTGACTTTAAAAGAAGTAGCCCTGC
TACAGTCAGGGCTGATAGTTGCTGGCCTTTCAATTGTGGTTCTGGCTCTATTTATTATGGGAGCTCGTAAAACCAA
GTTAGCTAGTTTTAATTTTTCTTTTTTTAGAGCTAAAGATTTGGCACGTTTGGGCTTGAGTTATCTAGTTATTGTCG
GGTCAAATATACTTGGTTCCATTTTATTGCAACTGTCAAATGAGACGACAACAGCTAACCAGTCTCAGATTAATGA
TATGGTTCAAAATAGTTCGTTGATTTCCAGTTTCTTCTTGCTAGCCTTGCTTGCTCCGATTTGTGAGGAAATCTTGT
GTCGTGGGATTGTTCCTAAAAAGATTTTCCGAGGCAAGGAGAACTTGGGATTTGTAGTCGGTACGATTGTGTTTGC
TTTATTGCATCAACCAAGTAATTTACCTTCTTTATTGATTTATGGAGGTATGTCGACAGTTCTATCTTGGACAGCCT
ACAAGACCCAACGTTTGGAAATGTCGATCTTGCTTCACATGATTGTTAATGGGATTGCTTTCTGTTTGTTGGCTCTT
GTGGTGATTATGAGTCGGACATTAGGAATTTCTGTTTAA

4156.4
ATGGATACACAAAAGATTGAAGCGGCTGTAAAAATGATTATCGAGGCTGTAGGAGAGGACGCTAATCGCGAGGGC
TTGCAGGAAACACCTGCTCGTGTAGCCCGTATGTATCAAGAGATTTTTTCAGGTCTTGGTCAAACAGCAGAGGAAC
ATTTGTCAAAATCCTTTGAAATTATTGACGATAATATGGTGGTAGAAAAGGATATCTTTTTCCATACCATGTGTGA
ACACCACTTCTTGCCATTTTATGGTAGAGCGCACATTGCCTACATTCCAGATGGTCGTGTGGCAGGCTTGTCTAAG
CTAGCCCGTACGGTTGAAGTTTATTCGAAAAAACCACAAATTCAAGAACGTTTGAATATCGAAGTGGCCGATGCC
TTGATGGACTATCTAGGTGCTAAAGGAGCCTTTGTTGTCATTGAGGCGGAACATATGTGTATGAGTATGCGTGGTG
TTAGAAAACCAGGCACTGCAACCTTGACGACAGTAGCTCGTGGTCTATTTGAAACAGATAAGGATCTCCGTGACC
AAGCTTATCGTTTAATGGGGCTATAA

4157.2

ATGAAAGACTTGTTTTTAAAGAGAAAGCAGGCCTTTCGTAAGGAGTGTCTTGGTTATCTGCGCTATGTGCTCAATG
ACCACTTTGTCTTGTTCCTGCTTGTCCTGTTGGGCTTTCTAGCCTACCAGTACAGTCAACTCTTACAACATTTTCCT
GAAAATCATTGGCCTATCCTTTTGTTTGTAGGAATTACGTCTGTTTTACTTTTACTTGGGGAGGAACTGCCACCTA
TATGGAGGCTCCAGACAAGCTCTTTCTCTTAGTTGGAGAAGAGGAAATTAAGCTCCATCTCAAGCGTCAAACTGG
CATTTCCCTAGTCTTTTGGCTCTTTGTACAGACCCTTTTCTTGCTGTTATTTGCGCCTTTATTTTTAGCAATGGGTTA
TGGCTTGCCAGTTTTTCTGCTCTATGTGCTTTTATTGGGGGTAGGAAAATATTTCCACTTTTGTCAAAAGGCCAGCA
AATTTTTCACTGAAACTGGACTGGACTGGGACTATGTTATTTCTCAAGAAAGCAAGCGTAAGCAAGTCTTGCTTCG
TTTCTTTGCCCTCTTTACGCAGGTCAAGGGAATTTCAAACAGCGTTAAGCGTCGTGCCTATCTGGACTTTATTTTAA
AGGCTGTTCAGAAGGTGCCTGGGAAGATTTGGCAAAATCTCTATCTGCGTTCTTATCTGCGAAATGGCGACCTCTT
TGCTCTCAGTCTTCGTCTTCTCTTGCTTTCCTTGCTGGCGCAGGTTTTTATCGAGCAAGCTTGGATTGCGACAGCAG
TGGTAGTTCTCTTTAACTACCTCTTGCTCTTCCAGTTGCTGGCCCTCTATCATGCCTTTGACTACCAGTATTTGACC
CAACTCTTTCCGCTGGACAAGGGGCAAAAGGAAAAAGGCTTACAGGAGGTAGTTCGAGGATTGACCAGTTTTGTT
TTACTTGTGGAATTAGTTGTTGGGTTGATTACCTTCCAAGAAAAACTAGCCCTTCTAGCCTTACTAGGAGCTGGTT
TGGTTTTACTAGTCTTGTATTTGCCTTATCAGGTAAAACGTCAGATGCAGGACTAA

4158.1
ATGAGAAAATCAATAGTATTAGCGGCAGATAATGCCTATCTTATTCCTTTAGAGACGACTATAAAGTCTGTATTGT
ATCACAATAGAGATGTTGATTTTTATATTCTCAACAGTGATATAGCTCCTGAATGGTTTAAATTATTGGGGAGAAA
AATGGAAGTTGTGAATTCTACAATTCGCAGTGTACACATTGATAAAGAACTTTTTGAAAGCTATAAAACAGGACCT
CATATAAATTATGCTTCTTACTTTAGATTTTTTGCGACAGAAGTGGTTGAATCTGATAGGGTATTGTATCTGGATTC
CGATATCATTGTAACTGGGGAACTAGCTACTTTGTTTGAGATAGATCTCAAAGGATATTCAATTGGTGCTGTTGAT
GATGTCTATGCCTATGAAGGACGAAAATCTGGATTTAATACTGGTATGTTACTAATGGATGTTGCAAAGTGGAAAG
AACATTCTATTGTCAATAGTTTATTGGAATTAGCGGCCGAGCAGAATCAAGTTGTTCATCTTGGGGATCAGAGTAT
TTTAAATATTTATTTTGAGGATAATTGGCTAGCCTTAGATAAAACATATAATTATATGGTGGGTATTGATATTTATC
ACCTTGCTCAAGAATGTGAACGTCTAGATGACAATCCACCTACAATTGTTCACTATGCTAGTCATGATAAACCTTG
GAATACATATAGTATATCTAGACTACGTGAATTATGGTGGGTTTATAGAGATTTGGATTGGTCAGAGATTGCTTTT
CAACGTTCCGATTTAAATTATTTTGAAAGAAGCAATCAGTCTAAAAAACAAGTGATGCTTGTGACATGGAGTGCA
GATATAAAACATTTAGAGTATTTAGTACAACGGTTACCTGATTGGCATTTTCATTTGGCTGCACCGTGTGATTGTTC
TGAGGAGCTGACCTCTCTATCACAGTATACGAATGTAACAGTATATCAAAATGTATTACATAGTAGAATTGATTGG
CTATTGGACGATTCTATAGTTTATTTAGATATTAATACAGGTGGAGAGGTTTTTAATGTAGTTACAAGGGCACAAG
AAAGTGGCAAGAAAATCTTCGCTTTTGATATCACACGTAAAAGTATGGATGATGGACTCTATGACGGTATTTTTTC
TGTGGAGAGACCAGATGATTTAGTGGATAGAATGAAGAATATAGAGATAGAGTAA

4158.2
ATGACTAAGATTTATTCGTCAATAGCAGTAAAAAAAGGACTATTTACCTCATTTCTACTGTTTATCTATGTATTGG
GAAGTCGTATTATTCTCCCTTTTGTTGACCTAAATACTAAAGATTTTTTAGGAGGTTCAACAGCCTATCTAGCCTTC
TCAGCCGCCCTAACAGGTGGGAATCTAAGAAGTTTATCAATTTTTTCTGTTGGATTATCCCCTTGGATGTCCGCCA
TGATTTTATGGCAGATGTTTTCTTTTTCTAAACGGTTGGGTTTAACATCTACGTCTATAGAAATACAAGATCGCCGT
AAAATGTACCTGACCTTGCTAATTGCTGTGATTCAATCCTTGGCAGTTAGCTTGAGACTGCCAGTACAATCCTCCT
ATTCTGCAATATTGGTTGTTCTAATGAATACAATATTGCTGATAGCAGGAACATTTTTTCTTGTTTGGTTGTCAGAT
TTAAATGCGAGTATGGGGATTGGAGGTTCTATTGTAATCCTCCTATCCAGTATGGTTTTAAATATTCCTCAGGATG
TTTTGGAAACATTTCAGACAGTACACATTCCAACAGGGATTATTGTGTTACTTGCTTTATTAACCCTTGTCTTTTCT
TATTTACTTGCCCTTATGTATCGAGCTCGCTATTTGGTTCCTGTTAATAAAATTGGCTTACACAATCGATTTAAACG
CTATTCTTATCTCGAAATCATGTTGAATCCTGCAGGTGGGATGCCTTATATGTATGTGATGAGTTTTCTTAGTGTAC
CAGCTTATTTGTTCATCTTGTTGGGATTTATTTTCCCTAATCATTCAGGGTTAGCGGCTTTATCAAAGGAATTTATG
GTTGGAAAGCCTTTGTGGGTCTATGTTTATATTTCGGTCTTATTTTTATTTAGTATCATTTTTGCTTTTGTTACGATG
AATGGAGAAGAGATTGCAGACCGTATGAAAAAATCTGGAGAATACATTTATGGTATTTATCCAGGTGCGGATACT
AGTCGATTTATTAATCGATTGGTCCTTCGTTTCTCAGTCATAGGTGGTCTCTTTAATGTGATTATGGCAGGTGGTCC
CATGCTTTTTTGTTTTGTTTGATGAAAAGTTATTACGATTGGCAATGATTCCAGGCTTATTTATGATGTTCGGGGGCA
TGATTTTTACGATTAGAGACGAGGTCAAGGCTTTAAGGCTAAATGAGACCTATAGACCTTTGATTTAG

4158.3
ATGTCCTCTCTTTCGGATCAAGAATTAGTAGCTAAAACAGTAGAGTTTCGTCAGCGTCTTTCCGAGGGAGAAAGTC
TAGACGATATTTTGGTTGAAGCTTTTGCTGTGGTGCGTGAAGCAGATAAGCGGATTTTAGGGATGTTTCCTTATGA
TGTTCAAGTCATGGGAGCTATTGTCATGCACTATGGAAATGTTGCTGAGATGAATACGGGGGAAGGTAAGACCTT
GACAGCTACCATGCCTGTCTATTTGAACGCTTTTTCAGGAGAAGGAGTGATGGTTGTGACTCCTAATGAGTATTTA
TCAAAGCGTGATGCCGAGGAAATGGGTCAAGTTTATCGTTTTCTAGGATTGACCATTGGTGTACCATTTACGGAAG
ATCCAAAGAAGGAGATGAAAGCTGAAGAAAAGAAGCTTATCTATGCTTCGGATATCATCTACACAACCAATAGTA
ATTTAGGTTTTGATTATCTAAATGATAACCTAGCCTCGAATGAAGAAGGTAAGTTTTTACGACCGTTTAACTATGT
GATTATTGATGAAATTGATGATATCTTGCTTGATAGTGCACAAACTCCTCTGATTATTGCGGGTTCTCCTCGTGTTC
AGTCTAATTACTATGCGATCATTGATACACTTGTAACAACCTTGGTCGAAGGAGAGGATTATATCTTTAAAGAGGA
GAAAGAGGAGGTTTGGCTCACTACTAAGGGGGCCAAGTCTGCTGAGAATTTCCTAGGGATTGATAATTTATACAA
GGAAGAGCATGCGTCTTTTGCTCGTCATTTGGTTTATGCGATTCGAGCTCATAAGCTCTTTACTAAAGATAAGGAC
TATATCATTCGTGGAAATGAGATGGTACTGGTTGATAAGGGAACAGGGCGTCTAATGGAAATGACTAAACTTCAA
GGAGGTCTCCATCAGGCTATTGAAGCCAAGGAACATGTCAAATTATCTCCTGAGACGCGGGCTATGGCCTCGATC

ACCTATCAGAGTCTTTTTAAGATGTTTAATAAGATATCTGGTATGACAGGGACAGGTAAGGTCGCGGAAAAAGAG
TTTATTGAAACTTACAATATGTCTGTAGTACGCATTCCAACCAATCGTCCGAGACAACGGA
TTGACTATCCAGATAATCTATATATCACTTTACCTGAAAAAGTGTATGCATCCTTGGAGTACATCAAGCAATACCA
TGCTAAGGGAAATCCTTTACTCGTTTTTGTAGGCTCAGTTGAAATGTCTCAACTCTATTCGTCTCTCTTGTTTCGTG
AAGGGATTGCCCATAATGTCCTAAATGCTAATAATGCGGCGCGTGAGGCTCAGATTATCTCCGAGTCAGGTCAGA
TGGGGGCTGTGACAGTGGCTACCTCTATGGCAGGACGTGGTACGGATATCAAGCTTGGTAAAGGAGTCGCAGAGC
TTGGGGGCTTGATTGTTATTGGGACTGAGCGGATGGAAAGTCAGCGGATCGACCTACAAATTCGTGGCCGTTCTGG
TCGTCAGGGAGATCCTGGTATGAGTAAATTTTTTGTATCCTTAGAGGATGATGTTATCAAGAAATTTGGTCCATCT
TGGGTGCATAAAAAGTACAAAGACTATCAGGTTCAAGATATGACTCAACCGGAAGTATTGAAAGGTCGTAAATAC
CGGAAACTAGTCGAAAAGGCTCAGCATGCCAGTGATAGTGCTGGACGTTCAGCACGTCGTCAGACTCTGGAGTAT
GCTGAAAGTATGAATATACAACGGGATATAGTCTATAAAGAGAGAAATCGTCTAATAGATGGTTCTCGTGACTTA
GAGGATGTTGTTGTGGATATCATTGAGAGATATACAGAAGAGGTAGCGGCTGATCACTATGCTAGTCGTGAATTAT
TGTTTCACTTTATTGTGACCAATATTAGTTTTCATGTTAAAGAGGTTCCAGATTATATAGATGTAACTGACAAAACT
GCAGTTCGTAGCTTTATGAAGCAGGTGATTGATAAAGAACTTTCTGAAAAGAAAGAATTACTTAATCAACATGACT
TATATGAACAGTTTTTACGACTTTCACTGCTTAAAGCCATTGATGACAACTGGGTAGAGCAGGTAGACTATCTACA
ACAGCTATCCATGGCTATCGGTGGTCAATCTGCTAGTCAGAAAAATCCAATCGTAGAGTACTATCAAGAAGCCTA
CGCGGGCTTTGAAGCTATGAAAGAACAGATTCATGCGGATATGGTGCGTAATCTCCTGATGGGGCTGGTTGAGGT
CACTCCAAAAGGTGAAATCGTGACTCATTTTCCATAA

4158.4
ATGATAGGGACTTTCGCCGCTGCTCTTGTAGCTGTACTAGCAAATTTCATCGTCCCTATTGAAATTACCCCAAATA
GTGCCAATACTGAAATTGCACCACCAGATGGGATTGGGCAGGTTCTCAGCAACCTCTTGCTCAAACTGGTTGACA
ACCCAGTCAACGCCCTGCTTACTGCTAACTATATTAGAATCTTATCTTGGGCAGTCATTTTTGGAATCGCTATGAG
AGAAGCCAGTAAAAATAGTCAAGAATTGCTAAAAACTATCGCTGACGTGACTTCTAAAATTGTCGAATGGATCAT
CAATCTGGCTCCATTTGGAATCCTTGGTCTTGTTTTTAAAACCATTTCTGACAAGGGAGTCGGAAGCCTTGCCAAC
TACGGTATTTTATTGGTTCTATTAGTAACGACTATGCTTTTTGTTGCCCCTGTGGTCAACCCTTTGATTGCCTTCTTC
TTTATGAGACGCAATCCTTACCCTCTAGTTTGGAACTGCCTCCGTGTCAGCGGTGTGACAGCCTTTTTCACTCGTA
GTTCTGCGACTAACATTCCTGTCAACATGAAACTCTGCCATGACCTTGGACTCAACCCAGATACCTATTCTGTTTC
TATCCCACTCGGTTCTACTATCAATATGGCTGGAGTAGCGATTACCATTAACCTTTTGACCCTTGCTGCAGTTAAC
ACTCTTGGAATTCCTGTTGACTTTGCCACAGCCTTTGTCCTCAGTGTGGTAGCAGCTATCTCATCCTGTGATGCTTC
AGGTATTGCCGGAGGTTCCCTCCTTCTTATCCCAGTTGCTTGTAGCCTTTTCGGTATTTCTAACGATATTGCCATAC
AAATTGTTGGGGTTGGTTTTGTGATTGGTGTCATCCAAGACTCATGTGAAACAGCCCTTAACTCTTCTACAGATGT
CCTCTTTACCGCCGTTGCCGAATACGCAGCAACCCGTAAAAAATAA

4158.5
ATGTCTATTAGCCAACGTACGACCAAGCTCATCTTAGCTACCTGTCTTGCCTGCCTGCTTGCTTATTTTCTCAATCT
TTCGTCAGCAGTTTCGGCTGGAATTATCGCTCTCTTGAGCCTATCTGATACGCGTAGAAGTACTTTAAAACTGGCT
CGCAATCGTCTTTTTTCTATGCTTCTAGCTCTGGCTATCGGTGTTCTAGCTTTTCACTTGAGCGGATTTCATATCTG
GAGTCTCGGCCTCTATCTGGCCTTCTACGTTCCTTTAGCCTACAAGATGGGCTGGGAAATTGGCATCACACCAAGC
ACTGTTTTGGTTAGCCATCTCTTGGTTCAAGAGTCAACCTCTCCAGACCTTCTAGTCAATGAATTCCTTCTCTTTGC
TATTGGTACAGGATTTGCCTTGCTTGTTAATCTCTATATGCCTTCACGAGAAGAGGAAATCCAGCACTACCACACG
CTGGTGGAAGAAAAGTTAAAAGATATCCTCCAGCGCTTCAAATACTATTTATCCAGAGGAGACGGACGCAACCGA
GCACAGCTGGTAGCAGAATTAGACACGCTTTTGAAAGAAGCCCTCAGACTGGTCTATTTGGATCACTCTGACCACC
TCTTTCACCAGACAGACTACCATATCCACTACTTTGAGATGAGACAGCGACAAAGTCGTATCCTGAGAAACATGG
CCCAACAGATTAACACTTGTCACCTTGCCGCCAGTGAAAGCCTGATCTTAGCGCAACTCTTTTCAAAAATTGCAGG
TCAACTGAGCCAGACCAATCCTGCTTCTGATTTGCTAGATGAAATTGAACGTTATCTGGAAGTCTTCCGGAACCGC
AGTCTGCCCAAGACAAGAGAAGAATTTGAAACCCGCGCCACCCTTCTTCAACTCCTACGTGAAGCCAAAACCTTC
ATCCAAGTAAAAGTTGATTTTTACCAAAAATATAGACAGTAA

4158.6
ATGGAAATCATGTCGCTTGCGATTGCTGTTTTTGCCGTCATCATTGGTTTAGTCATTGGATATGTCAGCATCTCAGC
TAAGATGAAATCATCTCAGGAAGCTGCAGAGTTGATGCTTTTAAATGCTGAACAAGAAGCAACTAATTTACGTGG
ACAAGCTGAGCGTGAAGCGGATTTACTTGTTAATGAAGCCAAACGTGAAAGCAAGTCTCTTAAAAAAGAAGCACT
ATTGGAGGCCAAAGAAGAAGCCAGAAAATACCGTGAAGAAGTGGACGCTGAATTCAAATCAGAACGTCAAGAAC
TCAAACAAATCGAAAGTCGTTTGACAGAGAGAGCTACTAGCCTTGACCGTAAGGACGACAATTTGACGAGTAAAG
AACAAACACTTGAACAAAAAGAACAAAGTATTTCTGATAGAGCGAAAAACCTTGATGCGCGTGAAGAGCAATTAG
AGGAAGTCGAAAGACAAAAAGAAGCAGAACTAGAGCGTATTGGTGCGCTGTCTCAGGCAGAAGCACGAGATATT
ATCTTGGCTCAGACAGAGGAAAACTTGACCAGGGAGATTGCCAGTCGCATTCGCGAAGCTGAGCAAGAGGTCAAG
GAACGTTCTGACAAAATGGCCAAGGACATCTTGGTTCAAGCTATGCAACGTATCGCTGGTGAATATGTAGCGGAG
TCAACAAACTCAACAGTTCATCTGCCAGACGATACTATGAAGGGACGCATTATTGGTCGTGAAGGTCGTAACATT
CGTACCTTTGAAAGTTTGACAGGGGTCGATGTGATTATCGACGATACACCAGAAGTGGTGACCTTGTCAGGATTTG
ATCCGATTCGTCGTGAGATTGCCCGTATGACTATGGAAATGTTGCTCAAAGATGGTCGTATACATCCAGCTCGTAT
CGAAGAGTTGGTTGAGAAAAACCGTCAAGAGATTGACAATAAGATTCGTGAATACGGTGAGGCTGCTGCCTATGA
AATTGGTGCGCCAAACCTTCATCCAGACTTGATGAAGATTATGGGACGTTTGCAGTTCCGTACTTCATATGGACAA
AATGTTTTGCGCCATTCGATTGAGGTTGCTAAGTTGGCTGGTATCATGGCGAGCGAACTTGGTGAAAATGCGGCTC

TTGCCCGTCGTGCTGGATTCCTTCACGATATCGGGAAAGCCATTGACCATGAGGTTGAAGGTAGCCACGTTGAAAT
CGGTATGGAATTGGCCCGTAAGTACAAGGAACCCCCAGTTGTGGTGAATACGATTGCTAGTCACCACGGAGATGT
TGAAGCTGAGAGCGTGATAGCAGTTATCGTCGCTGCAGCAGATGCCTTGAGCGCAGCCCGTCCAGGTGCTCGTAG
TGAGTCTCTTGAAAGCTACATCAAGCGTCTCCATGATTTGGAAGAAATTGCTAACGGCTTTGAAGGAGTGCAAACT
AGCTTTGCCCTTCAAGCAGGACGTGAAATTCGTATCATGGTCAATCCAGGAAAAATCAAGGACGACAAAGTCACA
ATCTTGGCTCACAAAGTTCGTAAGAAAATTGAAAACAATCTCGATTATCCAGGAAATATCAAGGTAACCGTGATT
CGCGAGCTTCGTGCAGTAGATTATGCTAAATAA

4158.7
ATGATGTTAAAACCCTCTATTGATACCTTGCTCGACAAGGTTCCTTCAAAATATTCACTCGTAATCTTGGAAGCAA
AACGTGCCCACGAATTGGAAGCAGGTGCCCCAGCAACTCAAGGTTTCAAGTCTGAAAAATCAACTCTTCGCGCTT
TAGAAGAAATCGAATCAGGAAACGTTACAATTCACCCAGATCCAGAAGGAAAACGTGAAGCAGTGCGTCGCCGTA
TCGAAGAAGAAAAACGCCGCAAAGAAGAAGAAAAGAAAATCAAAGAGCAAATTGCTAAAGAAAAAGAAGA
TGGTGAAAAAATTTAA

4161.1
ATGTCAGCATATCAATTACCGACCGTATGGCAGGATGAAGCTAGTAATCAAGGAGCTTTTACGGGGCTAAACAGA
CCAACAGCAGGTGCCCGTTTCGAACAAAACTTGCCAAAAGGAGAACAAGCTTTTCAGCTTTATTCACTGGGAACA
CCAAATGGTGTGAAGGTTACTATCTTATTGGAAGAATTACTAGAAGCTGGTTTTAAGGAAGCGGCTTACGACTTGT
ATAAGATTGCTATCATGGATGGGGATCAATTCGGATCAGACTTTGTGAAGCTCAATCCAAATTCCAAGATTCCAGC
CTTATTGGACCAGTCAGGTACTGAAAACGTAAGAGTCTTTGAGTCTGCTCATATTCTTCTTTACCTTGCTGAGAAA
TTTGGAGCCTTTTTACCAAGTAATCCTGTGGAAAAGGTAGAAGTTTTGAATTGGCTATTCTGGCAAGCAGGTGCAG
CACCTTTTCTAGGTGGGGGATTTGGACATTTCTTCAATTATGCTCCTGAAAAATTGGAATATCCTATTAACCGTTTT
ACGATGGAAGTGAAACGCCAGTTGGATTTATTGGATAAGGAATTGGCTCAGAAACCTTATATTGCAGGCAATGAC
TATACGATTGCAGATATTGCTATCTGGTCTTGGTATGGACAGTTAGTTCAAGGAAATCTTTACCAAGGTTCTGCAA
AATTCTTGGATGCCTCAAGTTATCAAAATCTAGTAAAATGGGCAGAAAAAATTGCCAATCGTCCAGCTGTTAAGC
GTGGCTTGGAAGTAACTTATACAGAAATTAAATAG

4161.2
TTGGCAAGCTTGATCACTTCTATCATCATGTTCTATGTCGGTTTCGATGTTCTAAGAGATACCATTCAAAAGATTCT
CAGTCGGGAAGAAACGGTCATTGATCCTCTTGGTGCAACTCTAGGAATCATTTCTGCAGCGATTATGTTTGTGGTC
TATCTCTACAATACTCGCCTCAGTAAGAAATCCAACTCCAATGCGCTGAAGGCAGCTGCTAAGGACAATCTTTCTG
ACGCTGTTACCTCACTTGGAACCGCCATTGCCATCCTAGCTAGTAGTTTCAATTATCCGATTGTGGATAAACTGGT
TGCTATCATCATCACTTTCTTTATCTTGAAGACTGCCTATGATATCTTCATCGAGTCTTCCTTTAGTCTTTCAGATG
GCTTTGACGACCGCCTGCTCGAGGACTACCAAAAGGCTATCATGGAAATTCCCAAAATCAGCAAGGTCAAATCGC
AAAGAGGTCGCACCTACGGTAGCAACATCTACCTGGATATTACACTAGAGATGAATCCTGACTTGTCTGTTTTTGA
AAGCCATGAAATCGCGGATCAGGTCGAGTCTATGCTGGAGGAGCGTTTTGGCGTCTTTGATACCGATGTCCATATC
GAACCAGCACCTATCCCTGAGGATGAAATTTTAGACAATGTCTATAAAAAATTGCTTATGCGTGAACAATTGATTG
ACCAAGGAAACCAACTAGAAGAACTCTTGACTGATGATTTTGTCTATATTCGCCAAGATGGAGAGCAGATGGATA
AAGAGGCTTATAAGACCAAAAAAGAGTTAAATTCTGCTATCAAGGACATTCAAATTACTTCCATCAGTCAAAAAA
CCAAACTCATCTGCTATGAGTTAGATGGTATCATCCATACCAGTATCTGGCGTCGCCACGAAACCTGGCAAAATAT
CTTTCATCAAGAAACCAAAAAAGAATAG

4162.1
ATGACAATTAAACTAGTAGCAACGGATATGGACGGAACCTTCCTAGATGGGAATGGACGCTTTGATATGGATCGT
CTCAAGTCTCTCTTGGTTTCCTACAAGGAAAAAGGGATTTACTTTGCGGTAGCTTCGGGTCGGGGATTTCTGTCTC
TAGAAAAATTATTTGCTGGTGTTCGTGATGACATTATTTTCATCGCGGAAAATGGCAGTTTGGTAGAGTATCAAGG
TCAGGACTTGTATGAAGCGACTATGTCTCGTGACTTTTATCTGGCAACTTTTGAAAAGCTGAAAACTTCACCTTAT
GTAGATATCAATAAACTGCTCTTGACGGGTAAGAAGGGTTCATATGTTCTAGATACGGTTGATGAGACCTATTTGA
AAGTGAGTCAGCACTATAATGAAAATATCCAAAAAGTAGCGAGTTTGGAAGATATCACAGATGACATTTTCAAAT
TTACAACCAACTTCACAGAAGAAACGCTGGAAGATGGGGAGGCTTGGGTAAACGAAAACGTTCCTGGTGTTAAGG
CCATGACAACTGGCTTTGAATCCATTGATATTGTTCTGGACTATGTCGATAAGGGAGTGGCCATTGTTGAATTAGT
TAAAAAACTTGGTATCACAATGGATCAGGTCATGGCTTTTGGAGACAATCTTAATGACTTACATATGATGCAGGTT
GTGGGACATCCTGTAGCTCCTGAAAATGCACGACCTGAAATTTTAGAATTAGCAAAGACTGTGATTGGTCACCATA
AGGAACGGTCGGTTATAGCTTATATGGAGGGCTTATAA

4162.2
ATGGCAGATATAAAATTGATTGCATTGGACTTGGACGGGACCTTGCTGACTACTGATAAAAGGCTGACGGATCGT
ACCAAGGAAACCTTGCAAGCTGCGCGTGATCGTGGTATCAAGGTCGTATTGACAACTGGTCGTCCCTTAAAAGCC
ATGGATTTCTTTCTCCATGAGTTAGGGACTGACGGTCAGGAAGATGAGTATACCATTACTTTTAATGGTGGATTAG
TTCAGAAAAATACAGGAGAAATCCTTGATAAAACAGTCTTTTCATATGATGATGTGGCACGTTTGTATGAAGAAAC
AGAGAAATTATCACTGCCTCTTGATGCCATCTCAGAAGGAACAGTTTATCAAATCCAATCGGACCAAGAAAGTCT
TTATGCCAAATTCAATCCAGCTTTGACCTTTGTTCCAGTGGACTTTGAAGACTTATCTAGTCAAATGACCTACAAC
AAATGCGTGACTGCCTTTGCTCAAGAACCCTTGGATGCAGCCATTCAGAAGATTTCTCCAGAATTGTTTGACCAAT
ATGAAATCTTTAAATCACGTGAAATGTTGCTAGAATGGTCACCAAAGAATGTTCATAAAGCAACAGGTTTGGCAA

AACTAATCAGCCATCTTGGAATCGACCAAAGTCAAGTGATGGCTTGTGGTGACGAGGCCAATGACCTCTCTATGA
TTGAATGGGCAGGTCTTGGTGTTGCTATGCAAAACGCTGTTCCTGAAGTAAAGGCAGCCGCAAATGTAGTGACGC
CGATGACCAACGATGAGGAAGCTGTCGCCTGGGCTATCGAAGAATATGTGCTAAAGGAGAACTAA

4164.2
ATGGAAAGTTTACTTATTCTATTATTAATTGCCAATCTAGCTGGTCTCTTTCTGATTTGGCAAAGGCAGGATAGGC
AGGAGAAACACTTAAGTAAGAGCTTGGAGGATCAGGCAGATCATTTGTCAGACCAGTTGGATTACCGCTTTGACC
AAGCCAGACAAGCCAGCCAGTTAGACCAAAAAGATTTGGAAGTGGTTGTCAGCGACCGTTTGCAAGAAGTGCGGA
TTGAATTGCACCAAGGTCTGACCCAAGTCCGTCAAGAAATGACAGATAATCTCCTCCAAACTAGAGACAAGACAG
ACCAACGTCTCCAAGCCTTGCAGGAATCAAATGAGCAACGTTTGGAACAAATGCGCCAGACGGTCGAGGAAAAAC
TAGAAAAGACCTTGCAGACACGCTTACAGGCTTCCTTTGAGACAGTTTCTAAACAACTGGAGTCTGTCAATCGTGG
CCTTGGAGAAATGCAGACAGTTGCCCGTGATGTCGGAGCTCTTAACAAGGTTCTCTCTGGAACCAAGACGCGAGG
GATTCTGGGAGAATTGCAACTGGGGCAAATTATTGAAGACATCATGACACCTGCCCAGTACGAACGAGAATACGC
AACGGTTGAAAACTCTAGTGAACGAGTGGAGTATGCCATCAAGTTACCCGGACAAGGCGACCAAGAATACGTCTA
TCTGCCAATTGACTCTAAGTTTCCACTGGCAGATTATTACCGCTTGGAAGAAGCCTATGAGACAGGTGACAAGGAT
GAGATTGAACGCTGTCGTAAGTCACTCCTAGCAAGCGTCAAGCGCTTTGCTAGGGATATTAGGAACAAGTACATA
GCACCACCTCGGACGACCAATTTTGGAGTTTTGTTTGTTCCGACAGAAGGTCTCTACTCAGAAATCGTCCGCAATC
CGGTCTTCTTTGATGATTTGAGACGGAAGAACAGATTATTGTTGCAGGACCAAGTACCCTATCAGCCCTTCTTAA
CTCCCTATCAGTTGGTTTCAAGACCCTTAATATCCAAAAGAGTGCCGACCATATCAGCAAGACTCTTGCCAGTGTC
AAGACCGAGTTTGGCAAGTTTGGTGGTATTCTGGTCAAGGCACAAAAACATCTCCAACATGCCTCTGGCAATATTG
ATGAATTATTAAACCGTCGTACCATAGCTATCGAGCGGACGCTCCGTCACATTGAGTTGTCAGAAGGTGAGCCTGC
GCTTGATCTACTCCATTTTCAAGAAAATGAGGAAGAATATGAAGATTAG

4164.3
ATGAAGATTAGTCACATGAAAAAAGATGAGTTATTTGAAGGCTTTTACCTAATCAAATCAGCTGACCTGAGGCAA
ACTCGAGCTGGGAAAAACTACCTAGCCTTTACCTTCCAAGATGATAGTGGCGAGATTGATGGAAGCTCTGGGAT
GCCCAACCTCATAACATTGAGGCCTTTACCGCAGGTAAGGTTGTCCACATGAAAGGACGCCGAGAAGTTTATAAC
AATACCCCTCAAGTCAATCAAATTACTCTCCGCCTGCCTCAAGCTGGTGAACCCAATGACCCAGCTGATTTCAAGG
TCAAGTCACCAGTTGATGTCAAGGAAATTCGTGACTACATGTCGCAAATGATTTTCAAAATTGAAAATCCTGTCTG
GCAACGGATTGTCCGAAATCTCTACACCAAGTATGATAAGGAATTCTACTCCTATCCAGCTGCCAAGACCAACCA
CCATGCCTTTGAAACGGGCTTGGCCTATCATACGGCGACCATGGTGCGTTTGGCAGACGCTATTAGCGAAGTTTAT
CCTCAGCTCAATAAGAGCCTGCTCTATGCGGGGATTATGTTGCATGACTTAGCTAAGGTCATCGAGTTGACGGGGC
CAGACCAGACAGAGTACACAGTGCGAGGTAATCTTCTTGGACATATCGCTCTCATTGATAGCGAAATTACCAAGA
CAGTTATGGAACTCGGCATCGATGATACCAAGGAAGAAGTCGTTTTGCTTCGTCATGTCATCCTCAGTCACCACGG
CTTGCTTGAGTATGGAAGCCCAGTCCGTCCACGCATTATGGAAGCAGAGATTATCCATATGATTGACAATCTGGAT
GCAAGCATGATGATGATGTCAACAGCTCTTGCTTTGGTGGATAAAGGAGAGATGACCAATAAAATCTTCGCTATG
GATAATCGTTCCTTCTATAAACCAGATTTAGATTAA

4166.2
ATGAGTGAAAAAGCTAAAAAAGGGTTTAAGATGCCTTCATCTTACACCGTATTATTGATAATCATTGCTATTATGG
CAGTGCTAACTTGGTTTATCCCTGCGGGGGCCTTTATAGAAGGTATTTACGAGACTCAGCCTCAAAATCCACAAGG
GATTTGGGATGTCCTCATGGCACCGATTCGGGCTATGCTAGGTACTCATCCAGAGGAAGGTTCGCTCATTAAAGAA
ACGAGCGCAGCGATTGATGTAGCCTTCTTCATCCTTATGGTTGGTGGTTTCCTTGGCATTGTCAACAAAACTGGTG
CTCTTGACGTAGGGATTGCCTCTATCGTGAAGAAGTATAAGGGCCGCGAAAAAATGTTAATTTTGGTACTGATGCC
TTTGTTTGCCCTCGGTGGTACAACTTATGGTATGGGTGAAGAAACAATGGCCTTCTATCCACTCCTTGTGCCAGTT
ATGATGGCCGTTGGTTTTGATAGCCTGACTGGTGTTGCAATTATTTTGCTCGGTTCTCAAATCGGCTGTTTGGCATC
TACTCTGAATCCATTTGCGACAGGTATTGCTTCAGCGACTGCGGGAGTTGGTACAGGGGACGGTATCGTACTTCGT
CTGATCTTCTGGGTTACCTTGACTGCTCTTAGTACTTGGTTTGTTTACCGTTATGCGGATAAGATTCAAAAAGATCC
GACTAAGTCACTGGTTTATAGTACTCGCAAAGAAGATTTGAAACACTTTAACGTAGAAGAATCTTCATCTGTAGAA
TCTACACTTAGCAGCAAACAAAAATCAGTTCTCTTCTTATTTGTGTTGACATTCATCTTGATGGTATTGAGCTTCAT
TCCATGGACAGACCTTGGCGTTACCATTTTTGATGACTTTAATACTTGGTTGACTGGTCTTCCAGTTATTGGTAATA
TTGTCGGTTCATCTACTTCTGCACTAGGTACTTGGTACTTCCCAGAAGGCGCAATGCTCTTTGCCTTTATGGGTATC
CTGATTGGTGTTATTTATGGTCTTAAAGAAGATAAGATTATCTCTTCCTTCATGAATGGTGCTGCTGACTTGCTCAG
TGTTGCCTTGATCGTAGCGATTGCTCGTGGTATTCAAGTTATCATGAACGACGGTATGATTACCGATACAATCCTC
AACTGGGGTAAAGAAGGCTTGAGCGGTCTATCTTCACAAGTCTTTATCGTTGTAACTTATATCTTCTATCTACCTAT
GTCATTCTTGATCCCATCTTCATCTGGTCTTGCCAGCGCAACTATGGGTATCATGGC
TCCACTTGGAGAATTTGTAAATGTCCGTCCTAGCTTGATTATCACTGCTTACCAATCTGCTTCAGGTGTCTTGAACT
TGATTGCACCAACATCTGGTATTGTGATGGGAGCTCTTGCACTTGGACGTATCAACATTGGTACTTGGTGGAAATT
CATGGGCAAACTCGTAGTCGCTATTATTGTAGTGACCATCGCCCTTCTTCTCCTTGGAACCTTCCTTCCATTCCTAT
AA

4166.3
ATGAAAATAGATATAACAAATCAAGTTAAAGATGAATTTCTTATATCATTAAAAACCTTGATTTCCTATCCTTCAG
TACTCAATGAAGGAGAAAATGGAACACCTTTTGGACAAGCAATCCAAGATGTCCTAGAAAAAACTTTAGAGATTT
GTCGAGACATAGGTTTCACTACCTATCTTGACCCTAAAGGTTATTACGGATATGCAGAAATCGGTCAGGGAGCAG

AGCTTCTGGCCATTCTCTGTCATTTGGATGTTGTTCCATCAGGTGATGAAGCAGATTGGCAGACACCGCCATTTGA
AGCAACTATCAAAGACGGCTGGGTATTCGGACGTGGTGTCCAAGATGATAAAGGCCCTTCGCTCGCAGCTCTCTA
TGCAGTAAAAAGCTTGCTGGACCAAGGTATTCAGTTCAAAAAGCGCGTACGCTTTATCTTTGGTACCGATGAGGA
AACCCTCTGGCGCTGCATGGCACGCTACAATACCATCGAAGAACAGGCCAGTATGGGCTTTGCACCTGACTCATC
TTTTCCTCTGACCTATGCTGAAAAAGGGCTTCTACAGGTCAAACTTCATGGCCCTGGATCGGATCAACTAGAGCTT
GAAGTAGGAGGCGCCTTTAACGTTGTACCAGACAAGGCCAACTACCAAGGTCTCCTCTATGAACAGGTTTGTAAC
GGTCTCAAAGAAGCTGGTTATGATTACCAAACCACTGAACAAACCGTAACGGTTCTCGGAGTGCCAAAGCATGCT
AAGGATGCTAGTCAAGGTATCAATGCTGTCATCCGACTAGCTACCATTCTTGCTCCTCTCCAAGAACACCCTGCTC
TCAGTTTTCTTGCAACACAAGCAGGTCAAGACGGCACAGGAAGACAAATCTTTGGTGATATAGCAGATGAACCTT
CTGGTCACCTATCCTTTAATGTCGCAGGTCTCATGATCAATCATGAACGTTCTGAAATCCGTATTGACATTCGGAC
TCCTGTCTTAGCTGACAAGGAAGAACTAGTAGAGTTGCTTACAAGATGTGCACAAAACTACCAACTCCGCTACGA
AGAGTTTGACTATCTAGCGCCTCTATACGTCGCAGAAGACAGTAAACTCGTTAGCACACTGATGCAAATCTACCA
AGAAAAGACTGGCGATAACAGTCCTGCTATTTCATCCGGTGGTGCCACTTTTGCTCGCACCATGCCAAATTGTGTA
GCCTTCGGCGCCTTATTCCCAGGAGCGAAGCAGACAGAACATCAGGCAAATGAATGTGCCGTTCTAGAAGATTTG
TACCGTGCTATGGATATTTATGCCGAAGCCGTCTATCGACTTGCAACTTAA

4169.1
ATGTCTAATTCATTTGTCAAGTTGTTAGTCTCTCAATTATTTGCAAATTTAGCAGATATTTTCTTTAGAGTAACAAT
CATTGCTAACATATACATTATTTCAAAATCAGTAATTGCCACATCACTAGTTCCTATCTTAATAGGAATATCCTCTT
TTGTTGCGAGTCTTTTAGTTCCGTTGGTTACTAAAAGGTTAGCGCTAAATAGGGTTTTATCTTTATCTCAATTTGGA
AAGACTATATTATTGGCGATACTGGTAGGAATGTTTACCGTAATGCAATCCGTAGCGCCTTTGGTGACCTATCTAT
TTGTTGTTGCAATTTCCATACTAGATGGTTTTGCAGCACCCGTTTCCTATGCTATTGTGCCACGCTATGCGACCGAT
TTGGGTAAGGCTAATTCAGCCTTATCAATGACTGGTGAAGCTGTTCAATTGATAGGTTGGGGATTAGGTGGACTCT
TGTTTGCAACAATTGGTCTGTTACCTACCACGTGTATCAATTTAGTCTTGTATATCATTTCTAGCTTTCTGATGTTA
TTTCTTCCTAACGCTGAAGTGGAGGTGTTAGAGTCAGAAACTAATCTTGAAATTTTGCTCAAAGGTTGGAAGTTAG
TTGCTAGAAATCCTAGATTAAGACTTTTTGTATCAGCAAATTTATTGGAAATTTTTCAAATACGATTTGGGTTTCT
TCCATTATACTTGTTTTTGTAACGGAGTTATTAAATAAAACGGAAAGTTACTGGGGATATTCTAATACAGCATACT
CTATTGGTATTATAATTAGTGGCTTAATTGCTTTTAGGCTATCTGAAAAGTTCCTTGCTGCTAAATGGGAACCCCA
ATTATTCACCCCAAATCTAAAAACCATCCAGAATCCTTGCCTTAGCTTAGATCCTGGATGGTTTCTTTTTTCACCCA
ATGGGTGTTTTTTACTAGACAAAAAAGAGTTTCCCCTTTATGGTATAAGTGTAGAAAAAAACACAAAAAGAAAGG
AAACTCACATGAACAGTTTACCAAATCATCACTTCCAAAACAAGTCTTTTTACCAACTATCTTTCGATGGAGGTCA
TTTAACCCAGTATGGTGGTCTTATCTTTTTTCAGGAACTTTTTTCCCAGTTGAAACTAAAAGAGCGGATTTCTAAGT
ATTTAGTAACGAATGACCAACGCCGCTACTGTCGTTATTCGGATTCAGATATCCTTGTCCAGTTCCTCTTTCAACTG
TTAACAGGTTATGGAACGGACTATGCTTGTAAAGAATTGTCAGCTGATGCCTACTT
TCCAAAAATTGTTGGAAGGAGGGCAGCTTGCTTCACAGCCAACCTTATCCCGTTTTCTTTCCAGAACTGACGAGGAA
ACAGTCCATAGTTTGCGATGCCTCAACCTTGAATTGGTCGAATTCTTTTTACAGTTTCACCAGCTAAACCAACTCA
TTGTAGATATCGATTCTACCCATTTCACAACTTATGGCAAGCAAGAAGGTGTTGCTTATAACGCCCACTATCGTGC
TCATGGCTATCATCCTCTTTATGCTTTCGAGGGGAAGACAGGTTATTGTTTCAATGCCCAGCTTCGTCCTGGTAATC
GTTATTGTTCTGAAGAGGCAGACAGCTTTATCACACCTGTTTTAGAACGGTTTAATCAACTTCTCTTTCGAATGGA
TAGTGGCTTTGCGACCCCAAAATTATACGATTTAATTGAAAAAACAGGGCAATACTACCTCATAAAACTCAAGAA
AAATACTGTTCTGAGCCGTCTTGGAGACCTTTCCCTCCCTTGCCCACAGGATGAGGACTTAACCATCTTGCCCCAC
TCCGCCTACTCAGAAACTCTCTATCAAGCAGGATCTTGGTCGCACAAGCGTCGTGTCTGCCAGTTCTCTGAACGAA
AAGAAGGAAACTTGTTCTACGATGTTATTTCTCTCGTTACAAATATGACGAGTGGAACAAGCCAAGACCAGTTTCA
GCTTTATCGTGGACGTGGTCAAGCCGAGAATTTCATCAAGGAGATGAAGGAGGGATTTTTTGGCGATAAAACGGA
TAGTTCAACCTTAATCAAAAACGAAGTTCGTATGATGATGAGCTGTATCGCCTACAATCTCTATCTTTTTCTCAAA
CATCTAGCTGGAGGTGACTTCCAAACTTTAACAATCAAACGCTTCCGCCATCTTTTTCTTCACGTGGTGGGAAAAT
GTGTTCGAACAGGACGCAAGCAGCTCCTCAAATTGTCTAGTCTCTATGCCTATTCCGAATTGTTTTCAGCACTTTA
TTCTAGGATTAGAAAAGTCAACCTGAATCTTCCTGTTCCTTATGAACCACCTAGAAGAAAAGCGTCGTTAATGATG
CATTAA

4169.3
ATGATGGAGTTTTTTCAACAGCTTCCTCATTTAGAGCCATATGGCAATCCTCAGTATTTTGTTTATGTGATTGCTGC
AACCTTGCCCATCTTTATAGGTCTCTTTTTCAAGAAACGCTTTGCCTGGTATGAAGTGTTGGTAAGTCTCTTCTTTA
TTGTCACCATGTTGGTGGGTGGAAAGACCAATCAACTAGCTGCCTTGGGTATTTACCTTTGCTGGGAAATATTGCT
CCTGCTTTTCTACAAGCATTATCGAAAAAGCAAGGATGGCAAGTGGGTCTTCTACTTAGTTAGTTTTCTGTCCCTA
CTTCCGATTATCTTTGTCAAGGTGCAACCAGCTATCAATGGAACGCAGTCTTTGCTTGGGTTCTTGGGAATTTCTTA
CCTGACCTTTCGTTCGGTTGGAATTGTCATCGAGCTGAGAGATGGAGTGATTAAGGATTTTACCCTCTGGGAATTC
CTCCGTTTCCTTCTCTTCATGCCAACTTTCTCGAGTGGTCCAATCGATCGCTTTAAGCGATTTAATGAAAATTATCA
GGCTATTCCTGAGCGAGATGAGTTGATGGATATGCTGGATGAATCTGTCCGCTATATCATGTGGGGCTTTTTGTAT
AAGTTTATCCTAGCTCATGTTTTAGGAGAGACCTTACTACCTCCTCTGAAGAATTTAGCCTTGCAGTCAGGTGGCT
TCTTTAATCTCTATGCCTTGGCAGTTATGTATACTTTGGTCTGGAACTCTTCTTTGACTTTGCAGGTTATTCTATGT
TTGCTTTGGCCATCTCAAACTTGATGGGAATCCGTAGCCCTATCAACTTTAACAAGCCCTTTTTATCAAGGGATTT
AAAGGAGTTTTGGAATCGCTGGCATATGAGTCTGTCCTTCTGGTTCCGTGACTTTGTCTTTATGCGAATGGTGATG
GTGTTAACCAGAAAGAAAGTCTTTAAAAATCGTAATGTAACCTCAAGCATGGCCTACATTGTAAATATGCTGATTA
TGGGATTTTGGCATGGTGTGACCTGGTACTATATCGCCTATGGACTCTTTCATGGACTAGGCTTGGTCATCAATGA

TGCCTGGGTTCGCAAGAAAAAAACGCTCAATAAGGAACGGAAAAAAGCAGGGAAGGCTGCCCTACCTGAGAATC
GCTGGATTCAGTTGCTTGGCATGGTTGTCACTTTCCATGTTGTCATGTTGTCATTCTTAATCTTTTCTGGATTCTTGA
ATAATCTATGGTTTAAAAAATAA

4169.4
ATGCTTAAACGCTTATGGATGATCTTCGGACCGGTCTTGATCGCTGGTTTGTTGGTTTTTCTGCTCATTTTCTTTTAT
CCTACTGAGATGCATCATAATCTAGGAGCTGAAAAGCGTTCAGCAGTGGCTACTACTATCGATAGTTTTAAGGAGC
GAAGTCAAAAAGTCAGAGCACTATCTGATCCAAATGTGCGTTTTGTTCCCTTCTTTGGCTCTAGTGAATGGCTTCG
TTTTGACGGTGCTCATCCTGCGGTATTAGCTGAGAAATACAATCGTTCCTACCGTCCTTATCTTTTAGGACAGGGG
GGAGCTGCATCGCTTAACCAATATTTTGGAATGCAACAGATGTTACCACAGCTGGAGAATAAACAAGTTGTGTAT
GTTATCTCACCTCAGTGGTTCAGTAAAAATGGCTATGATCCAGCAGCCTTCCAGCAGTATTTTAATGGAGACCAGT
TGACTAGTTTTCTGAAACATCAATCTGGGGATCAGGCTAGTCAATATGCAGCGACTCGCTTACTGCAACAGTTCCC
AAACGTAGCTATGAAGGACCTGGTTCAGAAGTTGGCAAGTAAAGAAGAATTGTCGACAGCAGACAATGAAATGAT
TGAATTATTGGCTCGTTTTAATGAACGCCAAGCTTCCTTTTTTGGTCAGTTTTCGGTTAGAGGCTATGTTAACTACG
ATAAGCATGTAGCTAAGTATTTAAAAATCTTGCCAGACCAGTTTTCTTATCAGGCAATAGAAGATGTTGTCAAAGC
AGATGCTGAAAAAAATACTTCCAATAATGAGATGGGAATGGAAAATTATTTCTATAATGAGCAGATCAAGAAGGA
TTTGAAGAAATTAAAGGATTCTCAGAAAAGCTTTACCTATCTCAAGTCGCCAGAGTATAATGACTTGCAGTTGGTT
TTAACACAGTTTTCTAAATCTAAGGTAAACCCGATTTTTATCATTCCACCTGTTAATAAAAAATGGATGAACTATG
CTGGTCTACGAGAGGATATGTACCAACAAACGGTGCAGAAGATTCGCTACCAGTTAGAAAGTCAAGGTTTTACCA
ATATAGCAGATTTTTCTAAGGACGGCGGGGAGCCTTTCTTTATGAAGGACACCATTCACCTTGGTTGGTTGGGTTG
GTTGGCTTTTGACAAGGCAGTTGATCCTTTCCTATCCAATCCCACACCAGCTCCGACTTACCATCTGAATGAGCGC
TTTTTCAGCAAAGATTGGGCGACTTATGATGGAGATGTCAAAGAATTTCAATAG

4169.6
ATGGAGAAAAACCTCAAGGCTTTGAAACAAACAACAGACCAAGAAGGCCCAGCAATTGAACCTGAAAAGGCAGA
GGATACCAAGACAGTCCAAAATGGTTACTTCGAGGATGCAGCTGTCAAGGACCGCACCTTGAGTGACTATGCAGG
TAACTGGCAATCAGTTTATCCTTTCCTTGAAGACGGCACGTTTGACCAAGTCTTTGACTACAAGGCTAAGTTGACT
GGTAAGATGACCCAGGCTGAGTACAAGGCTTACTATACAAAAGGCTATCATACAGATGTGACTAAGATTAACATT
ACTGATAATACTATGGAATTTGTTCAAGGTGGACAAAGCAAGAAATACACTTACAAGTATGTCGGTAAGAAAATT
TTGACTTACAAGAAAGGCAATCGTGGCGTGCGTTTCCTCTTTGAAGCCACAGATGCTGACGCTGGACAATTCAAGT
ATGTTCAGTTTAGTGACCACAATGTTGCCCCAGTTAAGGCAGAACATTTCCATATCTTCTTTGGAGGCACAAGCCA
AGAAGCCCTCTTTGAAGAAATGGACAACTGGCCAACCTACTACCCAGATAACCTATCTGGCCAAGAAATCGCCCA
AGAAATGTTGGCGCATTGA

4170.3
ATGAAAGATGGTCATTTGCTAGCCCATCATATTCGTTTGTTGAATGGGCGGATTTTTCAAAAGTTACTGAGTCAAG
ATCCTGAGGCTCTTTATAGGGGTGAACAGGGCAAGATTTTAGCGGTTTTATGGAATAGTGAAACTGGCTGCGCAA
CTGCGACAGATATCGCGCTTGCGACTGGACTTGCGAATAATACGCTGACGACTATGATAAAAAAGCTAGAGGAAC
AAAAGCTTGTAATTGTTAGTCCGTGTGGAAAAGACAAGCGTAAGAAGTATTTAGTTTTAACGGAGTTAGGCAAGT
CCCAGAAAGAAGTGGGGCATCGTGTCAGTCAGAAATTGGATACTATCTTTTACAAAGGATTTTCAGAGGAAGAAA
TTCACCAATTTGAAGGTTTTCAAGAAAGAATTTTGGCGAATCTGAAAGAGAAGGGAAATGAGGTTTAG

4170.4
ATGACTAATTTAATTGCAACTTTTCAGGATCGTTTTAGTGATTGGTTGACAGCTCTATCTCAACATTTGCAGTTGTC
GCTTTTGACCTTGTTACTAGCTATTTTGCTTGCGATTCCCTTGGCTGTTTTTCTTCGCTATCATGAGAAGCTGGCCG
ACTGGGTCTTGCAGATTGCAGGTATTTTCCAGACCATCCCGTCTCTGGCCTTGTTGGGGCTCTTTATCCCTTTGATG
GGAATTGGGACCTTGCCGGCTTTGACAGCTCTAGTGATTTATGCGATTTTCCCTATTTTGCAAAATACTATCACTG
GGCTGAAGGGAATTGATCCGAACCTGCAAGAGGCTGGGATTGCCTTTGGGATGACCAGATGGGAACGTCTCAAGA
AATTTGAAATTCCACTCGCCATGCCTGTTATCATGTCTGGGATTCGGACGGCAGCTGTTTTGATTATCGGTACGGC
AACCTTGGCGGCCTTGATTGGTGCAGGGGGACTAGGTTCCTTTATTCTTTTGGGAATTGACCGTAATAATGCCAGT
TTGATTTTGATTGGGGCACTTTCTTCTGCAGTGCTAGCCATTGCCTTTAACTTCCTACTAAAAGTGATGGAAAAAG
CAAAATTACGGACGATTTTCTCAGGTTTTGCCTTGGTGGCTTTATTACTGGGTCTGTCTTATAGTCCAGCTCTTTTG
GTTCAAAAAGAGAAGGAAAACTTGGTTATTGCTGGGAAAATAGGTCCAGAACCAGAAATTTTGGCCAATATGTAT
AAGTTGCTGATTGAAGAAAATACCAGCATGACTGCGACTGTTAAACCGAATTTTGGGAAGACAAGCTTCCTTTATG
AAGCTCTGAAAAAAGGCGATATTGACATCTATCCTGAATTTACTGGTACGGTGACTGAAAGTTTGCTTCAACCATC
ACCCAAGGTGAGTCATGAACCAGAACAGGTTTATCAGGTGGCGCGTGATGGCATTGCTAAGCAGGATCATCTAGC
CTATCTCAAACCCATGTCTTATCAAAACACCTATGCTGTAGCTGTTCCGAAAAAGATTGCTCAAGAATATGGCTTG
AAGACCATTTCAGACTTGAAAAAAGTGGAAGGGCAGTTGAAGGCAGGTTTTACACTCGAGTTTAACGACCGTGAA
GATGGAAATAAGGGCTTGCAATCAATGTATGGTCTCAATCTCAATGTAGCGACCATTGAGCCAGCCCTTCGCTATC
AGGCTATTCAGTCAGGGGATATTCAAATCACGGATGCCTATTCGACTGATGCGGAATTGGAGCGTTATGATTTACA
GGTCTTGGAAGATGACAAGCAACTCTTCCCACCTTATCAAGGGGCTCCACTCATGAAAGAAGCTCTTCTCAAGAA
ACACCCAGAGTTGGAAAGAGTTCTTAATACATTGGCTGGTAAGATTACAGAAAGCCAGATGAGCCAGCTCAACTA
CCAAGTCGGTGTTGAAGGCAAGTCAGCAAAGCAAGTAGCCAAGGAGTTTCTCCAAGAACAAGGTTTGTTGAAGAA
ATGA

4170.5
ATGATGCATACTTATTTGCAAAAGAAAATTGAAAATATCAAAACAACCCTAGGTGAAATGTCAGGTGGTTACCGT
CGTATGGTTGCGGCTATGGCTGATTTAGGATTTTCAGGAACTATGAAGGCTATCTGGGATGACCTCTTTGCCCATC
GTAGTTTTGCCCAGTGGATTTATTTGCTGGTTTTAGGAAGTTTTCCTCTCTGGCTGGAGTTGGTTTACGAACATCGT
ATTGTTGACTGGATTGGGATGATTTGTAGCTTGACAGGGATTATCTGTGTAATCTTTGTATCGGAAGGTCGAGCAA
GTAATTATCTTTTTGGCTTGATTAACTCTGTTATTTACCTTATTTTGGCCCTACAGAAAGGCTTTTATGGTGAGGTG
CTGACGACACTTTACTTCACAGTCATGCAGCCAATTGGACTTCTAGTTTGGATTTATCAGGCACAGTTTAAGAAGG
AAAAGCAGGAGTTTGTCGCGCGTAAACTGGACGGCAAGGGCTGGACAAAGTATCTTTCCATTAGTGTGCTTTGGT
GGTTGGCCTTTGGCTTCATTTATCAGTCTATTGGTGCCAATCGTCCCTATCGTGATTCAATCACAGATGCAACCAA
TGGGGTAGGGCAAATCCTCATGACAGCTGTTTACCGTGAACAGTGGATATTCTGGGCGGCTACCAATGTCTTTTCA
ATCTATCTCTGGTGGGGAGAAAGCCTGCAAATTCAAGGGAAATATCTAATTTATCTCATTAACAGTCTAGTTGGTT
GGTATCAATGGAGCAAGGCAGCTAAGCAGAATACTGATTTACTTAACTAG

4170.6
ATGAGAAATATGAAGGCAAAATATGCTGTTTGGGTGGCTTTTTTCTTAAATTTGACTTATGCCATTGTTGAGTTTAT
TGCAGGTGGAGTATTTGGTTCTAGCGCTGTTCTTGCTGACTCTGTGCATGACTTGGGAGATGCGATTGCAATTGGA
ATATCAGCTTTTCTAGAAACAATCTCCAATCGTGAAGAAGACAATCAGTACACCTTGGGCTATAAGCGGTTTAGCC
TGCTAGGAGCCTTGGTAACAGCTGTGATTCTCGTAACGGGCTCTGTTCTAGTCATTTTGGAAAATGTCACGAAGAT
TTTGCATCCGCAACCAGTCAATGATGAGGGGATTCTCTGGTTAGGAATTATTGCGATTACTATCAATCTGTTAGCG
AGTCTGGTGGTTGGTAAGGGAAAGACAAAGAATGAGTCTATTCTGAGTCTGCATTTTCTGGAAGATACGCTAGGG
TGGGTAGCTGTTATCCTGATGGCGATTGTTCTTCGATTTACGGACTGGTATATCCTAGATCCTCTTTTGTCCCTTGT
CATTTCTTTCTTTATTCTTTCAAAAGCCCTTCCACGTTTTTGGTCTACACTCAAGATTTTCTTGGATGCTGTGCCAG
AAGGTCTTGATATCAAGCAAGTAAAGAGTGGCCTGGAGCGATTGGACAATGTGGCCAGCCTTAATCAGCTTAATC
TCTGGACTATGGATGCTTTGGAAAAAAATGCCATTGTCCATGTTTGTCTAAAAGAAATGGAACATATGGAAACTTG
TAAAGAGTCTATTCGAATTTTCCTAAAAGATTGTGGTTTTCAAAATATTACCATTGAAATTGATGCTGACCTAGAA
ACTCACCAAACCCATAAGCGAAAGGTGTGTGACTTGGAACGGAGTTATGAGCATCAACATTAG

4170.8
ATGATTGAATACAAAAATGTAGCACTGCGCTACACAGAAAAGGATGTCTTGAGAGATGTCAACTTACAGATTGAG
GATGGGGAATTTATGGTTTTAGTAGGGCCTTCTGGGTCAGGTAAGACGACCATGCTCAAGATGATTAACCGTCTTT
TGGAACCAACTGATGGAAATATTTATATGGATGGAAGCGCATCAAAGACTATGATGAGCGTGAACTTCGTCTTT
CTACTGGTTATGTTTTACAGGCTATTGCTCTTTTTCCAAATCTAACAGTTGCGGAAAATATTGCTCTCATTCCTGAA
ATGAAGGGGTGGAGCAAGGAAGAAATTACGAAGAAAACAGAAGAGCTTTTGGCTAAGGTTGGTTTACCAGTAGCC
GAGTATGGGCATCGCTTACCTAGTGAATTATCTGGTGGAGAACAGCAACGGGTCGGTATTGTCCGAGCTATGATTG
GTCAGCCCAAGATTTTCCTCATGGATGAACCCTTTTCGGCCTTGGATGCTATTTCGAGAAAACAGTTGCAGGTTCT
GACAAAAGAATTGCATAAAGAGTTTGGGATGACAACGATTTTTGTAACCCATGATACGGATGAAGCCTTGAAGTT
GGCGGACCGTATTGCTGTCTTGCAGGATGGAGAAATTCGCCAGGTAGCGAATCCCGAGACAATTTTAAAAGCGCC
TGCAACAGACTTTGTAGCAGACTTGTTTGGAGGTAGTGTTCATGACTAA

4171.1
ATGTCAGCAGTTGCTATTTCAGCTATGACCAAGGTTATGCAAGAAACCCACGGAAATCCTTCTAGTATTCATGGTC
ATGGTCGTCAAGCTGGCAAACTCTTGCGAGAAGCCCGTCAGGAACTAGCCCAGTTACTAAGGACAAAACCTCAAC
ATATCTTTTTCACTTCTGGTGGGACTGAAGGCAATAATACTACCATCATTGGCTACTGTCTTCGTCACCAAGAACA
AGGAAAAACATATCATCACAACTGCCATCGAGCACCATGCTGTCCTTGAAACAATTGATTACTTGGTTCAACACTTT
GGGTTTGAAGCAACCATTATCCAGCCAGAAAATCAAGAAATCACAGCCCAGCAAATTCAAAAGGCTTTACGTGAC
GATACGATTTTGGTTTCTACCATGTTTGTCAATAATGAGACAGGAAACCTACTGCCCATCGCTGAAATTGGCCAAA
TACTCAAGCAACACCCTGCTGCCTATCATGTTGATGCAGTTCAGGCTATTGGTAAAATCCCAATTCATTCAGAAGA
ATTGGGCATTGATTTTCTCACTGCTTCTGCCCACAAATTCCATGGTCCTAAGGGAATCGGTTTTCTCTACGCATCTA
GCATGGACTTTGATTCCTATCTACATGGCGGAGACCAGGAACAGAAAAAACGTGCAGGAACTGAAAATCTGCCTG
CCATTGTAGGCATGGTTGCAGCCCTAAAAGAAGACCTAGAAAAACAAGAAGAACATTTTCAACATGTACAAAATC
TAGAAACTGCCTTTCTGGCAGAGCTGGAGGGCATTCAGTATTACCTGAATAGAGGAAAACATCATCTCCCTTATGT
TCTCAATATTGGATTTCCTGGTCAGAAAAATGACCTCTTACTCCTTCGGCTAGATTTAGCTGGAATTTCAATCTCTA
CTGGCTCAGCCTGTACTGCAGGCGTTGTCCAATCCAGCCATGTTCTTGAAGCCATGTATGGCGCAAATTCAGAACG
CTTGAAGGAATCCCTTCGCATCAGTTTGTCGCCACAAAATACCGTTGAAGACCTACAAACCCTCGCAAAAACCTTA
AAAGAAATTATCGGAGGTTAG

4172.1
ATGTTATTCAAATTATCTAAGGAAAAAATAGAGCTAGGCTTATCTCGTTTATCGCCAGCCCGTCGTATTTTTTTGA
GTTTTGCCTTGGTCATTTTACTAGGCTCTCTTCTTTTGAGCTTGCCCTTTGTCCAAGTTGAAAGCTCACGAGCGACT
TATTTTGATCATCTTTTCACTGCTGTCTCTGCAGTCTGTGTGACGGGTCTCTCAACCCTTCCAGTAGCTCACACCTA
TAATATCTGGGGTCAAATAATCTGTTTGCTCTTGATTCAGATCGGTGGTCTAGGGCTCATGACCTTTATTGGGGTTT
TCTATATCCAGAGCAAGCAAAAGCTTAGTCTTCGTAGCCGTGCAACTATTCAGGATAGTTTTAGTTATGGAGAAAC

TCGATCTTTGAGAAAGTTTGTCTATTCTATTTTTCTCACGACCTTTTTGGTTGAGAGCTTGGGAGCTATTTTGCTTA
GTTTTCGCCTTATTCCTCAACTTGGCTGGGGACGTGGTCTTTTTAGTTCCATTTTTCTAGCGATCTCAGCCTTCTGT
AATGCCGGTTTTGATAATTTAGGGAGCACCAGTTTATTTGCTTTTCAGACCGATTTACTGGTCAATCTGGTGATTGC
AGGCTTGATTATTACAGGCGGCCTTGGTTTTATGGTCTGGTTTGATTTGGCTGGTCATGTAGGAAGAAAGAAAAAA
GGACGTCTGCACTTTCATACGAAGCTTGTACTATTATTGACTATAGGTTTGTTGTTATTTGGAACAGCAACTACTCT
CTTTCTTGAGTGGAACAATGCTGGAACGATTGGCAATCTCCCTGTTGCCGATAAGGTTTTAGTTAGCTTTTTTCAA
ACAGTGACGATGCGAACAGCTGGCTTTTCTACGATAGATTATACTCAGGCTCATCCTGTGACTCTTTTGATTTATA
TCTTACAGATGTTTCTAGGTGGGGCACCTGGAGGAACAGCTGGGGGACTCAAGATTACGACATTTTTTGTCCTCTT
GGTCTTTGCACGAAGTGAGCTTCTAGGCTTGCCTCATGCCAATGTTGCGAGACGAACGATCGCGCCGCGAACGGTT
CAAAAATCCTTTAGTGTCTTTATTATCTTTTTGATGAGCTTCTTGATAGGATTGATTCTGCTAGGGATAACAGCCAA
AGGCAATCCTCCCTTTATCCACCTCGTATTTGAAACCATTTCAGCTCTTAGTACAGTTGGTGTAACGGCAAATCTG
ACTCCTGACCTTGGGAAATTGGCTCTCAGTGTTATCATGCCACTTATGTTTATGGG
ACGAATTGGTCCCTTGACCTTGTTTGTTAGCTTGGCAGATTACCATCCAGAAAAGAAAGATATGATTCACTATATG
AAAGCAGATATTAGTATTGGTTAA

4172.2
ATGTCAGATCGTACGATTGGAATTTTGGGCTTGGGAATTTTTGGGAGCAGTGTCCTAGCTGCCCTAGCCAAGCAGG
ATATGAATATTATCGCTATTGATGACCACGCAGAGCGCATCAATCAGTTTGAGCCAGTTTTGGCGCGTGGAGTGAT
TGGTGACATCACAGATGAAGAATTATTGAGATCAGCAGGGATTGATACCTGCGATACCGTTGTAGTCGCGACAGG
TGAAAATCTGGAGTCGAGTGTGCTTGCGGTTATGCACTGTAAGAGTTTGGGGGTACCGACTGTTATTGCTAAGGTC
AAAAGTCAGACCGCTAAGAAAGTGCTAGAAAAGATTGGAGCTGACTCGGTTATCTCGCCAGAGTATGAAATGGGG
CAGTCTCTAGCACAGACCATTCTTTTCCATAATAGTGTTGATGTCTTTCAGTTGGATAAAAATGTGTCTATCGTGG
AGATGAAAATTCCTCAGTCTTGGGCAGGTCAAAGTCTGAGTAAATTAGACCTCCGTGGCAAATACAATCTGAATA
TTTTGGGTTTCCGAGAGCAGGAAAATTCCCCATTGGATGTTGAATTTGGACCAGATGACCTCTTGAAAGCAGATAC
CTATATTTTGGCAGTCATCAACAACCAGTATTTGGATACCCTAGTAGCATTGAATTCGTAA

4172.3
ATGAAGTTATTGTCTATCGCAATTTCTAGCTATAATGCAGCAGCCTATCTTCATTACTGTGTGGAGTCGCTAGTGA
TTGGTGGTGAGCAAGTTGGGATTTTGATTATCAATGACGGGTCTCAGGATCAGACTCAGGAAATCGCTGAGTGTTT
AGCTAGCAAGTATCCTAATATCGTTAGAGCCATCTATCAGGAAAATAAATGCCATGGCGGTGCGGTCAATCGTGG
CTTGGTAGAGGCTTCTGGGCGCTATTTTAAAGTAGTTGACAGTGATGACTGGGTGGATCCTCGTGCCTACTTGAAA
ATTCTTGAAACCTTGCAGGAACTTGAGAGCAAAGGTCAAGAGGTGGATGTCTTTGTGACCAATTTTGTCTATGAAA
AGGAAGGGCAGTCTCGTAAGAAGAGTATGAGTTACGATTCAGTCTTGCCTGTTCGGCAGATTTTTGGCTGGGACCA
GGTCGGAAATTTCTCCAAAGGCCAGTATACCATGATGCACTCGCTGATTTATCGGACAGATTTGTTGCGTGCTAGC
CAGTTCTAA

4172.4
ATGAAATTCAATCCAAATCAAAGATATACTCGTTGGTCTATTCGCCGTCTCAGTGTCGGTGTTGCCTCAGTTGTTG
TGGCTAGTGGCTTCTTTGTCCTAGTTGGTCAGCCAAGTTCTGTACGTGCCGATGGGCTCAATCCAACCCCAGGTCA
AGTCTTACCTGAAGAGACATCGGGAACGAAAGAGGGTGACTTATCAGAAAAACCAGGAGACACCGTTCTCACTCA
AGCGAAACCTGAGGGCGTTACTGGAAATACGAATTCACTTCCGACACCTACAGAAAGAACTGAAGTGAGCGAGGA
AACAAGCCCTTCTAGTCTGGATACACTTTTTGAAAAAGATGAAGAAGCTCAAAAAAATCCAGAGCTAACAGATGT
CTTAAAAGAAACTGTAGATACAGCTGATGTGGATGGACACAAGCAAGTCCAGCAGAAACTACTCCTGAACAAGT
AAAAGGTGGAGTGAAAGAAAATACAAAAGACAGCATCGATGTTCCTGCTGCTTATCTTGAAAAAGCTGAAGGGAA
AGGTCCTTTCACTGCCGGTGTAAACCAAGTAATTCCTTATGAACTATTCGCTGGTGATGGTATGTTAACTCGTCTA
TTACTAAAAGCTTCGGATAATGCTCCTTGGTCTGACAATGGTACTGCTAAAAATCCTGCTTTACCTCCTCTTGAAG
GATTAACAAAAGGGAAATACTTCTATGAAGTAGACTTAAATGGCAATACTGTTGGTAAACAAGGTCAAGCTTTAA
TTGATCAACTTCGCGCTAATGGTACTCAAACTTATAAAGCTACTGTTAAAGTTTACGGAAATAAAGACGGTAAAGC
TGACTTGACTAATCTAGTTGCTACTAAAAATGTAGACATCAACATCAATGGATTAGTTGCTAAAGAAACAGTTCAA
AAAGCCGTTGCAGACAACGTTAAAGACAGTATCGATGTTCCAGCAGCCTACCTAGAAAAAGCCAAGGGTGAAGGT
CCATTCACAGCAGGTGTCAACCATGTGATTCCATACGAACTCTTCGCAGGTGATGGCATGTTGACTCGTCTCTTGC
TCAAGGCATCTGACAAGGCACCATGGTCAGATAACGGCGACGCTAAAAACCCAGCCCTATCTCCACTAGGCGAAA
ACGTGAAGACCAAAGGTCAATACTTCTATCAAGTAGCCTTGGACGGAAATGTAGCTGGCAAAGAAAAACAAGCGC
TCATTGACCAGTTCCGAGCAAATGGTACTCAAACTTACAGCGCTACAGTCAATGTCTATGGTAACAAAGACGGTA
AACCAGACTTGGACAACATCGTAGCAACTAAAAAAGTCACTATTAACATAAACGGTTTAATTTCTAAAGAAACAG
TTCAAAAAGCCGTTGCAGACAACGTTAAAGACAGTATCGATGTTCCAGCAGCCTACCTAGAAAAAGCCAAGGGTG
AAGGTCCATTCACAGCAGGTGTCAACCATGTGATTCCATACGAACTCTTCGCAGGTGATGGTATGTTGACTCGTCT
CTTGCTCAAGGCATCTGACAAGGCACCATGGTCAGATAACGGTGACGCTAAAAACCCAGCCCTATCTCCACTAGG
TGAAAACGTGAAGACCAAAGGTCAATACTTCTATCAATTAGCCTTGGACGGAAATGTAGCTGGCAAAGAAAAACA
AGCGCTCATTGACCAGTTCCGAGCAAACGGTACTCAAACTTACAGCGCTACAGTCAATGTCTATGGTAACAAAGA
CGGTAAACCAGACTTGGACAACATCGTAGCAACTAAAAAAGTCACTATTAACATAAACGGTTTAATTTCTAAAGA
AACAGTTCAAAAAGCCGTTGCAGACAACGTTAAGACAGTATCGATGTTCCAGCAGCCTACCTAG

4172.5

ATGAAACTAAAAAGTTATATTTTGGTTGGATATATTATTTCAACCCTCTTAACCATTTTGGTTGTTTTTGGGCTGT
TCAAAAAATGCTGATTGCGAAAGGCGAGATTTACTTTTGCTTGGGATGACCATCGTTGCCAGCCTTGTCGGTGCT
GGGATTAGTCTCTTTCTCCTATTGCCAGTCTTTACGTCGTTGGGCAAACTCAAGGAGCATGCCAAGCGGGTAGCGG
CCAAGGATTTTCCTTCAAATTTGGAGGTTCAAGGTCCTGTAGAATTTCAGCAATTAGGGCAAACTTTTAATGAGAT
GTCCCATGATTTGCAGGTAAGCTTTGATTCCTTGGAAGAAAGCGAACGAGAAAAGGGCTTGATGATTGCCCAGTT
GTCGCATGATATTAAGACTCCTATCACTTCGATCCAAGCGACGGTAGAAGGGATTTTGGATGGGATTATCAAGGA
GTCGGAGCAAGCTCATTATCTAGCAACCATTGGACGCCAGACGGAGAGGCTCAATAAACTGGTTGAGGAGTTGAA
TTTTTTGACCCTAAACACAGCTAGAAATCAGGTGGAAACTACCAGTAAAGACAGTATTTTTCTGGACAAGCTCTTA
ATTGAGTGCATGAGTGAATTTCAGTTTTTGATTGAGCAGGAGAGAAGAGATGTCCACTTGCAGGTAATCCCAGAGT
CTGCCCGGATTGAGGGAGATTATGCTAAGCTTTCTCGTATCTTGGTGAATCTGGTCGATAACGCTTTTAAATATTC
TGCTCCAGGAACCAAGCTGGAAGTGGTGGCTAAGCTGGAGAAGGACCAGCTTTCAATCAGTGTGACCGATGAAGG
GCAGGGTATTGCCCCAGAGGATTTGGAAAATATTTTCAAACGCCTTTATCGTGTCGAAACTTCGCGTAACATGAAG
ACAGGTGGTCATGGATTAGGACTTGCGATTGCGCGTGAATTGGCCCATCAATTGGGTGGGGAAATCACAGTCAGC
AGCCAGTACGGTCTAGGAAGTACCTTTACCCTCGTTCTCAACCTCTCTGGTAGTGAAAATAAAGCCTAA

4172.6
ATGTTTGGTCAAACGGCTCAACATGGTCTTACGAATAGCCTGAAAGACTTCTGGATTTTTCTGCTGAATATAGGTC
CACAATTGGCGTTTTTTTGCCAGATGCTCCGCTGTTCCAGATCGGTTGAGCAGGGTACTGGAAATCACCGTCGTGA
GTTCAATATGATTCAGCAGATATTCTCGCATTTTGGGATGACTCACTTGGGACAAATCAAGTTGGTCTATCAAGAG
TCGATTGACCTTGAGTTGCTGGTCAATGCACTTAATCATCACTTGCTCATTGACAGACTGGTCCTCACGCCCAATC
AAATAACGATAGAAATCGACAGGCAGATAGTACATGGTCTTGACCTGCTGAAGGGGCGTAAAGACAAAGAGATTA
TCGACATAAAAAGTATGTTCAGGCAGTTAGAACTGGCTAGCACGCAACAAATCTGTCCGATAAATCAGCGAGTGC
ATCATGGTATACTGGCCTTTGGAGAAATTTCCGACCTGGTCCCAGCCAAAAATCTGCCGAACAGGCAAGACTGA

4174.1
ATGGAACATTTAGCAACTTATTTTTCAACCTATGGAGGAGCTTTCTTCGCTGCATTGGGAATTGTATTGGCGGTTG
GATTAAGCGGTATGGGGTCTGCTTATGGAGTTGGTAAGGCTGGGCAATCTGCCGCAGCTTTACTGAAAGAACAGC
CTGAAAAGTTTGCCTCAGCTTTGATATTGCAATTATTGCCCGGAACACAAGGATTATATGGTTTTGTTATTGGAAT
TTTAATTTGGTTGCAATTAACTCCAGAACTTCCTTTAGAAAAAGGCGTTGCTTATTTCTTTGTAGCTCTTCCAATTG
CTATTGTAGGATACTTTTCAGCTAAGCATCAAGGAAATGTAGCAGTAGCGGGAATGCAAATCTTGGCTAAAAGAC
CAAAAGAATTCATGAAGGGAGCAATTTTAGCTGCCATGGTAGAAACCTATGCAATTCTTGCTTTTGTCGTATCATT
CATTTTGACCCTTCGTGTATAA
4175.2
ATGTTAAAATCAGAAAAACAATCACGTTATCAAATGTTAAATGAAGAATTGTCCTTCCTATTGGAAGGCGAAACC
AATGTTTTGGCTAATCTTTCCAACGCCAGTGCTCTCATAAAATCACGTTTTCCTAATACCGTATTTGCAGGCTTTTA
TTTGTTCGATGGAAAGGAATTGGTTTTAGGCCCCTTCCAAGGAGGTGTTTCCTGCATCCGTATTGCACTAGGCAAG
GGTGTTTGTGGTGAGGCAGCTCACTTTCAGGAAACTGTTATTGTTGGAGATGTGACGACCTATCTCAACTATATTT
CTTGTGATAGTCTAGCTAAAAGTGAAATTGTGGTGCCGATGATGAAGAATGGTCAGTTACTTGGAGTTCTGGATCT
GGATTCTTCAGAGATTGAGGATTACGATGCTATGGATCGAGATTATTTGGAACAATTTGTCGCTATTTTGCTTGAA
AAGACAGCATGGGACTTTACGATGTTTGAGGAAAAATCTTAA

4175.3
ATGTCAGTATTAGAGATCAAAGATCTTCACGTTGAGATTGAAGGAAAAGAAATTTTAAAAGGGGTTAACCTGACC
CTGAAAACAGGAGAAATTGCCGCTATCATGGGACCAAATGGTACAGGTAAATCGACTCTTTCTGCCGCTATCATG
GGAAATCCAAACTATGAAGTAACTAAAGGTGAAGTTTTGTTTGATGGCGTAAACATCCTTGAGTTGGAAGTGGAT
GAGCGTGCGCGTATGGGACTTTTCCTTGCTATGCAATACCCATCAGAAATCCCTGGAATTACCAATGCTGAGTTTC
TTCGTGCCGCTATGAATGCGGGTAAAGAAGATGATGAGAAGATTTCAGTTCGTGAGTTTATTACTAAGCTAGATGA
AAAAATGGAATTGCTCAACATGAAAGAAGAAATGGCAGAGCGTTACCTCAACGAAGGCTTCTCTGGTGGTGAGAA
AAAACGCAATGAAATTCTTCAACTTTTGATGTTGGAGCCAACATTTGCTCTTTTGGACGAGATTGACTCAGGTCTT
GATATTGACGCTCTTAAAGTTGTGTCTAAAGGTGTCAATGCCATGCGTGGTGAAGGTTTTGGTGCTATGATCATCA
CTCACTACCAACGTCTTTTGAACTATATCACACCTGATGTGGTACACGTGATGATGGAAGGTCGTGTTGTCCTTTC
TGGTGGTCCAGAATTGGCTGCGCGTTTGGAACGTGAAGGATACGCAAAATTAGCTGAAGAACTTGGCTACGACTA
CAAGGAAGAATTGTAA

4174.4
ATGCCCTACAAAAGACAAAGGAGTTTTTCAATGGCACTTTCTAAACTAGATAGCCTTTATATGGCAGTGGTAGCAG
ACCATTCGAAAAATCCACATCACCAAGGGAAGTTAGAAGATGCTGAGCAAATCAGTCTCAACAATCCGACTTGTG
GGGATGTCATCAACCTCTCTGTCAAGTTTGATGCAGAGGACCGTTTGGAAGATATTGCTTTTCTAAATTCAGGATG
CACGATTTCAACTGCTTCTGCTAGTATGATGACAGATGCCGTTTTAGGAAAAACCAAACAAGAAATTTTAGAACTG
GCGACTATTTTTTCTGAAATGGTTCAAGGGCAAAAAGATGAGCGTCAAGACCAACTTGGAGACGCGGCATTCTTG
TCAGGTGTTGCCAAATTCCCTCAAAGAATCAAGTGTGCAACCCTAGCTTGGAATGCCCTTAAGAAAACAATTGAA
AATCAAGAAAAACAGTAA

4175.5

ATGAAAATTCAAGACCTATTGAGAAAAGATGTCATGTTGCTAGATTTGCAGGCAACTGAAAAAACAGCTGTCATC
GACGAGATGATTAAAAATTTGACAGACCACGGTTATGTAACAGATTTTGAAACATTTAAAGAAGGAATTTTGGCG
CGTGAAGCTTTGACTTCTACTGGTTTGGGTGATGGAATCGCAATGCCTCACAGCAAAAACGCTGCTGTCAAAGAA
GCGACAGTTCTATTTGCTAAGTCAAATAAGGGTGTTGACTACGAGAGCTTGGATGGACAAGCAACTGACCTCTTCT
TCATGATTGCAGCTCCAGAAGGTGCCAATGATACTCACTTGGCAGCCTTGGCAGAATTGTCTCAATACTTGATGAA
AGACGGTTTTGCAGACAAACTTCGTCAAGCAACATCTGCAGACCAAGTTATCGAACTTTTTGACCAAGCTTCAGAA
AAAACTGAGGAACTTGTTCAAGCACCTGCTAATGACTCTGGTGACTTTATCGTAGCTGTTACAGCTTGTACAACAG
GTATTGCCCACACTTACATGGCCCAAGAAGCCCTTCAAAAAGTAGCTGCTGAAATGGGGGTTGGTATCAAGGTCG
AAACCAACGGTGCTAGCGGTGTTGGAAATCAACTAACTGCAGAAGATATCCGTAAGGCTAAAGCTATTATCATTG
CAGCAGACAAGGCCGTTGAAATGGATCGATTTGATGGAAAACCATTGATCAATCGTCCAGTTGCTGACGGTATCC
GTAAGACAGAAGAGCTAATTAACTTGGCTCTTTCAGGAGATACTGAAGTCTACCGTGCCGCTAATGGTGCCAAAG
CTGCAACAGCCTCTAACGAAAAACAAAGCCTTGGTGGTGCCTTGTACAAACACTTGATGAGTGGTGTATCTCAAA
TGTTACCATTCGTTATCGGTGGTGGTATCATGATTGCCCTTGCCTTCTTGATTGACGGTGCTTTGGGTGTTCCAAAT
GAAAACCTTGGCAATCTTGGTTCTTACCATGAGTTAGCTTCTATGTTCATGAAAATTGGTGGAGCTGCCTTTGGTTT
GATGCTTCCAGTCTTTGCGGGTTATGTTGCCTACTCTATTGCTGAAAAACCGGGTTTGGTAGCAGGTTTCGTGGCT
GGTGCTATTGCCAAAGAAGGTTTTGCCTTTGGTAAAATTCCTTATGCCGCAGGTGGTGAAGCAACTTCAACTCTTG
CAGGTGTCTCATCTGGTTTCCTAGGTGCCCTTGTTGGTGGATTTATCGCAGGTGCCTTGGTTCTTGCCATCAAGAAA
TACGTTAAAGTTCCTCGTTCACTCGAAGGTGCTAAATCAATCCTTCTATTGCCACTTCTTGGAACAATCTTGACAG
GATTTGTTATGCTAGCTGTGAATATCCCAATGGCTGCAATCAACACTGCTATGAATGACTTCCTAGGCGGTCTTGG
AGGAGGTTCAGCTGTCCTTCTTGGTATCGTCCTTGGTGGAATGATGGCTGTTGACATGGGTGGACCAGTTAATAAA
GCAGCTTATGTCTTTGGTACAGGTACGCTTGCAGCAACTGTTTCTTCAGGTGGTTCTGTAGCCATGGCAGCAGTTA
TGGCTGGAGGAATGGTGCCACCACTTGCAATCTTTGTCGCAACTCTTCTTTTCAAAGATAAATTTACTAAGGAAGA
ACGTAACTCTGGTTTGACAAACATCATCATGGGCTTGTCATTTATCACTGAGGGAGCGATTCCATTTGGTGCCGCT
GACCCAGCTCGTGCGATTCCAAGCTTCATCCTTGGTTCAGCAGTAGCAGGTGGACTCGTTGGTCTTACTGGTATCA
AACTCATGGCGCCACACGGAGGAATCTTCGTTATCGCCCTTACTTCAAATGCTCTCCTTTACCTCGTTTCTGTCTTG
GTAGGAGCAATCGTAAGTGGTGTGGTTTATGGTTACCTACGCAAACCACAAGCATAA

4175.6
ATGGCAAACAAGAATACAAGTACAACAAGACGGAGACCGTCTAAAGCAGAACTGGAAAGAAAAGAAGCGATTCA
ACGAATGTTGATTTCGTTAGGAATTGCGATTTTATTGATTTTCGCAGCCTTCAAATTAGGGGCTGCAGGTATAACC
CTTTATAATTTAATTCGCTTGCTAGTGGGTAGCCTAGCTTATCTGGCGATATTCGGCCTATTAATCTATCTCTTCTT
TTTCAAGTGGATACGAAAACAGGAAGGACTCTTATCTGGCTTTTTCACCATATTTGCTGGCTTACTCTTGATTTTTG
AGGCCTACTTGGTTTGGAAATATGGTTTGGACAAGTCCGTTCTAAAAGGGACCATGGCTCAGGTTGTGACAGATCT
GACTGGTTTTCGAACGACTAGCTTTGCTGGAGGGGGCTTGATCGGGGTCGCTCTTTATATTCCAACAGCCTTTCTC
TTTTCAAATATCGGAACTTACTTTATTGGTTCTATCTTGATTTTAGTGGGTTCTCTCCTAGTCAGCCCTTGGTCTGTT
TACGATATTGCTGAATTTTTCAGTAGAGGCTTTGCCAAATGGTGGGAAGGGCACGAGCGTCGAAAAGAGGAACGC
TTTGTCAAACAAGAAGAAAAAGCTCGCCAAAAGGCTGAGAAAGAGGCTAGATTAGAACAAGAAGAGACTGAAAA
AGCCTTACTCGATTTGCCTCCTGTTGATATGGAAACGGGTGAAATTCTGACAGAGGAAGCTGTTCAAAATCTTCCA
CCTATTCCAGAAGAAAAGTGGGTGGAACCAGAAATCATCCTGCCTCAAGCTGAACTTAAATTCCCTGAACAGGAA
GATGACTCAGATGACGAAGATGTTCAGGTCGATTTTTCAGCCAAAGAAGCCCTTGAATACAAACTTCCAAGCTTA
CAACTCTTTGCACCAGATAAACCAAAAGATCAGTCTAAAGAGAAGAAAATTGTCAGAGAAAATATCAAAATCTTA
GAAGCAACCTTTGCTAGCTTTGGTATTAAGGTAACAGTTGAACGGGCCGAAATTGGGCCATCAGTGACCAAGTAT
GAAGTCAAGCCGGCTGTTGGTGTAAGGGTCAACCGCATTTCCAATCTATCAGATGACCTCGCTCTAGCCTTGGCTG
CCAAAGATGTCCGGATTGAAGCACCAATCCCTGGGAAATCCCTAATCGGAATTGAAGTGCCCAACTCCGATATTG
CCACTGTATCTTTCCGAGAACTATGGGAACAATCGCAAACGAAAGCAGAAAATTTCTTGGAAATTCCTTTAGGGA
AGGCTGTTAATGGAACCGCAAGAGCTTTTGACCTTTCTAAAATGCCCCACTTGCTAGTTGCAGGTTCAACGGGTTC
AGGGAAGTCAGTAGCAGTTAACGGCATTATTGCTAGCATTCTCATGAAGGCGAGACCAGATCAAGTTAAATTTAT
GATGGTCGATCCCAAGATGGTTGAGTTATCTGTTTACAATGATATTCCCCACCTCTTGATTCCAGTCGTGACCAAT
CCACGCAAAGCCAGCAAGGCTCTGCAAAAGGTTGTGGATGAAATGGAAAACCGTTATGAACTCTTTGCCAAGGTG
GGAGTTCGGAATATTGCAGGTTTTAATGCCAAGGTAGAAGAGTTCAATTCCCAGTCTGAGTACAAGCAAATTCCG
CTACCATTCATTGTCGTGATTGTGGATGAGTTGGCTGACCTCATGATGGTGGCCAGCAAGGAAGTGGAAGATGCTA
TCATCCGTCTTGGGCAGAAGGCGCGTGCTGCAGGTATCCACATGATTCTTGCAACTCAGCGTCCATCTGTTGATGT
CATCTCTGGTTTGATTAAGGCCAATGTTCCATCTCGTGTAGCATTTGCGGTTTCATCAGGAACAGACTCCCGTACG
ATTTTGGATGAAAATGGAGCAGAAAAACTTCTTGGTCGAGGAGACATGCTCTTTAAACCGATTGATGAAAATCAT
CCAGTTCGTCTCCAAGGCTCCTTTATCTCGGATGACGATGTTGAGCGCATTGTGAACTTCATCAAGACTCAGGCAG
ATGCAGACTACGATGAGAGTTTTGATCCAGGTGAGGTTTCTGAAAATGAAGGAGAATTTTCGGATGGAGATGCTG
GTGGTGATCCGCTTTTTGAAGAAGCTAAGTCTTTGGTTATCGAAACACAGAAAGCCAGTGCGTCTATGATTCAGCG
TCGTTTATCAGTTGGATTTAACCGTGCGACCCGTCTCATGGAAGAACTGGAGATAGCAGGTGTCATCGGTCCAGCT
GAAGGTACCAAACCTCGAAAAGTGTTACAACAATAA

4176.1
ATGAGTTATTTTAAAAAATATAAATTCGATAAATCCCAGTTCAAACTTGGTATGCGAACCTTTAAAACAGGTATTG
CTGTTTTTCTAGTTCTCTTGATTTTTGGCTTTTTTGGCTGGAAAGGTCTTCAAATTGGTGCTTTGACAGCCGTTTTTA
GCCTGAGGGAGAGTTTTGATGAGAGTGTTCATTTTGGGACTTCGCGTATTCTAGGAAATAGTATCGGTGGACTCTA
TGCCTTGGTCTTCTTCTTATTAAATACCTTTTTCCACGAAGCCTTTTGGGTGACCTTGGTAGTTGTTCCAATCTGCA

CCATGTTAACCATTATGACAAATGTAGCCATGAATAACAAAGCAGGGGTTATTGGTGGTGTAGCAGCTATGTTAAT
CATTACCCTATCAATTCCAAGTGGTGAGACAATTTTGTACGTGTTTGTGCGTGTATTAGAAACGTTTATGGGAGTT
TTTGTCGCAATTATCGTAAATTACGATATTGATCGTATTCGTCTCTTTTTAGAGAAAAAAGAAAAATAA

4178.2
ATGAATAAATCAGAACACCGCCACCAACTTATACGCGCTCTTATCACAAAAAACAAGATTCATACACAGGCTGAG
TTGCAAGCCCTTCTTGCTGAGAACGACATTCAAGTAACCCAGGCAACCCTCTCACGCGACATCAAAAATATGAAC
CTATCAAAAGTCCGCGAAGAAGATAGCGCTTATTATGTTCTTAACAATGGTTCCATCTCAAAATGGAAAAACGTC
TCGAACTCTACATGGAAGACGCCCTTGTCTGGATGCGCCCAGTTCAACACCAAGTCCTACTAAAAACCCTTCCTGG
ACTGGCTCAATCCTTTGGTTCTATCATTGATACTTTGAGCTTCCCTGACGCTATCGCTACCCTTTGTGGTAATGATG
TCTGTCTTATCATCTGTGAAGATGCAGATACTGCTCAAAAGTGCTTTGAAGAACTGAAAAAATTCGCCCCACCATT
TTTCTTTGAAGAATAA

4179.1
ATGAAAAGTATAAAATTAAATGCTCTATCTTACATGGGAATTCGTGTCTTGAATATTATTTTTCCCATCCTAACTGG
AACCTATGTCGCGCGTGTCTTGGACCGAACTGACTATGGTTACTTCAACTCAGTCGACACTATTTTGTCATTTTTCT
TGCCCTTTGCAACTTATGGTGTCTATAACTACGGTTTAAGGGCTATCAGTAATGTCAAGGATAACAAAAAAGATCT
TAACAGAACCTTTTCTAGTCTTTTTTATTTGTGCATCGCTTGTACGATTTTGACCACTGCTGTCTATATCCTAGCCT
ATCCTCTCTTCTTTACTGATAATCCAATCGTCAAAAAGGTCTACCTTGTTATGGGGATTCAACTCATTGCCCAGATT
TTTTCAATCGAATGGGTCAATGAAGCTCTGGAAAATTACAGTTTTCTCTTTTACAAAACTGCCTTCATCCGTATCCT
GATGCTGGTCTCTATTTTCTTATTTGTTAAAAATGAACACGATATTGTTGTCTATACACTTGTGATGAGTTTATCGA
CGCTGATTAACTACCTGATTAGTTATTTTTGGATTAAAAGAGACATCAAACTTGTTAAAATTCACCTAAGTGATTT
TAAACCACTCTTTCTCCCTCTGACAGCCATGTTAGTCTTTGCCAATGCCAATATGCTCTTCACTTTTTTAGATCGCC
TCTTCCTCGTTAAAACAGGGATTGATGTCAACGTTAGTTACTATACCATAGCTCAGCGAATTGTGACCGTTATAGC
TGGGGTTGTAACAGGTGCAATTGGAGTGAGTGTGCCTCGTCTCAGTTACTATCTGGGGAAAGGAGACAAAGAAGC
CTATGTTTCTCTGGTTAATAGAGGTAGTCGAATCTTTAACTTCTTTATCATTCCACTGAGTTTTGGACTCATGGTTT
TAGGACCAAATGCCATCCTACTTTACGGTAGTGAAAAATATATCGGAGGCGGCATCTTGACCTCTCTCTTCGCTTT
TCGTACGATTATCCTGGCCTTAGATACCATTCTTGGTTCCCAAATTCTCTTTACCAATGGCTATGAAAAACGTATC
ACAGTCTATACAGTCTTTGCTGGGCTACTCAATTTGGGCTTGAATAGTCTCCTTTTTTTCAACCATATCGTGGCTCC
TGAATACTACTTACTGACAACTATGCTATCAGAGACTTCTCTACTTGTTTCTATATCATTTTCATCCATAGAAAAC
AACTCATCCACTTGGGACATATCTTTAGCTATACTGTTCGATACTCTCTCTTTTCACTTTCCTTTGTAGCAATTTATT
TCCTGATTAATTTCGTGTATCCTGTAGATATGGTCATTAATTTGCCATTTTTGATTA
ATACTGGTTTGATTGTCTTGCTATCAGCTATCTCTTATATTAGTCTACTTGTCTTCACAAAAGATAGCATTTTCTAT
GAATTTTTAAACCATGTCCTAGCCTTAAAAAATAAATTTAAAAAATCATAG

4179.2
ATGAAACAACTAACCGTTGAAGATGCCAAACAAATTGAATTAGAAATTTTGGATTATATTGATACTCTCTGTAAAA
AGCACAATATCAACTATATTATTAACTACGGTACTCTGATTGGGGCGGTTCGACATGAGGGCTTTATCCCTTGGGA
CGACGATATTGATCTGTCCATGCCTAGAGAAGACTACCAACGATTTATTAACATTTTTCAAAAGGAAAAAAGCAA
GTATAAGCTCCTATCCTTAGAAACTGATAAGAACTACTTTAACAACTTTATCAAGATAACCGACAGTACGACTAAA
ATTATTGATACTCGAAATACAAAAACCTATGAGTCTGGTATCTTTATCGATATTTTCCCTATAGATCGCTTTGATGA
TCCTAAGGTCATTGATACTTGTTATAAACTGGAAAGCTTCAAACTGCTGTCTTTCAGTAAACATAAAAAATATTGTC
TATAAGGATAGCCTTTTAAAAGATTGGATACGAACAGCCTTCTGGTTACTCCTTCGACCGGTTTCTCCTCGTTATTT
TGCAAATAAAATCGAGAAAGAAATTCAAAAATATAGTCGTGAAAATGGGCAATATATGGCTTTTATCCCTTCAAA
ATTTAAGGAAAAGGAAGTCTTCCCAAGTGGTACCTTTGATAAAACAATCGATTTACCCTTTGAGAATTTAAGCCTT
CCTGCACCTGAAAAATTTGATACTATTTTGACACAATTTTATGGAGATTATATGACCCTACCACCAGAAGAAAAAC
GCTTCTACAGTCATGAATTTCACGCTTATAAATTGGAGGATTAG

4179.3
ATGATAAAAATCAATCATCTAACCATCACACAAAACAAAGATTTACGAGATCTTGTATCTGACCTAACCATGACC
ATCCAAGACGGGGAAAAGGTTGCTATTATTGGTGAAGAAGGAAATGGCAAATCAACCTTACTTAAAATTTTAATG
GGGGAAGCTTTGTCTGATTTCACTATCAAGGGAAACATCCAATCTGACTATCAGTCACTGGCCTACATTCCTCAAA
AAGTCCCTGAGGACCTAAAAAAGAAAACTTTACACGACTACTTCTTTTTAGATTCTATTGATTTAGACTACAGTAT
CCTCTATCGTTTGGCGGAGGAATTGCATTTTGATAGCAATCGTTTCGCAAGTGACCAAGAGATTGGCAATCTATCA
GGGGGCGAAGCTTTGAAAATTCAGCTTATCCATGAGTTAGCCAAACCCTTTGAGATTCTATTTTTAGATGAACCTT
CAAATGACCTAGACCTTGAGACAGTTGATTGGCTAAAAGGCCAGATTCAAAAGACCAGGCAAACCGTTATTTTCA
TTTCCCATGATGAAGACTTTCTTTCTGAAACGGCAGACACTATTGTTCACTTGCGACTGGTCAAACACCGTAAAGA
AGCGGAAACGCTAGTAGAGCATTTAGACTATGATAGCTATAGTGAGCAGAGAAAGGCTAATTTTGCCAAACAAAG
TCAGCAAGCTGCTAACAACCAAAGAGCCTACGATAAAACCATGGAAAAACATCGGAGAGTTAAGCAAAATGTAG
AAACTGCGCTTCGAGCTACCAAAGATAGTACTGCCGGTCGCCTATTGGCTAAAAAGATGAAAACTGTCCTCTCAC
AAGAAAAACGCTACGAAAAGGCAGCTCAGTCCATGACTCAAAAGCCACTTGAAGAGGAACAAATCCAACTTTTCT
TTTCAGACATCCAACCATTACCAGCTTCTAAAGTCTTAGTCCAACTGGAAAAAGAAAATTTGTCCATTGACGACCG
AGTTTTGGTTCAAAAACTACAACTAACTGTCCGTGGCCAAGAAAAAATCGGTATTATCGG
GCCAAATGGTGTTGGGAAATCAACTCTGTTAGCCAAGTTACAGAGACTTCTGAATGATAAAAGAGAGATTTCACT
TGGTTTTATGCCACAAGATTACCACAAAAAACTGCAATTGGATTTATCCCCAATAGCCTATCTCAGTAAAACTGGG

GAAAAAGAGGAACTACAGAAAATCCAATCTCACCTAGCTAGTCTCAATTTCAGTTATCCAGAAATGCAGCATCAA
ATTCGCTCCTTATCTGGCGGACAACAGGGAAAACTCCTGCTTTTGGATTTAGTCCTGCGCAAACCAAACTTTCTCC
TGCTGGATGAACCCACACGAAACTTTTCTCCCACTTCTCAACCCCAAATCAGAAAACTCTTTGCTACCTATCCAGG
CGGTCTCATCACTGTTTCGCATGACCGTCGTTTCTTAAAAGAAGTCTGCTCGATCATCTATCGCATGACAGAACAC
GGTTTGAAGCTAGTTAATTTAGAAGATTTATAA

4179.4
ATGAAACCAAAAACATTTTACAACTTGCTTGCCGAGCAGAATCTTCCACTTTCGGACCAGCAAAAAGAACAATTT
GAACGTTATTTTGAGCTCTTGGTCGAGTGGAATGAGAAGATTAATTTGACGGCGATTACGGACAAGGAAGAAGTT
TATCTCAAACATTTTTACGATTCGATTGCACCCATTCTTCAAGGTTTGATTCCCAATGAAACTATCAAACTTCTTGA
TATCGGGGCTGGGGCAGGATTTCCTAGTCTACCAATGAAAATTCTCTATCCGGAGTTAGATGTGACCATTATTGAT
TCACTCAATAAGCGCATCAACTTCCTACAACTCTTGGCTCAAGAACTGGATTTGAACGGAGTTCATTTCTACCACG
GACGTGCCGAAGATTTTGCCCAAGACAAGAACTTCCGTGCTCAATATGATTTTGTAACAGCTCGTGCGGTTGCCCG
TATGCAGGTCCTATCTGAATTGACTATTCCCTACCTTAAGGTTGGTGGCAAACTATTAGCACTCAAGGCTAGCAAT
GCGCCTGAGGAATTATTAGAAGCTAAGAATGCCCTCAATCTCCTTTTTAGTAAGGTCGAAGACAATCTCAGctACG
CCCTACCGAATAGAGATCCGCGCTATATCACAGTGGTAGAAAAGAAAAAAGAAACACCAAATAAATATCCACGTA
AGGCTGGTATGCCAAATAAACGCCCACTTTAA

4179.6
ATGAGTATTAAACTAATTGCCGTTGATATCGACGGAACCCTTGTCAACAGCCAAAAGGAAATCACTCCTGAAGTTT
TTTCTGCCATCCAAGATGCCAAAGAAGCTGGTGTCAAAGTCGTGATTGCAACTGGCCGCCCTATCGCAGGCGTTGC
CAAACTTCTAGACGACTTGCAGTTGAGAGACGAGGGGGACTATGTGGTAACCTTCAACGGTGCCCTTGTCCAAGA
AACTGCTACAGGACATGAGATTATCAGCGAATCCTTGACTTATGAGGATTATCTAGATATGGAATTCCTCAGTCGC
AAGCTCGGTGTCCACATGCATGCCATTACCAAGGACGGTATCTATACTGCAAATCGCAATATCGGAAAATACACT
GTACACGAATCAACCCTCGTCAGCATGCCTATCTTCTACCGTACCCCTGAAGAAATGGCTGGCAAAGAAATTGTTA
AATGTATGTTTATCGATGAACCAGAAATTCTCGATGCTGCGATTGAAAAAATTCCAGCAGAATTTTACGAGCGCTA
CTCCATCAACAAATCTGCTCCTTTCTACCTCGAACTCCTTAAAAAGAATGTAGACAAGGGTTCAGCCATTACTCAC
TTGGCTGAAAAACTCGGATTGACCAAAGATGAAACCATGGCAATCGGTGATGAAGAAAATGACCGTGCCATGCTG
GAAGTCGTTGGAAACCCCGTTGTCATGGAAAATGGAAATCCAGAAATCAAAAAAATCGCCAAATACATCACCAAA
ACAAATGACGAATCCGGCGTTGCCCATGCCATCCGAACATGGGTACTGTAA

4179.7
ATGACTTGGATTATTCTTGGAGTTATCGCTCTTATTGTTATTTTTGTGATTGTTAGCTATAACGGTTTGGTTAAAAA
TCGTATGCAAACCAAGGAGGCTTGGAGTCAGATTGATGTTCAGTTGAAACGTCGCAATGACCTCTTGCCAAACTTG
ATTGAGACTGTAAAAGGTTATGCCAAATATGAAGGTTCTACCCTTGAAAAGGTGGCAGAACTACGTAACCAAGTG
GCGGCAGCGACTTCACCAGCAGAAGCTATGAAAGCCAGTGATGCCCTCACTCGTCAGGTTTCAGGTATTTTTGCAG
TTGCAGAAAGCTATCCAGATTTGAAAGCTAGTGCTAACTTTGTTAAATTGCAAGAGGAGTTGACAAACACAGAAA
ATAAAATTTCTTACTCTCGTCAACTCTATAACAGTGTTGTCAGCAACTACAATGTAAAATTAGAAACTTTCCCGAG
CAATATTATCGCTGGAATGTTTGGATTTAAAGCGGCAGATTTCCTTCAAACACCTGAAGAGGAAAAGTCGGTTCCT
AAAGTTGATTTTAGCGGTTTAGGTGACTAA

4179.8
ATGTTGTTTGATCAAATTGCAAGCAATAAACGAAAAACCTGGATTTTGTTGCTGGTATTTTTCCTACTCTTAGCTCT
TGTTGGTTATGCGGTTGGTTATCTCTTTATAAGATCTGGACTTGGTGGTTTGGTTATTGCACTGATTATCGGCTTTA
TCTACGCTTTGTCTATGATTTTTCAATCGACAGAGATTGTCATGTCCATGAATGGAGCGCGTGAGGTGGATGAGCA
AACGGCACCAGACCTCTACCATGTAGTGGAAGATATGGCTCTGGTCGCTCAGATTCCTATGCCCCGTGTTTTCATC
ATTGATGATCCAGCCTTAAATGCCTTTGCGACAGGTTCTAATCCTCAAAATGCGGCTGTTGCTGCGACTTCAGGTC
TACTAGCTATCATGAATCGTGAAGAACTAGAAGCTGTTATGGGACATGAAGTCAGTCATATTCGTAATTATGATAT
CCGTATTTCGACTATTGCAGTTGCCCTTGCTAGTGCTATCACCATGCTTTCTAGTATGGCAGGTCGTATGATGTGGT
GGGGTGGAGCAGGTCGCAGACGAAGTGATGATGACCGAGATGGAAATGGTCTTGAAATCATTATGCTAGTGGTTT
CCCTACTAGCTATTGTACTGGCACCTCTCGCTGCAACCTTGGTTCAGCTCGCTATTTCTCGTCAGAGGGAATTTCTG
GCAGATGCATCTAGTGTCGAGCTGACTCGCAATCCCCAGGGAATGATTAATGCCCTAGATAAGTTGGACAATAGC
AAACCTATGAGTCGCCACGTCGATGATGCTAGCAGTGCCCTTTATATCAATGATCCTAAGAAAGGTGGGGGGTTC
CAAAAACTCTTTTATACCCACCCACCTATCTCAGAACGGATTGAACGTTTAAAACAGATGTAA

4179.9
ATGAAATTAAATATTCAAGAAATTCGTAAGCAGTCTGAAGGTTTGAACTTTGAACAAACGTTAGATTTAGTTGATG
ACCTGCGTGCACGTAATCAAGAAATTTTAGATGTAAAAGATATCCTTGCAGTTGGGAAAGTACAATATGAAGACC
GTATGTATTTCTTAGATTATCAACTATCTTATACCATTGTTCTTGCTTCGAGTCGCAGTATGGAGCCAGTTGAGTTA
GTTGAATCTTATCCAGTCACGGAAGTTTTCATGGAAGGCGCAACTAACCAGCTAGATCAAGAAGTTTTAGATGATG
ACTTGGTCTTGCCCATCGAAAATGGGGAGCTTGACCTTGCTGAGAGTGTATCAGACAATATCCTGCTAAACATTCC
TATCAAGGTCTTGACGGCTGAAGAAGAAGCTGGTCAAGGATTTATCTCAGGAAATGACTGGCAAATCATGACAGA
GGAAGAATACCAAGCTCAAAAAGCAGTAAAGAAAGAAGAAAACAGTCCTTTTGCTGGCTTACAAGGACTATTTGA
CGGAGATGAATAA

4179.12
ATGGAGTTATTTATGAAAATCACAAACTATGAAATCTATAAGTTAAAAAAATCAGGTTTGACCAATCAACAGATTT
TGAAAGTGCTAGAATACGGTGAAAATGTTGATCAGGAGCTTTTGTTGGGTGATATTGCAGATATCTCAGGTTGCCG
TAATCCAGCCGTTTTTATGGAACGTTATTTTCAGATAGACGATGCGCATTTGTCGAAAGAGTTTCAAAAATTTCCA
TCTTTCTCTATTTTAGATGACTGTTATCCTTGGGATTTGAGTGAAATATATGATGCGCCTGTACTTTTATTTTACAA
GGGAAATCTTGACCTCCTGAAATTCCCGAAGGTAGCGGTCGTGGGCAGTCGTGCTTGTAGCAAACAGGGAGCTAA
GTCAGTTGAAAAAGTCATTCAAGGCTTGGAAAATGAACTGGTTATTGTCAGTGGTCTGGCCAAGGGCATTGACAC
AGCAGCTCATATGGCAGCTCTTCAGAATGGCGGAAAAACCATTGCAGTGATTGGAACAGGACTGGATGTGTTTTA
TCCTAAAGCCAATAAACGCTTGCAAGACTACATCGGCAATGACCATCTGGTTCTAAGTGAATATGGACCTGGTGA
ACAACCTCTGAAATTTCATTTTCCTGCCCGTAATCGCATCATTGCTGGACTTTGTCGTGGTGTGATTGTAGCAGAG
GCTAAGATGCGTTCAGGTAGTCTCATTACGTGTGAGCGAGCAATGGAAGAAGGACGCGATGTCTTTGCTATTCCTG
GTAGCATTTTAGATGGACTATCAGACGGTTGCCATCATTTGATTCAAGAAGGAGCAAAATTGGTCACCAGTGGGC
AAGATGTTCTTGCGGAATTTGAATTTTAA

4181.1
ATGAAACGTCAATTAGCCTTGGTCGTCTTTAGTGGTGGTCAAGATTCAACAACCTGCCTTTTCTGGGTCATGCAAC
ACTATGAAACAGTCGAAGCTGTCACCTTTGCCTACGGCCAACGTCATCACCTCGAAATTCAAATTACTAGAGAAAT
CGCTAAGGAACAGGGCATTCGTCACCATATCCTCGATATGTCTCTGCTGGGACAAATCACTGCTCAGCCAGACTTT
GCGACGATTCATATTTCCTACATTCCTGACAAGCTCTGTGTCGAGTCAAAATCCCTCAAACTATATCTATTTAGCT
ACCGAAACCACGGAGATTTCCACGAAAACTGTATCAACACCATCGGGAAAGACTTGGTCAACTTGCTAGACCCTC
GCTATTTAGAAGTCTGGGGAAAATTCACTCCGCGCGGTGGCATTTCAATCGACCCCTACTACAACTACGGTAAGCA
AGGAACTAAGTATGAGGGCTTGGCAGAACAACGCCTCTTCCAACACGACCTTTATCCAGAGAAAATTGACAACCG
CTAA

4181.2
ATGACCGAAACGGTAGAAGATAAAGTAAGTCATTCAATTACTGGGCTTGATATCCTCAAGGGGATAGTTGCTGCG
GGAGCTGTCATAAGTGGAACCGTTGCAACTCAAACGAAGGTATTTACAAATGAGTCAGCAGTACTTGAAAAAACT
GTAGAGAAAACGGATGCTTTGGCAACAAATGATACAGTAGTTCTAGGTACGATATCTACAAGTAATTCAGCGAGT
TCAACTAGTTTGTCAGCTTCAGAGTCGGCAAGTACATCTGCATCTGAGTCAGCCTCAACCAGCGCTTCGACCTCAG
CAAGTACAAGTGCATCAGAATCAGCAAGTACATCGGCTTCGACAAGTATTTCTGCATCATCTACTGTGGTAGGTTC
ACAAACAGCTGCCGCTACAGAAGCAACTGCTAAGAAGGTCGAAGAAGATCGTAAGAAACCAGCTAGTGATTATGT
AGCATCAGTTACAAATGTCAATCTCCAATCTTATGCTAAGCGACGCAAGCGTTCAGTGGATTCCATCGAGCAATTG
CTGGCTTCTATAAAAAATGCTGCTGTTTTTTCTGGCAATACGATTGTAAATGGCGCCCCTGCAATTAATGCAAGTC
TAAACATTGCTAAAAGTGAGACAAAAGTTTATACAGGTGAAGGTGTAGATTCGGTATATCGTGTTCCAATTTACTA
TAAATTGAAAGTGACAAATGATGGTTCAAAATTGACCTTTACCTATACGGTTACGTATGTGAATCCTAAAACAAAT
GATCTTGGTAATATATCAAGTATGCGTCCTGGATATTCTATCTATAATTCAGGTACTTCAACACAAACAATGTTAA
CCCTTGGCAGTGATCTTGGTAAACCTTCAGGTGTAAAGAACTACATTACTGACAAAAATGGTAGACAGGTTCTATC
CTATAATACATCTACAATGACGACGCAGGGTAGTGGGTATACTTGGGGAAATGGTGCCCAAATGAATGGTTTCTTT
GCTAAGAAAGGATATGGATTAACATCATCTTGGACTGTACCAATTACTGGAACGGA
TACATCCTTTACATTTACCCCTTACGCTGCTAGAACAGATAGAATTGGAATTAACTACTTCAATGGTGGAGGAAAG
GTAGTTGAATCTAGCACGACCAGTCAGTCACTTTCACAGTCTAAGTCACTCTCAGTAAGTGCTAGTCAAAGCGCCT
CAGCTTCAGCATCAACAAGTGCGTCGGCTTCAGCATCAACCAGTGCCTCGGCTTCAGCGTCAACCAGTGCGTCAG
CTTCAGCAAGTACCAGTGCTTCAGTCTCAGCATCAACAAGTGCTTCAGCCTCAGCATCGACAAGTGCCTCGGCTTC
AGCAAGCACATCAGCATCTGAATCAGCGTCAACCAGTGCTTCGGCTTCAGCAAGTACCAGTGCTTCAGCTTCAGC
ATCAACCAGCGCCTCGGCCTCAGCAAGCACCTCAGCTTCTGAATCGGCCTCAACCAGCGCCTCGGCCTCAGCAAG
CACCTCAGCTTCTGAATCGGCCTCAACCAGCGCCTCAGCCTCAGCATCAACGAGTGCTTCGGCTTCAGCAAGCAC
AAGCGCCTCGGGTTCAGCATCAACGAGTACGTCAGCTTCAGCGTCAACCAGTGCTTCAGCCTCAGCATCAACAAG
TGCGTCAGCCTCAGCAAGTATCTCAGCGTCTGAATCGGCATCAACGAGTGCGTCTGAGTCAGCATCAACGAGTAC
GTCAGCCTCAGCAAGCACCTCAGCTTCTGAATCGGCCTCAACCAGTGCGTCAGCCTCAGCATCGACAAGCGCCTC
AGCTTCAGCAAGTACCAGTGCTTCAGCCTCAGCGTCGACAAGTGCGTCGGCCTCAACCAGTGCATCTGAATCGGC
ATCAACCAGTGCGTCAGCCTCAGCAAGTACTAGTGCATCGGCTTCAGCATCAACCAGTGCCTCGGCTTCAGCGTCA
ACCAGTGCGTCAGCTTCAGCAAGTACCAGTGCTTCAGTCTCAGCATCAACAAGTGCTTCAGCCTCAGCATCGACA
AGTGCCTCGGCTTCAGCAAGCACATCAGCATCTGAATCAGCGTCGACAAGCGCCTCAGCTTCAGCAAGTACCAGT
GCGTCAGCTTCAGCATCAACCAGCGCCTCGGCCTCAGCAAGCACCTCAGCTTCTGAATCGGCCTCAACCAGCGCC
TCGGCCTCAGCAAGCACCTCAGCTTCTGAATCGGCCTCAACCAGCGCCTCAGCCTCAGCATCAACGAGTGCTTCG
GCTTCAGCAAGCACAAGCGCCTCGGGTTCAGCATCAACGAGTACGTCAGCTTCAGCGTCAACCAGTGCTTCAGCC
TCAGCATCAACAAGTGCGTCAGCCTCAGCAAGTATCTCAGCGTCTGAATCGGCATCAACGAGTGCGTCTGAGTCA
GCATCAACGAGTACGTCAGCCTCAGCAAGCACCTCAGCTTCTGAATCGGCCTCAACCAGTGCGTCAGCCTCAGCA
TCGACAAGCGCCTCAGCTTCAGCAAGTACCAGTGCTTCAGCCTCAGCTCGACAAGTGCGTCGGCCTCAACCAGTG
CATCTGAATCGGCATCAACCAGTGCGTCAGCCTCAGCAAGTACTAGTGCATCAGCTTCAGCATCAACGAGTGCAT
CGGCTTCAGCATCAACCAGTGCCTCGGCTTCAGCGTCAACCAGTGCGTCAGCTTCAGCAAGTACCAGTGCTTCAGT
CTCAGCATCAACAAGTGCTTCAGCCTCAGCATCGACAAGTGCcTCGGCTTCAGCAAGCACATCAGCATCTGAATCA
GCGTCGACAAGCGCcTCAGCTTCAGCAAGTACCAGTGCGTCAGCCTCAGCGTCGACAAGTGCGTCAGCCTCAGCA
AGTACTAGTGCATCAGCTTCAGCATCAACGAGTGCATCGGCTTCGGCGTCAACCAGTGCATCAGAGTCAGCAAGT
ACCAGTGCGTCAGCTTCCGCATCAACAAGTGCCTCGGCTTCAGCAAGCACCAGTGCGTCGGCTTCAGCAAGTACT

AGCGCCTCAGCCTCAGCCTCAACCAGTGCGTCAGCCTCAGCAAGTATCTCAGCGTCTGAATCGGCATCAACGAGT
GCGTCCGCTTCAGCAAGTACTAGCGCCTCAGCCTCAGCGTCAACAAGTGCATCGGCTTCAGCGTCAACGAGTGCG
TCTGAATCGGCATCAACGAGTGCGTCCGCTTCAGCAAGTACTAGCGCCTCAGCCTCAGCGTCAACAAGTGCATCG
GCTTCAGCATCAACGAGTGCGTCCGCTTCAGCAAGTACTAGCGCCTCAGCCTCAGCGTCAACAAGTGCATCGGCTT
CAGCGTCAACGAGTGCGTCTGAGTCAGCATCAACGAGTGCGTCAGCCTCAGCAAGCACATCAGCTTCTGAATCTG
CATCAACCAGTGCGTCAGCCTCAGCATCGACAAGCGCCTCAGCTTCAGCAAGTACCAGTGCGTCAGCCTCAGCGT
CGACAAGTGCGTCGGCTTCAGCAAGTACCAGTGCGTCAGCCTCAGCAAGTACCAGTGCGTCAGCCTCAGCGTCGA
CAAGTGCGTCGGCCTCAACCAGTGCATCTGAATCGGCATCAACCAGTGCGTCAGCCTCAGCAAGTACTAGTGCAT
CAGCTTCAGCATCAACGAGTGCATCGGCTTCAGCATCAACCAGTGCATCAGAGTCAGCAAGTACCAGTGCGTCAG
TTCCGCATCAACAAGTGCCTCGGCTTCAGCAAGTACTAG

4183.1
ATGGGGGTCGAAACTTGGTTTTATTCTAGCATCTGCTGGCTGGCCATCGGGCTTGGTTCCGTTTGGAAGTTTCCCT
ACATGACTGCTGCTAATGGCGGTGGAGGCTTTTTACTAATCTTTCTCATTTCCACTATTTTAATCGGTTTCCCTCTC
CTGCTGGCTGAGTTTGCCCTTGGCCGTAGTGCTGGCGTTTCCGCTATCAAAACCTTTGGAAAACTGGGCAAGAATA
ACAAGTACAACTTTATCGGTTGGATTGGCGCCTTTGCCCTCTTTATCCTCTTATCTTTTTACAGTGTTATCGGAGGA
TGGATTCTAGTCTATCTAGGTATTGAGTTTGGGAAATTGTTCCAACTTGGTGGAACGGGTGATTATGCTCAGTTAT
TTACTTCAATCATTTCAAATCCAGCCATTGCCCTAGGAGCTCAAGCGGCCTTTATCCTATTGAATATCTTCATTGTA
TCACGTGGGGTTCAAAAAGGGATTGAAAGAGCTTCGAAAGTCATGATGCCCCTGCTCTTTATCGTCTTTGTTTTTA
TCATCGGTCGCTCTCTCAGTTTGCCAAATGCCATGGAAGGGGTTCTTTACTTCCTCAAACCAGACTTTTCAAAACT
GACTAGCACTGGTCTCCTCTATGCTCTGGGACAATCTTTCTTTGCCCTCTCACTAGGGGTTACAGTCATGTTGACCT
ATGCTTCTTACTTAGACAAGAAAACCAATCTAGTCCAGTCAGGAATCTCCATCGTAGCCATGAATATCTCGATATC
CATCATGGCAGGTCTAGCCATTTTCCAAGCTCGATCCCCCTTCAATATCCAGTCTGAAGGGGGACCCAGCCTGCTC
TTTATCGTCTTGCCTCAACTCTTTGACAAGATGCCTTTTGGAACCATTTTCTACGTCCTCTTCCTCTTGCTCTTCCTT
TTTGCGACAGTCACTTTTTCTGTCGTGATGCTGGAAATCAATGTAGACAATATCACCAACCAGGATAACAGCAAAC
GTGCCAAATGGAGTGTTATTTTAGGAATTTTGACCTTTGTCTTTGGCATTCCTTCAGCCCTATCTTACGGTGTCATG
GCGGATGTTCACATTTTTGGTAAGACCTTCTTTGACGCTATGGACTTCTTGGTTTCCAATCTCCTCATGCCATTTGG
AGCTCTCTACCTTTCACTTTTTACAGGCTATATCTTTAAAAAGGCTCTTGCAATGGAGGAACTCCATCTCGATGAA
AGAGCATGGAAACAAGGACTGTTCCAAGTCTGGCTCTTCCTTCTTCGTTTCTTCGTTTCGTCATTCCAATCATCATC
ATTGTGGTCTTCATTGCCCAATTTATGTAATCAAAAAGGACTTGAGTAG

4183.5
ATGTTGAAAAAATGGCAGTTAAAAGATGTTATCTTGCTTGCTTTCTTGTCTATCTTTTTTGGTGGGGTTTTCGTTGG
TTCAGGATATGTGTATAATATTCTCAGTCTACTCTTAACACCTCTTGGTTTGCAGGCCTTTGCCAATGAAATCCTCT
TCGGTCTCTGGTGTATGGCTGCGCCCATTGCTGCCATCTTTGTTCCGAGAGTCGGAAGTGCAACGATTGGAGAAGT
GCTAGCTGCGCTTGCTGAAGTCCTTTATGGTAGCCAATTTGGTCTAGGAGCTCTTTTGTCTGGCTTTGTTCAAGGTT
TGGGAAGTGAATTTGGTTTTATCGTAACTAAGAATCGCTATGAAAGTTGGCTCTCTCTAACTGCTAATAGTATTGG
GATTACGCTTGTTAGCTTTGTCTATGAATACATTAAGTTAGGTTACTACGCCTTTTCCCTTCCGTTTGTCCTTTCCTT
GCTTGTGGTACGTTTTATTTCTGTTTATTTCTTCTGTACCATCTTGGTTCGTGCCATTGTCAAACTCTATCATCAGTT
TGCAACTGGAGGAAAAGCATAG

4183.6
ATGGTCAAAGTAGCAACCCAGACACCGATTATCAGTCTCTTCTTGCTGATTTTATCCTTGGAAACATCTTTCATTCC
TTCGATTGCTCTGACTCTTTCGGTAGTCGCATTTTGTATTCTCTTTATGCTCTATTACCGTCGATTTAAAATGTTAG
CTTGGATGATCATACTTGCCATTTTACCATCTTTTGCCAACTACTGGGCAGTTCAGTTACACGGAGATGCTTCACA
GGCAGTCATGCTTGGAACGAGGGCCTTTGTGACAGTTTGTATCGGCCTTGTCTTTGTTTCCTCTGTTTCACTAAAAG
AGCTTCTCTTGTACTTGGCTCAAAAGGGGCTATCACGCTCTTGGTCCTATGCCTTGATTGTGGTATTCAATTCTTTT
CCTCTCATTCAGCAAGAAATCAAGTCCCTCAAAGAAGCTTGCCTATTACGTGGTCAAGAACTACATTTTTGGTCGC
CCTTGATTTACAGTAAGGTTCTGATGACAGTCTTTAGGTGGCGCCATCTTTACCTGAGAGCTCTATCTGCTCACGG
ATATGACGAACATGCACAGTTGAAGAATAGCTATCGGACTTTTTATATTCCTAAAAAAACAAAATTAATCTACCTG
CTTTTCTTTTTATTGCTTCAAACCAGTCTATTTTTATAA

4183.7
ATGAGAAAGCACCAATTACAAGTTCACAAATTAACCATTTTATCTATGATGATTGCCCTTGATGTAGTCCTTACAC
CTATCTTTCGAATTGAGGGAATGGCACCGATGTCCAGTGTAGTCAATATTCTAGCAGGAATCATGATGGGACCTGT
TTATGCCTTGGCTATGGCTACAGTCACAGCCTTTATCCGTATGACGACTCAAGGGATTCCGCCTTTAGCTCTCACA
GGAGCGACTTTTGGAGCCCTTCTAGCAGGTCTCTTTTATAAGTACGGTCGAAAATTTCACTATTCTGCTCTAGGAG
AGATTTTGGGAACAGGTATTATTGGTTCCATTGTTTCCTATCCTGTTATGGTACTCTTTACAGGATCAGCTGCTAAG
CTTAGCTGGTTTATCTACACGCCTCGATTTTTCGGAGCAACCTTGATTGGTACAGCGATTTCCTTTATTGCCTTTCG
ATTTTTAATCAAGCAGGAATTCTTTAAAAAAGTGCAGGGATATTTCTTTAGTGAAAGGATAGACTGA

4183.8
ATGCAGGAATTTACAAATCCCTTTCCTATAGGCTCTAGTTCCCTCATTCACTGCATTACCAATGAGATTTCTTGTGA
GATGCTGGCAAATGGGATTTTGGCTCTGGGATGCAAACCTGTCATGGCAGATGATTCCCGTGAAGTTCTTGATTTT
ACTAAGCAAAGTCAGGCTCTCTTCATCAATTTGGGGCATTTGTCAGCTGAGAAGGAAAAAGCAATCCGCATGGCA

GCTTCGTATGCAAACCAATCTTCTCTCCCGATGGTAGTAGATGCGGTTGGCGTAACGACTTCATCCATTCGTAAGA
GCTTAGTTAAAGACCTTTTAGACTATAGACCTACGGTCCTTAAAGGAAACATGTCAGAAATTCGAAGTCTTGTTGG
ATTAAAGCACCACGGCGTTGGGGTCGATGCGAGTGCTAAAGATCAAGAAACGGAGGATTTGCTTCAAGTCTTGAA
AGACTGGTGTCAGACCTATCCTGGTATGTCTTTCTTAGTCACAGGTCCCAAGGACCTCGTCGTTTCGAAAAATCAG
GTCGCTGTACTGGGAAATGGCTGTACTGAATTAGACTGGATAACAGGGACAGGAGACTTGGTTGGAGCCTTAACA
GCTGTTTTTCTCAGCCAAGGAAAGACTGGTTTTGAAGCTTCTTGCTTAGCAGTCTCTTATCTCAATATCGCTGCTGA
GAAAATAGTTGTTCAAGGAATGGGATTGGAAGAATTTCGTTACCAAGTACTCAATCAGCTTTCGCTCCTAAGAAG
AGATGAAAATTGGCTAGATACCATCAAAGGAGAGGTTTATGAATAG

4185.3
ATGAACCATAAAATCGCAATTTTATCAGATGTTCATGGCAATGCGACGGCGCTAGAAGCAGTGATTGCAGATGCT
AAAAATCAAGGGGCCAGTGAATATTGGCTTCTGGGAGATATTTTTCTTCCTGGTCCAGGCGCAAATGACTTAGTCG
CCCTGCTAAAGGACCTTCCTATCACAGCAAGTGTTCGAGGCAATTGGGATGATCGTGTCCTTGAGGCTTTAGATGG
GCAATATGGCTTAGAAGACCCACAGGAAGTTCAGCTCTTGCGTATGACACAGTATTTGATGGAGCGAATGGATCC
TGCAACGATTGTCTGGCTACGAAGCTTGCCTTTGCTGGAAAAGAAAGAAATTGACGGATTGCGCTTTTCTATCTCT
CATAATTTACCTGACAAAAACTATGGTGGTGACTTGCTAGTTGAGAATGATACAGAGAAATTTGACCAACTGCTA
GATGCGGAAACGGACGTGGCAGTTTATGGTCATGTTCACAAGCAGTTGCTTCGTTATGGAAGTCAAGGGCAACAA
ATCATCAATCCAGGGTCGATTGGCATGCCCTATTTTAATTGGGAGGCGTTAAAAAATCACCGTTCCCAGTATGCCG
TGATAGAAGTTGAAGATGGGGAATTACTCAATATCCAATTTCGTAAAGTTGCTTATGATTACGAAGCTGAGTTAGA
ATTGGCCAAGTCCAAGGGGCTTCCCTTTATCGAAATGTATGAAGAACTGCGTCGTGACGATAACTATCAGGGGCA
CAATCTGGAATTATTAGCCAGCTTAATAGAAAAGCATGGGTATGTAGAGGATGTGAAGAATTTTTTTGATTTTTG
TAA

4186.1
ATGAATGTAAATCAGATTGTACGGATTATTCCTACTTTAAAAGCTAATAATAGAAAATTAAATGAAACATTTTATA
TTGAAACCCTTGGAATGAAGGCCTTGTTAGAAGAATCGGCCTTTCTGTCACTAGGTGACCAAACGGGTCTTGAAAA
GCTGGTTTTAGAAGAAGCTCCCAGTATGCGTACTCGTAAGGTAGAGGGAAGAAAAAAACTAGCTAGATTGATTGT
CAAGGTGGAAAATCCCTTAGAAATTGAAGGAATCTTATCTAAAACAGATTCGATTCATCGATTATATAAAGGTCA
AAATGGCTACGCTTTTGAAATTTTCTCACCAGAAGATGATTTGATTTTGATTCATGCGGAAGATGACATAGCAAGT
CTAGTAGAAGTAGGAGAAAAGCCTGAATTTCAAACAGATTTGGCATCAATTTCTTTAAGTAAATTTGAGATTTCTA
TGGAATTACATCTCCCAACTGATATCGAAAGTTTCTTGGAATCATCTGAAATTGGGGCATCCCTTGATTTTATTCC
AGCTCAGGGGCAGGATTTGACTGTGGACAATACGGTTACCTGGGACTTATCTATGCTCAAGTTCTTGGTCAATGAA
TTAGACATAGCAAGTCTTCGCCAGAAGTTTGAGTCTACTGAATATTTTATTCCTAAGTCTGAAAAATTCTTCCTTG
GTAAAGATAGAAATAATGTTGAATTGTGGTTTGAAGAAGTATGA

4186.2
ATGAAGTGGACCAAGATTATTAAAAAAATAGAAGAACAAATCGAGGCAGGGATTTATCCCGGAGCCTCTTTTGCG
TATTTTAAGGACAATCAATGGACAGAGTTCTATTTAGGCCAGAGTGACCCAGAGCATGGCTTGCAGACTGAGGCA
GGACTAGTTTATGACCTAGCTAGTGTCAGCAAGGTTGTTGGGGTTGGCACAGTTTGTACCTTCTTGTGGGAAATAG
GTCAATTAGATATTGATAGACTGGTAATAGATTTTTTACCTGAGAGTGATTATCCAGACATCACTATTCGCCAGCT
CTTGACTCATGCAACAGACCTTGATCCTTTTATTCCTAATCGTGATCTTTTAACAGCCCCTGAATTAAAGGAAGCG
ATGTTTCATCTCAACAGACGAAGTCAGCCAGCCTTTCTTTATTCGGATGTCCATTTTTTGCTGTTGGGCTTTATTTT
GGAAAGAATTTTTAATCAAGATTTGGATGTGATTTTAAAGGATCAAGTCTGGAAACCTTGGGGAATGACGGAAAC
TAAGTTTGGGCCAGTTGAGCTTGCTGTTCCAACAGTTAGAGGTGTAGAGGCAGGCATAGTGCATGATCCCAAGGC
TCGTCTCCTGGGTAGACATGCTGGGAGTGCTGGTTTATTTTCGACTATAAAGGATTTACAAATCTTTTTAGAACAC
TATTTAGCAGATGATTTTGCAAGAGACTTAAATCAAAATTTTTCTCCTTTGGATGACAAGGAACGTTCTTTAGCAT
GGAATTTGGAAGGAGATTGGCTAGACCATACGGGCTATACAGGTACCTTTATCATGTGGAATCGTCAGAAGCAAG
AAGCCACTATTTTCCTATCGAATCGTACCTATGAAAAGGACGAGAGAGCTCAATGGATATTAGACCGCAATCAAG
TGATGAACTTGATTCGCAAAGAAGAGTAA

4187.2
ATGATGAAGAAGACTTATAATCATATTTTGGTCTGGGGAGTCATTTTCTATAGCATTTGCATTGTCTGTTTTTGCTT
TACTCCTCAAGAACAATCTACCGTGGGAGTGGGAACTCCAGGTATTCAGCATCTTGGACGCCTGGTTTTTCTTTTG
ACTCCTTTCAATTCTCTCTGGAAACTGGGCGAAGTGAGTGACATTGGACAATTATGTTGGATTTTTTTACAAAATA
TCCTCAATGTCTTCTTGTTTTTTCCTCTGATTTTCCAACTCCTTTATCTATTTCCAAATTTGCGGAAAACAAAAAAG
GTCCTTCTTTTTAGTTTTCTTGTGAGTCTTGGAATCGAGTGTACGCAATTAATCTTGGACTTTTTCTTTGATTTCAAT
CGCGTCTTTGAGATTGATGATTTGTGGACCAACACTTTGGGTGGCTATCTGGCTTGGCTCCTTTATAAACGATTAC
ATAAAAACAAGGTAAGGAATTAA

4188.1
ATGAAGATTCCTCTCTTAACTTTTGCAAGGCATAAATTTGTTTATGTCTTGCTTACTTTGCTTTTTCTTGCTTTGGTT
TATCGTGATGTTTTGATGACTTATTTCTTTTTTGATATTCATGCGCCCGATCTAGCTAAATTCGATGGACAAGCAAT
TAAAAATGACTTATTAAAATCAGCATTAGATTTTCGTATTCTCCAGTTCAATCTAGGTTTTTATCAATCATTTATTA
TTCCAATCATCATTGTTTTGCTAGGTTTTCAATATATTGAGCTGAAAAATAAAGTTTTACGATTGAGTATTGGAAG
AGAAGTGAGTTATCAAGGGTTAAAAAGAAAGTTGACTTTGCAAGTTGCAAGTATCCCTTGTTTGATATATTTAGTG
ACTGTGCTGATAATTGCAATTATAACCTATTTCTTTGGGACTTTTTCTCCTCTTGGATGGAATTCTCTATTTTCTGAT
GGAAGTGGTTTACAAAGACTCCTAGATGGAGAGATAAAAAGCTATTTGTTCTTTACTTGTGTCCTACTAATCGGTA
TTTTCATCAATGCAATCTATTTTTTACAAATAGTTGATTATGTGGGGAATGTGACTCGTTCGGCAATCACCTATTTG
ATGTTTCTTTGGCTTGGTTCTATGCTGCTTTATAGTGCCTTGCCTTACTATATGGTTCCTATGACGAGTTTGATGCA
AGCTAGCTATGGGGATGTAAGTTTGATGAAACTCTTTACTCCTTATATCCTTTATATTGTCCCTTACATGGTGCTTG
AAAAATATGAAGATAATGTTTAA

4188.2
ATGAAGATAATGTTTAAGAATTTTAACAATATTTTGCTAAATAGAAAGATTGTTTTACTACTTCGTATAGTTCTGAT
GATGATTTTGATAAACCATCTATTGTCAACAGCGGTTCAAAAGCAGGATGCTGTTATCTTTTTCAAGAGAGAATTG
ATTTCAATTTTTTCCTATAATGACTATTCTGAAGCGAATTTAGAAATCCCCAAACTATTGTTAAACCTTTCGCTTTT
CATGGTAGGATGGCTCTCTGTCATTTTACTTGAAAGTGATTTGGCAGACCATTACCATCACTTGATTCGCTATCAA
TCAAGCTCCTTTTTCGATTATACAAGGAAACGATTGGTTGTCATTTCTAAATTTTTTACTCAAGATTTGTTTGTCTG
GTTTCTTGGTTTACTTCCTCTAGGAATTCATTTCAAAACAGTCGCACTTTTCTTTTTACTTGCTCAGTTAATGATGTT
GTACTTACTACTGTCTTATCTGATAGCACTGATTAGTGCGGGCGCTGGTTTTTCCTTTTTTCTCTATTTTTTAGCATT
TGTGGGACAAGAATGGATGATGGATCATATTGTAACAGTGTATTTAGTACTCTTAAGTTTATTAGTTATGTTGATT
GTTAGTCGCTTGGAAGAGAAATTTAAGAAAGGATAA

4188.5
ATGGGCAAAGGAGAGATGGGCAAAGGAGTTATTGGCTTGGAGTTCGACTCAGAAGTATTGGTCAACAAGGCTCCA
ACCCTTCAATTGGCAAATGGTAAAACAGCGACTTTCCTAACCCAGTATGATAGCAAGACCTTGTTGTTTGCAGTAG
ATAAGGAAGATATCGGACAGGAAATTATTGGTATAGCTAAAGGAAGCATCGAAAGTATGCATAATCTTCCTGTAA
ATCTAGCAGGTGCCAGAGTTCCTGGCGGAGTAAATGGTAGCAAAGCAGCGGTGCATGAAGTTCCAGAATTTACAG
GGGGAGTTAATGGTACAGAGCCAGCTGTTCATGAAATCGCAGAGTATAAGGGATCTGATTCGCTTGTAACTCTTAC
TACAAAAAAAGATTATACTTACAAAGCTCCTCTTGCTCAGCAGGCACTTCCTGAAACAGGAAACAAGGAGAGTGA
CCTCCTAGCTTCACTAGGACTAACAGCTTTCTTCCTTGGTCTGTTTACGCTAGGGAAAAAGAGAGAACAATAA

4188.10
ATGTTTAAAGTTTTACAAAAAGTTGGAAAAGCTTTTATGTTACCTATAGCTATACTTCCTGCAGCAGGTCTACTTTT
GGGGATTGGTGGTGCACTTTCAAACCCAACCACGATAGCAACTTATCCAATACTAGACAATAGTATTTTTCAATCA
ATATTCCAAGTAATGAGCTCTGCAGGAGAGGTTGTATTCAGTAATTTGTCACTACTTCTCTGTGTGGGATTATGTA
TTGGCTTAGCGAAACGAGATAAAGGAACCGCTGCGTTAGCAGGAGTAACTGGTTACTTAGTTATGACTGCAACGA
TCAAAGCTTTGGTAAAACTTTTTATGGCAGAAGGATCTGCAATTGATACTGGAGTTATTGGAGCATTAGTTGTCGG
AATAGTTGCCGTATATTTGCACAACCGATATAACAATATTCAATTACCTTCCGCTTTAGGATTCTTTGGAGGTTCA
CGCTTCGTTCCTATTGTTACATCGTTCTCTTCTATCTTGATTGGCTTTGTCTTCTTTGTTATTTGGCCACCTTTCCAA
CAACTTCTTGTTTCTACAGGTGGATATATTTCTCAGGCGGGTCCAATTGGAACTTTTCTATATGGATTTTTAATGAG
ACTTTCTGGAGCAGTAGGCTTACATCATATAATTTACCCTATGTTTTGGTATACTGAACTTGGTGGTGTTGAAACTG
TTGCAGGACAAACAGTGGTTGGAGCTCAAAAAATATTTTTTGCTCAATTAGCCGATTTGGCCCATTCTGGATTATT
TACAGAAGGAACAAGGTTTTTTGCAGGTCGTTTCTCAACAATGATGTTCGGTTTACCGGCTGCCTGTTTAGCGATG
TACCATAGTGTTCCTAAAAATCGTCGTAAAAAATACGCGGGTTTGTTTTTTGGAGTTGCTTTAACATCTTTTATTAC
CGGTATTACAGAACCAATTGAATTTATGTTTCTATTCGTCAGTCCGGTTCTATATGTTGTTCACGCATTCCTTGATG
GTGTTAGCTTCTTTATTGCAGACGTCTTAAATATTTCAATAGGAAACACATTTTCAGGAGGTGTAATCGATTTCACT
TTATTTGGAATTTTGCAGGGGAACGCTAAGACGAATTGGGTTCTTCAGATTCCATTTGGACTTATTTGGAGTGTTTT
GTATTATATTATTTTTAGATGGTTCATTACTCAATTCAACGTTCTAACGCCAGGGCGAGGAGAAGAAGTAGATTCT
AAAGAAATTTCTGAATCCGCAGATTCAACTTCAAATACTGCAGATTATTTAAAACAGGATAGCCTACAAATTATCA
GAGCCTTGGGTGGATCAAATAATATAGAAGATGTAGATGCTTGTGTGACACGTTTACGTGTAGCTGTAAAAGAAG
TTAATCAAGTTGATAAAGCACTTTTAAAACAAATTGGTGCAGTTGATGTCTTAGAAGTGAAGGGTGGCATTCAAGC
AATCTATGGAGCAAAAGCAATCTTATATAAAAATAGTATTAATGAAATTTTAGGTGTAGATGATTAA

4188.11
ATGAAATTTAGAAAATTAGCTTGTACAGTACTTGCGGGTGCTGCGGTTCTTGGTCTTGCTGCTTGTGGCAATTCTG
GCGGAAGTAAAGATGCTGCCAAATCAGGTGGTGACGGTGCCAAAACAGAAATCACTTGGTGGGCATTCCCAGTAT
TTACCCAAGAAAAAACTGGTGACGGTGTTGGAACTTATGAAAAATCAATCATCGAAGCGTTTGAAAAAGCAAACC
CAGATATAAAAGTGAAATTGGAAACCATCGACTTCAAGTCAGGTCCTGAAAAAATCACAACAGCCATCGAAGCAG
GAACAGCTCCAGACGTACTCTTTGATGCACCAGGACGTATCATCCAATACGGTAAAAACGGTAAATTGGCTGAGT
TGAATGACCTCTTCACAGATGAATTTGTTAAAGATGTCAACAATGAAAACATCGTACAAGCAAGTAAAGCTGGAG
ACAAGGCTTATATGTATCCGATTAGTTCTGCCCCATTCTACATGGCAATGAACAAGAAAATGTTAGAAGATGCTGG
AGTAGCAAACCTTGTAAAAGAAGGTTGGACAACTGATGATTTTGAAAAAGTATTGAAAGCACTTAAAGACAAGGG
TTACACACCAGGTTCATTGTTCAGTTCTGGTCAAGGGGGAGACCAAGGAACACGTGCCTTTATCTCTAACCTTTAT

AGCGGTTCTGTAACAGATGAAAAAGTTAGCAAATATACAACTGATGATCCTAAATTCGTCAAAGGTCTTGAAAAA
GCAACTAGCTGGATTAAAGACAATTTGATCAATAATGGTTCACAATTTGACGGTGGGGCAGATATCCAAAACTTT
GCCAACGGTCAAACATCTTACACAATCCTTTGGGCACCAGCTCAAAATGGTATCCAAGCTAAACTTTTAGAAGCA
AGTAAGGTAGAAGTGGTAGAAGTACCATTCCCATCAGACGAAGGTAAGCCAGCTCTTGAGTACCTTGTAAACGGG
TTTGCAGTATTCAACAATAAAGACGACAAGAAAGTCGCTGCATCTAAGAAATTCATCCAGTTTATCGCAGATGAC
AAGGAGTGGGGACCTAAAGACGTAGTTCGTACAGGTGCTTTCCCAGTCCGTACTTCATTTGGAAAACTTTATGAAG
ACAAACGCATGGAAACAATCAGCGGCTGGACTCAATACTACTCACCATACTACAACACTATTGATGGATTTGCTG
AAATGAGAACACTTTGGTTCCCAATGTTGCAATCTGTATCAAATGGTGACGAAAAACCAGCAGATGCTTTGAAAG
CCTTCACTGAAAAAGCGAACGAAACAATCAAAAAAGCTATGAAACAATAG

4188.12
ATGCAATCTACAGAAAAAAAACCATTAACAGCCTTTACTGTTATTTCAACAATCATTTTGCTCTTGTTGACTGTGC
TGTTCATCTTTCCATTCTACTGGATTTTGACAGGGGCATTCAAATCACAACCTGATACAATTGTTATTCCTCCTCAG
TGGTTCCCTAAAATGCCAACCATGGAAAACTTCCAACAACTCATGGTGCAGAACCCTGCCTTGCAATGGATGTGG
AACTCAGTATTTATCTCATTGGTAACCATGTTCTTAGTTTGTGCAACCTCATCTCTAGCAGGTTATGTATTGGCTAA
AAAACGTTTCTATGGTCAACGCATTCTATTTGCTATCTTTATCGCTGCTATGGCGCTTCCAAAACAAGTTGTCCTTG
TACCATTGGTACGTATCGTCAACTTCATGGGAATCCATGATACTCTCTGGGCAGTTATCTTGCCTTTGATTGGATG
GCCATTCGGTGTCTTCCTCATGAAACAGTTCAGTGAAAATATCCCTACAGAGTTGCTTGAATCAGCTAAAATCGAC
GGTTGTGGTGAGATTCGTACCTTCTGGAGTGTAGCCTTCCCGATTGTGAAACCAGGGTTTGCAGCCCTTGCAATCT
TTACCTTCATCAATACTTGGAATGACTACTTCATGCAATTGGTAATGTTGACTTCACGTAACAATTTGACCATCTCA
CTTGGGGTTGCGACCATGCAGGCTGAAATGGCAACCAACTATGGTTTGATTATGGCAGGAGCTGCCCTTGCTGCTG
TTCCAATCGTCACAGTCTTCCTAGTCTTCCAAAAATCCTTCACACAGGGTATTACTATGGGAGCGGTCAAAGGATA
A

4191.1
ATGAAAAAAACTTTTTTCTTACTGGTGTTAGGCTTGTTTTGCCTTCTTCCACTCTCTGTTTTTGCCATTGATTTCAAG
ATAAACTCTTATCAAGGGGATTTGTATATTCATGCAGACAATACGGCAGAGTTTAGACAGAAGATAGTTTACCAGT
TTGAGGAGGACTTTAAGGGCCAAATCGTGGGACTTGGACGTGCTGGTAAGATGCCTAGCGGGTTTGACATTGACC
CTCATCCAAAGATTCAGGCCGCGAAAAACGGTGCAGAACTAGCAGATGTGACTAGCGAAGTAACAGAAGAAGCG
GATGGTTATACTGTGAGAGTCTATAATCCAGGTCAGGAGGGCGACATAGTTGAAGTTGACCTCGTCTGGAACTTA
AAAAATTTACTTTTCCTTTATGATGATATCGCTGAATTAAATTGGCAACCTCTGACAGATAGTTCAGAGTCTATTG
AAAAGTTTGAATTTCATGTAAGGGGAGACAAGGGGGCTGAAAAACTCTTTTTCCATACAGGGAAACTTTTTAGAG
AGGGAACGATTGAAAAGAGTAACCTTGATTATACTATCCGTTTAGACAATCTTCCGGCTAAGCGTGGAGTTGAGTT
GCATGCCTATTGGCCTCGGACCGATTTTGCTAGCGCTAGGGATCAGGGATTGAAAGGGAATCGTTTAGAAGAGTTT
AATAAGATAGAAGACTCGATTGTTAGAGAAAAAGATCAGAGTAAACAACTCGTTACTTGGGTCCTCCCTTCGATC
CTTTCCATCTCCTTGTTATTGAGTGTCTGCTTCTATTTTATTTATAGAAGAAAGACCACTCCTTCAGTCAAATATGC
CAAAAATCATCGTCTCTATGAACCACCAATGGAATTAGAGCCTATGGTTTTATCAGAAGCAGTCTACTCGACCTCC
TTGGAGGAAGTGAGTCCCTTGGTCAAGGGAGCTGGAAAATTCACCTTTGATCAACTTATTCAAGCTACCTTGCTAG
ATGTGATAGACCGTGGGAATGTCTCTATCATTTCAGAAGGAGATGCAGTTGGTTTGAGGCTAGTAAAAGAAGATG
GTTTGTCAAGCTTTGAGAAAGACTGCCTAAATCTAGCTTTTTCAGGTAAAAAAGAAGAAACTCTTTCCAATTTGTT
TGCGGATTACAAGGTATCTGATAGTCTTTATCGTAGAGCCAAAGTTTCTGATGAAAAACGGATTCAAGCAAGAGG
GCTTCAACTCAAATCTTCTTTTGAAGAGGTATTGAACCAGATGCAAGAAGGAGTGAGAAAACGAGTTTCCTTCTGG
GGGCTCCCAGATTATTATCGTCCTTTAACTGGTGGGGAAAAGGCCTTGCAAGTGGGTATGGGTGCCTTGACTATCC
TGCCCCTATTTATCGGATTTGGTTTGTTCTTGTACAGTTTAGACGTTCATGGCTATCTTTACCTCCCTTTGCCAATA
CTTGGTTTTCTAGGGTTAGTTTTGTCTGTTTTCTATTATTGGAAGCTTCGACTAGATAATCGTGATGGTGTTCTAAA
TGAAGCGGGAGCTGAGGTCTACTATCTCTGGACCAGTTTTGAAAATATGTTGCGTGAGATTGCACGATTGGATCAG
GCTGAACTGGAAAGTATTGTGGTCTGGAATCGCCTCTTGGTCTATGCGACCTTATTTGGCTATGCGGACAAGGTTA
GTCATTTGATGAAGGTTCATCAGATTCAAGTGGAAAATCCAGATATCAATCTCTATGTAGCTTATGGCTGGCACAG
TACGTTTTATCATTCAACAGCACAAATGAGCCATTATGCTAGTGTCGCAAATACAGCAAGCACCTACTCTGTATCT
TCTGGAAGTGGAAGTTCTGGTGGTGGCTTCTCTGGAGGCGGAGGTGGCGGCAGTATCGGTGCCTTTTAA

4191.2
ATGAAAAAAGTAAGAAAGATATTTCAGAAGGCAGTTGCAGGACTGTGCTGTATATCTCAGTTGACAGCTTTTTCTT
CGATAGTTGCTTTAGCAGAAACGCCTGAAACCAGTCCAGCGATAGGAAAAGTAGTGATTAAGGAGACAGGCGAAG
GAGGAGCGCTTCTAGGAGATGCCGTCTTTGAGTTGAAAAACAATACGGATGGCACAACTGTTTCGCAAAGGACAG
AGGCGCAAACAGGAGAAGCGATATTTTCAAACATAAAACCTGGGACATACACCTTGACAGAAGCCCAACCTCCAG
TTGGTTATAAACCCTCTACTAAACAATGGACTGTTGAAGTTGAGAAGAATGGTCGGACGACTGTCCAAGGTGAAC
AGGTAGAAAATCGAGAAGAGGCTCTATCTGACCAGTATCCACAAACAGGGACTTATCCAGATGTTCAAACACCTT
ATCAGATTATTAAGGTAGATGGTTCGGAAAAAAACGGACAGCACAAGGCGTTGAATCCGAATCCATATGAACGTG
TGATTCCAGAAGGTACACTTTCAAAGAGAATTTATCAAGTGAATAATTTGGATGATAACCAATATGGAATCGAATT
GACGGTTAGTGGGAAAACAGTGTATGAACAAAAAGATAAGTCTGTGCCGCTGGATGTCGTTATCTTGCTCGATAA
CTCAAATAGTATGAGTAACATTCGAAACAAGAATGCTCGACGTGCGGAAAGAGCTGGTGAGGCGACACGTTCTCT
TATTGATAAAATTACATCTGATTCAGAAAATAGGGTAGCGCTTGTGACTTATGCTTCCACTATCTTTGATGGGACC
GAGTTTACAGTAGAAAAAGGGGTAGCAGATAAAAACGGAAAGCGATTGAATGATTCTCTTTTTTGGAATTATGAT
CAGACGAGTTTTACAACCAATACCAAAGATTATAGTTATTTAAAGCTGACTAATGATAAGAATGACATTGTAGAAT

TAAAAAATAAGGTACCTACCGAGGCAGAAGACCATGATGGAAATAGATTGATGTACCAATTCGGTGCCACTTTTA
CTCAGAAAGCTTTGATGAAGGCAGATGAGATTTTGACACAACAAGCGAGACAAAATAGTCAAAAGTCATTTTCC
ATATTACGGATGGTGTCCCAACTATGTCGTATCCGATTAATTTTAATCATGCTACGTTTGCTCCATCATATCAAAAT
CAACTAAATGCATTTTTTAGTAAATCTCCTAATAAAGATGGAATACTATTAAGTGATTTTATTACGCAAGCAACTA
GTGGAGAACATACAATTGTACGCGGAGATGGGCAAAGTTACCAGATGTTTACAGATAAGACAGTTTATGAAAAAG
GTGCTCCTGCAGCTTTCCCAGTTAAACCTGAAAAATATTCTGAAATGAAGGCGGCTGGTTATGCAGTTATAGGCGA
TCCAATTAATGGTGGATATATTTGGCTTAATTGGAGAGAGAGTATTCTGGCTTATCCGTTTAATTCTAATACTGCTA
AAATTACCAATCATGGTGACCCTACAAGATGGTACTATAACGGGAATATTGCTCCTGATGGGTATGATGTCTTTAC
GGTAGGTATTGGTATTAACGGAGATCCTGGTACGGATGAAGCAACGGCTACTAGTTTTATGCAAAGTATTTCTAGT
AAACCTGAAAACTATACCAATGTTACTGACACGACAAAAAATATTGGAACAGTTGAATCGTTATTTCCACACCATC
GTAACTGAAAAGAAATCAATTGAGAATGGTACGATTACAGATCCGATGGGTGAGTTAATTGATTTGCAATTGGGC
ACAGATGGAAGATTTGATCCAGCAGATTACACTTTAACTGCAAACGATGGTAGTCGCTTGGAGAATGGACAAGCT
GTAGGTGGTCCACAAAATGATGGTGGTTTGTTAAAAAATGCAAAAGTGCTCTATGATACGACTGAGAAAAGGATT
CGTGTAACAGGTCTGTACCTTGGAACGGATGAAAAAGTTACGTTGACCTACAATGTTCGTTTGAATGATGAGTTTG
TAAGCAATAAATTTTATGATACCAATGGTCGAACAACCTTACATCCTAAGGAAGTAGAACAGAACACAGTGCGCG
ACTTCCCGATTCCTAAGATTCGTGATGTGCGGAAGTATCCAGAAATCACAATTTCAAAAGAGAAAAAACTTGGTG
ACATTGAGTTTATTAAGGTCAATAAAAATGATAAAAAACCACTGAGAGGTGCGGTCTTTAGTCTTCAAAAACAAC
ATCCGGATTATCCAGATATTTATGGAGCTATTGATCAAAATGGCACTTATCAAAATGTGAGAACAGGTGAAGATG
GTAAGTTGACCTTTAAAAATCTGTCAGATGGGAAATATCGATTATTTGAAAATTCTGAACCAGCTGGTTATAAACC
CGTTCAAAATAAGCCTATCGTTGCCTTCCAAATAGTAAATGGAGAAGTCAGAGATGTGACTTCAATCGTTCCACAA
GATATACCAGCGGGTTACGAGTTTACGAATGATAAGCACTATATTACCAATGAACCTATTCCTCCAAAGAGAGAA
TATCCTCGAACTGGTGGTATCGGAATGTTGCCATTCTATCTGATAGGTTGCATGATGATGGGAGGAGTTCTATTAT
ACACACGGAAACATCCGTAA

4191.3
ATGAAATCAATCAACAAATTTTTAACAATGCTTGCTGCCTTATTACTGACAGCGAGTAGCCTGTTTTCAGCTGCAA
CAGTTTTTGCGGCTGGGACGACAACAACATCTGTTACCGTTCATAAACTATTGGCAACAGATGGGGATATGGATA
AAATTGCAAATGAGTTAGAAACAGGTAACTATGCTGGTAATAAAGTGGGTGTTCTACCTGCAAATGCAAAAGAAA
TTGCCGGTGTTATGTTCGTTTGGACAAATACTAATAATGAAATTATTGATGAAAATGGCCAAACTCTAGGAGTGAA
TATTGATCCACAAACATTTAAACTCTCAGGGGCAATGCCGGCAACTGCAATGAAAAAATTAACAGAAGCTGAAGG
AGCTAAATTTAACACGGCAAATTTACCAGCTGCTAAGTATAAAATTTATGAAATTCACAGTTTATCAACTTATGTC
GGTGAAGATGGAGCAACCTTAACAGGTTCTAAAGCAGTTCCAATTGAAATTGAATTACCATTGAACGATGTTGTG
GATGCGCATGTGTATCCAAAAAATACAGAAGCAAAGCCAAAAATTGATAAAGATTTCAAAGGTAAAGCAAATCCA
GATACACCACGTGTAGATAAAGATACACCTGTGAACCACCAAGTTGGAGATGTTGTAGAGTACGAAATTGTTACA
AAAATTCCAGCACTTGCTAATTATGCAACAGCAAACTGGAGCGATAGAATGACTGAAGGTTTGGCATTCAACAAA
GGTACAGTGAAAGTAACTGTTGATGATGTTGCACTTGAAGCAGGTGATTATGCTCTAACAGAAGTAGCAACTGGTT
TTGATTTGAAATTAACAGATGCTGGTTTAGCTAAAGTGAATGACCAAAACGCTGAAAAAACTGTGAAAATCACTT
ATTCGGCAACATTGAATGACAAAGCAATTGTAGAAGTACCAGAATCTAATGATGTAACATTTAACTATGGTAATA
ATCCAGATCACGGGAATACTCCAAAGCCGAATAAGCCAAATGAAAACGGCGATTTGACATTGACCAAGACATGGG
TTGATGCTACAGGTGCACCAATTCCGGCTGGAGCTGAAGCAACGTTCGATTTGGTTAATGCTCAGACTGGTAAAGT
TGTACAAACTGTAACTTTGACAACAGACAAAAATACAGTTACTGTTAACGGATTGGATAAAAATACAGAATATAA
ATTCGTTGAACGTAGTATAAAAGGGTATTCAGCAGATTATCAAGAAATCACTACAGCTGGAGAAATTGCTGTCAA
GAACTGGAAAGACGAAAATCCAAAACCACTTGATCCAACAGAGCCAAAAGTTGTTACATATGGTAAAAAGTTTGT
CAAAGTTAATGATAAAGATAATCGTTTAGCTGGGGCAGAATTTGTAATTGCAAATGCTGATAATGCTGGTCAATAT
TTAGCACGTAAAGCAGATAAAGTGAGTCAAGAAGAGAAGCAGTTGGTTGTTACAACAAAGGATGCTTTAGATAGA
GCAGTTGCTGCTTATAACGCTCTTACTGCACAACAACAAACTCAGCAAGAAAAAGAGAAAGTTGACAAAGCTCAA
GCTGCTTATAATGCTGCTGTGATTGCTGCCAACAATGCATTTGAATGGGTGGCAGATAAGGACAATGAAAATGTTG
TGAAATTAGTTTCTGATGCACAAGGTCGCTTTGAAATTACAGGCCTTCTTGCAGGTACATATTACTTAGAAGAAAC
AAAACAGCCTGCTGGTTATGCATTACTAACTAGCCGTCAGAAATTTGAAGTCACTGCAACTTCTTATTCAGCGACT
GGACAAGGCATTGAGTATACTGCTGGTTCAGGTAAAGATGACGCTACAAAAGTAGTCAACAAAAAAATCACTATC
CCACAAACGGGTGGTATTGGTACAATTATCTTTGCTGTAGCGGGGGCTGCGATTATGGGTATTGCAGTGTACGCAT
ATGTTAAAAACAACAAAGATGAGGATCAACTTGCTTAA

4191.4
ATGACAATGCAGAAAATGCAGAAAATGATTAGTCGTATCTTCTTTGTTATGGCTCTGTGTTTTTCTCTTGTATGGGG
TGCACATGCAGTCCAAGCGCAAGAAGATCACACGTTGGTCTTGCAATTGGAGAACTATCAGGAGGTGGTTAGTCA
ATTGCCATCTCGTGATGGTCATCGGTTGCAAGTATGGAAGTTGGATGATTCGTATTCCTATGATGATCGGGTGCAA
ATTGTAAGAGACTTGCATTCGTGGGATGAGAATAAACTTTCTTCTTTCAAAAAGACTTCGTTTGAGATGACCTTCC
TTGAGAATCAGATTGAAGTATCTCATATTCCAAATGGTCTTTACTATGTTCGCTCTATTATCCAGACGGATGCGGT
TTCTTATCCAGCTGAATTTCTTTTTGAAATGACAGATCAAACGGTAGAGCCTTTGGTCATTGTAGCGAAAAAAACA
GATACAATGACAACAAAGGTGAAGCTGATAAAGGTGGATCAAGACCACAATCGCTTGGAGGGTGTCGGCTTTAAA
TTGGTATCAGTAGCAAGAGATGTTTCTGAAAAAGAGGTTCCCTTGATTGGAGAATACCGTTACAGTTCTTCTGGTC
AAGTAGGGAGAACTCTCTATACTGATAAAAATGGAGAGATTTTTGTGACAAATCTTCCTCTTGGGAACTATCGTTT

CAAGGAGGTGGAGCCACTGGCAGGCTATGCTGTTACGACGCTGGATACGGATGTCCAGCTGGTAGATCATCAGCT
GGTGACGATTACGGTTGTCAATCAGAAATTACCACGTGGCAATGTTGACTTTATGAAGGTGGATGGTCGGACCAA
TACCTCTCTTCAAGGGGCAATGTTCAAAGTCATGAAAGAAGAAAGCGGACACTATACTCCTGTTCTTCAAAATGGT
AAGGAAGTAGTTGTAACATCAGGGAAAGATGGTCGTTTCCGAGTGGAAGGTCTAGAGTATGGGACATACTATTTA
TGGGAGCTCCAAGCTCCAACTGGTTATGTTCAATTAACATCGCCTGTTTCCTTTACAATCGGGAAAGATACTCGTA
AGGAACTGGTAACAGTGGTTAAAAATAACAAGCGACCACGGATTGATGTGCCAGATACAGGGGAAGAAACCCTTG
TATATCTTGATGCTTGTTGCCATTTTGTTGTTTGGTAG

4191.5
ATGAGCCACATATACTTATCTATTTTCACAAGTCTCTTGCTGATGCTAGGACTTGTCAATGTTGCTCAAGCCGATG
AATATTTACGCATCGGTATGGAAGCAGCATATGCTCCCTTTAACTGGACCCAGGATGATGATAGCAACGGAGCTG
TCAAAATCGATGGGACCAATCAGTATGCCAACGGATACGATGTTCAAATCGCCAAGAAAATCGCTAAGGACTTAG
GTAAAGAACCTTTGGTTGTTAAAACCAAGTGGGAAGGTCTAGTCCCTGCCCTTACTTCTGGTAAGATTGACATGAT
TATCGCAGGTATGAGTCCAACTGCAGAACGCAAACAAGAAATTGCCTTTTCGAGCAGTTACTATACTAGCGAACC
AGTTTTGCTTGTCAAAAAAGATTCTGCCTACGCAAGTGCTAAATCTTTGGATGACTTTAACGGTGCAAAAATCACT
TCTCAACAAGGGGTCTACCTTTATAACTTGATTGCACAAATCCCAGGTGCTAAAAAAGAAACAGCCATGGGAGAC
TTCGCTCAAATGCGACAAGCTCTTGAGGCTGGTGTCATTGATGCTTATGTTTCTGAACGTCCAGAAGCACTGACTG
CTGAAGCTGCGAACTCTAAGTTCAAGATGATTCAAGTAGAACCTGGTTTCAAAACTGGGGAAGAAGATACAGCTA
TCGCTATCGGGCTTCGTAAAAATGACAATCGTATTAGCCAAATCAATGCCAGCATTGAAACCATTTCAAAAGATG
ACCAAGTTGCCTTGATGGATCGTATGATCAAGGAACAACCTGCCGAAGCTACAACAACTGAAGAGACTAGCAGTA
GTTTCTTTAGCCAAGTTGCTAAAATTCTTTCTGAAAACTGGCAACAACTCTTGCGTGGTGCTGGTATCACTCTTTTA
ATCTCTATCGTCGGAACCATCATAGGTCTCATTATTGGACTTGCCATTGGTGTCTTCCGTACTGCTCCTCTCTCTGA
AAACAAAGTCATTTACGGCCTACAAAAACTAGTCGGCTGGGTTCTCAATGTCTACATTGAAATTTTCCGTGGTACG
CCAATGATTGTTCAATCGATGGTTATCTACTATGGAACTGCCCAAGCTTTCGGGATCAACC
TTGACCGTACACTGGCTGCTATCTTCATCGTTTCAATCAATACCGGTGCCTACATGACTGAAATCGTCCGTGGTGG
TATCCTAGCAGTTGACAAGGGACAATTTGAAGCTGCGACTGCTCTTGGTATGACCCATAACCAGACCATGCGTAA
GATTGTCCTACCTCAGGTAGTCCGTAACATCCTACCTGCAACTGGTAATGAATTTGTCATCAATATCAAAGATACA
TCTGTATTGAACGTTATCTCTGTTGTCGAACTTTATTTCTCAGGAAATACCGTGGCAACACAAACCTATCAATACTT
CCAGACATTTACAATCATCGCCGTGATTTACTTTGTCCTCACCTTCACCGTAACACGTATCCTACGCTTTATCGAGC
GCAGAATGGACATGGATACCTACACTACAGGTGCTAACCAAATGCAAACGGAGGATTTGAAATAA

4191.6
ATGACACAAGCAATCCTTGAAATTAAACACCTCAAAAAATCCTATGGACAAAACGAAGTGCTAAAAGACATTTCA
CTCACTGTCCACAAGGGAGAGGTCATCTCTATCATCGGAAGCTCTGGAAGCGGAAAATCGACCTTCCTACGCTCC
ATTAACCTACTTGAAACACCAACTGATGGACAAATCCTTTATCATGGACAAAACGTCCTCGAAAAAGGCTATGAC
CTCACGCAATACCGTGAAAAGTTGGGGATGGTTTTCCAATCCTTTAACCTCTTTGAAAATCTCAATGTTCTTGAAA
ACACAATCGTCGCTCAGACAACTGTCCTAAAACGCGAACGCACAGAAGCTGAAAAGATTGCCAAAGAAAACCTG
GAAAAGGTCGGCATGGGAGAACGCTACTGGCAAGCCAAACCAAAACAACTCTCAGGTGGTCAAAAACAACGTGT
GGCCATCGCTCGTGCCCTCTCCATGAATCCGGACGCTATTCTCTTTGATGAACCAACATCAGCTCTCGATCCAGAA
ATGGTTGGAGAAGTCCTCAAAATCATGCAGGACCTGGCTCAGGAAGGCTTGACCATGATTGTCGTAACCCATGAA
ATGGAATTTGCCCGTGATGTCTCTCACCGTGTTATCTTTATGGATAAGGGCGTGATCGCTGAAGAAGGTAAACCAG
AAGACCTCTTCACCAATCCTAAAGAAGACCGAACAAAAGAGTTCCTTCAACGCTATCTCAAATAA

4192.3
ATGAAAAAGTATCAACTTCTATTCAAAATAAGTGCAGTCTTCTCTTACTTATTTTTCGTATTTAGTCTTTCTCAGCT
GACGCTTATCGTCCAAAACTATTGGCAATTTTCTTCTCAGATAGGCAATTTATTCTGGATTCAAAATATCTTGAGTT
TACTTTTTATTGGAGTCATGATTGTGGTTCTTGTTAAGACAGGCCATGGTTATCTCTTCCGCATTCCAAGAAAAAA
ATGGCTTTGGTATTCGATTTTGACAGTATTAGTGCTAGTGTTCCAGATCTCTTTTAACGTTCAGACAGCTAAACATG
TTCAGTCAACTGCGGAAGGTTGGGCTGTATTGATTGGTTATAGTGGGACTAACTTTGCAGAGCTAGGTATTTATAT
AGCCCTGTTCTTTCTGGTTCCACTGATGGAAGAATTGATTTATAGAGGATTACTGCAACATGCTTTCTTTAAGCATT
CGCGATTTGGTCTTGATTTGCTTCTTCCTTCTATTTTATTTGCTCTCCCTCATTTTTCAAGCCTGCCTAGTCTGTTAG
ATATCTTCGTCTTTGCAACAGTTGGAATCATCTTTGCTGGTTTGACCCGCTATACCAAGAGCATTTATCCATCCTAT
GCGGTGCATGTGATCAATAATATTGTAGCGACCTTCCCGTTTTTGCTCACTTTTCTACATAGGGTCTTGGGGTAA

4193.1
ATGAACAAGAAACAATGGCTAGGTCTTGGCCTAGTTGCAGTGGCAGCAGTTGGACTTGCTGCATGTGGTAACCGC
TCTTCTCGTAACGCAGCTTCATCTTCTGATGTGAAGACAAAAGCAGCAATCGTCACTGATACTGGTGGTGTTGATG
ACAAATCATTCAACCAATCAGCTTGGGAAGGTTTGCAGGCTTGGGGTAAAGAACACAATCTTTCAAAAGATAACG
GTTTCACTTACTTCCAATCAACAAGTGAAGCTGACTACGCTAACAACTTGCAACAAGCGGCTGGAAGTTACAACCT
AATCTTCGGTGTTGGTTTTGCCCTTAATAATGCAGTTAAAGATGCAGCAAAAGAACACACTGACTTGAACTATGTC
TTGATTGATGATGTGATTAAAGACCAAAAGAATGTTGCGAGCGTAACTTTCGCTGATAATGAGTCAGGTTACcTTG
CAGGTGTGGCTGCAGCAAAAACAACTAAGACAAAACAAGTTGGTTTTGTAGGTGGTATCGAATCTGAAGTTATCT
CTCGTTTTGAAGCAGGATTCAAGGCTGGTGTTGCGTCAGTAGACCCATCTATCAAAGTCCAAGTTGACTACGCTGG
TTCATTTGGTGATGCGGCTAAAGGTAAAACAATTGCAGCCGCACAATACGCAGCCGGTGCAGATATTGTTTACCA
AGTAGCTGGTGGTACAGGTGCAGGTGTCTTTGCAGAGGCAAAATCTCTCAACGAAAGCCGTCCTGAAAATGAAAA

AGTTTGGGTTATCGGTGTTGATCGTGACCAAGAAGCAGAAGGTAAATACACTTCTAAAGATGGCAAAGAATCAAA
CTTTGTTCTTGTATCTACTTTGAAACAAGTTGGTACAACTGTAAAAGATATTTCTAACAAGGCAGAAAGAGGAGAA
TTCCCTGGCGGTCAAGTGATCGTTTACTCATTGAAGGATAAAGGGGTTGACTTGGCAGTAACAAACCTTTCAGAAG
AAGGTAAAAAAGCTGTCGAAGATGCAAAAGCTAAAATCCTTGATGGAAGCGTAAAAGT
TCCTGAAAAATAA

4193.3
ATGTCTAAAAAATTACAACAAATTTCGGTTCCCTTGATTTCTGTATTCCTAGGAATTTTACTCGGAGCCATTGTCAT
GTGGATCTTCGGTTATGATGCTATTTGGGGCTACGAAGAATTGTTCTATACAGCCTTTGGCAGTCTGCGTGGGATT
GGAGAAATCTTCCGTGCTATGGGTCCTCTGGTCTTGATTGGTCTTGGTTTTGCCGTTGCCAGTCGAGCTGGTTTCTT
TAACGTCGGACTTCCTGGTCAGGCTTTGGCAGGTTGGATTCTCAGTGGTTGGTTTGCCCTGTCGCATCCAGATATG
CCCCGTCCCTTGATGATTCTAGCAACCATCGTGATTGCCTTGATTGCTGGTGGGATTGTCGGAGCGATTCCAGGTA
TTCTTAGGGCCTATCTAGGGACGTCAGAGGTTATTGTAACCATCATGATGAACTACATTGTCTTGTATGTAGGGAA
TGCCTTTATCCATGCTTTCCCTAAAGACTTCATGCAAAGTACAGATTCGACCATTCGTGTTGGGGCTAATGCAACC
TATCAGACACCTTGGTTGGCTGAGTTGACTGGTAACTCACGGATGAATATTGGTATTTTCTTTGCCATCATTGCCGT
TGCAGTTATTTGGTTCATGCTCAAGAAAACAACTCTTGGTTTTGAAATCCGTGCAGTTGGTCTTAATCCACATGCTT
CAGAATATGCTGGTATTTCTGCCAAGCGGACTATTATCCTATCTATGATTATTTCAGGTGCCTTGGCAGGTCTTGGT
GGAGCTGTTGAAGGTTTGGGAACCTTCCAGAACGTCTATGTTCAAGGTTCGTCATTAGCTATCGGATTTAACGGAA
TGGCGGTTAGTTTGCTTGCGGCCAACTCACCAATTGGTATACTCTTTGCAGCCTTCCTATTTGGCGTTCTCCAAGTT
GGGGCTCCTGGTATGAATGCGGCGCAGGTACCATCTGAGCTTGTCAGCATTGTAACAGCGTCTATTATCTTCTTTG
TCAGTGTTCATTACCTTATCGAACGCTTTGTCAAACCGAAAAAACAAGTTAAAGGAGGTAAGTAA

4194.1
ATGGGAGTGAAAAAGAAACTAAAGTTGACTAGTTTGCTAGGACTGTCTCTGTTAATCATGACAGCCTGTGCGACT
AATGGGGTAACTAGCGATATTACAGCCGAATCGGCTGATTTTTGGAGTAAATTGGTTTACTTCTTTGCGGAAATCA
TTCGCTTTTTATCGTTTGATATTAGTATCGGAGTGGGGATTATTCTCTTTACGGTCTTGATTCGTACAGTCCTCTTG
CCAGTCTTTCAGGTGCAAATGGTGGCTTCTAGGAAAATGCAGGAAGCTCAGCCACGCATTAAGGCGCTTCGAGAA
CAATATCCAGGTCGAGATATGGAAAGCAGAACCAAACTAGAGCAGGAAATGCGTAAAGTATTTAAAGAAATGGG
TGTCAGACAGTCAGACTCTCTTTGGCCGATTTTGATTCAGATGCCGGTTATTTTGGCCCTGTTCCAAGCCCTATCAA
GAGTTGACTTTTTAAAGACAGGTCATTTCTTATGGATTAACCTTGGTAGTGTGGATACAACCCTTGTTCTTCCGATT
TTAGCAGCAGTATTCACCTTTTTAAGTACTTGGTTGTCCAACAAAGCTTTGTCTGAGCGAAATGGCGCTACGACTG
CGATGATGTATGGGATTCCAGTCTTGATTTTTATCTTTGCAGTTTATGCGCCAGGTGGAGTCGCCCTATACTGGAC
AGTGTCTAATGCTTATCAAGTCTTGCAAACCTATTTCTTGAATAATCCATTCAAGATTATCGCAGAGCGCGAGGCC
GTAGTACAGGCACAAAAAGATTTGGAAAATAGAAAAAGAAAAGCCAAGAAAAAGGCTCAGAAAACGAAATAA

4194.4
ATGGTTATCGATCCATTTGCTATCAACGAACTAGACTATTACTTAGTTTCACACTTCCACAGTGATCATATCGACC
CATACACAGCTGCAGCAATTCTCAATAATCCTAAGTTAGAGCATGTTAAGTTTATCGGTCCTTACCACTGTGGACG
AATCTGGGAAGGATGGGGTGTTCCAAAAGAACGTATCATCGTTGTTAAACCAGGTGACACTATCGAATTAAAAGA
TATGAAGATTCATGCAGTAGAATCATTTGACCGTACTTGCTTGGTAACTCTCCCAGTGAACGGTGCTGATGAGACA
GGCGGTGAACTTGCTGGCTTGGCTGTTACAGATGAAGAAATGGCTCAAAAGGCTGTTAACTATATCTTTGAAACAC
CAGGTGGAACCATCTATCATGGTGCAGATTCTCACTTCTCAAACTATTTTGCAAAACATGGTAAAGACTTTAAAAT
TGATGTTGCTTTGAATAACTATGGTGAAAATCCGGTAGGTATCCAAGACAAAATGACATCTATCGACCTTCTTCGT
ATGGCAGAAAATCTGCGTACCAAAGTCATTATCCCAGTTCACTATGATATCTGGTCTAACTTCATGGCTTCTACTA
ATGAGATTCTAGAACTTTGGAAAATGCGAAAAGATCGCTTGCAATACGATTTCCATCCATTTATCTGGGAAGTTGG
CGGTAAGTACACTTATCCTCAAGATCAACACTTAGTAGAATACCATCATCCACGTGGTTTTGATGATTGTTTTGAA
CAAGACTCTAACATTCAATTTAAAGCTTTGCTATAA

4196.2
ATGTTCCTTTCAGGCTGGTTGTCTAGTTTTGCTAATACTTATATCCATGATTTACTGGGGGTTCTTTTCCCAGATAG
TCCATTTTTAAATGCCTTTGAAAGTGCTATTGCGGCTCCTTTGGTAGAAGAACCCTTGAAATTATTGTCACTTGTTT
TTGTTTTGGCTTTGATTCCTGTGCGAAAATTAAAATCTTTGTTTTTACTTGGAATTGCTTCCGGTTTGGGATTCCAA
ATGATTAAGGATATTGGTTATATTCGTACGGATTTGCCAGAGGGCTTTGACTTTACTATTTCGCGAATTTTAGAGC
GTATCATCTCAGGAATTGCCTCTCACTGGACTTTTTCAGGTCTAGCTGTAGTAGGTGTTTACTTGCTTTACAGAGCC
TATAAAGGACAGAAGGTTGGCAAGAAACAGGGCCTTATTTTTCTAGGTTTAGCCTTGGGAACTCACTTCTTGTTTA
ACTCTCCTTTTGTGGAGTTGGAAACAGAGTTGCCTTTAGCGATTCCAGTGGTTACGGCTATTGCTCTCTATGGTTTT
TATCATGCTTATTGCTTTGTTGAGAAACACAATGAGTTGATGACCTAG

4197.1
ATGAAGGTGGAACCACGTTGCGACGTCCTTTCGAGGATGTCGCATTTTTTTATTAGGATACTAATTATGGAGTTGC
AAGAATTAGTGGAGCGCAGTTGGGCAATCCGACAAGCTTATCACGAACTGGAAGTTAAGCATCATGATTCCAAGT
GGACGGTAGAAGAAGACCTCTTGGCTTTATCTAATGATATTGGAAATTTCCAACGACTGGTGATGACAAAGCAAG

GACGCTACTATGATGAAACACCCTACACACTGGAACAAAAACTTTCAGAAAATATCTGGTGGCTATTAGAACTTT
CTCAACGTTTGGATATAGACATTCTGACGGAAATGGAAAACTTCCTCTCTGATAAAGAAAAGCAATTGAACGTTA
GGACTTGGAAGTAG

4197.4
ATGCTTGATTGGAAACAATTTTTTCTAGCCTATCTGCGCTCCCGTAGTCGTCTTTTTATCTATCTGCTTTCTTTGGC
ATTTCTTGTCTTACTCTTTCAGTTTTTATTTGCCAGTCTAGGAATTTACTTCCTCTACTTTTTCTTCTTGTGTTGCTTT
GTAACCATATTATTTTTCACTTGGGACATATTGGTGGAAACGCAGGTCTATCGCCAGGAACTTCTCTATGGAGAGA
GGGAAGCCAAGTCTCCTTTGGAAATAGCTTTAGCAGAAAAATTAGAAGCGCGTGAGATGGAACTCTATCAGCAGA
GGTCAAAAGCAGAAAGAAAACTGACGGATTTGCTGGATTACTATACCTTGTGGGTCCATCAGATAAAGACCCCCA
TTGCAGCCAGTCAACTCTTAGTTGCAGAAGTGGTCGACCGCCAACTGAAGCAGCAGCTAGAACAGGAAATTTTCA
AAATCGACTCCTATACCAACCTAGTTTTACAGTACCTGCGTTTAGAAAGTTTCCATGATGATTTGGTCTTAAAGCA
GGTTCAAATTGAGGACTTGGTCAAGGAAATAATTCGTAAATATGCTCTTTTCTTTATTCAAAAAGGCTTAAATGTC
AATCTACATGACCTTGATAAAGAAATCGTGACGGATAAAAAGTGGCTGCTAGTGGTTATTGAGCAAATCATCTCA
AACAGTCTCAAGTACACCAAGGAAGGTGGTCTGGAGATTTATATGGATGACCAAGAGCTTTGTATCAAAGATACG
GGAATCGGGATAAAAAACAGTGATGTCCTCCGAGTATTTGAACGTGGCTTTTCAGGATACAATGGCCGTTTGACCC
AGCAGTCCTCTGGACTTGGCCTTTATCTATCTAAGAAAATTTCTGAAGAACTGGGGCACCAGATTCGTATCGAGTC
TGAGGTCGGAAAAGGAACGACAGTGCGGATTCAGTTTGCTCAAGTGAACTTAGTCCTTGAGTAA

4211.2
ATGGAACTTAATACACACAATGCTGAAATCTTGCTCAGTGCAGCTAATAAGTCCCACTATCCGCAGGATGAACTG
CCAGAGATTGCCCTAGCAGGGCGTTCAAATGTTGGTAAATCCAGCTTTATCAACACTATGTTGAACCGTAAGAATC
TCGCCCGTACATCAGGAAAACCTGGTAAAACCCAGCTCCTGAACTTTTTTAACATTGATGACAAGATGCGCTTTGT
GGATGTGCCTGGTTATGGCTATGCTCGTGTTTCTAAAAAGGAACGTGAAAAGTGGGGGTGCATGATTGAGGAGTA
CTTAACGACTCGGGAAAATCTCCGTGCGGTTGTCAGTCTAGTTGACCTTCGTCATGACCCGTCAGCAGATGATGTG
CAGATGTACGAATTTCTCAAGTATTATGAGATTCCAGTCATCATTGTGGCGACCAAGGCGGACAAGATTCCTCGTG
GTAAATGGAACAAGCATGAATCAGCAATCAAAAAGAAATTAAACTTTGACCCGAGTGACGATTTCATCCTCTTTTC
ATCTGTCAGTAAGGCAGGGATGGATGAGGCTTGGGATGCAATCTTAGAAAAATTGTGA

4211.3
ATGACAAAGAAACAACTTCACTTGGTGATTGTGACAGGGATGAGTGGCGCAGGGAAAACTGTAGCCATTCAGTCC
TTCGAGGATCTAGGTTATTTCACCATTGATAATATGCCGCCAGCTCTCTTGCCTAAGTTTTTGCAGCTGGTTGAAAT
TAAGGAAGACAATCCTAAGTTGGCCTTGGTAGTGGATATGCGTAGCCGTTCTTTCTTTTCAGAGATTCAAGCTGTT
TTGGATGAGTTGGAAAATCAAGATGGTTTGGATTTCAAAATCCTCTTTTTGGATGCGGCTGATAAGGAATTGGTCG
CTCGTTACAAGGAAACCAGACGGAGTCACCCACTAGCAGCAGACGGTCGTATTTTAGATGGAATCAAGTTGGAAC
GTGAACTCTTGGCACCTTTGAAAAATATGAGCCAAAATGTGGTGGATACGACTGAACTCACTCCACGTGAGcTGCG
CAAAACCCTTGCAGAGCAGTTTTCAGACCAAGAACAAGCCCAGTCTTTCCGTATCGAAGTCATGTCTTTCGGATTT
AAGTATGGAATCCCGATTGATGCGGACTTGGTCTTTGATGTCCGTTTCTTGCCAAATCCCTATTATTTACCAGAACT
GAGAAACCAAACGGGTGTGGATGAACCTGTTTATGATTATGTCATGAACCATCCTGAGTCAGAAGACTTTTATCAA
CATTTATTGGCCTTGATTGAGCCGATTCTGCCAAGTTACCAAAAGGAAGGTAAGTCCGTTTTGACCATTGCCATGG
GATGTACGGGTGGACAACACCGTAGTGTGGCATTTGCTAAACGCTTGGCGCAGGACTTATCCAAGAATTGGTCTGT
TAATGAAGGGCATCGCGACAAAGACCGCAGAAAGGAAACGGTAAACCGTTCATGA

4211.4
ATGAGAAAACCAAAGATAACGGTGATTGGTGGAGGGACTGGAAGTCCCGTCATTCTAAAAAGTCTGCGGGAAAAA
GATGTGGAAATCGCAGCTATCGTGACGGTGGCAGATGATGGTGGTTCTTCAGGTGAACTCCGAAAAAATATGCAA
CAGTTGACACCGCCAGGTGATCTTCGTAATGTCCTTGTGGCCATGTCGGATATGCCTAAGTTTTATGAGAAGGTCT
TTCAGTATCGGTTCTCTGAGGATGCCGGAGCCTTTGCTGGCCATCCATTGGGAAATCTCATCATTGCTGGCTTGTC
AGAAATGCAGGGTTCAACCTATAATGCCATGCAGTTATTGAGCAAATTTTTCCATACAACAGGGAAAATTTATCCT
TCCAGTGACCATCCTTTGACCCTTCATGCAGTCTTTCAGGATGGGACAGAAGTGGCTGGAGAGAGTCATATTGTAG
ACCATCGAGGCATAATTGACAATGTCTATGTGACCAATGCCCTAAACGATGATACGCCTCTGGCCAGCCGTCGAG
TAGTGCAGACCATCCTTGAAAGTGACATGATTGTCCTAGGGCCAGGTTCCCTCTTTACCTCTATTTTGCCCAATAT
CGTGATTAAGGAAATTGGGCGGGCTCTTTTGGAAACCAAGGCAGAAATTGCCTATGTCTGCAATATCATGACCCA
ACGTGGGGAGACGGAACACTTTACAGATAGCGACCACGTGGAAGTCTTGCATCGTCACCTTGGTCGCCCTTTTATC
GACACTGTCTTGGTGAATATTGAAAAAGTGCCTCAGGAATACATGAATTCCAACCGTTTTGATGAATACTTAGTGC
AAGTGGAACACGATTTTGTAGGTCTTTGTAAGCAAGTTTCGCGCGTGATTTCATCTAACTTCCTTCGTCTGGAAAA
TGGCGGTGCCTTCCACGATGGAGATTTGATTGTGGACGAGTTGATGCGCATTATACAGGTGAAAAAATGA

4213.1
ATGAAAAATTTGATAAAGTTGCTAATAATTAGATTGATTGTTAACTTAGCAGACAGTGTATTTTATATAGTAGCAT
TGTGGCACGTTAGCAATAATTATTCTTCGAGCATGTTCTTAGGAATATTTATTGCAGTAAATTATCTACCGGATTTG
TTACTAATCTTTTTTGGACCAGTTATTGACAGAGTAAATCCGCAAAAAATTCTTATAATATCAATTTTGGTTCAATT
AGCAGTGGCTGTAATATTTTTATTATTATTAAACCAAATATCATTTTGGGTGATAATGAGTCTAGTGTTTATTTCAG
TAATGGCTAGCTCCATAAGTTACGTGATAGAAGATGTGTTGATTCCTCAAGTGGTAGAATATGATAAGATTGTATT
TGCAAATTCTCTTTTTAGTATTTCGTATAAAGTATTAGATTCTATTTTTAATTCATTCGCATCATTTTTACAGGTGG

CAGTAGGATTTATTTTATTGGTTAAGATAGATATAGGCATATTTTTACTTGCTCTATTTATATTGTTGTTGTTAAAA
TTTAGAACTAGCAATGCGAATATAGAAAACTTCTCTTTCAAATATTACAAGAGAGAAGTGTTGCAAGGTACAAAG
TTTATTTTAAATAATAAATTATTATTTAAAACCAGTATTTCTTTAACGCTTATAAACTTTTTTTATTCATTTCAGACA
GTAGTTGTACCGATTTTTTCTATTCGATATTTTGATGGTCCGATTTTTTATGGTATTTTTTAACTATTGCTGGTTTG
GGTGGTATATTGGGAAATATGCTAGCGCCAATCGTAATAAAATATTTAAAATCGAATCAAATTGTTGGTGTATTTC
TTTTTTTGAACGGCTCAAGTTGGTTAGTAGCAATTGTTATAAAAGACTATACTTTATCACTTATTTTATTTTTCGTTT
GTTTTATGTCTAAAGGAGTCTTCAATATTATTTTTAATTCGTTGTACCAACAAATACCTCCACATCAACTTCTTGGT
AGGGTAAATACTACCATTGATTCTATTATTTCTTTTGGAATGCCAATTGGTAGTTTAGTTGCAGGAACGCTTATTGA
TTTGAATATTGAATTAGTGTTAATTGCTATTAGCATACCTTATTTTTTGTTTTCTTATATTTTTTATACGGATAATGG
ATTGAAAGAATTTAGTATATATTAG

4213.2
ATGATGTCTAACAAAAATAAGGAAATTCTGATTTTTGCGATTCTCTATACAGTCCTCTTTATGTTTGATGGCGTTAA
ATTGCTGGCTTCTTTAATGCCATCTGCCATTGCAAATTATCTTGTTTATGTAGTTTTAGCTCTATATGGCTCCTTCTT
GTTCAAGGATAGATTGATCCAACAATGGAAGGAGATTAGAAAGACTAAAAGAAAATTCTTCTTTGGAGTCTTAAC
AGGATGGCTCTTTCTCATTCTGATGACTGTTGTCTTTGAATTTGTATCAGAGATGTTGAAGCAGTTTGTGGGACTAG
ATGGACAAGGTCTAAATCAGTCTAATATTCAAAGTACCTTTCAAGAACAACCACTACTGATAGCTGTTTTTGCTTG
TGTCATTGGACCTCTGGTAGAAGAATTATTTTTCCGTCAGGTCTTATTGCATTACTTGCAGGAACGGTTGTCAGGTT
TACTAAGCATTATTCTGGTAGGACTTGTTTTTGCTCTGACTCATATGCACAGTTTGGCTCTATCAGAGTGGATTGGT
GCAGTTGGTTACTTAGGTGGAGGCCTTGCCTTTTCTATTATTTATGTGAAAGAAAAAGAGAATATCTACTATCCCC
TACTTGTTCACATGTTAAGCAACAGCCTCTCCTTAATCATTTTAGCTATCAGTATAGTAAAATGA

4224.1
TTGAAAAAGCCAATTATCGAATTCAAAAACGTCTCTAAAGTTTTTGAAGACAGCAACACCAAGGTTCTCAAAGAC
ATCAACTTTGAGTTGGAAGAAGGGAAATTCTACACCCTTCTAGGTGCATCTGGTTCGGGGAAATCAACTATCCTAA
ACATTATTGCAGGTTTACTGGATGCGACGACAGGAGATATCATGCTAGACGGTGTTCGTATCAATGATATTCCAAC
CAACAAGCGCGACGTACATACCGTCTTCCAATCCTATGCCTTGTTCCCACATATGAATGTGTTTGAAAATGTTGCC
TTTCCACTTCGCTTGCGTAAAATTGATAAGAAAGAAATCGAGCAGCGTGTAGCGGAAGTTCTCAAGATGGTTCAGT
TGGAAGGTTATGAAAAACGTTCCATCCGCAAACTTTCTGGAGGACAACGTCAGCGTGTGGCCATCGCCCGTGCTA
TCATCAACCAACCCCGTGTGGTCTTGTTGGACGAGCCTTTATCAGCGCTGGACTTGAAATTGAGAACAGACATGCA
GTACGAATTGCGTGAATTACAACAACGATTGGGCATTACCTTTGTCTTTGTCACTCACGATCAGGAAGAAGCTCTT
GCCATGAGTGACTGGATTTTCGTTATGAATGATGGCGAGATTGTCCAGTCTGGAACCCCTGTGGACATCTACGATG
AGCCAATCAACCACTTTGTTGCCACCTTTATCGGGGAGTCAAACATCTTGCCAGGTACCATGATTGAGGACTACTT
GGTCGAATTTAACGGCAAACGCTTTGAAGCGGTTGATGGTGGATGAAGCCAAATGAACCTGTTGAGGTCGTTAT
TCGTCCAGAGGACTTGCGCATTACCCTTCCTGAAGAAGGCAAGCTCCAAGTTAAGGTCGATACCCAGCTTTTCCGT
GGAGTTCATTATGAAATTATCGCCTATGACGAACTTGGAAATGAATGGATGATCCACTCAACCCGTAAGGCTATCG
TGGGTGAGGAAATCGGTCTGGACTTTGAACCAGAAGACATCCACATCATGCGTCTCAATGAAACCGAAGAAGAGT
TCGATGCTCGTATTGAGGAGTACGTAGAAATCGAAGAGCAAGAAGCAGGTTTGATCAATGCAATCGAGGAGGAAA
GAGATGAAGAAAACAAGCTCTAA

4252.1
ATGAAATCAATGAGAATCTTATTTTTGTTAGCTTTAATTCAAATCAGTTTGAGTAGCTGTTTCCTATGGAAGGAAT
GCATCTTGTCCTTTAAACAAAGTACAGCTTTTTTCATCGGAAGCATGGTTTTCGTTTCAGGAATCTGTGCTGGAGT
AAATTATCTTTATACCCGTAAGCAAGAAGTCCATAGTGTCCTAGCCAGTAAGAAGTCGGTGAAGCTTTTTTACAGT
ATGTTACTCTTAATTAATTTGTTAGGAGCTGTTCTTGTTTTGTCAGATAACTTGTTCATCAAAAATACGCTGCAGCA
AGAATTAGTTGACTTTTTATTGCCATCCTTCTTTTTCCTATTTGGGCTAGATTTGCTGATTTTTTTACCCTTGAAAAA
ATACGTGCGCGATTTTCTTGCTATGCTGGACAGAAAAAAGACAGTGTTGGTGACTATTTTAGCAACACTTCTTTTC
TTAAGAAATCCAATGACCATTGTCTCACTTCTGATTTATATTGGACTGGGCTTGTTTTTTGCAGCCTATCTTGTCCC
AAATTCGGTTAAGAAGGAAGTTTCCTTTTATGGTCATATTTTCCGAGATCTTGTATTGGTCATTGTTACGCTCATTT
TCTTTTAG

4252.2
ATGGTTAAAAAAATTATTGGAATGGTGCTAGCTTTACTTTCTGTAACTGTAGTAGGAGTAGGTGTTTTTGCTTATAC
TATTTATCAACAAGGGACAGAAACCTTAGCTAAAACCTATAAAAAAATCGGTGAAGAAACCAAGGTTATTGAAGC
GACTGAACCTCTAACCATTCTGTTAATGGGAGTGGACACCGGAAATGTTGAACGAACTGAAACTTGGGTCGGTAG
AAGTGATAGCATGATCTTGATGACAGTGAATCCTAAAACGAAAAAAACAACAATGATGAGTTTAGAGCGGGATAT
TCTGACGCGCATTGAATCAGGGAATGGTCAGGCTCATGAAGCGAAACTGAACTCAGCATATGCAGATGGTGGAGC
AGAGCTTGCTATAGAAACCATTCAAAAAATGATGAATATCCATATTGATCGCTATGTGATGGTCAATATGAGAGG
ATTGCAAAAACTAGTGGATGCAGTAGGAGGTATTACAGTCAATAATATCCTAGGTTTCCCAATTTCTATCAGTGAC
CAAGAAGAATTTAATACTATTTCTATCGGTGTTGGGGAGCAACATATTGGGGGAGAAGAAGCCCTAGTCTATGCA
CGAATGCGTTACCAAGATCCTGAGGGGGATTATGGTCGTCAAAAACGTCAACGTGAAGTTATTCAAAAAGTCATG
GAAAAAGCTCTCAGTTTAAATAGCATTGGTCATTATCAAGAGATTCTAAAAGCTTTGAGTGACAATATGCAGACC

AATATTGATTTGTCTGCAAAAAGTATCCCTAACTTGCTAGGCTATAAAGATTCATTTAAAACCATTGAAACTCAGC
AGTTGCAGGGTGAAGGAGAGATACTTCAAGGTGTTTCTTACCAGATTGTTTCGAGAGCACATATGTTGGAAATGCA
AAATCTACTCCGACGTTCTTTGGGACAAGAAGAAGTTACTCAGCTTGAAACCAATGCGGTTTATTTGAAGATTTA
TTTGGCAGAGCACCTGTTGGTGATGAAGATAATTAA

4256.2
ATGAAAAAACAAGCCTATGTCATTATTGCTCTCACCTCCTTCCTATTTGTCTTTTTTTCTCCCACAGCTTGCTGGA
AATACTTGATTTTGACTGGTCTATCTTTTTGCACGATGTCGAAAAACAGAAAAATTTGTCTTTTTATTGTTGGTTT
TCAGCATGTCCATGACCTGTCTCTTAGCCCTGTTTTGGCGAGGGATCGAAGAGCTTTCTCTAAGAAAAATGCAGGC
TAATCTCAAGCGTTTATTAGCAGGGCAAGAAGTGGTTCAGGTTGCAGATCCAGATTTGGATGCCAGTTTCAAGTCC
TTATCAGGTAAACTTAACCTTTTGACAGAGGCTCTTCAAAAAGCTGAAAATCAGAGCCTTGCTCAGGAAGAGGAA
ATCATCGAGAAGGAACGGAAGCGAATTGCTCGGGATTTGCACGATACAGTCAGTCAGGAGTTGTTTGCGGCCCAC
ATGATTTTATCGGGTATCAGTCAGCAGGCTTTGAAATTGGATAGAGAAAAGATGCAGACCCAGTTGCAGAGTGTC
ACAGCTATTTTAGAAACAGCCCAGAAGGATTTGCGGGTTTTGCTCTTGCATTTGCGACCAGTTGAACTGGAGCAGA
AGAGCTTGATAGAAGGGATTCAAATTCTTTTAAAAGAGCTTGAGGACAAGAGTGATCTTAGGGTTAGTCTCAAGC
AGAATATGACGAAATTGCCTAAGAAAATCGAGGAGCATATCTTCCGTATCCTGCAAGAGTTGATTAGCAATACCC
TCCGCCATGCCCAGGCATCTTGCCTAGATGTCTACCTCTATCAGACAGATGTTGAATTGCAACTGAAGGTGGTGGA
CAATGGGATTGGTTTCCAGTTAGGGAGCTTAGACGACTTGAGTTATGGACTGCGAAATATCAAGGAGCGGGTTGA
AGATATGGCTGGAACAGTTCAACTCTTGACAGCTCCCAAGCAAGGGCTGGCGGTTGATATCCGTATTCCCCTGTTA
GATAAGGAATGA

4263.1
ATGATTGTTTCCATTATTTCTCAAGGATTTGTCTGGGCTATTCTAGGTCTGGGAATCTTTATGACATTTAGGATTTT
AAACTTTCCAGATATGACGACAGAAGGTTCCTTCCCTCTTGGGGGAGCTGTTGCTGTCACTTTGATAACCAAAGGC
GTGAACCCATTTTTAGCGACACTTGTTGCTGTAGGAGCAGGTTGTTTGGCTGGAATGGCAGCAGGCCTTCTTTATA
CAAAAGGGAAGATCCCAACCTTGCTCTCAGGGATTTTGGTGATGACTTCTTGTCACTCAATCATGCTCTTGATTAT
GGGACGTGCGAATTTAGGCCTGCTTGGAACCAAGCAAATTCAGGATGTTTTGCCTTTTGATTCGGATTTGAATCAA
CTCTTGACAGGTCTCATCTTTGTGAGTATTGTTATTGCTCTCATGCTCTTTTTCTTGGACACTAAACTCGGACAAGC
CTATATTGCTACAGGGGATAATCCTGATATGGCTAGAAGTTTCGGGATTCATACTGGACGCATGGAGCTCATGGGC
TTGGTCTTATCAAATGGTGTGATTGCCCTTGCAGGTGCCCTCATTGCTCAGCAAGAAGGTTATGCCGATGTGTCTC
GAGGGATCGGGGTTATCGTTGTGGGGCTTGCAAGTTTGATTATTGGAGAAGTTATTTTCAAGAGTTTGAGCTTGGC
AGAGCGTTTGGTTACTATCGTTGTAGGTTCTATCGCTTATCAATTTTTAGTGTGGGCAGTTATCGCACTTGGCTTTA
ATACAAGTTACCTTCGTTTATACAGTGCCTTGATTTTAGCAGTCTGCCTCATGATTCCAACATTTAAGCAAACAAT
CTTGAAAGGAGCCAAGTTAAGCAAATGA

4346.1
ATGAAAAAAATGAAAGTTTGGTCTACTGTACTTGCAACGGGAGTTGCTCTTACTACACTTGCTGCTTGCTCTGGAG
GTTCAAATTCTACGACTGCTTCTTCATCTGAAGAAAAAGCTGATAAAGTCAAGAATTAGTTATCTATTCGAACTC
AGTCTCAAATGGTCGTGGTGATTGGTTAACTGCTAAAGCAAAAGAAGCTGGTTTTAATATAAAAATGGTTGATATC
GCTGGCGCTCAATTAGCAGACCGTGTTATTGCTGAGAAGAATAATGCAGTTGCAGATATGGTATTTGGAATTGGTG
CTGTTGATTCAAATAAAATTAGAGATCAAAAATTACTAGTACAGTACAAGCCTAAATGGTTAGATAAAATTGATC
AATCTTTATCAGATAAAGATAATTATTATAATCCTGTGATTGTTCAACCATTAGTTTTAATTGGGGCGCCTGATGTA
AAAGAAATGCCTAAAGATTGGACTGAATTAGGTAGTAAGTATAAAGGTAAATATTCAATTTCTGGTCTTCAAGGA
GGTACAGGACGGGCAATTCTAGCAAGTATCTTAGTTCGATACCTTGATGATAAAGGTGAATTAGGTGTTTCCGAAA
AAGGTTGGGAAGTAGCAAAAGAATATTTGAAAAATGCATACACTCTTCAAAAGGGAGAAAGTTCAATTGTTAAGA
TGTTAGACAAAGAAGATCCAATACAATATGGAATGATGTGGGGTTCTGGTGCATTAGTTGGACAAAAAGAACAAA
ATGTTGTTTTCAAAGTTATGACTCCTGAGATTGGTGTACCATTTGTAACTGAACAAACTATGGTTTTAAGCACTAG
TAAAAAACAAGCGTTAGCTAAAGAATTTATTGATTGGTTTGGTCAATCAGAAATTCAAGTAGAATATAGTAAGAA
CTTTGGATCTATTCCTGCAAATAAAGATGCCCTCAAAGATCTACCTGAAGATACGAAGAAATTTGTTGATCAAGTG
AAACCACAAAATATTGACTGGGAAGCTGTTGGAAAGCATTTGGATGAATGGGTAGAAAAAGCTGAATTAGAATAC
GTACAATAA

4346.2
ATGATTAAATTTGATAATATTCAAATTAAATATGGTGATTTTGTTGCAATTGATAATCTGAATTTAGATATACATG
AAGGGGAATTTTTTACATTTCTTGGGCCTTCAGGATGTGGTAAATCAACTACTTTGAGAGCATTGGTAGGTTTTCT
AGATCCATCATCAGGAAGTATTGAAGTTAATGGAACAGATGTCACTCATTTGGAACCTGAAAAGCGTGGAATTGG
TATTGTATTTCAATCTTATGCGCTATTTCCAACTATGACTGTTTTTGATAATATTGCATTTGGTTTAAAAGTTAAGA
AGGTAGCTCCAGATGTTATTAAAGCTAAAGTATCAGCAGTGGCAGCAAAAATTAAGATCTCTGATCAACAGTTAC
AGCGTAATGTATCAGAATTATCTGGGGGTCAACAACAAAGGGTAGCATTGGCTCGTGCTCTGGTTCTTGAACCTAA
AATTCTTTGTCTAGATGAACCATTGTCAAACCTTGACGCAAAATTACGTGTAGATTTGAGAAAAGAGTTGAAAAGA
CTTCAAAAAGAGTTAGGTATTACTACTTTATATGTTACTCATGATCAAGAGGAAGCCTTGACTTTATCTGATAGAA
TTGCAGTCTTTAACAATGGATACATCGAACAGGTCGGTACACCAGTAGAGATTTATCATAATTCTCAAACTGAATT
TGTATGTGATTTTATTGGAGATATTAATGTTTTGACCGATGAAACAGTCCACGAAGTATTATTGAAAAATACAAGC
GTTTTCTTAGAGGATAAAAAAGGATACATTCGATTAGAGAAAGTTCGATTCAATCGTGAAACTGAACAAGATTTTA
TTCTAAAAGGGACAATTATTGATGTTGAGTTTCTGGAGTTACAATTCACTATACAATAAAAGTTTCTGAAAGTCA

GATTCTTAATGTAACAAGTATTGATAGTCAGGCTGCTATTAGATCTGTCGGAGAAAGTGTGGAATTATTTATCACA
CCATCAGACGTTCTGCAATTTTAA

4346.3
ATGCGTCATAAATTAAATTTAAAAGATTGGCTTATTCGTTTAGGGTTAATCTGGTTCTTAGTAACATTTATTATTTA
TCCAAACTTTGATCTAGTAGTGAATGTATTTGTAAAAGGAGGAGAATTTTCCCTTGATGCTGTACATCGTGTTCTA
AAATCTCAGAGGGCACTTCAGAGTATTATGAACAGTTTTAAGTTAGCATTTTCACTCATTATTACAGTTAATGTCG
TAGGTATTCTTTGTGTTCTATTTACAGAGTACTTTGATATTAAAGGTGCTAAAATTTTAAAATTAGGTTATATGACC
TCTTTAATTTATGGAGGAGTGGTTTTAGCGACTGGATATAAATTTGTCTATGGTCCTTATGGATTGATTACAAAATT
TTTACAAAATGTTATCCCTTCTTTAGACCCTAACTGGTTTATTGGGTATGGTGCAGTCTTATTCATTATGACATTTT
CAGGAACTGCTAATCATACATTGTTTTTAACAAATACAATTCGAAGCGTTGACTATCACACTATTGAGGCTGCTCG
AAATATGGGAGCAAAACCATTTACTGTTTTCCGAAAAGTAGTGTTACCAACCTTAATTCCAACTCTATTTGCACTT
ACTATTATGGTTTTTCTTAGTGGTTTATCTGCAGTAGCAGCACCCATGATTGTTGGTGGTAAAGAATTTCAAACTAT
AAATCCAATGATTATTACATTTGCAGGGATGGGGAATTCTCGTGATTTAGCTGCCCTACTTGCAATTATTTTAGGT
ATTGCAACTACAATTTTGCTTACTATCATGAATAAGATAGAAAAAGGTGGAAATTATATTTCTATCTCTAAGACTA
AAGCGCCTCTTAAAAAACAAAAAATTGCGTCTAAGCCTTGGAATATCATTGCTCACATTGTAGCATATGGATTGTT
CACAGTTTTCATGCTTCCACTAATTTTTATAGTATTATACTCATTTACAGATCCAGTTGCAATTCAAACAGGTAACT
TAACATTATCAAACTTTACTTTAGAAAATTATCGCTTATTCTTTAGTAATAGTGCGGCATTCTCTCCATTCTTGGTC
AGCTTTATTTATTCTATTATTGCTGCGACAACAGCAACAATTCTCGCAGTTGTATTTGCTCGTGTTGTCAGAAAACA
TAAATCTCGTTTTGATTTCTTATTTGAATATGGTGCTCTACTTCCTTGGTTACTACCAAGTACACTTTTAGCAGTAA
GTTTATTATTTACTTTTAATCAGCCACAATTTCTTGTCTTGAATCAGATTTTGGTAGGTAGTTTGGTAATTCTACTT
ATTGCATATATAGTTGTAAAAATCCCATTTTCTTATAGAATGGTACGTGCTATTTTATTTAGTGTTGATGATGAGAT
GGAAGATGCAGCAAGAAGTATGGGTGCTTCACCTTTTTATACTATGATGAAGGTTATCATTCCATTTATTTTACCG
GTTGTTCTCTCTGTTATTGCTTTAAACTTTAACTCTTTATTAACTGACTTCGACTTATCTGTATTCCTTTACCATCCC
CTAGCTCAACCATTAGGTATTACGATTCGATCTGCAGGTGATGAAACAGCAACATCTAATGCACAAGCTCTGGTAT
TTGTTTATACAATTGTTCTGATGATTATTTCTGGAACGGTATTATACTTCACACAAAGACCGGGGCGTAAAGTAAG
GAAATAA

Table 2

MEELVTLDCLFIDRTKIEANANKYSFVWKKTTEKFSAKLQEQIQVYFQEEITPLLIKYAMFDKKQKRGYKESAKNLANW
HYNDKEDSYTHPDGWYYRFHHTKYQKTQTDFQQEIKVYYADEPESAPQKGLYMNERYQNLKAKECQALLSPQGRQIF
AQRKIDVEPVFGQIKASLGYKRCNLRGKRQVRIDMGLVLMANNLLKYSKMKZ

MGKGHWNRKRVYSIRKFAVGACSVMIGTCAVLLGGNIAGESVVYADETLITHTAEKPKEEKMIVEEKADKALETKNIV
ERTEQSEPSSTEAIASEKKEDEAVTPKEEKVSAKPEEKAPRIESQASNQEKPLKEDAKAVTNEEVNQMIEDRKVDFNQN
WYFKLNANSKEAIKPDADVSTWKKLDLPYDWSIFNDFDHESPAQNEGGQLNGGEAWYRKTFKLDEKDLKKNVRLTF
DGVYMDSQVYVNGQLVGHYPNGYNQFSYDITKYLQKDGRENVIAVHAVNKQPSSRWYSGSGIYRDVTLQVTDKVHV
EKNGTTILTPKLEEQQHGKVETHVTSKIVNTDDKDHELVAEYQIVERGGHAVTGLVRTASRTLKAHESTSLDAILEVER
PKLWTVLNDKPALYELITRVYRDGQLVDAKKDLFGYRYYHWTPNEGFSLNGERIKFHGVSLHHDHGALGAEENYKAE
YRRLKQMKEMGVNSIRTTHNPASEQTLQIAAELGLLVQEEAFDTWYGGKKPYDYGRFFEKDATHPEARKGEKWSDFD
LRTMVERGKNNPAIFMWSIGNEIGEANGDAHSLATVKRLVKVIKDVDKTRYVTMGADKFRFGNGSGGHEKIADELDA
VGFNYSEDNYKALRAKHPKWLIYGSETSSATRTRGSYYRPERELKHSNGPERNYEQSDYGNDRVGWGKTATASWTFD
RDNAGYAGQFIWTGTDYIGEPTPWHNQNQTPVKSSYFGIVDTAGIPKHDFYLYQSQWVSVKKKPMVHLLPHWNWENK
ELASKVADSEGKIPVRAYSNASSVELFLNGKSLGLKTFNKKQTSDGRTYQEGANANELYLEWKVAYQPGTLEAIARDES
GKEIARDKITTAGKPAAVRLIKEDHAIAADGKDLTYIYYEIVDSQGNVVPTANNLVRFQLHGQGQLVGVDNGEQASRER
YKAQADGSWIRKAFNGKGVAIVKSTEQAGKFTLTAHSDLLKSNQVTVFTGKKEGQEKTVLGTEVPKVQTIIGEAPEMPT
TVPFVYSDGSRAERPVTWSSVDVSKPGIVTVKGMADGREVEARVEVIALKSELPVVKRIAPNTDLNSVDKSVSYVLIDGS
VEEYEVDKWEIAEEDKAKLAIPGSRIQATGYLEGQPIHATLVVEEGNPAAPAVPTVTVGGEAVTGLTSQKPMQYRTLA
YGAKLPEVTASAKNAAVTVLQASAANGMRASIFIQPKDGGPLQTYAIQFLEEAPKIAHLSLQVEKADSLKEDQTVKLSV
RAHYQDGTQAVLPADKVTFSTSGEGEVAIRKGMLELHKPGAVTLNAEYEGAKDQVELTIQANTEKKIAQSIRPVNVVT
DLHQEPSLPATVTVEYDKGFPKTHKVTWQAIPKEKLDSYQTFEVLGKVEGIDLEARAKVSVEGIVSVEEVSVTTPIAEAP
QLPESVRTYDSNGHVSSAKVAWDAIRPEQYAKEGVFTVNGRLEGTQLTTKLHVRVSAQTEQGANISDQWTGSELPLAF
ASDSNPSDPVSNVNDKLISYNNQPANRWTNWNRTNPEASVGVLFGDSGILSKRSVDNLSVGFHEDHGVGVPKSYVIEY
YVGKTVPTAPKNPSFVGNEDHVFNDSANWKPVTNLKAPAQLKAGEMNHFSFDKVETYAVRIRMVKADNKRGTSITEV
QIFAKQVAAAKQGQTRIQVDGKDLANFNPDLTDYYLESVDGKVPAVTASVSNNGLATVVPSVREGEPVRVIAKAENGD
ILGEYRLHFTKDKSLLSHKPVAAVKQARLLQVGQALELPTKVPVYFTGKDGYETKDLTVEWEEVPAENLTKAGQFTVR
GRVLGSNLVAEITVRVTDKLGETLSDNPNYDENSNQAFASATNDIDKNSHDRVDYLNDGDHSENRRWTNWSPTPSSNP
EVSAGVIFRENGKIVERTVTQGKVQFFADSGTDAPSKLVLERYVGPEFEVPTYYSNYQAYDADHPFNNPENWEAVPYR
ADKDIAAGDEINVTFKAIKAKAMRWRMERKADKSGVAMIEMTFLAPSELPQESTQSKILVDGKELADFAENRQDYQIT
YKGQRPKVSVEENNQVASTVVDSGEDSFPVLVRLVSESGKQVKEYRIHLTKEKPVSEKTVAAVQEDLPKIEFVEKDLAY
KTVEKKDSTLYLGETRVEQEGKVGKERIFTAINPDGSKEEKLREVVEVPTDRIVLVGTKPVAQEAKKPQVSEKADTKPID
SSEASQTNKAQLPSTGSAASQAAVAAGLTLLGLSAGLVVTKGKKEDZ

MKIMKKKYWTLAILFFCLFNNSVTAQEIPKNLDGNITHTQTSESFSESDEKQVDYSNKNQEEVDQNKFRIQIDKTELFVT
TDKHLEKNCCKLELEPQINNDIVNSESNNLLGEDNLDNKIKENVSHLDNRGGNIEHDKDNLESSIVRKYEWDIDKVTGG
GESYKLYSKSNSKVSIAILDSGVDLQNTGLLKNLSNHSKNYVPNKGYLGKEEGEEGIISDIQDRLGHGTAVVAQIVGDDN
INGVNPHVNINVYRIFGKSSASPDWIVKAIFDAVDDGNDIINLSTGQYLMIDGEYEDGTNDFETFLKYKKAIDYANQKGV
IIVAALGNDSLNVSNQSDLLKLISSRKKVRKPGLVVDVPSYFSSTISVGGIDRLGNLSDFSNKGDSDAIYAPAGSTLSLSEL
GLNNFINAEKYKEDWIFSATLGGYTYLYGNSFAAPKVSGAIAMIIDKYKLKDQPYNYMFVKKFWKKHYQZ

MKKTWKVFLTLVTALVAVVLVACGQGTASKDNKEAELKKVDFILDWTPNTNHTGLYVAKEKGYFKEAGVDVDLKLP
PEESSSDLVINGKAPFAVYFQDYMAKKLEKGAGITAVAAIVEHNTSGIISRKSDNVSSPKDLVGKKYGTWNDPTELAML
KTLVESQGGDFEKVEKVPNNDSNSITPIANGVFDTAWIYYGWDGILAKSQGVDANFMYLKDYVKEFDYYSPVIIANND
YLKDNKEEARKVIQAIKKGYQYAMEHPEEAADILIKNAPELKEKRDFVIESQKYLSKEYASDKEKWGQFDAARWNAFY
KWDKENGILKEDLTDKGFTNEFVKZ

MKRTWRNSFVTNLNTPFMIGNIEIPNRTVLAPMAGVTNSAFRTIAKELGAGLVVMEMVSDKGIQYNNEKTLHMLHIDE
GENPVSIQLFGSDEDSLARAAEFIQENTKTDIVDINMGCPVNKIVKNEAGAMWLKDPDKIYSIINKVQSVLDIPLTVKMR
TGWADPSLAVENALAAEAAGVSALAMHGRTREQMYTGHADLETLYKVAQALTKIPFIANGDIRTVQEAKQRIEEVGA
DAVMIGRAAMGNPYLFNQINHYFETGEILPDLTFEDKMKIAYEHLKRLINLKGENVAVREFRGLAPHYLRGTSGAAKL
RGAISQASTLAEIETLLQLEKAZ

MIKNPKLLTKSFLRSFAILGGVGLVIHIAIYLTFPFYYIQLEGEKFNESARVFTEYLKTKTSDEIPSLLQSYSKSLTISAHLK
RDIVDKRLPLVHDLDIKDGKLSNYIVMLDMSVSTADGKQVTVQFVHGVDVYKEAKNILLLYLPYTFLVTIAFSFVFSYF
YTKRLLNPLFYISEVTSKMQDLDDNIRFDESRKDEVGEVGKQINGMYEHLLKVIYELESRNEQIVKLQNQKVSFVRGAS
HELKTPLASLRIILENMQHNIGDYKDHPKYIAKSINKIDQMSHLLEEVLESSKFQEWTECRETTVKPVLVDILSRYQELAH
SIGVTIENQLTDATRVVMSLRALDKVLTNLISNAIKYSDKNGRVIISEQDGYLSIKNTCAPLSDQELEHLFDIFYHSQIVTD
KDESSGLGLYIVNNILESYQMDYSFLPYEHGMEFKISLZ

MYLGDLMEKAECGQFSILSFLLQESQTTVKAVMEETGFSKATLTKYVTLLNDKALDSGLELAIHSEDENLRLSIGAATK
GRDIRSLFLESAVKYQILVYLLYHQQFLAHQLAQELVISEATLGRHLAGLNQILSEFDLSIQNGRWRGPEHQIHYFYFCL

FRKVWSSQEWEGHMQKPERKQEIANLEEICGASLSAGQKLDLVLWAHISQQRLRVNACQFQVIEEKMRGYFDNIFYLR
LLRKVPSFFAGQHIPLGVEDGEMMIFFSFLLSHRILPLHTMEYILGFGGQLADLLTQLIQEMKKEELLGDYTEDHVTYEL
SQLCAQVYLYKGYILQDRYKYQLENRHPYLLMEHDFKETAEEIFHALPAFQQGTDLDKKILWEWLQLIEYMAENGGQ
HMRIGLDLTSGFLVFSRMAAILKRYLEYNRFITIEAYDPSRHYDLLVTNNPIHKKEQTPVYYLKNDLDMEDLVAIRQLLF
TZ

MEFSKKTRELSIKKMQERTLDLLIIGGGITGAGVALQAAASGLETGLIEMQDFAEGTSSRSTKLVHGGLRYLKQFDVEV
VSDTVSERAVVQQIAPHIPKSDPMLLPVYDEDGATFSLFRLKVAMDLYDLLAGVSNTPAANKVLSKDQVLERQPNLKK
EGLVGGGVYLDFRNNDARLVIENIKRANQDGALIANHVKAEGFLFDESGKITGVVARDLLTDQVFEIKARLVINTTGPW
SDKVRNLSNKGTQFSQMRPTKGVHLVVDSSKIKVSQPVYFDTGLGDGRMVFVLPRENKTYFGTTDTDYTGDLEHPKVT
QEDVDYLLGIVNNRFPESNITIDDIESSWAGLRPLIAGNSASDYNGGNNGTISDESFDNLIATVESYLSKEKTREDVESAV
SKLESSTSEKHLDPSAVSRGSSLDRDDNGLLTLAGGKITDYRKMAEGAMERVVDILKAEFDRSFKLINSKTYPVSGGELN
PANVDSEIEAFAQLGVSRGLDSKEAHYLANLYGSNAPKVFALAHSLEQAPGLSLADTLSLHYAMRNELTLSPVDFLLRR
TNHMLFMRDSLDSIVEPILDEMGRFYDWTEEEKATYRADVEAALANNDLAELKNZ

MMNELFGEFLGTLILILLGNGVVAGVVLPKTKSNSSGWIVITMGWGIAVAVAVFVSGKLSPAYLNPAVTIGVALKGGLP
WASVLPYILAQFAGAMLGQILVWLQFKPHYEAEENAGNILATFSTGPAIKDTVSNLISEILGTFVLVLTIFALGLYDFQA
GIGTFAVGTLIVGIGLSLGGTTGYALNPARDLGPRIMHSILPIPNKGDGDWSYAWIPVVGPVIGAALAVLVFSLFZ

MTKKKIERISVIHREKILWLKWYFMRDKEQPKYSVLERKMFDAAKNQDMLAYQKYATIKQITDIRVQTSEADILEAVKE
VYVYNHMNVIGACQRILFISQSPAYDKLNKWFNIYSDLYFSVVPLPKMGVYHEMVGIZ

MKNSNEAEMKLLYTDIRTSLTEILTREAEELVAAGKRVFYIAPNSLSFEKERAVLEYLSQQASFSITVTRFAQMARYLVL
NDLPAKTTTLDDIGLGLAFYKCLAELDPKDLRVYGAIKQDPQLIQQLIELYHEMTKSQMSFLDLENLTDEDKRADLLLIF
EKVTAYLNQGQLAQESQLSHLIEAIENDKVSSDFNQIALVIDGFTRFSAEEERVVDLLHGKGVEIVIGAYASKKAYTSPFS
EGNLYQASVKFLHHLASKYQTPAQDCSQTHEKMDSFDKASRLLESSYDFSELALDVDEKDRENLQIWSCLTQKEELEL
VARSIRQKLHENSDLSYKHFRILLGDVASYQLSLKTIFDQYQIPFYLGRSEAMAHHPLTQFVESILALKRYRFRQEDLINL
LRTDLYTDLSQSDIDAFEQYIRYLGINGLPAFQQTFTKSHHGKFNLERLNVLRLRILAPLETLFASRKQKAEKLLQKWSV
FLKEGAVTKQLQDLTTTLEAVEQERQAEVWKAFCHVLEQFATVFAGSQVSLEDFLALLHSGMSLSQYRTIPATVDTVL
VQSYDLIAPLTADFVYAIGLTQDNLPKISQNTSLLTDEERQNLNQATEEGVQLLIASSENLKKNRYTMLSLVNSARKQLF
LSAPSLFNESESKESAYLQELIHFGFRRREKRMNHKGLSKEDMGSYHSLLSSLVAYHQQGEMSDTEQDLTFVKVLSRVI
GKKLDQQGLENPAIPTSPSSKTLAKDTLQALYPAKQEFYLSTSGLTEFYRNEYSYFLRYVLGLQEELRLHPDARSHGNFL
HRIFERALQLPNEDSFDQRLEQAIQETSQEREFEAIYQESLEAQFTKEVLLDVARTTGHILRHNPAIETIKEEANFGGKDQ
AFIQLDNGRSVFVRGKVDRIDRLKANGAIGVVDYKSSLTQFQFPHFFNGLNSQLPTYLAALKREGEQNFFGAMYLEMA
EPVQSLMAVKSLAGAVVEASKSMKYQGLFLEKESSYLGEFYNKNKANQLTDEEFQLLLDYNAYLYKKAAEKILAGRF
AINPYTENGRSIAPYVQQHQAITGFEANYHLGQARFLEKLDLADGKRLVGEKLKQAWLEKIREELNRZ

MKLIPFLSEEEIQKLQEAEANSSKEQKKTAEQIEAIYTSAQNILVSASAGSGKTFVMAERILDQLARGVEISQLFISTFTVK
AATELKERLEKKISKKIQETDDVDLKQHLGRQLADLPNAAIGTMDSFTQKFLGKHGYLLDIAPNFRILQNQSEQLILENE
VFHEVFEAHYQGKQKETFSHLLKNFAGRGKDERGLRQQVYKIYDFLQSTSNPQKWLSESFLKGFEKADFTSEKEKLTE
QIKQALWDLESFFRYHLDNDAKEFAKAAYLENVQLILDEIGSLNQESDSQAYQAVLARVVAISKEKNGRALTNASRKA
DLKPLADAYNEERKTQFAKLGQLSDQIAILDYQERYHGDTWKLAKTFQSFMSDFVEAYRQRKRQENAFEFADISHYTIE
ILENFPQVRESYQERFHEVMVDEYQDTNHIQERMLELLSNGHNRFMVGDIKQSIYRFRQADPQIFNEKFQRYAQNPQEG
RLIILKENFRSSSEVLSATNDVFERLMDQEVGEINYDNKHQLVFANTKLTPNPDNKAAFLLYDKDDTGEEEESQTETKL
TGEMRLVIKEILKLHQEKGVAFKEIALLTSSRSRNDQILLALSEYGIPVKTDGEQNNYLQSLEVQVMLDTLRVIHNPLQD
YALVALMKSPMFGFDEDELARLSLQKAEDKVHENLYEKLVNAQKMASSQKGLIHTALAEKLKQFMDILASWRLYAKT
HSLYDLIWKIYNDRFYYDYVGALPNGPARQANLYALALRADQFEKSNFKGLSRFIRMIDQVLEAQHDLASVAVAPPKD
AVELMTIHKSKGLEFPYVFILNMDQDFNKQDSMSEVILSRQNGLGVKYIAKMETGAVEDHYPKTIKLSIPSLTYRQNEEE
LQLASYSEQMRLLYVAMTRAEKKLYLVGKGSREKLESKEYPAAKNGKLNSNTRLQARNFQDWLWAISKVFTKDKLNF
SYRFIGEDQLTREAIGELETKSPLQDSSQADNRQSDTIKEALEMLKEVEVYNTLHRAAIELPSVQTPSQIKKFYEPVMDM
EGVEIAGQGQSVGKKISFDLPDFSTKEKVTGAEIGSATHELMQRIDLSQQLTLASLTETLKQVQTSQAVRDKINLDKILAF
FDTVLGQEILANTDHLYREQPFSMLKRDQKSQEDFVVRGILDGYLLYENKIVLFDYKTDRYDEPSQLVDRYRGQLALY
EEALSRAYSIENIEKYLILLGKDEVQVVKVZ

MELARHAESLGVDAIATIPPIYFRLPEYSVAKYWNDISSAAPNTDYVIYNIPQLAGVALTPSLYTEMLKNPRVIGVKNSS
MPVQDIQTFVSLGGEDHIVFNGPDEQFLGGRLMGARAGIGGTYGAMPELFLKLNQLIADKDLETARELQYAINAIIGKL
TSAHGNMYGVIKEVLKINEGLNIGSVRSPLTPVTEEDRPVVEAAAALIRETKERFLZ

MYKTKCLREKLVLFLKIFFPILIYQFANYSASFVDTAMTGQYNTMDLAGVSMATSIWNPFFTFLTGIVSALVPIIGHHLG
RGKKEEVASDFYQFIYLALGLSVVLLGMVLFLAPIILNHIGLEAAVAAVAVRYLWFLSIGIIPLLLFSVIRSLLDSLGLTKL
SMYLMLLLLPLNSGFNYLLIYGAFGVPELGGAGAGLGTSLAYWVLLGISVLVLFKQEKLKALHLEKRIPLNMDKIKEGV
RLGLPIGGTVFAEVAIFSVVGLIMAKFSPLIIASHQSAMNFSSLMYAFPMSISSAMAIVVSYEVGAKRFDDAKTYIGLGRW

TALIFAAFTLTFLYIFRGNVASLYGNDPKFIDLTVRFLTYSLFFQLADTFAAPLQGILRGYKDTVIPFYLGLLGYWGVAIP
VYAIZ

MSTLAKIEALLFVAGEDGIRVRQLAELLSLPPTGIQQSLGKLAQKYEKDPDSSLALIETSGAYRLVTKPQFAEILKEYSKA
PINQSLSRAALETLSIIAYKQPITRIEIDAIRGVNSSGALAKLQAFDLIKEDGKKEVLGRPNLYVTTDYFLDYMGINHLEEL
PVIDELEIQAQESQLFGERIEEDENQZ

MDTMISRFFRHLFEALKSLKRNGWMTVAAVSSVMITLTLVAIFASVIFNTAKLATDIENNVRVVVYIRKDVEDNSQTIE
KEGQTVTNNDYHKVYDSLKNMSTVKSVTFSSKEEQYEKLTEIMGDNWKIFEGDANPLYDAYIVEANTPNDVKTIAEDA
KKIEGVSEVQDGGANTERLFKLASFIRVWGLGIAALLIFIAVFLISNTIRITIISRSREIQIMRLVGAKNSYIRGPFLLEGAFIG
LLGAIAPSVLVFIVYQIVYQSVNKSLVGQNLSMISPDLFSPLMIALLFVIGVFIGSLGSGISMRRFLKIZ

MKKVRFIFLALLFFLASPEGAMASDGTWQGKQYLKEDGSQAANEWVFDTHYQSWFYIKADANYAENEWLKQGDDYF
YLKSGGYMAKSEWVEDKGAFYYLDQDGKMKRNAWVGTSYVGATGAKVIEDWVYDSQYDAWFYIKADGQHAEKEW
LQIKGKDYYFKSGGYLLTSQWINQAYVNASGAKVQQGWLFDKQYQSWFYIKENGNYADKEWIFENGHYYYLKSGGY
MAANEWIWDKESWFYLKFDGKMAEKEWVYDSHSQAWYYFKSGGYMTANEWIWDKESWFYLKSDGKIAEKEWVYD
SHSQAWYYFKSGGYMTANEWIWDKESWFYLKSDGKIAEKEWVYDSHSQAWYYFKSGGYMAKNETVDGYQLGSDGK
WLGGKTTNENAAYYQVVPVTANVYDSDGEKLSYISQGSVVWLDKDRKSDDKRLAITISGLSGYMKTEDLQALDASKD
FIPYYESDGHRFYHYVAQNASIPVASHLSDMEVGKKYYSADGLHFDGFKLENPFLFKDLTEATNYSAEELDKVFSLLNI
NNSLLENKGATFKEAEEHYHINALYLLAHSALESNWGRSKIAKDKNNFFGITAYDTTPYLSAKTFDDVDKGILGATKWI
KENYIDRGRTFLGNKASGMNVEYASDPYWGEKIASVMMKINEKLGGKDZ

MKKVLQKYWAWAFVVIPLLLQAIFFYVPMFQGAFYSFTNWTGLTYNYKFVGLNNFKLLFMDPKFMNAIGFTAIIAIAM
VVGEIALGIFIARVLNSKIKGQTFFRAWFFFPAVLSGLTVALIFKQVFNYGLPAIGNALHIEFFQTSLLGTKWGAIFAAVF
VLLWQGVAMPIIIFLAGLQSIPTEITEAARIDGATSKQVFWNIELPYLLPSVSMVFILALKGGLTAFDQVFAMTGGGPNN
ATTSLGLLVYNYAFKNNQFGYANAIAVILFFLIVVISIIQLRVSKKFEIZ

MMKQDERKALIGKYILLILGSVLILVPLLATLFSSFKPTKDIVDNFFGFPTNFTWDNFSRLLADGIGGYYWNSVVITVLSL
LAVMIFIPMAAYSIARNMSKRKAFTIMYTLLILGIFVPFQVIMIPITVMMSKLGLANTFGLILLYLTYAIPQTLFLYVGYIKI
SIPESLDEAAEIDGANQFTTYFRIIFPMMKPMHATTMIINALWFWNDFMLPLLVLNRDSKMWTLPLFQYNYAGQYFND
YGPSFASYVVGIISITIVYLFFQRHIISGMSNGAVKZ

MKSILQKMGEHPMLLLFLSYSTVISILAQNWMGLVASVGMFLFTIFFLHYQSILSHKFFRLILQFVLFGSVLSAAFASLEH
FQIVKKFNYAFLSPNMQVWHQNRAEVTFFNPNYYGIICCFCIMIAFYLFTTTKLNWLKVFCVIAGFVNLFGLNFTQNRT
AFPAIIAGAIIYLFTTIKNWKAFWLSIGVFAIGLSFLFSSDLGVRMGTLDSSMEERISIWDAGMALFKQNPFWGEGPLTYM
NSYPRIHAPYHEHAHSLYIDTILSYGIVGTILLVLSSVAPVRLMMDMSQESGKRPIIGLYLSFLTVVAVHGIFDLALFWIQS
GFIFLLVMCSIPLEHRMLVSDMTDZ

MSKMDVQKIIAPMMKFVNMRGIIALKDGMLAILPLTVVGSLFLIMGQLPFEGLNKSIASVFGANWTEPFMQVYSGTFAI
MGLISCFSIAYSYAKNSGVEALPAGVLSVSAFFILLRSSYIPKQGEAIGDAISKVWFGGQGIIGAIIGLVVGSIYTFFIKRKIV
IKMPEQVPQAIAKQFEAMIPAFVIFLSSMIVYILAKSLTNGGTFIEMIYSAIQVPLQGLTGSLYGAIGIAFFISFLWWFGVH
GQSVVNGVVTALLLSNLDANKAMLASANLSLENGAHIVTQQFLDSFLILSGSGITFGLVVAMLFAAKSKQYQALGKVA
AFPAIFNVNEPVVFGFPIVMNPVMFVPFILVPVLAAVIVYGAIATGFMQPFSGVTLPWSTPAILSGFLVGGWQGVITQLVI
LAMSTLVYFPFFKVQDRLAYQNEIKQSZ

MKKKDLVDQLVSEIETGKVRTLGIYGHGASGKSTFAQELYQALDSTTVNLLETDPYITSGRHLVVPKDAPNQKVTASLP
VAHELESLQRDILACRRVWMSZ

MKKRYLVLTALLALSLAACSQEKTKNEDGETKTEQTAKADGTVGSKSQGAAQKKAEVVNKGDYYSIQGKYDEIIVAN
KHYPLSKDYNPGENPTAKAELVKLIKAMQEAGFPISDHYSGFRSYETQTKLYQDYVNQDGKAAADRYSARPGYSEHQT
GLAFDVIGTDGDLVTEEKAAQWLLDHAADYGFVVRYLKGKEKETGYMAEEWHLRYVGKEAKEIAASGLSLEEYYGF
EGGDYVDZ

MREPDFLNHFLKKGYFKKHAKAVLALSGGLDSMFLFKVLSTYQKELEIELILAHVNHKQRIESDWEEKELRKLAAEAE
LPIYISNFSGEFSEARARNFRYDFFQEVMKKTGATALVTAHHADDQVETIFMRLIRGTRLRYLSGIKEKQVVGEIEIIRPFL
HFQKKDFPSIFHFEDTSNQENHYFRNRIRNSYLPELEKENPRFRDAILGIGNEILDYDLAIAELSNNINVEDLQQLFSYSES
TQRVLLQTYLNRFPDLNLTKAQFAEVQQILKSKSQYRHPIKNGYELIKEYQQFQICKISPQADEKEDELVLHYQNQVAY
QGYLFSFGLPLEGELIQQIPVSRETSIHIRHRKTGDVLIKNGHRKKLRRLFIDLKIPMEKRNSALIIEQFGEIVSILGIATNNL
SKKTKNDIMNTVLYIEKIDRZ

MRKFLIILLLLPSFLTISKVVSTEKEVVYTSKEIYYLSQSDFGIYFREKLSSPMVYGEVPVYANEDLVVESGKLTPKTSFQIT
EWRLNKQGIPVFKLSNHQFIAADKRFLYDQSEVTPTIKKVWLESDFKLYNSPYDLKEVKSSLSAYSQVSIDKTMFVEGRE
FLHIDQAGWVAKESTSEEDNRMSKVQEMLSEKYQKDSFSIYVKQLTTGKEAGINQDEKMYAASVLKLSYLYYTQEKIN
EGLYQLDTTVKYVSAVNDFPGSYKPEGSGSLPKKEDNKEYSLKDLITKVSKESDNVAHNLLGYYISNQSDATFKSKMSA

IMGDDWDPKEKLISSKMAGKFMEAIYNQNGFVLESLTKTDFDSQRIAKGVSVKVAHKIGDADEFKHDTGVVYADSPFIL
SIFTKNSDYDTISKIAKDVYEVLKZ

MKKQNNGLIKNPFLWLLFIFFLVTGFQYFYSGNNSGGSQQINYTELVQEITDGNVKELTYQPNGSVIEVSGVYKNPKTSK
EETGIQFFTPSVTKVEKFTSTILPADTTVSELQKLATDHKAEVTVKHESSSGIWINLLVSIVPFGILFFFLFSMMGNMGGG
NGRNPMSFGRSKAKAANKEDIKVRFSDVAGAEEEKQELVEVVEFLKDPKRFTKLGARIPAGVLLEGPPGTGKTLLAKA
VAGEAGVPFFSISGSDFVEMFVGVGASRVRSLFEDAKKAAPAIIFIDEIDAVGRQRGVGLGGGNDEREQTLNQLLIEMDG
FEGNEGIIVIAATNRSDVLDPALLRPGRFDRKVLVGRPDVKGREAILKVHAKNKPLAEDVDLKLVAQQTPGFVGADLEN
VLNEAALVAARRNKSIIDASDIDEAEDRVIAGPSKKDKTVSQKERELVAYHEAGHTIVGLVLSNARVVHKVTIVPRGRA
GGYMIALPKEDQMLLSKEDMKEQLAGLMGGRVAEEIIFNVQTTGASNDFEQATQMARAMVTEYGMSEKLGPVQYEG
NHAMLGAQSPQKSISEQTAYEIDEEVRSLLNEARNKAAEIIQSNRETHKLIAEALLKYETLDSTQIKALYETGKMPEAVE
EESHALSYDEVKSKMNDEKZ

MKRSSLLVRMVISIFLVFLILLALVGTFYYQSSSSAIEATIEGNSQTTISQTSHFIQSYIKKLETTSTGLTQQTDVLAYAENP
SQDKVEGIRDLFLTILKSDKDLKTVVLVTKSGQVISTDDSVQMKTSSDMMAEDWYQKAIHQGAMPVLTPARKSDSQW
VISVTQELVDAKGANLGVLRLDISYETLEAYLNQLQLGQQGFAFIINENHEFVYHPQHTVYSSSSKMEAMKPYIDTGQG
YTPGHKSYVSQEKIAGTDWTVLGVSSLEKLDQVRSQLLWTLLGASVTSLLVCLCLVWFSLKRWIAPLKDLRETMLEIAS
GAQNLRAKEVGAYELREVTRQFNAMLDQIDQLMVAIRSQEETTRQYQLQALSSQINPHFLYNTLDTIIWMAEFHDSQR
VVQVTKSLATYFRLALNQGKDLICLSDEINHVRQYLFIQKQRYGDKLEYEINENVAFDNLVLPKLVLQPLVENALYHGI
KEKEGQGHIKLSVQKQDSGLVIRIEDDGVGFQDAGDSSQSQLKRGGVGLQNVDQRLKLHFGANYHMKIDSRPQKGTKV
EIYINRIETSZ

MKRSSLLVRMVISIFLVFLILLALVGTFYYQSSSSAIEATIEGNSQTTISQTSHFIQSYIKKLETTSTGLTQQTDVLAYAENP
SQDKVEGIRDLFLTILKSDKDLKTVVLVTKSGQVISTDDSVQMKTSSDMMAEDWYQKAIHQGAMPVLTPARKSDSQW
VISVTQELVDAKGANLGVLRLDISYETLEAYLNQLQLGQQGFAFIINENHEFVYHPQHTVYSSSSKMEAMKPYIDTGQG
YTPGHKSYVSQEKIAGTDWTVLGVSSLEKLDQVRSQLLWTLLGASVTSLLVCLCLVWFSLKRWIAPLKDLRETMLEIAS
GAQNLRAKEVGAYELREVTRQFNAMLDQIDQLMVAIRSQEETTRQYQLQALSSQINPHFLYNTLDTIIWMAEFHDSQR
VVQVTKSLATYFRLALNQGKDLICLSDEINHVRQYLFIQKQRYGDKLEYEINENVAFDNLVLPKLVLQPLVENALYHGI
KEKEGQGHIKLSVQKQDSGLVIRIEDDGVGFQDAGDSSQSQLKRGGVGLQNVDQRLKLHFGANYHMKIDSRPQKGTKV
EIYINRIETSZ

MFFKLLREALKVKQVRSKILFTIFIVLVFRIGTSITVPGVNANSLNALSGLSFLNMLSLVSGNALKNFSIFALGVSPYITASI
VVQLLQMDILPKFVEWGKQGEVGRRKLNQATRYIALVLAFVQSIGITAGFNTLAGAQLIKTALTPQVFLTIGIILTAGSMI
VTWLGEQITDKGYGNGVSMIIFAGIVSSIPEMIQGIYVDYFVNVPSSRITSSIIFVIILIITVLLIIYFTTYVQQAEYKIPIQYTK
VAQGAPSSSYLPLKVNPAGVIPVIFASSITAAPAAILQFLSATGHDWAWVRVAQEMLATTSPTGIAMYALLIILFTFFYTF
VQINPEKAAETYKRVVPISMEFVLVKVQKNICLNFFVVLQLLVPSSLVZ

MDIRQVTETIAMIEEQNFDIRTITMGISLLDCIDPDINRAAEKIYQKITTKAANLVAVGDEIAAELGIPIVNKRVSVTPISLIG
AATDATDYVVLAKALDKAAKEIGVDFIGGFSALVQKGYQKGDEILINSIPRALAETDKVCSSVNIGSTKSGINMTAVAD
MGRIIKETANLSDMGVAKLVVFANAVEDNPFMAGAFHGVGEADVIINVGVSGPGVVKRALEKVRGQSFDVVAETVKK
TAFKITRIGQLVGQMASERLGVEFGIVDLSLAPTPAVGDSVARVLEEMGLETVGTHGTTAALALLNDQVKKGGVMAC
NQVGGLSGAFIPVSEDEGMIAAVQNGSLNLEKLEAMTAICSVGLDMIAIPEDTPAETIAAMIADEAAIGVINMKTTAVRII
PKGKEGDMIEFGGLLGTAPVMKVNGASSVDFISRGGQIPAPIHSFKNZ

MTQIIDGKALAAKLQGQLAEKTAKLKEETGLVPGLVVILVGDNPASQVYVRNKERSALAAGFRSEVVRVPETITQEELL
DLIAKYNQDPAWHGILVQLPLPKHIDEEAVLLAIDPEKDVDGFHPLNMGRLWSGHPVMIPSTPAGIMEMFHEYGIDLEG
KNAVVIGRSNIVGKPMAQLLLAKNATVTLTHSRTHNLSKVAAKADILVVAIGRAKFVTADFVKPGAVVIDVGMNRDEN
GKLCGDVDYEAVAPLASHITPVPGGVGPMTITMLMEQTYQAALRTLDRKZ

MSKFNRIHLVVLDSVGIGAAPDANNFVNAGVPDGASDTLGHISKTVGLNVPNMAKIGLGNIPRETPLKTVAAESNPTGY
ATKLEEVSLGKDTMTGHWEIMGLNITEPFDTFWNGFPEEILTKIEEFSGRKVIREANKPYSGTAVIYDFGPRQMETGELII
YTSADPVLQIAAHEDIIPLDELYRICEYARSITLERPALLGRIIARPYVGEPGNFTRTANRRDLAVSPFFPTVLDKLNEAGI
DTYAVGKINDIFNGAGINHDMGHNKSNSHGIDTLLKTMGLAEFEKGFSFTNLVDFDALYGHRRNAHGYRDCLHEFDE
RLPEIIAAMRENDLLLITADHGNDPTYAGTDHTREYIPLLAYSPAFKGNGLIPVGHFADISATVADNFGVETAMIGESFL
DKLVZ

MFISISAGIVTFLLTLVEIPAFIQFYRKAQITGQQMHEDVKQHQAKAGTPTMGGLVFLITSVLVAFFFALFSSQFSNNVGM
ILFILVLYGLVGFLDDFLKVFRKINEGLNPKQKLALQLLGGVIFYLFYERGGDILSVFGYPVHLGFFYIFFALFWLVGFSN
AVNLTDGVDGLASISVVISLSAYGVIAYVQGQMDILLVILAMIGGLLGFFIFNHKPAKVFMGDVGSLALGGMLAAISMA
LHQEWTLLIIGIVYVFETTSVMMQVSYFKLTGGKRIFRMTPVHHHFELGGLSGKGNPWSEWKVDFFFWGVGLLASLLT
LAILYLMZ

LFKKNKDILNIALPAMGENFLQMLMGMVDSYLVAHLGLIAISGVSVAGNIITIYQAIFIALGAAISSVISKSIGQKDQSKLA
YHVTEALKITLLLSFLLGFLSIFAGKEMIGLLGTERDVAESGGLYLSLVGGSIVLLGLMTSLGALIRATHNPRLPLYVSFL
SNALNILFSSLAIFVLDMGIAGVAWGTIVSRLVGLVILWSQLKLPYGKPTFGLDKELLTLALPAAGERLMMRAGDVVIIA

LVVSFGTEAVAGNAIGEVLTQFNYMPAFGVATATVMLLARAVGEDDWKRVASLSKQTFWLSLFLMLPLSFSIYVLGVP
LTHLYTTDSLAVEASVLVTLFSLLGTPMTTGTVIYTAVWQGLGNARLPFYATSIGMWCIRIGTGYLMGIVLGWGLPGIW
AGSLLDNGFRWLFLRYRYQRYMSLKGZ

MQTQEKHSQAAVLGLQHLLAMYSGSILVPIMIATALGYSAEQLTYLISTDIFMCGVATFLQLQLNKYFGIGLPVVLGVA
FQSVAPLIMIGQSHGSGAMFGALIASGIYVVLVSGIFSKVANLFPSIVTGSVITTIGLTLIPVAIGNMGNNVPEPTGQSLLLA
AITVLIILLINIFTKGFIKSISILIGLVVGTAIAATMGLVDFSPVAVAPLVHVPTPLYFGMPTFEISSIVMMCIIATVSMVEST
GVYLALSDITKDPIDSTRLRNGYRAEGLAVLLGGIFNTFPYTGFSQNVGLVKLSGIKKRLPIYYAAGFLVLLGLLPKFGA
LAQIIPSSVLGGAMLVMFGFVSIQGMQILARVDFANNEHNFLIAAVSIAAGVGLNNSNLFVSMPTAFQMFFSNGIVVASL
LAIVLNAVLNHKKKZ

MKDRIKEYLQDKGKVTVNDLAQALGKDSSKDFRELIKTLSLMERKHQIRFEEDGSLTLEIKKKHEITLKGIFHAHKNGFG
FVSLEGEEDDLFVGKNDVNYAIDGDTVEVVIKKVADRNKGTAAEAKIIDILEHSLTTVVGQIVLDQEKPKYAGYIRSKN
QKISQPIYVKKPALKLEGTEVLKVFIDKYPSKKHDFFVASVLDVVGHSTDVGIDVLEVLESMDIVSEFPEAVVKEAESVP
DAPSQKDMEGRLDLRDEITFTIDGADAKDLDDAVHIKALKNGNLEFGVHIADVSYYVTEGSALDKEALNRATSVYVTD
RVVPMLPERLSNGICSLNPQVDRLTQSAIMEIDKHGRVVNYTITQTVIKTSFRMTYSDVNDILAGDEEKRKEYHKIVSSIE
LMAKLHETLENMRVKRGALNFDTNEAKILVDKQGKPVDIVLRQRGIAERMIESFMLMANETVAEHFSKLDLPFIYRIHE
EPKAEKVQKFIDYASSFGLRIYGTASEISQEALQDIMRAVEGEPYADVLSMMLLRSMQQARYSEHNHGHYGLAADYYT
HFTSPIRRYPDLLVHRMIRDYGRSKEIAEHFEQVIPEIATQSSNRERRAIEAEREVEAMKKAEYMEEYVGEEYDAVVSSIV
KFGLFVELPNTVEGLIHITNLPEFYHFNERDLTLRGEKSGITFRVGQQIRIRVERADKMTGEIDFSFVPSEFDVIEKGLKQS
SRSGRGRDSNRRSDKKEDKRKSGRSNDKRKHSQKDKKKKGKKPFYKEVAKKGAKHGKGRGKGRRTKZ

MGTTGFTIIDLIILIVYLLAVLVAGIYFSKKEMKGKEFFKGDGSVPWYVTSVSIFATMLSPISFLGLAGSSYAGSWILWFA
QLGMVVAIPLTIRFILPIFARIDIDTAYDYLDKRFNSKALRIISALLFIIYQLGRMSIIMYLPSAGLSVLTGIDINILIILMGVV
AIVYSYTGGLKSVLWTDFIQGVILISGVVLALFVLIANIKGGFGAVAETLANGKFLAANEKLFDPNLLSNSIFLIVMGSGF
TILSSYASSQDLVQRFTTTQNIKKLNKMLFTNGVLSLATATVFYLIGTGLYVFYQVQNADSAASNIPQDQIFMYFIAYQL
PVGITGLILAAIYAASQSTISTGLNSVATSWTLDIQDVISKNMSDNRRTKIAQFVSLAVGLFSIGVSIVMAHSDIKSAYEWF
NSFMGLVLGLLGGVFILGFVSKKANKQGAYAALIVSTIVMVFIKYFLPPTAVSYWAYSLISISVSVVSGYIVSVLTGNKVS
APKYTTIHDITEIKADSSWEVRHZ

MKFSKKYIAAGSAVIVSLSLCAYALNQHRSQENKDNNRVSYVDGSQSSQKSENLTPDQVSQKEGIQAEQIVIKITDQGYV
TSHGDHYHYYNGKVPYDALFSEELLMKDPNYQLKDADIVNEVKGGYIIKVDGKYYVYLKDAAHADNVRTKDEINRQK
QEHVKDNEKVNSNVAVARSQGRYTTNDGYVFNPADIIEDTGNAYIVPHGGHYHYIPKSDLSASELAAAKAHLAGKNM
QPSQLSYSSTASDNNTQSVAKGSTSKPANKSENLQSLLKELYDSPSAQRYSESDGLVFDPAKIISRTPNGVAIPHGDHYHF
IPYSKLSALEEKIARMVPISGTGSTVSTNAKPNEVVSSLGSLSSNPSSLTTSKELSSASDGYIFNPKDIVEETATAYIVRHGD
HFHYIPKSNQIGQPTLPNNSLATPSPSLPINPGTSHEKHEEDGYGFDANRIIAEDESGFVMSHGDHNHYFFKKDLTEEQIK
VRKNIZ

MKKRAIVAVIVLLLIGLDQLVKSYIVQQIPLGEVRSWIPNFVSLTYLQNRGAAFSILQDQQLLFAVITLVVVIGAIWYLHK
HMEDSFWMVLGLTLIIAGGLGNFIDRVSQGFVVDMFHLDFINFAIFNVADSYLTVGVIILLIAMLKEEINGNZ

MNTNLASFIVGLIIDENDRFYFVQKDGQTYALAKEEGQHTVGDTVKGFAYTDMKQKLRLTTLEVTATQDQFGWGRVT
EVRKDLGVFVDTGLPDKEIVVSLDILPELKELWPKKGDQLYIRLEVDKKDRIWGLLAYQEDFQRLARPAYNNMQNQN
WPAIVYRLKLSGTFVYLPENNMLGFIHPSERYAEPRLGQVLDARVIGFREVDRTLNLSLKPRSFEMLENDAQMILTYLE
SNGGFMTLNDKSSPDDIKATFGISKGQFKKALGGLMKAGKIKQDQFGTELIZ

MKDVSLFLLKKVFKSRLNWIVLALFVSVLGVTFYLNSQTANSHSLESRLESRIAANERAINENEEKLSQMSDTSSEEYQF
AKNNLDVQKNLLTRKTEILTLLKEGRWKEAYYLQWQDEEKNYEFVSNDPTASPGLKMGVDRERKIYQALYPLNIKAH
TLEFPTHGIDQIVWILEVIIPSLFVVAIIFMLTQLFAERYQNHLDTAHLYPVSKVTFAISSLGVGVGYVTVLFIGICGFSFLV
GSLISGFGQLDYPYPIYSLVNQEVTIGKIQDVLFPGLLLAFLAFIVIVEVVYLIAYFFKQKMPVLFLSLIGIVGLLFGIQTIQP
LQRIAHLIPFTYLRSVEILSGRLPKQIDNVDLNWSMGMVLLPCLIIFLLLGILFIERWGSSQKKEFFNRFZ

MMKFILDIVSTPAILVALIAILGLVLQKKKLPDIIKGGIKTFVGFLVVSGGAGIVQNSLNPFGTMFEHAFHLSGVVPNNEAI
VAVALTTYGSATAMIMFAGMVFNILIARFTRFKYIFLTGHHTLYMACMIAVILSVAGFTSLPLILLGGLALGIIMSISPAF
VQKYMVQLTGNDKVALGHFSSLGYWLSGFTGSLIGDKSKSTEDIKFPKSLAFLRDSTVSITLSMAVIYIIVAIFAGSEYIEK
EISSGTSGLVYALQLAGQFAAGVFVILAGVRLILGEIVPAFKGISERLVPNSKPALDCPIVYTYAPNAVLIGFISSFVGGLVS
MVIMIASGTVVILPGVVPHFFCGATAGVIGNASGGVRGATIGAFLQGILISFLPVFLMPVLGGLGFQGSTFSDADFGLSGII
LGMLNQFGSQAGIVIGLVLILAVMFGVSFIKKPSATEEZ

MIKTFLSALSVILFSIPIITYSFFPSSNLNIWLSTQPILAQIYAFPLATATMAAILSFLFFFLSFYKKNKQIRFYSGILLLLLSLIL
LLFGTDKTLSSASNKTKTLKLVTWNVANQIEAQHIERIFSHFDADMAIFPELATNIRGEQENQRIKLLFHQVGLSMANYD
IFTSPPTNSGIAPVTVIVKKSYGFYTEAKTFHTTRFGTIVLHSRKQNIPDIIALHTAPPLPGLMEIWKQDLNIIHNQLASKYP
KAIIAGDFNATMRHGALAKISSHRDALNALPPFERGTWNSQSPKLFNATIDHILLPKNHYYVKDLDIVSFQNSDHRCIFT
EITFZ

MNPIQRSWAYVSRKRLRSFILFLILLVLLAGISACLTLMKSNKTVESNLYKSLNTSFSIKKIENGQTFKLSDLASVSKIKGL
ENVSPELETVAKLKDKEAVTGEQSVERDDLSAADNNLVSLTALEDSSKDVTFTSSAFNLKEGRHLQKGDSKKILIHEEL
AKKNGLSLHDKIGLDAGQSESGKGQTVEFEIIGIFSGKKQEKFTGLSSDFSENQVFTDYESSQTLLGNSEAQVSAARFYVE
NPKEMDGLMKQVENLALENQGYQVEKENKAFEQIKDSVATFQTFLTIFLYGMLIAGAGALILVLSLWLRERVYEVGIL
LALGKGKSSIFLQFCLEVVLVSLGALLPAFVAGNAITTYLLQTLLASGDQASLQDTLAKASSLSTSILSFAESYVFLVLLS
CLSVALCFLFLFRKSPKEILSSISZ

MLHNAFAYVTRKFFKSIVIFLIILLMASLSLVGLSIKGATAKASQETFKNITNSFSMQINRRVNQGTPRGAGNIKGEDIKKI
TENKAIESYVKRINAIGDLTGYDLIETPETKKNLTADRAKRFGSSLMITGVNDSSKEDKFVSGSYKLVEGEHLTNDDKDK
ILLHKDLAAKHGWKVGDKVKLDSNIYDADNEKGAKETVEVTIKGLFDGHNKSAVTYSQELYENTAITDIHTAAKLYGY
TEDTAIYGDATFFVTADKNLDDVMKELNGISGINWKSYTLVKSSSNYPALEQSISGMYKMANLLFWGSLSFSVLLLALL
LSLWINARRKEVGILLSIGLKQASILGQFITESILIAIPALVSAYFLANYTARAIGNTVLANVTSGVAKQASKAAQASNLGG
GAEVDGFSKTLSSLDISIQTSDFIIIFVLALVLVVLVMALASSNLLRKQPKELLLDGEZ

MSQDKQMKAVSPLLQRVINISSIVGGVGSLIFCIWAYQAGILQSKETLSAFIQQAGIWGPPLFIFLQILQTVVPIIPGALTSV
AGVFIYGHIIGTIYNYIGIVIGCAIIFYLVRLYGAAFVQSVVSKRTYDKYIDWLDKGNRFDRFFIFMMIWPISPADFLCMLA
ALTKMSFKRYMTIIILTKPFTLVVYTYGLTYIIDFFWQMLZ

MRNMWVVIKETYLRHVESWSFFFMVISPFLFLGISVGIGHLQGSSMAKNNKVAVVTTVPSVAEGLKNVNGVNFDYKDE
ASAKEAIKEEKLKGYLTIDQEDSVLKAVYHGETSLENGIKFEVTGTLNELQNQLNRSTASLSQEQEKRLAQTIQFTEKIDE
AKENKKFIQTIAAGALGFFLYMILITYAGVTAQEVASEKGTKIMEVVFSSIRASHYFYARMMALFLVILTHIGIYVVGGL
AAVLLFKDLPFLAQSGILDHLGDAISLNTLLFILISLFMYVVLAAFLGSMVSRPEDSGKALSPLMILIMGGFFGVTALGAA
GDNLLLKIGSYIPFISTFFMPFRTINDYAGGAEAWISLAITVIFAVVATGFIGRMYASLVLQTDDLGIWKTFKRALSYKZ

MTETIKLMKAHTSVRRFKEQEIPQVDLNEILTAAQMASSWKNFQSYSVIVVRSQEKKDALYELVPQEAIRQSAVFLLFV
GDLNRAEKGARLHTDTFQPQGVEGLLISSVDAALAGQNALLAAESLGYGGVIIGLVRYKSEEVAELFNLPDYTYSVFG
MALGVPNQHHDMKPRLPLENVVFEEEYQEQSTEAIQAYDRVQADYAGARATTSWSQRLAEQFGQAEPSSTRKNLEQK
KLLZMLKLIAIVGTNSKRSTNRQLLQYMQKHFTDKAEIELVEIKAIPVFNKPADKQVPAEILEIAAKIEEADGVIIGTPEYD
HSIPAVLMSALAWLSYGIYPLLNKPIMITGASYGTLGSSRAQLQLRQILNAPEIKANVLPDEFLLSHSLQAFNPSGDLVDL
DVIKKLDAIFDDFRIFVKITEKLRNAQELLRKDAEDFDWENLZ

MNTYQLNNGVEIPVLGFGTFKAKDGEEAYRAVLEALKAGYRHIDTAAIYQNEESVGQAIKDSGVPREEMFVTTKLWNS
QQTYEQTRQALEKSIEKLGLDYLDLYLIHWPNPKPLRENDAWKTRNAEVWRAMEDLYQEGKIRAIGVSNFLPHHLDAL
LETATIVPAVNQVRLAPGVYQDQVVAYCREKGILLEAWGPFGQGELFDSKQVQEIAANHGKSVAQIALAWSLAEGFLP
LPKSVTTSRIQANLDCFGIELSHEERETLKTIAVQSGAPRVDDVDFZ

MRCKMLDPIAIQLGPLAIRWYALCIVTGLILAVYLTMKEAPRKKIIPDDILDFILVAFPLAILGARLYYVIFRFDYYSQNLG
EIFAIWNGGLAIYGGLITGALVLYIFADRKLINTWDFLDIAAPSVMIAQSLGRWGNFFNQEAYGATVDNLDYLPGFIRDQ
MYIEGSYRQPTFLYESLWNLLGFALILIFRRKWKSLRRGHITAFYLIWYGFGRMVIEGMRTDSLMFFGFRVSQWLSVVLI
GLGIMIVIYQNRKKAPYYITEEENZ

MGKLSSILLGTVSGAALALFLTSDKGKQVCSQAQDFLDDLREDPEYAKEQVCEKLTEVKEQATDFVLKTKEQVESGEIT
VDSILAQTKSYAFQATEASKNQLNNLKEQWQEKAEALDDSEEIVIDITEEZ

MKTKLIFWGSMLFLLSLSILLTIYLAWIFYPMEIQWLNLTNRVYLKPETIQYNFHILMNYLTNPFSQVLQMPDFRSSAAG
LHHFAVVKNLFHLVQLVALVTLPSFYVFVNRIVKKDFLSLYRKSLLALVVLPVMIGLGGVLIGFDQFFTLFHQILFVGD
DTWLFDPAKDPVIMILPETFFLHAFLLFFALYENFFGYLYLKSRRKZ

MTYHFTEEYDIIVIGAGHAGVEASLAASRMGCKVLLATINIEMLAFMPCNPSIGGSAKGIVVREVDALGGEMAKTIDKT
YIQMKMLNTGKGPAVRALRAQADKELYSKEMRKTVENQENLTLRQTMIDEILVEDGKVVGVRTATHQEYAAKAVIVT
TGTALRGEIIIGDLKYSSGPNHSLASINLADNLKELGLEIGRFKTGTPPRVKASSINYDVTEIQPGDEVPNHFSYTSRDEDY
VKDQVPCWLTYTNGTSHEIIQNNLHRAPMFTGVVKGVGPRYCPSIEDKIVRFADKERHQLFLEPEGRNTEEVYVQGLST
SLPEDVQRDLVHSIKGLENAEMMRTGYAIEYDMVLPHQLRATLETKKISGLFTAGQTNGTSGYEEAAGQGIIAGINAAL
KIQGKPELILKRSDGYIGVMIDDLVTKGTIEPYRLLTSRAEYRLILRHDNADMRLTEMGREIGLVDDERWARFEIKKNQF
DNEMKRLDSIKLKPVKETNAKVEEMGFKPLTDAVTAKEFLRRPEVSYQDVVAFIGPAAEDLDDKIIELIETEIKYEGYISK
AMDQVAKMKRMEEKRIPANIDWDDIDSIATEARQKFKLINPETIGQASRISGVNPADISILMVYLEGKNRSISKTLQKSKZ

MTKQVLLVDDEEHILKLLDYHLSKEGFSTQLVTNGRKALALAETEPFDFILLDIMLPQLDGMEVCKRLRAKGVKTPIM
MVSAKSDEFDKVLALELGADDYLTKPFSPRELLARVKAVLRRTKGEQEGDDSDNIADDSWLFGTLKVYPERHEVYKA
NKLLSLTPKEFESDKNPFFEVFKVSKVTAQZ

MTTFKDGFLWGGAVAAHQLEGGWQEGGKGISVADVMTAGRHGVAREITLGVLEGKYYPNHEAIDFYHRYKEDIALF
AEMGFKCFRTSIAWTRIFPKGDELEPNEEGLQFYDNLFDECLKNGIEPVITLSHFEMPYHLVTEYGGWKNRKLIDFFARF
AEVVFKRYKDKVKYWMTFNEINNQANYQEDFAPFTNSGIVYEEGDNREAIMYQAAHYELVASARAVKIGHEINPDFQI

GCMIAMCPIYPVTCNPKDILMAMKAMQKRYYFADVHVLGKYPEHIFKYWERKGISVDFTAQDKEDLLGGTVDYIGFS
YYMSFAIDSHRENNPYFDYLETEDLVKNNYVKASEWEWQIDPEGLRYALNWFTDHYHLPLFIVENGFGAIDQVAADG
MVHDDYRIEYLGAHIREMKKAVVEDGVDLMGYTPWGCIDLVSAGTGEMRKRYGFIYVDKDDNGKGSYNRSPKKSFG
WYKEVISSNGESVEZ

MDQQNGLFGFLENHVMGPMGKLAQFKVVRAITAAGMAAVPFTIVGSMFLVFSILPQAFSFWPIVADIFSASFDKFTSLY
MVANYATMGSLSLYFVLSLAYELTKIYAEEEELNMNPLNGALLALMAFVMTVPQIIFDGGMMKTVTSLKEGAVIADG
WAMGNVVARFGTTGIFTAIIMAIVTVLIYRMCVKHNWVIKMPEAVPEGVSRGFTALVPGFVVAFVVIFINGLLVAMGT
DIFKVIAIPFGFVSNLTNSWIGLMIIYLLTQLLWIVGIHGANIVFAFVSPIALANMAENAAGGHFAVAGEFSNMFVIAGGS
GATLGLCLYIAFASKSEQLKAIGRASVVPALFNINEPLIFGLPIIYNPALAIPFILAPMVTATIYYVANSLNFIKPIIAQVPWP
TPVGIGAFLGTADLRAVLVALVCAFAAFLVYLPFIRVYDQKLVKEEQGIZ

MKKFYVSPIFPILVGLIAFGVLSTFIIFVNNNLLTVLILFLFVGGYVFLFKKLRVHYTRSDVEQIQYVNHQAEESLTALLEQ
MPVGVMKLNLSSGEVEWFNPYAELILTKEDGDFDLEAVQTIIKASVGNPSTYAKLGEKRYAVHMDASSGVLYFVDVSR
EQAITDELVTSRPVIGIVSVDNYDDLEDETSESDISQINSFVANFISEFSEKHMMFSRRVSMDRFYLFTDYTVLEGLMNDK
FSVIDAFREESKQRQLPLTLSMGFSYGDGNHDEIGKVALLNLNLAEVRGGDQVVVKENDETKNPVYFGGGSAASIKRT
RTRTRAMMTAISDKIRSVDQVFVVGHKNLDMDALGSAVGMQLFASNVIENSYALYDEEQMSPDIERAVSFIEKEGVTK
LLSVKDAMGMVTNRSLLILVDHSKTALTLSKEFYDLFTQTIVIDHHRRDQDFPDNAVITYIESGASSASELVTELIQFQNS
KKNRLSRMQASVLMAGMMLDTKNFTSRVTSRTFDVASYLRTRGSDSIAIQEIAATDFEEYREVNELILQGRKLGSDVLI
AEAKDMKCYDTVVISKAADAMLAMSGIEASFVLAKNTQGFISISARSRSKLNVQRIMEELGGGGHFNLAAAQIKDVTLS
EAGEKLTEIVLNEMKEKEKEEZ

MKEKNMWKELLNRAGWILVFLLAVLLYQVPLVVTSILTLKEVALLQSGLIVAGLSIVVLALFIMGARKTKLASFNFSFF
RAKDLARLGLSYLVIVGSNILGSILLQLSNETTTANQSQINDMVQNSSLISSFFLLALLAPICEEILCRGIVPKKIFRGKENL
GFVVGTIVFALLHQPSNLPSLLIYGGMSTVLSWTAYKTQRLEMSILLHMIVNGIAFCLLALVVIMSRTLGISVZ

MKEKNMWKELLNRAGWILVFLLAVLLYQVPLVVTSILTLKEVALLQSGLIVAGLSIVVLALFIMGARKTKLASFNFSFF
RAKDLARLGLSYLVIVGSNILGSILLQLSNETTTANQSQINDMVQNSSLISSFFLLALLAPICEEILCRGIVPKKIFRGKENL
GFVVGTIVFALLHQPSNLPSLLIYGGMSTVLSWTAYKTQRLEMSILLHMIVNGIAFCLLALVVIMSRTLGISVZ

MDTQKIEAAVKMIIEAVGEDANREGLQETPARVARMYQEIFSGLGQTAEEHLSKSFEIIDDNMVVEKDIFFHTMCEHHF
LPFYGRAHIAYIPDGRVAGLSKLARTVEVYSKKPQIQERLNIEVADALMDYLGAKGAFVVIEAEHMCMSMRGVRKPGT
ATLTTVARGLFETDKDLRDQAYRLMGLZMKDLFLKRKQAFRKECLGYLRYVLNDHFVLFLLVLLGFLAYQYSQLLQH
FPENHWPILLFVGITSVLLLLWGGTATYMEAPDKLFLLVGEEEIKLHLKRQTGISLVFWLFVQTLFLLLFAPLFLAMGY
GLPVFLLYVLLLGVGKYFHFCQKASKFFTETGLDWDYVISQESKRKQVLLRFFALFTQVKGISNSVKRRAYLDFILKAV
QKVPGKIWQNLYLRSYLRNGDLFALSLRLLLLSLLAQVFIEQAWIATAVVVLFNYLLLFQLLALYHAFDYQYLTQLFPL
DKGQKEKGLQEVVRGLTSFVLLVELVVGLITFQEKLALLALLGAGLVLLVLYLPYQVKRQMQDZ

MRKSIVLAADNAYLIPLETTIKSVLYHNRDVDFYILNSDIAPEWFKLLGRKMEVVNSTIRSVHIDKELFESYKTGPHINYA
SYFRFFATEVVESDRVLYLDSDIIVTGELATLFEIDLKGYSIGAVDDVYAYEGRKSGFNTGMLLMDVAKWKEHSIVNSL
LELAAEQNQVVHLGDQSILNIYFEDNWLALDKTYNYMVGIDIYHLAQECERLDDNPPTIVHYASHDKPWNTYSISRLRE
LWWVYRDLDWSEIAFQRSDLNYFERSNQSKKQVMLVTWSADIKHLEYLVQRLPDWHFHLAAPCDCSEELTSLSQYTN
VTVYQNVLHSRIDWLLDDSIVYLDINTGGEVFNVVTRAQESGKKIFAFDITRKSMDDGLYDGIFSVERPDDLVDRMKNI
EIEZ

MTKIYSSIAVKKGLFTSFLLFIYVLGSRIILPFVDLNTKDFLGGSTAYLAFSAALTGGNLRSLSIFSVGLSPWMSAMILWQ
MFSFSKRLGLTSTSIEIQDRRKMYLTLLIAVIQSLAVSLRLPVQSSYSAILVVLMNTILLIAGTFFLVWLSDLNASMGIGGSI
VILLSSMVLNIPQDVLETFQTVHIPTGIIVLLALLTLVFSYLLALMYRARYLVPVNKIGLHNRFKRYSYLEIMLNPAGGMP
YMYVMSFLSVPAYLFILLGFIFPNHSGLAALSKEFMVGKPLWVYVYISVLFLFSIIFAFVTMNGEEIADRMKKSGEYIYGI
YPGADTSRFINRLVLRFSVIGGLFNVIMAGGPMLFVLFDEKLLRLAMIPGLFMMFGGMIFTIRDEVKALRLNETYRPLIZ

MSSLSDQELVAKTVEFRQRLSEGESLDDILVEAFAVVREADKRILGMFPYDVQVMGAIVMHYGNVAEMNTGEGKTLT
ATMPVYLNAFSGEGVMVVTPNEYLSKRDAEEMGQVYRFLGLTIGVPFTEDPKKEMKAEEKKLIYASDIIYTTNSNLGFD
YLNDNLASNEEGKFLRPFNYVIIDEIDDILLDSAQTPLIIAGSPRVQSNYYAIIDTLVTTLVEGEDYIFKEEKEEVWLTTKG
AKSAENFLGIDNLYKEEHASFARHLVYAIRAHKLFTKDKDYIIRGNEMVLVDKGTGRLMEMTKLQGGLHQAIEAKEHV
KLSPETRAMASITYQSLFKMFNKISGMTGTGKVAEKEFIETYNMSVVRIPTNRPRQRIDYPDNLYITLPEKVYASLEYIKQ
YHAKGNPLLVFVGSVEMSQLYSSLLFREGIAHNVLNANNAAREAQIISESGQMGAVTVATSMAGRGTDIKLGKGVAEL
GGLIVIGTERMESQRIDLQIRGRSGRQGDPGMSKFFVSLEDDVIKKFGPSWVHKKYKDYQVQDMTQPEVLKGRKYRKL
VEKAQHASDSAGRSARRQTLEYAESMNIQRDIVYKERNRLIDGSRDLEDVVVDIIERYTEEVAADHYASRELLFHFIVTN
ISFHVKEVPDYIDVTDKTAVRSFMKQVIDKELSEKKELLNQHDLYEQFLRLSLLKAIDDNWVEQVDYLQQLSMAIGGQS
ASQKNPIVEYYQEAYAGFEAMKEQIHADMVRNLLMGLVEVTPKGEIVTHFPZ

MIGTFAAALVAVLANFIVPIEITPNSANTEIAPPDGIGQVLSNLLLKLVDNPVNALLTANYIRILSWAVIFGIAMREASKNS
QELLKTIADVTSKIVEWIINLAPFGILGLVFKTISDKGVGSLANYGILLVLLVTTMLFVAPVVNPLIAFFFMRRNPYPLVW

NCLRVSGVTAFFTRSSATNIPVNMKLCHDLGLNPDTYSVSIPLGSTINMAGVAITINLLTLAAVNTLGIPVDFATAFVLSV
VAAISSCDASGIAGGSLLLIPVACSLFGISNDIAIQIVGVGFVIGVIQDSCETALNSSTDVLFTAVAEYAATRKKZ

MSISQRTTKLILATCLACLLAYFLNLSSAVSAGIIALLSLSDTRRSTLKLARNRLFSMLLALAIGVLAFHLSGFHIWSLGLY
LAFYVPLAYKMGWEIGITPSTVLVSHLLVQESTSPDLLVNEFLLFAIGTGFALLVNLYMPSREEEIQHYHTLVEEKLKDI
LQRFKYYLSRGDGRNRAQLVAELDTLLKEALRLVYLDHSDHLFHQTDYHIHYFEMRQRQSRILRNMAQQINTCHLAAS
ESLILAQLFSKIAGQLSQTNPASDLLDEIERYLEVFRNRSLPKTREEFETRATLLQLLREAKTFIQVKVDFYQKYRQZ

MEIMSLAIAVFAVIIGLVIGYVSISAKMKSSQEAAELMLLNAEQEATNLRGQAEREADLLVNEAKRESKSLKKEALLEAK
EEARKYREEVDAEFKSERQELKQIESRLTERATSLDRKDDNLTSKEQTLEQKEQSISDRAKNLDAREEQLEEVERQKEAE
LERIGALSQAEARDIILAQTEENLTREIASRIREAEQEVKERSDKMAKDILVQAMQRIAGEYVAESTNSTVHLPDDTMKG
RIIGREGRNIRTFESLTGVDVIIDDTPEVVTLSGFDPIRREIARMTMEMLLKDGRIHPARIEELVEKNRQEIDNKIREYGEA
AAYEIGAPNLHPDLMKIMGRLQFRTSYGQNVLRHSIEVAKLAGIMASELGENAALARRAGFLHDIGKAIDHEVEGSHVE
IGMELARKYKEPPVVVNTIASHHGDVEAESVIAVIVAAADALSAARPGARSESLESYIKRLHDLEEIANGFEGVQTSFAL
QAGREIRIMVNPGKIKDDKVTILAHKVRKKIENNLDYPGNIKVTVIRELRAVDYAKZ

MMLKPSIDTLLDKVPSKYSLVILEAKRAHELEAGAPATQGFKSEKSTLRALEEIESGNVTIHPDPEGKREAVRRRIEEEKR
RKEEEEKKIKEQIAKEKEDGEKIZ

MSAYQLPTVWQDEASNQGAFTGLNRPTAGARFEQNLPKGEQAFQLYSLGTPNGVKVTILLEELLEAGFKEAAYDLYKI
AIMDGDQFGSDFVKLNPNSKIPALLDQSGTENVRVFESAHILLYLAEKFGAFLPSNPVEKVEVLNWLFWQAGAAPFLG
GGFGHFFNYAPEKLEYPINRFTMEVKRQLDLLDKELAQKPYIAGNDYTIADIAIWSWYGQLVQGNLYQGSAKFLDASS
YQNLVKWAEKIANRPAVKRGLEVTYTEIKZ

LASLITSIIMFYVGFDVLRDTIQKILSREETVIDPLGATLGIISAAIMFVVYLYNTRLSKKSNSNALKAAAKDNLSDAVTSL
GTAIAILASSFNYPIVDKLVAIIITFFILKTAYDIFIESSFSLSDGFDDRLLEDYQKAIMEIPKISKVKSQRGRTYGSNIYLDIT
LEMNPDLSVFESHEIADQVESMLEERFGVFDTDVHIEPAPIPEDEILDNVYKKLLMREQLIDQGNQLEELLTDDFVYIRQ
DGEQMDKEAYKTKKELNSAIKDIQITSISQKTKLICYELDGIIHTSIWRRHETWQNIFHQETKKEZ

MTIKLVATDMDGTFLDGNGRFDMDRLKSLLVSYKEKGIYFAVASGRGFLSLEKLFAGVRDDIIFIAENGSLVEYQGQDL
YEATMSRDFYLATFEKLKTSPYVDINKLLLTGKKGSYVLDTVDETYLKVSQHYNENIQKVASLEDITDDIFKFTTNFTEE
TLEDGEAWVNENVPGVKAMTTGFESIDIVLDYVDKGVAIVELVKKLGITMDQVMAFGDNLNDLHMMQVVGHPVAPE
NARPEILELAKTVIGHHKERSVIAYMEGLZ

MADIKLIALDLDGTLLTTDKRLTDRTKETLQAARDRGIKVVLTTGRPLKAMDFFLHELGTDGQEDEYTITFNGGLVQK
NTGEILDKTVFSYDDVARLYEETEKLSLPLDAISEGTVYQIQSDQESLYAKFNPALTFVPVDFEDLSSQMTYNKCVTAFA
QEPLDAAIQKISPELFDQYEIFKSREMLLEWSPKNVHKATGLAKLISHLGIDQSQVMACGDEANDLSMIEWAGLGVAM
QNAVPEVKAAANVVTPMTNDEEAVAWAIEEYVLKENZ

MESLLILLLIANLAGLFLIWQRQDRQEKHLSKSLEDQADHLSDQLDYRFDQARQASQLDQKDLEVVVSDRLQEVRIELH
QGLTQVRQEMTDNLLQTRDKTDQRLQALQESNEQRLEQMRQTVEEKLEKTLQTRLQASFETVSKQLESVNRGLGEMQ
TVARDVGALNKVLSGTKTRGILGELQLGQIIEDIMTPAQYEREYATVENSSERVEYAIKLPGQGDQEYVYLPIDSKFPLA
DYYRLEEAYETGDKDEIERCRKSLLASVKRFARDIRNKYIAPPRTTNFGVLFVPTEGLYSEIVRNPVFFDDLRREEQIIVA
GPSTLSALLNSLSVGFKTLNIQKSADHISKTLASVKTEFGKFGGILVKAQKHLQHASGNIDELLNRRTIAIERTLRHIELSE
GEPALDLLHFQENEEEYEDZ

MKISHMKKDELFEGFYLIKSADLRQTRAGKNYLAFTFQDDSGEIDGKLWDAQPHNIEAFTAGKVVHMKGRREVYNNT
PQVNQITLRLPQAGEPNDPADFKVKSPVDVKEIRDYMSQMIFKIENPVWQRIVRNLYTKYDKEFYSYPAAKTNHHAFET
GLAYHTATMVRLADAISEVYPQLNKSLLYAGIMLHDLAKVIELTGPDQTEYTVRGNLLGHIALIDSEITKTVMELGIDDT
KEEVVLLRHVILSHHGLLEYGSPVRPRIMEAEIIHMIDNLDASMMMMSTALALVDKGEMTNKIFAMDNRSFYKPDLDZ

MSEKAKKGFKMPSSYTVLLIIIAIMAVLTWFIPAGAFIEGIYETQPQNPQGIWDVLMAPIRAMLGTHPEEGSLIKETSAAID
VAFFILMVGGFLGIVNKTGALDVGIASIVKKYKGREKMLILVLMPLFALGGTTYGMGEETMAFYPLLVPVMMAVGFDS
LTGVAIILLGSQIGCLASTLNPFATGIASATAGVGTGDGIVLRLIFWVTLTALSTWFVYRYADKIQKDPTKSLVYSTRKED
LKHFNVEESSSVESTLSSKQKSVLFLFVLTFILMVLSFIPWTDLGVTIFDDFNTWLTGLPVIGNIVGSSTSALGTWYFPEG
AMLFAFMGILIGVIYGLKEDKIISSFMNGAADLLSVALIVAIARGIQVIMNDGMITDTILNWGKEGLSGLSSQVFIVVTYIF
YLPMSFLIPSSSGLASATMGIMAPLGEFVNVRPSLIITAYQSASGVLNLIAPTSGIVMGALALGRINIGTWWKFMGKLVVA
IIVVTIALLLLGTFLPFLZ

MSNSFVKLLVSQLFANLADIFFRVTIIANIYIISKSVIATSLVPILIGISSFVASLLVPLVTKRLALNRVLSLSQFGKTILLAIL
VGMFTVMQSVAPLVTYLFVVAISILDGFAAPVSYAIVPRYATDLGKANSALSMTGEAVQLIGWGLGGLLFATIGLLPTT
CINLVLYIISSFLMLFLPNAEVEVLESETNLEILLKGWKLVARNPRLRLFVSANLLEIFSNTIWVSSIILVFVTELLNKTESY
WGYSNTAYSIGIIISGLIAFRLSEKFLAAKWEPQLFTPNLKTIQNPCLSLDPGWFLFSPNGCFLLDKKEFPLYGISVEKNTK
RKETHMNSLPNHHFQNKSFYQLSFDGGHLTQYGGLIFFQELFSQLKLKERISKYLVTNDQRRYCRYSDSDILVQFLFQLL

TGYGTDYACKELSADAYFPKLLEGGQLASQPTLSRFLSRTDEETVHSLRCLNLELVEFFLQFHQLNQLIVDIDSTHFTTY
GKQEGVAYNAHYRAHGYHPLYAFEGKTGYCFNAQLRPGNRYCSEEADSFITPVLERFNQLLFRMDSGFATPKLYDLIE
KTGQYYLIKLKKNTVLSRLGDLSLPCPQDEDLTILPHSAYSETLYQAGSWSHKRRVCQFSERKEGNLFYDVISLVTNMTS
GTSQDQFQLYRGRGQAENFIKEMKEGFFGDKTDSSTLIKNEVRMMMSCIAYNLYLFLKHLAGGDFQTLTIKRFRHLFL
HVVGKCVRTGRKQLLKLSSLYAYSELFSALYSRIRKVNLNLPVPYEPPRRKASLMMHZ

MMEFFQQLPHLEPYGNPQYFVYVIAATLPIFIGLFFKKRFAWYEVLVSLFFIVTMLVGGKTNQLAALGIYLCWEILLLLF
YKHYRKSKDGKWVFYLVSFLSLLPIIFVKVQPAINGTQSLLGFLGISYLTFRSVGIVIELRDGVIKDFTLWEFLRFLLFMPT
FSSGPIDRFKRFNENYQAIPERDELMDMLDESVRYIMWGFLYKFILAHVLGETLLPPLKNLALQSGGFFNLYALAVMYT
FGLELFFDFAGYSMFALAISNLMGIRSPINFNKPFLSRDLKEFWNRWHMSLSFWFRDFVFMRMVMVLTRKKVFKNRN
VTSSMAYIVNMLIMGFWHGVTWYYIAYGLFHGLGLVINDAWVRKKKTLNKERKKAGKAALPENRWIQLLGMVVTFH
VVMLSFLIFSGFLNNLWFKKZ

MLKRLWMIFGPVLIAGLLVFLLIFFYPTEMHHNLGAEKRSAVATTIDSFKERSQKVRALSDPNVRFVPFFGSSEWLRFD
GAHPAVLAEKYNRSYRPYLLGQGGAASLNQYFGMQQMLPQLENKQVVYVISPQWFSKNGYDPAAFQQYFNGDQLTS
FLKHQSGDQASQYAATRLLQQFPNVAMKDLVQKLASKEELSTADNEMIELLARFNERQASFFGQFSVRGYVNYDKHV
AKYLKILPDQFSYQAIEDVVKADAEKNTSNNEMGMENYFYNEQIKKDLKKLKDSQKSFTYLKSPEYNDLQLVLTQFSK
SKVNPIFIIPPVNKKWMNYAGLREDMYQQTVQKIRYQLESQGFTNIADFSKDGGEPFFMKDTIHLGWLGWLAFDKAVD
PFLSNPTPAPTYHLNERFFSKDWATYDGDVKEFQZ

MEKNLKALKQTTDQEGPAIEPEKAEDTKTVQNGYFEDAAVKDRTLSDYAGNWQSVYPFLEDGTFDQVFDYKAKLTG
KMTQAEYKAYYTKGYHTDVTKINITDNTMEFVQGGQSKKYTYKYVGKKILTYKKGNRGVRFLFEATDADAGQFKYV
QFSDHNVAPVKAEHFHIFFGGTSQEALFEEMDNWPTYYPDNLSGQEIAQEMLAHZ

MKDGHLLAHHIRLLNGRIFQKLLSQDPEALYRGEQGKILAVLWNSETGCATATDIALATGLANNTLTTMIKKLEEQKL
VIVSPCGKDKRKKYLVLTELGKSQKEVGHRVSQKLDTIFYKGFSEEEIHQFEGFQERILANLKEKGNEVZ

MTNLIATFQDRFSDWLTALSQHLQLSLLTLLLAILLAIPLAVFLRYHEKLADWVLQIAGIFQTIPSLALLGLFIPLMGIGTL
PALTALVIYAIFPILQNTITGLKGIDPNLQEAGIAFGMTRWERLKKFEIPLAMPVIMSGIRTAAVLIIGTATLAALIGAGGL
GSFILLGIDRNNASLILIGALSSAVLAIAFNFLLKVMEKAKLRTIFSGFALVALLLGLSYSPALLVQKEKENLVIAGKIGPEP
EILANMYKLLIEENTSMTATVKPNFGKTSFLYEALKKGDIDIYPEFTGTVTESLLQPSPKVSHEPEQVYQVARDGIAKQD
HLAYLKPMSYQNTYAVAVPKKIAQEYGLKTISDLKKVEGQLKAGFTLEFNDREDGNKGLQSMYGLNLNVATIEPALRY
QAIQSGDIQITDAYSTDAELERYDLQVLEDDKQLFPPYQGAPLMKEALLKKHPELERVLNTLAGKITESQMSQLNYQVG
VEGKSAKQVAKEFLQEQGLLKKZ

MMHTYLQKKIENIKTTLGEMSGGYRRMVAAMADLGFSGTMKAIWDDLFAHRSFAQWIYLLVLGSFPLWLELVYEHRI
VDWIGMICSLTGIICVIFVSEGRASNYLFGLINSVIYLILALQKGFYGEVLTTLYFTVMQPIGLLVWIYQAQFKKEKQEFV
ARKLDGKGWTKYLSISVLWWLAFGFIYQSIGANRPYRDSITDATNGVGQILMTAVYREQWIFWAATNVFSIYLWWGES
LQIQGKYLIYLINSLVGWYQWSKAAKQNTDLLNZ

MRNMKAKYAVWVAFFLNLTYAIVEFIAGGVFGSSAVLADSVHDLGDAIAIGISAFLETISNREEDNQYTLGYKRFSLLG
ALVTAVILVTGSVLVILENVTKILHPQPVNDEGILWLGIIAITINLLASLVVGKGKTKNESILSLHFLEDTLGWVAVILMAI
VLRFTDWYILDPLLSLVISFFILSKALPRFWSTLKIFLDAVPEGLDIKQVKSGLERLDNVASLNQLNLWTMDALEKNAIV
HVCLKEMEHMETCKESIRIFLKDCGFQNITIEIDADLETHQTHKRKVCDLERSYEHQHZ

MIEYKNVALRYTEKDVLRDVNLQIEDGEFMVLVGPSGSGKTTMLKMINRLLEPTDGNIYMDGKRIKDYDERELRLSTG
YVLQAIALFPNLTVAENIALIPEMKGWSKEEITKKTEELLAKVGLPVAEYGHRLPSELSGGEQQRVGIVRAMIGQPKIFL
MDEPFSALDAISRKQLQVLTKELHKEFGMTTIFVTHDTDEALKLADRIAVLQDGEIRQVANPETILKAPATDFVADLFG
GSVHDZ

MSAVAISAMTKVMQETHGNPSSIHGHGRQAGKLLREARQELAQLLRTKPQHIFFTSGGTEGNNTTIIGYCLRHQEQGKH
IITTAIEHHAVLETIDYLVQHFGFEATIIQPENQEITAQQIQKALRDDTILVSTMFVNNETGNLLPIAEIGQILKQHPAAYH
VDAVQAIGKIPIHSEELGIDFLTASAHKFHGPKGIGFLYASSMDFDSYLHGGDQEQKKRAGTENLPAIVGMVAALKEDL
EKQEEHFQHVQNLETAFLAELEGIQYYLNRGKHHLPYVLNIGFPGQKNDLLLLRLDLAGISISTGSACTAGVVQSSHVLE
AMYGANSERLKESLRISLSPQNTVEDLQTLAKTLKEIIGGZ

MLFKLSKEKIELGLSRLSPARRIFLSFALVILLGSLLLSLPFVQVESSRATYFDHLFTAVSAVCVTGLSTLPVAHTYNIWG
QIICLLLIQIGGLGLMTFIGVFYIQSKQKLSLRSRATIQDSFSYGETRSLRKFVYSIFLTTFLVESLGAILLSFRLIPQLGWGR
GLFSSIFLAISAFCNAGFDNLGSTSLFAFQTDLLVNLVIAGLIITGGLGFMVWFDLAGHVGRKKKGRLHFHTKLVLLLTI
GLLLFGTATTLFLEWNNAGTIGNLPVADKVLVSFFQTVTMRTAGFSTIDYTQAHPVTLLIYILQMFLGGAPGGTAGGLK
ITTFFVLLVFARSELLGLPHANVARRTIAPRTVQKSFSVFIIFLMSFLIGLILLGITAKGNPPFIHLVFETISALSTVGVTANL
TPDLGKLALSVIMPLMFMGRIGPLTLFVSLADYHPEKKDMIHYMKADISIGZ

MSDRTIGILGLGIFGSSVLAALAKQDMNIIAIDDHAERINQFEPVLARGVIGDITDEELLRSAGIDTCDTVVVATGENLESS
VLAVMHCKSLGVPTVIAKVKSQTAKKVLEKIGADSVISPEYEMGQSLAQTILFHNSVDVFQLDKNVSIVEMKIPQSWAG
QSLSKLDLRGKYNLNILGFREQENSPLDVEFGPDDLLKADTYILAVINNQYLDTLVALNSZ

MKLLSIAISSYNAAAYLHYCVESLVIGGEQVGILIINDGSQDQTQEIAECLASKYPNIVRAIYQENKCHGGAVNRGLVEAS
GRYFKVVDSDDWVDPRAYLKILETLQELESKGQEVDVFVTNFVYEKEGQSRKKSMSYDSVLPVRQIFGWDQVGNFSK
GQYTMMHSLIYRTDLLRASQFZ

MKFNPNQRYTRWSIRRLSVGVASVVVASGFFVLVGQPSSVRADGLNPTPGQVLPEETSGTKEGDLSEKPGDTVLTQAKP
EGVTGNTNSLPTPTERTEVSEETSPSSLDTLFEKDEEAQKNPELTDVLKETVDTADVDGTQASPAETTPEQVKGGVKEN
TKDSIDVPAAYLEKAEGKGPFTAGVNQVIPYELFAGDGMLTRLLLKASDNAPWSDNGTAKNPALPPLEGLTKGKYFYE
VDLNGNTVGKQGQALIDQLRANGTQTYKATVKVYGNKDGKADLTNLVATKNVDININGLVAKETVQKAVADNVKDS
IDVPAAYLEKAKGEGPFTAGVNHVIPYELFAGDGMLTRLLLKASDKAPWSDNGDAKNPALSPLGENVKTKGQYFYQV
ALDGNVAGKEKQALIDQFRANGTQTYSATVNVYGNKDGKPDLDNIVATKKVTININGLISKETVQKAVADNVKDSIDV
PAAYLEKAKGEGPFTAGVNHVIPYELFAGDGMLTRLLLKASDKAPWSDNGDAKNPALSPLGENVKTKGQYFYQLALD
GNVAGKEKQALIDQFRANGTQTYSATVNVYGNKDGKPDLDNIVATKKVTININGLISKETVQKAVADNVKTVSMFQQP
TZ

MKLKSYILVGYIISTLLTILVVFWAVQKMLIAKGEIYFLLGMTIVASLVGAGISLFLLLPVFTSLGKLKEHAKRVAAKDFP
SNLEVQGPVEFQQLGQTFNEMSHDLQVSFDSLEESEREKGLMIAQLSHDIKTPITSIQATVEGILDGIIKESEQAHYLATIG
RQTERLNKLVEELNFLTLNTARNQVETTSKDSIFLDKLLIECMSEFQFLIEQERRDVHLQVIPESARIEGDYAKLSRILVN
LVDNAFKYSAPGTKLEVVAKLEKDQLSISVTDEGQGIAPEDLENIFKRLYRVETSRNMKTGGHGLGLAIARELAHQLGG
EITVSSQYGLGSTFTLVLNLSGSENKAZ

MFGQTAQHGLTNSLKDFWIFLLNIGPQLAFFCQMLRCSRSVEQGTGNHRREFNMIQQIFSHFGMTHLGQIKLVYQESID
LELLVNALNHHLLIDRLVLTPNQITIEIDRQIVHGLDLLKGRKDKEIIDIKSMFRQLELASTQQICPINQRVHHGILAFGEIS
DLVPAKNLPNRQDZ

MEHLATYFSTYGGAFFAALGIVLAVGLSGMGSAYGVGKAGQSAAALLKEQPEKFASALILQLLPGTQGLYGFVIGILIW
LQLTPELPLEKGVAYFFVALPIAIVGYFSAKHQGNVAVAGMQILAKRPKEFMKGAILAAMVETYAILAFVVSFILTLRVZ

MLKSEKQSRYQMLNEELSFLLEGETNVLANLSNASALIKSRFPNTVFAGFYLFDGKELVLGPFQGGVSCIRIALGKGVC
GEAAHFQETVIVGDVTTYLNYISCDSLAKSEIVVPMMKNGQLLGVLDLDSSEIEDYDAMDRDYLEQFVAILLEKTAWD
FTMFEEKSZ

MSVLEIKDLHVEIEGKEILKGVNLTLKTGEIAAIMGPNGTGKSTLSAAIMGNPNYEVTKGEVLFDGVNILELEVDERAR
MGLFLAMQYPSEIPGITNAEFLRAAMNAGKEDDEKISVREFITKLDEKMELLNMKEEMAERYLNEGFSGGEKKRNEIL
QLLMLEPTFALLDEIDSGLDIDALKVVSKGVNAMRGEGFGAMIITHYQRLLNYITPDVVHVMMEGRVVLSGGPELAAR
LEREGYAKLAEELGYDYKEELZ

MPYKRQRSFSMALSKLDSLYMAVVADHSKNPHHQGKLEDAEQISLNNPTCGDVINLSVKFDAEDRLEDIAFLNSGCTIS
TASASMMTDAVLGKTKQEILELATIFSEMVQGQKDERQDQLGDAAFLSGVAKFPQRIKCATLAWNALKKTIENQEKQZ

MKIQDLLRKDVMLLDLQATEKTAVIDEMIKNLTDHGYVTDFETFKEGILAREALTSTGLGDGIAMPHSKNAAVKEATV
LFAKSNKGVDYESLDGQATDLFFMIAAPEGANDTHLAALAELSQYLMKDGFADKLRQATSADQVIELFDQASEKTEEL
VQAPANDSGDFIVAVTACTTGIAHTYMAQEALQKVAAEMGVGIKVETNGASGVGNQLTAEDIRKAKAIIIAADKAVEM
DRFDGKPLINRPVADGIRKTEELINLALSGDTEVYRAANGAKAATASNEKQSLGGALYKHLMSGVSQMLPFVIGGGIMI
ALAFLIDGALGVPNENLGNLGSYHELASMFMKIGGAAFGLMLPVFAGYVAYSIAEKPGLVAGFVAGAIAKEGFAFGKIP
YAAGGEATSTLAGVSSGFLGALVGGFIAGALVLAIKKYVKVPRSLEGAKSILLLPLLGTILTGFVMLAVNIPMAAINTAM
NDFLGGLGGGSAVLLGIVLGGMMAVDMGGPVNKAAYVFGTGTLAATVSSGGSVAMAAVMAGGMVPPLAIFVATLLF
KDKFTKEERNSGLTNIIMGLSFITEGAIPFGAADPARAIPSFILGSAVAGGLVGLTGIKLMAPHGGIFVIALTSNALLYLVS
VLVGAIVSGVVYGYLRKPQAZ

MANKNTSTTRRRPSKAELERKEAIQRMLISLGIAILLIFAAFKLGAAGITLYNLIRLLVGSLAYLAIFGLLIYLFFFKWIRK
QEGLLSGFFTIFAGLLLIFEAYLVWKYGLDKSVLKGTMAQVVTDLTGFRTTSFAGGGLIGVALYIPTAFLFSNIGTYFIGS

ILILVGSLLVSPWSVYDIAEFFSRGFAKWWEGHERRKEERFVKQEEKARQKAEKEARLEQEETEKALLDLPPVDMETGE
ILTEEAVQNLPPIPEEKWVEPEIILPQAELKFPEQEDDSDDEDVQVDFSAKEALEYKLPSLQLFAPDKPKDQSKEKKIVRE
NIKILEATFASFGIKVTVERAEIGPSVTKYEVKPAVGVRVNRISNLSDDLALALAAKDVRIEAPIPGKSLIGIEVPNSDIATV
SFRELWEQSQTKAENFLEIPLGKAVNGTARAFDLSKMPHLLVAGSTGSGKSVAVNGIIASILMKARPDQVKFMMVDPK
MVELSVYNDIPHLLIPVVTNPRKASKALQKVVDEMENRYELFAKVGVRNIAGFNAKVEEFNSQSEYKQIPLPFIVVIVDE
LADLMMVASKEVEDAIIRLGQKARAAGIHMILATQRPSVDVISGLIKANVPSRVAFAVSSGTDSRTILDENGAEKLLGRG
DMLFKPIDENHPVRLQGSFISDDDVERIVNFIKTQADADYDESFDPGEVSENEGEFSDGDAGGDPLFEEAKSLVIETQKA
SASMIQRRLSVGFNRATRLMEELEIAGVIGPAEGTKPRKVLQQZ

MSYFKKYKFDKSQFKLGMRTFKTGIAVFLVLLIFGFFGWKGLQIGALTAVFSLRESFDESVHFGTSRILGNSIGGLYALV
FFLLNTFFHEAFWVTLVVVPICTMLTIMTNVAMNNKAGVIGGVAAMLIITLSIPSGETILYVFVRVLETFMGVFVAIIVN
YDIDRIRLFLEKKEKZ

MNKSEHRHQLIRALITKNKIHTQAELQALLAENDIQVTQATLSRDIKNMNLSKVREEDSAYYVLNNGSISKWEKRLELY
MEDALVWMRPVQHQVLLKTLPGLAQSFGSIIDTLSFPDAIATLCGNDVCLIICEDADTAQKCFEELKKFAPPFFFEEZ

MKSIKLNALSYMGIRVLNIIFPILTGTYVARVLDRTDYGYFNSVDTILSFFLPFATYGVYNYGLRAISNVKDNKKDLNRT
FSSLFYLCIACTILTTAVYILAYPLFFTDNPIVKKVYLVMGIQLIAQIFSIEWVNEALENYSFLFYKTAFIRILMLVSIFLFVK
NEHDIVVYTLVMSLSTLINYLISYFWIKRDIKLVKIHLSDFKPLFLPLTAMLVFANANMLFTFLDRLFLVKTGIDVNVSY
YTIAQRIVTVIAGVVTGAIGVSVPRLSYYLGKGDKEAYVSLVNRGSRIFNFFIIPLSFGLMVLGPNAILLYGSEKYIGGGIL
TSLFAFRTIILALDTILGSQILFTNGYEKRITVYYTVFAGLLNLGLNSLLFFNHIVAPEYYLLTTMLSETSLLVFYIIFIHRKQL
IHLGHIFSYTVRYSLFSLSFVAIYFLINFVYPVDMVINLPFLINTGLIVLLSAISYISLLVFTKDSIFYEFLNHVLALKNKFKK
SZ

MELFMKITNYEIYKLKKSGLTNQQILKVLEYGENVDQELLLGDIADISGCRNPAVFMERYFQIDDAHLSKEFQKFPSFSIL
DDCYPWDLSEIYDAPVLLFYKGNLDLLKFPKVAVVGSRACSKQGAKSVEKVIQGLENELVIVSGLAKGIDTAAHMAAL
QNGGKTIAVIGTGLDVFYPKANKRLQDYIGNDHLVLSEYGPGEQPLKFHFPARNRIIAGLCRGVIVAEAKMRSGSLITCE
RAMEEGRDVFAIPGSILDGLSDGCHHLIQEGAKLVTSGQDVLAEFEFZ

MKQLTVEDAKQIELEILDYIDTLCKKHNINYIINYGTLIGAVRHEGFIPWDDDIDLSMPREDYQRFINIFQKEKSKYKLLS
LETDKNYFNNFIKITDSTTKIIDTRNTKTYESGIFIDIFPIDRFDDPKVIDTCYKLESFKLLSFSKHKNIVYKDSLLKDWIRT
AFWLLLRPVSPRYFANKIEKEIQKYSRENGQYMAFIPSKFKEKEVFPSGTFDKTIDLPFENLSLPAPEKFDTILTQFYGDY
MTLPPEEKRFYSHEFHAYKLEDZ

MIKINHLTITQNKDLRDLVSDLTMTIQDGEKVAIIGEEGNGKSTLLKILMGEALSDFTIKGNIQSDYQSLAYIPQKVPEDL
KKKTLHDYFFLDSIDLDYSILYRLAEELHFDSNRFASDQEIGNLSGGEALKIQLIHELAKPFEILFLDEPSNDLDLETVDW
LKGQIQKTRQTVIFISHDEDFLSETADTIVHLRLVKHRKEAETLVEHLDYDSYSEQRKANFAKQSQQAANNQRAYDKT
MEKHRRVKQNVETALRATKDSTAGRLLAKKMKTVLSQEKRYEKAAQSMTQKPLEEEQIQLFFSDIQPLPASKVLVQLE
KENLSIDDRVLVQKLQLTVRGQEKIGIIGPNGVGKSTLLAKLQRLLNDKREISLGFMPQDYHKKLQLDLSPIAYLSKTGE
KEELQKIQSHLASLNFSYPEMQHQIRSLSGGQQGKLLLLDLVLRKPNFLLLDEPTRNFSPTSQPQIRKLFATYPGGLITVS
HDRRFLKEVCSIIYRMTEHGLKLVNLEDLZ

MKPKTFYNLLAEQNLPLSDQQKEQFERYFELLVEWNEKINLTAITDKEEVYLKHFYDSIAPILQGLIPNETIKLLDIGAGA
GFPSLPMKILYPELDVTIIDSLNKRINFLQLLAQELDLNGVHFYHGRAEDFAQDKNFRAQYDFVTARAVARMQVLSELT
IPYLKVGGKLLALKASNAPEELLEAKNALNLLFSKVEDNLSYALPNRDPRYITVVEKKKETPNKYPRKAGMPNKRPLZ

MSIKLIAVDIDGTLVNSQKEITPEVFSAIQDAKEAGVKVVIATGRPIAGVAKLLDDLQLRDEGDYVVTFNGALVQETATG
HEIISESLTYEDYLDMEFLSRKLGVHMHAITKDGIYTANRNIGKYTVHESTLVSMPIFYRTPEEMAGKEIVKCMFIDEPEI
LDAAIEKIPAEFYERYSINKSAPFYLELLKKNVDKGSAITHLAEKLGLTKDETMAIGDEENDRAMLEVVGNPVVMENGN
PEIKKIAKYITKTNDESGVAHAIRTWVLZ

MTWIILGVIALIVIFVIVSYNGLVKNRMQTKEAWSQIDVQLKRRNDLLPNLIETVKGYAKYEGSTLEKVAELRNQVAAA
TSPAEAMKASDALTRQVSGIFAVAESYPDLKASANFVKLQEELTNTENKISYSRQLYNSVVSNYNVKLETFPSNIIAGMF
GFKAADFLQTPEEEKSVPKVDFSGLGDZ

MLFDQIASNKRKTWILLLVFFLLLALVGYAVGYLFIRSGLGGLVIALIIGFIYALSMIFQSTEIVMSMNGAREVDEQTAPD
LYHVVEDMALVAQIPMPRVFIIDDPALNAFATGSNPQNAAVAATSGLLAIMNREELEAVMGHEVSHIRNYDIRISTIAV
ALASAITMLSSMAGRMMWWGGAGRRRSDDDRDGNGLEIIMLVVSLLAIVLAPLAATLVQLAISRQREFLADASSVELT
RNPQGMINALDKLDNSKPMSRHVDDASSALYINDPKKGGGFQKLFYTHPPISERIERLKQMZ

MKLNIQEIRKQSEGLNFEQTLDLVDDLRARNQEILDVKDILAVGKVQYEDRMYFLDYQLSYTIVLASSRSMEPVELVES
YPVTEVFMEGATNQLDQEVLDDDLVLPIENGELDLAESVSDNILLNIPIKVLTAEEEAGQGFISGNDWQIMTEEEYQAQ
KAVKKEENSPFAGLQGLFDGDEZ

MKRQLALVVFSGGQDSTTCLFWVMQHYETVEAVTFAYGQRHHLEIQITREIAKEQGIRHHILDMSLLGQITAQPDFATI
HISYIPDKLCVESKSLKLYLFSYRNHGDFHENCINTIGKDLVNLLDPRYLEVWGKFTPRGGISIDPYYNYGKQGTKYEGL
AEQRLFQHDLYPEKIDNRZ

MTETVEDKVSHSITGLDILKGIVAAGAVISGTVATQTKVFTNESAVLEKTVEKTDALATNDTVVLGTISTSNSASSTSLSA
SESASTSASESASTSASTSASTSASESASTSASTSISASSTVVGSQTAAATEATAKKVEEDRKKPASDYVASVTNVNLQSYA
KRRKRSVDSIEQLLASIKNAAVFSGNTIVNGAPAINASLNIAKSETKVYTGEGVDSVYRVPIYYKLKVTNDGSKLTFTYT
VTYVNPKTNDLGNISSMRPGYSIYNSGTSTQTMLTLGSDLGKPSGVKNYITDKNGRQVLSYNTSTMTTQGSGYTWGNG
AQMNGFFAKKGYGLTSSWTVPITGTDTSFTFTPYAARTDRIGINYFNGGGKVVESSTTSQSLSQSKSLSVSASQSASASAS
TSASASASTSASASASTSASASASTSASVSASTSASASASTSASASASTSASESASTSASASASTSASASASTSASASASTSASE
SASTSASASASTSASESASTSASASASTSASASASTSASGSASTSTSASASTSASASASTSASASASISASESASTSASESASTST
SASASTSASESASTSASASASTSASASASTSASASASTSASASTSASESASTSASASASTSASASASTSASASASTSASASASTS
ASVSASTSASASASTSASASASTSASESASTSASASASTSASASASTSASASASTSASESASTSASASASTSASESASTSASASA
STSASASASTSASGSASTSTSASASTSASASASTSASASASISASESASTSASESASTSTSASASTSASESASTSASASASTSASA
SASTSASASASARQVRRPQPVHLNRHQPVRQPQQVLVHQLQHQRVHRLQHQPVPRLQRQPVRQLQQVPVLQSQHQQVLQ
PQHRQVPRLQQAHQHLNQRRQAPQLQQVPVRQPQRRQVRQPQQVLVHQLQHQRVHRLRRQPVHQSQQVPVRQLPHQ
QVPRLQQAPVRRLQQVLAPQPQPQPVRQPQQVSQRLNRHQRVRPLQQVLAPQPQRQQVHRLQRQRVRLNRHQRVRPL
QQVLAPQPQRQQVHRLQHQRVRPLQQVLAPQPQRQQVHRLQRQRVRLSQHQRVRQPQQAHQLLNLHQPVRQPQHRQ
APQLQQVPVRQPQRRQVRRLQQVPVRQPQQVPVRQPQRRQVRRPQPVHLNRHQPVRQPQQVLVHQLQHQRVHRLQH
QPVHQSQQVPVRQFRINKCLGFSKYZ

MGVETWFYSSICWLAIGLGSVWKFPYMTAANGGGGFLLIFLISTILIGFPLLLAEFALGRSAGVSAIKTFGKLGKNNKYN
FIGWIGAFALFILLSFYSVIGGWILVYLGIEFGKLFQLGGTGDYAQLFTSIISNPAIALGAQAAFILLNIFIVSRGVQKGIERA
SKVMMPLLFIVFVFIIGRSLSLPNAMEGVLYFLKPDFSKLTSTGLLYALGQSFFALSLGVTVMLTYASYLDKKTNLVQSG
ISIVAMNISISIMAGLAIFQARSPFNIQSEGGPSLLFIVLPQLFDKMPFGTIFYVLFLLLFLFATVTFSVVMLEINVDNITNQD
NSKRAKWSVILGILTFVFGIPSALSYGVMADVHIFGKTFFDAMDFLVSNLLMPFGALYLSLFTGYIFKKALAMEELHLD
ERAWKQGLFQVWLFLLRFFVSSFQSSSLWSSLPNLCNQKGLEZ

MLKKWQLKDVILLAFLSIFFGGVFVGSGYVYNILSLLLTPLGLQAFANEILFGLWCMAAPIAAIFVPRVGSATIGEVLAA
LAEVLYGSQFGLGALLSGFVQGLGSEFGFIVTKNRYESWLSLTANSIGITLVSFVYEYIKLGYYAFSLPFVLSLLVVRFISV
YFFCTILVRAIVKLYHQFATGGKAZ

MVKVATQTPIISLFLLILSLETSFIPSIALTLSVVAFCILFMLYYRRFKMLAWMIILAILPSFANYWAVQLHGDASQAVML
GTRAFVTVCIGLVFVSSVSLKELLLYLAQKGLSRSWSYALIVVFNSFPLIQQEIKSLKEACLLRGQELHFWSPLIYSKVLM
TVFRWRHLYLRALSAHGYDEHAQLKNSYRTFYIPKKTKLIYLLFFLLLQTSLFLZ

MRKHQLQVHKLTILSMMIALDVVLTPIFRIEGMAPMSSVVNILAGIMMGPVYALAMATVTAFIRMTTQGIPPLALTGAT
FGALLAGLFYKYGRKFHYSALGEILGTGIIGSIVSYPVMVLFTGSAAKLSWFIYTPRFFGATLIGTAISFIAFRFLIKQEFFK
KVQGYFFSERIDZ

MQEFTNPFPIGSSSLIHCITNEISCEMLANGILALGCKPVMADDSREVLDFTKQSQALFINLGHLSAEKEKAIRMAASYAN
QSSLPMVVDAVGVTTSSIRKSLVKDLLDYRPTVLKGNMSEIRSLVGLKHHGVGVDASAKDQETEDLLQVLKDWCQTYP
GMSFLVTGPKDLVVSKNQVAVLGNGCTELDWITGTGDLVGALTAVFLSQGKTGFEASCLAVSYLNIAAEKIVVQGMG
LEEFRYQVLNQLSLLRRDENWLDTIKGEVYEZ

MNHKIAILSDVHGNATALEAVIADAKNQGASEYWLLGDIFLPGPGANDLVALLKDLPITASVRGNWDDRVLEALDGQ
YGLEDPQEVQLLRMTQYLMERMDPATIVWLRSLPLLEKKEIDGLRFSISHNLPDKNYGGDLLVENDTEKFDQLLDAET

DVAVYGHVHKQLLRYGSQGQQIINPGSIGMPYFNWEALKNHRSQYAVIEVEDGELLNIQFRKVAYDYEAELELAKSKG
LPFIEMYEELRRDDNYQGHNLELLASLIEKHGYVEDVKNFFDFLZ

MNVNQIVRIIPTLKANNRKLNETFYIETLGMKALLEESAFLSLGDQTGLEKLVLEEAPSMRTRKVEGRKKLARLIVKVE
NPLEIEGILSKTDSIHRLYKGQNGYAFEIFSPEDDLILIHAEDDIASLVEVGEKPEFQTDLASISLSKFEISMELHLPTDIESF
LESSEIGASLDFIPAQGQDLTVDNTVTWDLSMLKFLVNELDIASLRQKFESTEYFIPKSEKFFLGKDRNNVELWFEEVZ

MKWTKIIKKIEEQIEAGIYPGASFAYFKDNQWTEFYLGQSDPEHGLQTEAGLVYDLASVSKVVGVGTVCTFLWEIGQLD
IDRLVIDFLPESDYPDITIRQLLTHATDLDPFIPNRDLLTAPELKEAMFHLNRRSQPAFLYSDVHFLLLGFILERIFNQDLD
VILKDQVWKPWGMTETKFGPVELAVPTVRGVEAGIVHDPKARLLGRHAGSAGLFSTIKDLQIFLEHYLADDFARDLNQ
NFSPLDDKERSLAWNLEGDWLDHTGYTGTFIMWNRQKQEATIFLSNRTYEKDERAQWILDRNQVMNLIRKEEZ

MMKKTYNHILVWGVIFYSICIVCFCFTPQEQSTVGVGTPGIQHLGRLVFLLTPFNSLWKLGEVSDIGQLCWIFLQNILNV
FLFFPLIFQLLYLFPNLRKTKKVLLFSFLVSLGIECTQLILDFFFDFNRVFEIDDLWTNTLGGYLAWLLYKRLHKNKVRN
Z

MKIPLLTFARHKFVYVLLTLLFLALVYRDVLMTYFFFDIHAPDLAKFDGQAIKNDLLKSALDFRILQFNLGFYQSFIIPIII
VLLGFQYIELKNKVLRLSIGREVSYQGLKRKLTLQVASIPCLIYLVTVLIIAIITYFFGTFSPLGWNSLFSDGSGLQRLLDGE
IKSYLFFTCVLLIGIFINAIYFLQIVDYVGNVTRSAITYLMFLWLGSMLLYSALPYYMVPMTSLMQASYGDVSLMKLFTP
YILYIVPYMVLEKYEDNVZ

MFKVLQKVGKAFMLPIAILPAAGLLLGIGGALSNPTTIATYPILDNSIFQSIFQVMSSAGEVVFSNLSLLLCVGLCIGLAKR
DKGTAALAGVTGYLVMTATIKALVKLFMAEGSAIDTGVIGALVVGIVAVYLHNRYNNIQLPSALGFFGGSRFVPIVTSF
SSILIGFVFFVIWPPFQQLLVSTGGYISQAGPIGTFLYGFLMRLSGAVGLHHIIYPMFWYTELGGVETVAGQTVVGAQKIF
FAQLADLAHSGLFTEGTRFFAGRFSTMMFGLPAACLAMYHSVPKNRRKKYAGLFFGVALTSFITGITEPIEFMFLFVSPV
LYVVHAFLDGVSFFIADVLNISIGNTFSGGVIDFTLFGILQGNAKTNWVLQIPFGLIWSVLYYIIFRWFITQFNVLTPGRGE
EVDSKEISESADSTSNTADYLKQDSLQIIRALGGSNNIEDVDACVTRLRVAVKEVNQVDKALLKQIGAVDVLEVKGGIQ
AIYGAKAILYKNSINEILGVDDZ

MKFRKLACTVLAGAAVLGLAACGNSGGSKDAAKSGGDGAKTEITWWAFPVFTQEKTGDGVGTYEKSIIEAFEKANPDI
KVKLETIDFKSGPEKITTAIEAGTAPDVLFDAPGRIIQYGKNGKLAELNDLFTDEFVKDVNNENIVQASKAGDKAYMYPI
SSAPFYMAMNKKMLEDAGVANLVKEGWTTDDFEKVLKALKDKGYTPGSLFSSGQGGDQGTRAFISNLYSGSVTDEKV
SKYTTDDPKFVKGLEKATSWIKDNLINNGSQFDGGADIQNFANGQTSYTILWAPAQNGIQAKLLEASKVEVVEVPFPSD
EGKPALEYLVNGFAVFNNKDDKKVAASKKFIQFIADDKEWGPKDVVRTGAFPVRTSFGKLYEDKRMETISGWTQYYSP
YYNTIDGFAEMRTLWFPMLQSVSNGDEKPADALKAFTEKANETIKKAMKQZ

MQSTEKKPLTAFTVISTIILLLLTVLFIFPFYWILTGAFKSQPDTIVIPPQWFPKMPTMENFQQLMVQNPALQWMWNSVFI
SLVTMFLVCATSSLAGYVLAKKRFYGQRILFAIFIAAMALPKQVVLVPLVRIVNFMGIHDTLWAVILPLIGWPFGVFLM
KQFSENIPTELLESAKIDGCGEIRTFWSVAFPIVKPGFAALAIFTFINTWNDYFMQLVMLTSRNNLTISLGVATMQAEMA
TNYGLIMAGAALAAVPIVTVFLVFQKSFTQGITMGAVKGZ

MKIMFKNFNNILLNRKIVLLLRIVLMMILINHLLSTAVQKQDAVIFFKRELISIFSYNDYSEANLEIPKLLLNLSLFMVGW
LSVILLESDLADHYHHLIRYQSSSFFDYTRKRLVVISKFFTQDLFVWFLGLLPLGIHFKTVALFFLLAQLMMLYLLLSYLI
ALISAGAGFSFFLYFLAFVGQEWMMDHIVTVYLVLLSLLVMLIVSRLEEKFKKGZ

MGKGEMGKGVIGLEFDSEVLVNKAPTLQLANGKTATFLTQYDSKTLLFAVDKEDIGQEIIGIAKGSIESMHNLPVNLAG
ARVPGGVNGSKAAVHEVPEFTGGVNGTEPAVHEIAEYKGSDSLVTLTTKKDYTYKAPLAQQALPETGNKESDLLASLG
LTAFFLGLFTLGKKREQZ

MKKTFFLLVLGLFCLLPLSVFAIDFKINSYQGDLYIHADNTAEFRQKIVYQFEEDFKGQIVGLGRAGKMPSGFDIDPHPKI
QAAKNGAELADVTSEVTEEADGYTVRVYNPGQEGDIVEVDLVWNLKNLLFLYDDIAELNWQPLTDSSESIEKFEFHVR
GDKGAEKLFFHTGKLFREGTIEKSNLDYTIRLDNLPAKRGVELHAYWPRTDFASARDQGLKGNRLEEFNKIEDSIVREK
DQSKQLVTWVLPSILSISLLLSVCFYFIYRRKTTPSVKYAKNHRLYEPPMELEPMVLSEAVYSTSLEEVSPLVKGAGKFTF
DQLIQATLLDVIDRGNVSIISEGDAVGLRLVKEDGLSSFEKDCLNLAFSGKKEETLSNLFADYKVSDSLYRRAKVSDEKR
IQARGLQLKSSFEEVLNQMQEGVRKRVSFWGLPDYYRPLTGGEKALQVGMGALTILPLFIGFGLFLYSLDVHGYLYLPL

PILGFLGLVLSVFYYWKLRLDNRDGVLNEAGAEVYYLWTSFENMLREIARLDQAELESIVVWNRLLVYATLFGYADK
VSHLMKVHQIQVENPDINLYVAYGWHSTFYHSTAQMSHYASVANTASTYSVSSGSGSSGGGFSGGGGGGSIGAFZ

MKKVRKIFQKAVAGLCCISQLTAFSSIVALAETPETSPAIGKVVIKETGEGGALLGDAVFELKNNTDGTTVSQRTEAQTG
EAIFSNIKPGTYTLTEAQPPVGYKPSTKQWTVEVEKNGRTTVQGEQVENREEALSDQYPQTGTYPDVQTPYQIIKVDGS
EKNGQHKALNPNPYERVIPEGTLSKRIYQVNNLDDNQYGIELTVSGKTVYEQKDKSVPLDVVILLDNSNSMSNIRNKNA
RRAERAGEATRSLIDKITSDSENRVALVTYASTIFDGTEFTVEKGVADKNGKRLNDSLFWNYDQTSFTTNTKDYSYLKL
TNDKNDIVELKNKVPTEAEDHDGNRLMYQFGATFTQKALMKADEILTQQARQNSQKVIFHITDGVPTMSYPINFNHAT
FAPSYQNQLNAFFSKSPNKDGILLSDFITQATSGEHTIVRGDGQSYQMFTDKTVYEKGAPAAFPVKPEKYSEMKAAGYA
VIGDPINGGYIWLNWRESILAYPFNSNTAKITNHGDPTRWYYNGNIAPDGYDVFTVGIGINGDPGTDEATATSFMQSISS
KPENYTNVTDTTKILEQLNRYFHTIVTEKKSIENGTITDPMGELIDLQLGTDGRFDPADYTLTANDGSRLENGQAVGGP
QNDGGLLKNAKVLYDTTEKRIRVTGLYLGTDEKVTLTYNVRLNDEFVSNKFYDTNGRTTLHPKEVEQNTVRDFPIPKI
RDVRKYPEITISKEKKLGDIEFIKVNKNDKKPLRGAVFSLQKQHPDYPDIYGAIDQNGTYQNVRTGEDGKLTFKNLSDG
KYRLFENSEPAGYKPVQNKPIVAFQIVNGEVRDVTSIVPQDIPAGYEFTNDKHYITNEPIPPKREYPRTGGIGMLPFYLIG
CMMMGGVLLYTRKHPZ

MKSINKFLTMLAALLLTASSLFSAATVFAAGTTTTSVTVHKLLATDGDMDKIANELETGNYAGNKVGVLPANAKEIAG
VMFVWTNTNNNEIIDENGQTLGVNIDPQTFKLSGAMPATAMKKLTEAEGAKFNTANLPAAKYKIYEIHSLSTYVGEDGA
TLTGSKAVPIEIELPLNDVVDAHVYPKNTEAKPKIDKDFKGKANPDTPRVDKDTPVNHQVGDVVEYEIVTKIPALANYA
TANWSDRMTEGLAFNKGTVKVTVDDVALEAGDYALTEVATGFDLKLTDAGLAKVNDQNAEKTVKITYSATLNDKAI
VEVPESNDVTFNYGNNPDHGNTPKPNKPNENGDLTLTKTWVDATGAPIPAGAEATFDLVNAQTGKVVQTVTLTTDKN
TVTVNGLDKNTEYKFVERSIKGYSADYQEITTAGEIAVKNWKDENPKPLDPTEPKVVTYGKKFVKVNDKDNRLAGAEF
VIANADNAGQYLARKADKVSQEEKQLVVTTKDALDRAVAAYNALTAQQQTQQEKEKVDKAQAAYNAAVIAANNAF
EWVADKDNENVVKLVSDAQGRFEITGLLAGTYYLEETKQPAGYALLTSRQKFEVTATSYSATGQGIEYTAGSGKDDAT
KVVNKKITIPQTGGIGTIIFAVAGAAIMGIAVYAYVKNNKDEDQLAZ

MTMQKMQKMISRIFFVMALCFSLVWGAHAVQAQEDHTLVLQLENYQEVVSQLPSRDGHRLQVWKLDDSYSYDDRV
QIVRDLHSWDENKLSSFKKTSFEMTFLENQIEVSHIPNGLYYVRSIIQTDAVSYPAEFLFEMTDQTVEPLVIVAKKTDTM
TTKVKLIKVDQDHNRLEGVGFKLVSVARDVSEKEVPLIGEYRYSSSGQVGRTLYTDKNGEIFVTNLPLGNYRFKEVEPL
AGYAVTTLDTDVQLVDHQLVTITVVNQKLPRGNVDFMKVDGRTNTSLQGAMFKVMKEESGHYTPVLQNGKEVVVTS
GKDGRFRVEGLEYGTYYLWELQAPTGYVQLTSPVSFTIGKDTRKELVTVVKNNKRPRIDVPDTGEETLVYLDACCHFV
VWZ

MSHIYLSIFTSLLLMLGLVNVAQADEYLRIGMEAAYAPFNWTQDDDSNGAVKIDGTNQYANGYDVQIAKKIAKDLGKE
PLVVKTKWEGLVPALTSGKIDMIIAGMSPTAERKQEIAFSSSYYTSEPVLLVKKDSAYASAKSLDDFNGAKITSQQGVYL
YNLIAQIPGAKKETAMGDFAQMRQALEAGVIDAYVSERPEALTAEAANSKFKMIQVEPGFKTGEEDTAIAIGLRKNDNR
ISQINASIETISKDDQVALMDRMIKEQPAEATTTEETSSSFFSQVAKILSENWQQLLRGAGITLLISIVGTIIGLIIGLAIGVFR
TAPLSENKVIYGLQKLVGWVLNVYIEIFRGTPMIVQSMVIYYGTAQAFGINLDRTLAAIFIVSINTGAYMTEIVRGGILAV
DKGQFEAATALGMTHNQTMRKIVLPQVVRNILPATGNEFVINIKDTSVLNVISVVELYFSGNTVATQTYQYFQTFTIIAV
IYFVLTFTVTRILRFIERRMDMDTYTTGANQMQTEDLKZ

MTQAILEIKHLKKSYGQNEVLKDISLTVHKGEVISIIGSSGSGKSTFLRSINLLETPTDGQILYHGQNVLEKGYDLTQYREK
LGMVFQSFNLFENLNVLENTIVAQTTVLKRERTEAEKIAKENLEKVGMGERYWQAKPKQLSGGQKQRVAIARALSMN
PDAILFDEPTSALDPEMVGEVLKIMQDLAQEGLTMIVVTHEMEFARDVSHRVIFMDKGVIAEEGKPEDLFTNPKEDRTK
EFLQRYLKZ

MKKYQLLFKISAVFSYLFFVFSLSQLTLIVQNYWQFSSQIGNLFWIQNILSLLFIGVMIVVLVKTGHGYLFRIPRKKWLW
YSILTVLVLVFQISFNVQTAKHVQSTAEGWAVLIGYSGTNFAELGIYIALFFLVPLMEELIYRGLLQHAFFKHSRFGLDLL
LPSILFALPHFSSLPSLLDIFVFATVGIIFAGLTRYTKSIYPSYAVHVINNIVATFPFLLTFLHRVLGZ

MNKKQWLGLGLVAVAAVGLAACGNRSSRNAASSSDVKTKAAIVTDTGGVDDKSFNQSAWEGLQAWGKEHNLSKDN
GFTYFQSTSEADYANNLQQAAGSYNLIFGVGFALNNAVKDAAKEHTDLNYVLIDDVIKDQKNVASVTFADNESGYLA
GVAAAKTTKTKQVGFVGGIESEVISRFEAGFKAGVASVDPSIKVQVDYAGSFGDAAKGKTIAAAQYAAGADIVYQVAG
GTGAGVFAEAKSLNESRPENEKVWVIGVDRDQEAEGKYTSKDGKESNFVLVSTLKQVGTTVKDISNKAERGEFPGGQV
IVYSLKDKGVDLAVTNLSEEGKKAVEDAKAKILDGSVKVPEKZ

MSKKLQQISVPLISVFLGILLGAIVMWIFGYDAIWGYEELFYTAFGSLRGIGEIFRAMGPLVLIGLGFAVASRAGFFNVGL
PGQALAGWILSGWFALSHPDMPRPLMILATIVIALIAGGIVGAIPGILRAYLGTSEVIVTIMMNYIVLYVGNAFIHAFPKD
FMQSTDSTIRVGANATYQTPWLAELTGNSRMNIGIFFAIIAVAVIWFMLKKTTLGFEIRAVGLNPHASEYAGISAKRTIIL
SMIISGALAGLGGAVEGLGTFQNVYVQGSSLAIGFNGMAVSLLAANSPIGILFAAFLFGVLQVGAPGMNAAQVPSELVSI
VTASIIFFVSVHYLIERFVKPKKQVKGGKZ

MGVKKKLKLTSLLGLSLLIMTACATNGVTSDITAESADFWSKLVYFFAEIIRFLSFDISIGVGIILFTVLIRTVLLPVFQVQ
MVASRKMQEAQPRIKALREQYPGRDMESRTKLEQEMRKVFKEMGVRQSDSLWPILIQMPVILALFQALSRVDFLKTGH
FLWINLGSVDTTLVLPILAAVFTFLSTWLSNKALSERNGATTAMMYGIPVLIFIFAVYAPGGVALYWTVSNAYQVLQTY
FLNNPFKIIAEREAVVQAQKDLENRKRKAKKKAQKTKZ

MVIDPFAINELDYYLVSHFHSDHIDPYTAAAILNNPKLEHVKFIGPYHCGRIWEGWGVPKERIIVVKPGDTIELKDMKIH
AVESFDRTCLVTLPVNGADETGGELAGLAVTDEEMAQKAVNYIFETPGGTIYHGADSHFSNYFAKHGKDFKIDVALNN
YGENPVGIQDKMTSIDLLRMAENLRTKVIIPVHYDIWSNFMASTNEILELWKMRKDRLQYDFHPFIWEVGGKYTYPQD
QHLVEYHHPRGFDDCFEQDSNIQFKALLZ

MFLSGWLSSFANTYIHDLLGVLFPDSPFLNAFESAIAAPLVEEPLKLLSLVFVLALIPVRKLKSLFLLGIASGLGFQMIKDI
GYIRTDLPEGFDFTISRILERIISGIASHWTFSGLAVVGVYLLYRAYKGQKVGKKQGLIFLGLALGTHFLFNSPFVELETEL
PLAIPVVTAIALYGFYHAYCFVEKHNELMTZ

MKVEPRCDVLSRMSHFFIRILIMELQELVERSWAIRQAYHELEVKHHDSKWTVEEDLLALSNDIGNFQRLVMTKQGRY
YDETPYTLEQKLSENIWWLLELSQRLDIDILTEMENFLSDKEKQLNVRTWKZ

MLDWKQFFLAYLRSRSRLFIYLLSLAFLVLLFQFLFASLGIYFLYFFFLCCFVTILFFTWDILVETQVYRQELLYGEREAK
SPLEIALAEKLEAREMELYQQRSKAERKLTDLLDYYTLWVHQIKTPIAASQLLVAEVVDRQLKQQLEQEIFKIDSYTNLV
LQYLRLESFHDDLVLKQVQIEDLVKEIIRKYALFFIQKGLNVNLHDLDKEIVTDKKWLLVVIEQIISNSLKYTKEGGLEIY
MDDQELCIKDTGIGIKNSDVLRVFERGFSGYNGRLTQQSSGLGLYLSKKISEELGHQIRIESEVGKGTTVRIQFAQVNLVL
EZ

MELNTHNAEILLSAANKSHYPQDELPEIALAGRSNVGKSSFINTMLNRKNLARTSGKPGKTQLLNFFNIDDKMRFVDVP
GYGYARVSKKEREKWGCMIEEYLTTRENLRAVVSLVDLRHDPSADDVQMYEFLKYYEIPVIIVATKADKIPRGKWNKH
ESAIKKKLNFDPSDDFILFSSVSKAGMDEAWDAILEKLZ

MTKKQLHLVIVTGMSGAGKTVAIQSFEDLGYFTIDNMPPALLPKFLQLVEIKEDNPKLALVVDMRSRSFFSEIQAVLDEL
ENQDGLDFKILFLDAADKELVARYKETRRSHPLAADGRILDGIKLERELLAPLKNMSQNVVDTTELTPRELRKTLAEQF
SDQEQAQSFRIEVMSFGFKYGIPIDADLVFDVRFLPNPYYLPELRNQTGVDEPVYDYVMNHPESEDFYQHLLALIEPILP
SYQKEGKSVLTIAMGCTGGQHRSVAFAKRLAQDLSKNWSVNEGHRDKDRRKETVNRSZ

MRKPKITVIGGGTGSPVILKSLREKDVEIAAIVTVADDGGSSGELRKNMQQLTPPGDLRNVLVAMSDMPKFYEKVFQYR
FSEDAGAFAGHPLGNLIIAGLSEMQGSTYNAMQLLSKFFHTTGKIYPSSDHPLTLHAVFQDGTEVAGESHIVDHRGIIDN
VYVTNALNDDTPLASRRVVQTILESDMIVLGPGSLFTSILPNIVIKEIGRALLETKAEIAYVCNIMTQRGETEHFTDSDHV
EVLHRHLGRPFIDTVLVNIEKVPQEYMNSNRFDEYLVQVEHDFVGLCKQVSRVISSNFLRLENGGAFHDGDLIVDELMR
I
IQVKKZ

MKNLIKLLIIRLIVNLADSVFYIVALWHVSNNYSSSMFLGIFIAVNYLPDLLLIFFGPVIDRVNPQKILIISILVQLAVAVIFL
LLLNQISFWVIMSLVFISVMASSISYVIEDVLIPQVVEYDKIVFANSLFSISYKVLDSIFNSFASFLQVAVGFILLVKIDIGIFL
LALFILLLLKFRTSNANIENFSFKYYKREVLQGTKFILNNKLLFKTSISLTLINFFYSFQTVVVPIFSIRYFDGPIFYGIFLTIA
GLGGILGNMLAPIVIKYLKSNQIVGVFLFLNGSSWLVAIVIKDYTLSLILFFVCFMSKGVFNIIFNSLYQQIPPHQLLGRVN
TTIDSIISFGMPIGSLVAGTLIDLNIELVLIAISIPYFLFSYIFYTDNGLKEFSIYZ

MMSNKNKEILIFAILYTVLFMFDGVKLLASLMPSAIANYLVYVVLALYGSFLFKDRLIQQWKEIRKTKRKFFFGVLTGW
LFLILMTVVFEFVSEMLKQFVGLDGQGLNQSNIQSTFQEQPLLIAVFACVIGPLVEELFFRQVLLHYLQERLSGLLSIILV
GLVFALTHMHSLALSEWIGAVGYLGGGLAFSIIYVKEKENIYYPLLVHMLSNSLSLIILAISIVKZ

LKKPIIEFKNVSKVFEDSNTKVLKDINFELEEGKFYTLLGASGSGKSTILNIIAGLLDATTGDIMLDGVRINDIPTNKRDVH
TVFQSYALFPHMNVFENVAFPLRLRKIDKKEIEQRVAEVLKMVQLEGYEKRSIRKLSGGQRQRVAIARAIINQPRVVLLD
EPLSALDLKLRTDMQYELRELQQRLGITFVFVTHDQEEALAMSDWIFVMNDGEIVQSGTPVDIYDEPINHFVATFIGESN
ILPGTMIEDYLVEFNGKRFEAVDGGMKPNEPVEVVIRPEDLRITLPEEGKLQVKVDTQLFRGVHYEIIAYDELGNEWMI
HSTRKAIVGEEIGLDFEPEDIHIMRLNETEEEFDARIEEYVEIEEQEAGLINAIEEERDEENKLZ

MKSMRILFLLALIQISLSSCFLWKECILSFKQSTAFFIGSMVFVSGICAGVNYLYTRKQEVHSVLASKKSVKLFYSMLLLIN
LLGAVLVLSDNLFIKNTLQQELVDFLLPSFFFLFGLDLLIFLPLKKYVRDFLAMLDRKKTVLVTILATLLFLRNPMTIVSL
LIYIGLGLFFAAYLVPNSVKKEVSFYGHIFRDLVLVIVTLIFFZ

MVKKIIGMVLALLSVTVVGVGVFAYTIYQQGTETLAKTYKKIGEETKVIEATEPLTILLMGVDTGNVERTETWVGRSDS
MILMTVNPKTKKTTMMSLERDILTRIESGNGQAHEAKLNSAYADGGAELAIETIQKMMNIHIDRYVMVNMRGLQKLV
DAVGGITVNNILGFPISISDQEEFNTISIGVGEQHIGGEEALVYARMRYQDPEGDYGRQKRQREVIQKVMEKALSLNSIGH
YQEILKALSDNMQTNIDLSAKSIPNLLGYKDSFKTIETQQLQGEGEILQGVSYQIVSRAHMLEMQNLLRRSLGQEEVTQL
ETNAVLFEDLFGRAPVGDEDNZ

MKKQAYVIIALTSFLFVFFFSHSLLEILDFDWSIFLHDVEKTEKFVFLLLVFSMSMTCLLALFWRGIEELSLRKMQANLK
RLLAGQEVVQVADPDLDASFKSLSGKLNLLTEALQKAENQSLAQEEEIIEKERKRIARDLHDTVSQELFAAHMILSGISQ
QALKLDREKMQTQLQSVTAILETAQKDLRVLLLHLRPVELEQKSLIEGIQILLKELEDKSDLRVSLKQNMTKLPKKIEEHI
FRILQELISNTLRHAQASCLDVYLYQTDVELQLKVVDNGIGFQLGSLDDLSYGLRNIKERVEDMAGTVQLLTAPKQGLA
VDIRIPLLDKEZ

MIVSIISQGFVWAILGLGIFMTFRILNFPDMTTEGSFPLGGAVAVTLITKGVNPFLATLVAVGAGCLAGMAAGLLYTKGK
IPTLLSGILVMTSCHSIMLLIMGRANLGLLGTKQIQDVLPFDSDLNQLLTGLIFVSIVIALMLFFLDTKLGQAYIATGDNP
DMARSFGIHTGRMELMGLVLSNGVIALAGALIAQQEGYADVSRGIGVIVVGLASLIIGEVIFKSLSLAERLVTIVVGSIAY
QFLVWAVIALGFNTSYLRLYSALILAVCLMIPTFKQTILKGAKLSKZ

MKKMKVWSTVLATGVALTTLAACSGGSNSTTASSSEEKADKSQELVIYSNSVSNGRGDWLTAKAKEAGFNIKMVDIAG
AQLADRVIAEKNNAVADMVFGIGAVDSNKIRDQKLLVQYKPKWLDKIDQSLSDKDNYYNPVIVQPLVLIGAPDVKEMP
KDWTELGSKYKGKYSISGLQGGTGRAILASILVRYLDDKGELGVSEKGWEVAKEYLKNAYTLQKGESSIVKMLDKEDPI
QYGMMWGSGALVGQKEQNVVFKVMTPEIGVPFVTEQTMVLSTSKKQALAKEFIDWFGQSEIQVEYSKNFGSIPANKD
ALKDLPEDTKKFVDQVKPQNIDWEAVGKHLDEWVEKAELEYVQZ

MIKFDNIQIKYGDFVAIDNLNLDIHEGEFFTFLGPSGCGKSTTLRALVGFLDPSSGSIEVNGTDVTHLEPEKRGIGIVFQSY
ALFPTMTVFDNIAFGLKVKKVAPDVIKAKVSAVAAKIKISDQQLQRNVSELSGGQQQRVALARALVLEPKILCLDEPLS
NLDAKLRVDLRKELKRLQKELGITTLYVTHDQEEALTLSDRIAVFNNGYIEQVGTPVEIYHNSQTEFVCDFIGDINVLTD
ETVHEVLLKNTSVFLEDKKGYIRLEKVRFNRETEQDFILKGTIIDVEFSGVTIHYTIKVSESQILNVTSIDSQAAIRSVGESV
ELFITPSDVLQFZ

MRHKLNLKDWLIRLGLIWFLVTFIIYPNFDLVVNVFVKGGEFSLDAVHRVLKSQRALQSIMNSFKLAFSLIITVNVVGIL
CVLFTEYFDIKGAKILKLGYMTSLIYGGVVLATGYKFVYGPYGLITKFLQNVIPSLDPNWFIGYGAVLFIMTFSGTANHT
LFLTNTIRSVDYHTIEAARNMGAKPFTVFRKVVLPTLIPTLFALTIMVFLSGLSAVAAPMIVGGKEFQTINPMIITFAGMG
NSRDLAALLAIILGIATTILLTIMNKIEKGGNYISISKTKAPLKKQKIASKPWNIIAHIVAYGLFTVFMLPLIFIVLYSFTDPV
AIQTGNLTLSNFTLENYRLFFSNSAAFSPFLVSFIYSIIAATTATILAVVFARVVRKHKSRFDFLFEYGALLPWLLPSTLLA
VSLLFTFNQPQFLVLNQILVGSLVILLIAYIVVKIPFSYRMVRAILFSVDDEMEDAARSMGASPFYTMMKVIIPFILPVVLS
VIALNFNSLLTDFDLSVFLYHPLAQPLGITIRSAGDETATSNAQALVFVYTIVLMIISGTVLYFTQRPGRKVRKZ

Table 3

ID201 - 4106.4

ATGATAAAAAATCCTAAATTATTAACCAAGTCTTTTTTAAGAAGTTTTGCAATTCTAGGTGGTGTTGGTCTAGTCAT
TCATATAGCTATTTATTTGACCTTTCCTTTTTATTATATTCAACTGGAGGGGGAAAAGTTTAATGAGAGCGCAAGAG
TGTTTACGGAGTATTTAAAGACTAAGACATCTGATGAAATTCCAAGCTTACTCCAGTCTTATTCAAAGTCCTTGACC
ATATCTGCTCACCTTAAAAGAGATATTGTAGATAAGCGGCTCCCTCTTGTGCATGACTTGGATATTAAAGATGGAAA
GCTATCAAATTATATCGTGATGTTAGATATGTCTGTTAGTACAGCAGATGGTAAACAGGTAACCGTGCAATTTGTTC
ACGGGGTGGATGTCTACAAAGAAGCAAAGAATATTTTGCTTTTGTATCTCCCATATACATTTTTGGTTACAATTGCT
TTTTCCTTTGTTTTTTCTTATTTTTATACTAAACGCTTGCTCAATCCTCTTTTTTACATTTCAGAAGTGACTAGTAA
AATGCAAGATTTGGATGACAATATTCGTTTTGATGAAAGTAGGAAAGATGAAGTTGGTGAAGTTGGAAAACAGATTA
ATGGTATGTATGAGCACTTGTTGAAGGTTATTTATGAGTTGGAAAGTCGTAATGAGCAAATTGTAAAATTGCAAAAT
CAAAAGGTTTCCTTTGTCCGCGGAGCATCACATGAGTTGAAAACCCCTTTAGCCAGTCTTAGAATTATCCTAGAGAA
TATGCAGCATAATATTGGAGATTACAAAGATCATCCAAAATATATTGCAAAGAGTATAAATAAGATTGACCAGATGA
GCCACTTATTAGAAGAAGTACTGGAGTCTTCTAAATTCCAAGAGTGGACAGAGTGTCGTGAGACCTTGACTGTTAAG
CCAGTTTTAGTAGATATTTTATCACGTTATCAAGAATTAGCTCATTCAATAGGTGTTACAATTGAAAATCAATTGAC
AGATGCTACCAGGGTCGTCATGAGTCTTAGGGCATTGGATAAGGTTTTGACAAACCTGATTAGTAATGCAATTAAAT
ATTCAGATAAAAATGGGCGTGTAATCATATCCGAGCAAGATGGCTATCTCTCTATCAAAAATACATGTGCGCCTCTA
AGTGACCAAGAACTAGAACATTTATTTGATATATTCTATCATTCTCAAATCGTGACAGATAAGGATGAAAGTTCCGG
TTTGGGTCTTTACATTGTGAATAATATTTTAGAAAGCTATCAAATGGATTATAGTTTTCTCCCTTATGAACACGGTA
TGGAATTTAAGATTAGCTTGTAG

MIKNPKLLTKSFLRSFAILGGVGLVIHIAIYLTFPFYYIQLEGEKFNESARVFTEYLKTKTSDEIPSLLQSYSKSLT
ISAHLKRDIVDKRLPLVHDLDIKDGKLSNYIVMLDMSVSTADGKQVTVQFVHGVDVYKEAKNILLLYLPYTFLVTIA
FSFVFSYFYTKRLLNPLFYISEVTSKMQDLDDNIRFDESRKDEVGEVGKQINGMYEHLLKVIYELESRNEQIVKLQN
QKVSFVRGASHELKTPLASLRIILENMQHNIGDYKDHPKYIAKSINKIDQMSHLLEEVLESSKFQEWTECRETLTVK
PVLVDILSRYQELAHSIGVTIENQLTDATRVVMSLRALDKVLTNLISNAIKYSDKNGRVIISEQDGYLSIKNTCAPL
SDQELEHLFDIFYHSQIVTDKDESSGLGLYIVNNILESYQMDYSFLPYEHGMEFKISLZ

ID202 - 4106.9

ATGGATAAAATTATTAAAACTATATCAGAAAGCGGAGCCTTTCGTGCTTTTGTCCTTGATAGCACTGAAACCGTCCG
CACTGCTCAAGAAAAACATCAAACCCAAGCTAGCTCAACTGTAGCGCTTGGTCGAACTCTTATCGCTAGCCAGATTC
TCGCAGCCAATGAAAAAGGAAATACCAAACTTACAGTTAAGGTGTTGGGATCTAGCTCTCTAGGTGCTATTATCACC
GTCGCTGATACCAAGGGGAACGTCAAAGGCTATGTTCAAAATCCTGGTGTTGACATCAAAAAGACTGCGACTGGTGA
AGTCCTAGTCGGACCTTTTGTTGGAAATGGTCAATTCCTCGTTATCACAGACTACGGTACTGGAAATCCTTACAACT
CTATAACTCCCCTCATCTCTGGAGAAATCGGTGAAGACCTTGCCTTTTACCTTACTGAAAGCCAACAAACGCCTTCA
GCGGTCGGCCTCAATGTCCTTTTGGACGAGGAAGACAAGGTCAAGGTTGCAGGTGGTTTCCTAGTTCAAGTCTTGCC
AGGAGCCAAGAAAGAAGAGATTGCTCGCTTTGAAAAACGCATCCAAGAAATGCCAGCTATCTCTACTCTTCTCGAAA
GCGACGACCATATCGAAGCCCTCCTCAAGGCTATCTACGGGGACGAAGCCTACAAGCGTCTTTCTGAAGAAGAAATC
CGTTTCCAATGTGACTGTAGCCATGAACGCTTTATGAACGCTCTTGCCAGCCTTCCAAGCTCAGACTTACAGGAAAT
GAAAGAGGAAGACCACGGGGCAGAAATCACTTGTCAATTCTGCCAAACTACTTACAACTTTGATGAAAAGGACCTGG
AGGAACTCATTCGTGACAAATCTTAA

MDKIIKTISESGAFRAFVLDSTETVRTAQEKHQTQASSTVALGRTLIASQILAANEKGNTKLTVKVLGSSSLGAIIT
VADTKGNVKGYVQNPGVDIKKTATGEVLVGPFVGNGQFLVITDYGTGNPYNSITPLISGEIGEDLAFYLTESQQTPS
AVGLNVLLDEEDKVKVAGGFLVQVLPGAKKEEIARFEKRIQEMPAISTLLESDDHIEALLKAIYGDEAYKRLSEEEI
RFQCDCSHERFMNALASLPSSDLQEMKEEDHGAEITCQFCQTTYNFDEKDLEELIRDKSZ

ID203 - 4115

ATGAAATCAATAACTAAAAAGATTAAAGCAACTCTTGCAGGAGTAGCTGCCTTGTTTGCAGTATTTGCTCCATCATT
TGTATCTGCTCAAGAATCATCAACTTACACTGTTAAAGAAGGTGATACACTTTCAGAAATCGCTGAAACTCACAACA
CAACAGTTGAAAAATTGGCAGAAAACAACCACATTGATAACATTCATTTGATTTATGTTGATCAAGAGTTGGTTATC
GATGGCCCTGTAGCGCCTGTTGCAACACCAGCGCCAGCTACTTATGCGGCACCAGCCGCTCAAGATGAAACTGTTTC

AGCTCCAGTAGCAGAAACTCCAGTAGTAAGTGAAACAGTTGTTTCAACTGTAAGCGGATCTGAAGCAGAAGCCAAAG
AATGGATCGCTCAAAAAGAATCAGGTGGTAGTATACAGCTACAAATGGACGTTATATCGGACGTTACCAATTAA

MKSITKKIKATLAGVAALFAVFAPSFVSAQESSTYTVKEGDTLSEIAETHNTTVEKLAENNHIDNIHLIYVDQELVI
DGPVAPVATPAPATYAAPAAQDETVSAPVAETPVVSETVVSTVSGSEAEAKEWIAQKESGGSIQLQMDVISDVTNZ

ID204 - 4117.1

ATGAATTTAGGAGAATTTTGGTACAATAAAATAAATAAGAACAGAGGAAGAAGGTTAATGAAGAAAGTAAGATTTAT
TTTTTTAGCTCTGCTATTTTTCTTAGCTAGTCCAGAGGGTGCAATGGCTAGTGATGGTACTTGGCAAGGAAAACAGT
ATCTGAAAGAAGATGGCAGTCAAGCAGCAAATGAGTGGGTTTTTGATACTCATTATCAATCTTGGTTCTATATAAAA
GCAGATGCTAACTATGCTGAAAATGAATGGCTAAAGCAAGGTGACGACTATTTTTACCTCAAATCTGGTGGCTATAT
GGCCAAATCAGAATGGGTAGAAGACAAGGGAGCCTTTTATTATCTTGACCAAGATGGAAAGATGAAAAGAAATGCTT
GGGTAGGAACTTCCTATGTTGGTGCAACAGGTGCCAAAGTAATAGAAGACTGGGTCTATGATTCTCAATACGATGCT
TGGTTTTATATCAAAGCAGATGGACAGCACGCAGAGAAAGAATGGCTCCAAATTAAAGGGAAGGACTATTATTTCAA
ATCCGGTGGTTATCTACTGACAAGTCAGTGGATTAATCAAGCTTATGTGAATGCTAGTGGTGCCAAAGTACAGCAAG
GTTGGCTTTTTGACAAACAATACCAATCTTGGTTTTACATCAAAGAAAATGGAAACTATGCTGATAAAGAATGGATT
TTCGAGAATGGTCACTATTATTATCTAAAATCCGGTGGCTACATGGCAGCCAATGAATGGATTTGGGATAAGGAATC
TTGGTTTTATCTCAAATTTGATGGAAAAATGGCTGAAAAAGAATGGGTCTACGATTCTCATAGTCAAGCTTGGTACT
ACTTCAAATCCGGTGGTTACATGACAGCCAATGAATGGATTTGGGATAAGGAATCTTGGTTTTATCTCAAATCTGAT
GGGAAAATAGCTGAAAAAGAATGGGTCTACGATTCTCATAGTCAAGCTTGGTACTACTTCAAATCCGGTGGTTACAT
GACAGCCAATGAATGGATTTGGGATAAGGAATCTTGGTTTTACCTCAAATCTGATGGGAAAATAGCTGAAAAAGAAT
GGGTCTACGATTCTCATAGTCAAGCTTGGTACTACTTCAAATCTGGTGGCTACATGGCGAAAAATGAGACAGTAGAT
GGTTATCAGCTTGGAAGCGATGGTAAATGGCTTGGAGGAAAAACTACAAATGAAAATGCTGCTTACTATCAAGTAGT
GCCTGTTACAGCCAATGTTTATGATTCAGATGGTGAAAAGCTTTCCTATATATCGCAAGGTAGTGTCGTATGGCTAG
ATAAGGATAGAAAAAGTGATGACAAGCGCTTGGCTATTACTATTTCTGGTTTGTCAGGCTATATGAAAACAGAAGAT
TTACAAGCGCTAGATGCTAGTAAGGACTTTATCCCTTATTATGAGAGTGATGGCCACCGTTTTTATCACTATGTGGC
TCAGAATGCTAGTATCCCAGTAGCTTCTCATCTTTCTGATATGGAAGTAGGCAAGAAATATTATTCGGCAGATGGCC
TGCATTTTGATGGTTTTAAGCTTGAGAATCCCTTCCTTTTCAAAGATTTAACAGAGGCTACAAACTACAGTGCTGAA
GAATTGGATAAGGTATTTAGTTTGCTAAACATTAACAATAGCCTTTTGGAGAACAAGGGCGCTACTTTTAAGGAAGC
CGAAGAACATTACCATATCAATGCTCTTTATCTCCTTGCCCATAGTGCCCTAGAAAGTAACTGGGGAAGAAGTAAAA
TTGCCAAAGATAAGAATAATTTCTTTGGCATTACAGCCTATGATACGACCCCTTACCTTTCTGCTAAGACATTTGAT
GATGTGGATAAGGGAATTTTAGGTGCAACCAAGTGGATTAAGGAAAATTATATCGATAGGGGAAGAACTTTCCTTGG
AAACAAGGCTTCTGGTATGAATGTGGAATATGCTTCAGACCCTTATTGGGGCGAAAAAATTGCTAGTGTGATGATGA
AAATCAATGAGAAGCTAGGTGGCAAAGATTAG

MNLGEFWYNKINKNRGRRLMKKVRFIFLALLFFLASPEGAMASDGTWQGKQYLKEDGSQAANEWVFDTHYQSWFYIK
ADANYAENEWLKQGDDYFYLKSGGYMAKSEWVEDKGAFYYLDQDGKMKRNAWVGTSYVGATGAKVIEDWVYDSQYDA
WFYIKADGQHAEKEWLQIKGKDYYFKSGGYLLTSQWINQAYVNASGAKVQQGWLFDKQYQSWFYIKENGNYADKEWI
FENGHYYYLKSGGYMAANEWIWDKESWFYLKFDGKMAEKEWVYDSHSQAWYYFKSGGYMTANEWIWDKESWFYLKSD
GKIAEKEWVYDSHSQAWYYFKSGGYMTANEWIWDKESWFYLKSDGKIAEKEWVYDSHSQAWYYFKSGGYMAKNETVD
GYQLGSDGKWLGGKTTNENAAYYQVVPVTANVYDSDGEKLSYISQGSVVWLDKDRKSDDKRLAITISGLSGYMKTED
LQALDASKDFIPYYESDGHRFYHYVAQNASIPVASHLSDMEVGKKYYSADGLHFDGFKLENPFLFKDLTEATNYSAE
ELDKVFSLLNINNSLLENKGATFKEAEEHYHINALYLLAHSALESNWGRSKIAKDKNNFFGITAYDTTPYLSAKTFD
DVDKGILGATKWIKENYIDRGRTFLGNKASGMNVEYASDPYWGEKIASVMMKINEKLGGKDZ

ID205 - 4118.1
ATGAAAAAATTAGGTACATTACTCGTTCTCTTTCTTTCTGCAATCATTCTTGTAGCATGTGCTAGCGGAAAAAAAGA
TACAACTTCTGGTCAAAAACTAAAAGTTGTTGCTACAAACTCAATCATCGCTGATATTACTAAAAATATTGCTGGTG
ACAAAATTGACCTTCATAGTATCGTTCCGATTGGGCAAGACCCACACGAATACGAACCACTTCCTGAAGACGTTAAG
AAAACTTCTGAGGCTAATTTGATTTTCTATAACGGTATCAACCTTGAAACAGGTGGCAATGCTTGGTTTACAAAATT
GGTAGAAAATGCCAAGAAAACTGAAAACAAAGACTACTTCGCAGTCAGCGACGGCGTTGATGTTATCTACCTTGAAG
GTCAAAATGAAAAAGGAAAAGAAGACCCACACGCTTGGCTTAACCTTGAAAACGGTATTATTTTTGCTAAAAATATC
GCCAAACAATTGAGCGCCAAAGACCCTAACAATAAAGAATTCTATGAAAAAAATCTCAAAGAATATACTGATAAGTT
AGACAAACTTGATAAAGAAAGTAAGGATAAATTTAATAAGATCCCTGCTGAAAAGAAACTCATTGTAACCAGCGAAG
GAGCATTCAAATACTTCTCTAAAGCCTATGGTGTCCCAAGTGCTTACATCTGGGAAATCAATACTGAAGAAGAAGGA
ACTCCTGAACAAATCAAGACCTTGGTTGAAAAACTTCGCCAAACAAAAGTTCCATCACTCTTTGTAGAATCAAGTGT
GGATGACCGTCCAATGAAAACTGTTTCTCAAGACACAAACATCCCAATCTACGCTCAAATCTTTACTGACTCTATCG

CAGAACAAGGTAAAGAAGGCGACAGCTACTACAGCATGATGAAATACAACCTTGACAAGATTGCTGAAGGATTGGCA
AAATAA

MKKLGTLLVLFLSAIILVACASGKKDTTSGQKLKVVATNSIIADITKNIAGDKIDLHSIVPIGQDPHEYEPLPEDVK
KTSEANLIFYNGINLETGGNAWFTKLVENAKKTENKDYFAVSDGVDVIYLEGQNEKGKEDPHAWLNLENGIIFAKNI
AKQLSAKDPNNKEFYEKNLKEYTDKLDKLDKESKDKFNKIPAEKKLIVTSEGAFKYFSKAYGVPSAYIWEINTEEEG
TPEQIKTLVEKLRQTKVPSLFVESSVDDRPMKTVSQDTNIPIYAQIFTDSIAEQGKEGDSYYSMMKYNLDKIAEGLA
KZ

ID206 - 4119.1
ATGGAATGGTATAAAAAAATCGGACTTCTTGCAACTACAGGTTTAGCTTTGTTTGGGCTCGGCGCTTGCTCCAACTA
TGGTAAATCTGCGGATGGCACAGTGACCATCGAGTATTTCAACCAGAAAAAAGAAATGACCAAAACCTTGGAAGAAA
TCACTCGTGATTTTGAGAAGGAAAACCCTAAGATCAAGGTCAAAGTCGTCAATGTACCAAATGCTGGTGAAGTATTG
AAGACACGCGTTCTCGCAGGAGATGTGCCTGATGTGGTCAATATTTACCCACAGTCCATCGAACTGCAAGAATGGGC
AAAAGCAGGTGTTTTTGAAGATTTGAGCAACAAAGACTACCTGAAACGCGTGAAAAATGGCTACGCTGAAAAATATG
CTGTAAACGAAAAAGTTTACAACGTTCCTTTTACAGCTAATGCTTATGGAATTTACTACAACAAAGATAAATTCGAA
GAACTGGGCTTGAAGGTTCCTGAAACCTGGGATGAATTTGAACAGTTAGTCAAAGATATCGTTGCTAAAGGACAAAC
ACCATTTGGAATTGCAGGTGCAGATGCTTGGACACTCAATGGTTACAATCAATTAGCCTTTGCGACAGCAACAGGTG
GAGGAAAAGAAGCAAATCAATACCTTCGTTATTCTCAACCAAATGCCATTAAATTGTCGGATCCGATTATGAAAGAT
GATATCAAGGTCATGGACATCCTTCGCATCAATGGATCTAAGCAAAAGAACTGGGAAGGTGCTGGCTATACCGATGT
TATCGGAGCCTTCGCACGTGGGGATGTCCTCATGACACCAAATGGGTCTTGGGCGATCACAGCGATTAATGAACAAA
AACCGAACTTTAAGATTGGGACCTTCATGATTCCAGGAAAAGAAAAAGGACAAAGCTTAACCGTTGGTGCGGGAGAC
TTGGCATGGTCTATCTCAGCCACCACCAAACATCCAAAAGAAGCCAATGCCTTTGTGGAATATATGACCCGTCCAGA
AGTCATGCAAAAATACTACGATGTGGACGGATCTCCAACAGCGATCGAAGGGGTCAAACAAGCAGGAGAAGATTCAC
CGCTTGCTGGTATGACCGAATATGCCTTTACGGATCGTCACTTGGTCTGGTTGCAACAATACTGGACCAGTGAAGCA
GACTTCCATACCTTGACCATGAACTATGTCTTGACCGGTGATAAACAAGGCATGGTCAATGATTTGAATGCCTTCTT
TAACCCGATGAAAGCGGATGTGGATTAG

MEWYKKIGLLATTGLALFGLGACSNYGKSADGTVTIEYFNQKKEMTKTLEEITRDFEKENPKIKVKVVNVPNAGEVL
KTRVLAGDVPDVVNIYPQSIELQEWAKAGVFEDLSNKDYLKRVKNGYAEKYAVNEKVYNVPFTANAYGIYYNKDKFE
ELGLKVPETWDEFEQLVKDIVAKGQTPFGIAGADAWTLNGYNQLAFATATGGGKEANQYLRYSQPNAIKLSDPIMKD
DIKVMDILRINGSKQKNWEGAGYTDVIGAFARGDVLMTPNGSWAITAINEQKPNFKIGTFMIPGKEKGQSLTVGAGD
LAWSISATTKHPKEANAFVEYMTRPEVMQKYYDVDGSPTAIEGVKQAGEDSPLAGMTEYAFTDRHLVWLQQYWTSEA
DFHTLTMNYVLTGDKQGMVNDLNAFFNPMKADVDZ

ID207 - 4123.1
ATGAAGAAAATCAAACCGCATGGACCGTTACCAAGTCAGACTCAGCTAGCTTATCTGGGAGATGAACTAGCAGCTTT
TATCCACTTCGGTCCTAATACCTTTTATGACCAAGAATGGGGGACTGGACAGGAGGATCCTGAGCGCTTTAACCCGA
GTCAGTTGGATGCGCGTGAGTGGGTTCGTGTGCTCAAGGAAACGGGCTTCAAAAAGTTGATTTTGGTGGTCAAGCAC
CACGATGGCTTTGTCCTTTATCCGACAGCTCACACAGATTATTCGGTTAAGGTCAGTCCTTGGAGGAGAGGAAAGGG
CGACTTGCTCCTTGAAGTATCCCAAGCTGCCACAGAGTTTGATATGGATATGGGGGTCTACCTGTCACCGTGGGATG
CCCATAGTCCCCTCTATCATGTGGACCGAGAAGCGGACTACAATGCCTATTATCTGGCTCAGTTGAAGGAAATCTTA
TCAAATCCTAACTATGGGAATGCTGGTAAGTTCGCTGAGGTTTGGATGGATGGTGCCAGAGGAGAGGGCGCGCAAAA
GGTTAATTATGAATTTGAAAAATGGTTTGAAACCATTCGTGACCTGCAGGGCGATTGCTTGATTTTTTCAACAGAAG
GCACCAGTATCCGCTGGATTGGCAATGAACGAGGGTATGCAGGTGATCCACTGTGGCAAAAGGTGAATCCTGATAAA
CTAGGAACAGAAGCAGAGCTGAACTATCTTCAGCACGGGGATCCCTCGGGCACGATTTTTTCAATCGGAGAGGCAGA
TGTTTCCATCCGTCCAGGCTGGTTCTACCATGAGGATCAGGATCCTAAGTCTCTCGAGGAGTTGGTCGAAATCTACT
TTCACTCAGTAGGGCGAGGAACTCCACTCTTGCTTAATATTCCGCCGAATCAAGCTGGGCTCTTTGATGCAAAGGAT
ATTGAACGACTTTATGAATTTGCGACCTATCGCAATGAGCTCTATAAAGAAGATTTGGCTCTGGGAGCTGAGGTATC
TGGTCCAGCTCTTTCCGCAGACTTTGCTTGTCGCCATTTGACAGACGGCCTTGAGACCAGCTCTTGGGCAAGCGATG
CAGACTTGCCCATCCAGTTAGAACTCGACTTAGGTTCTCCTAAAACTTTTGATGTAATTGAGTTAAGAGAAGATTTG
AAGCTAGGGCAACGAATCGCTGCTTTTCATGTGCAAGTAGAGGTGGATGGTGTCTGGCAGGAGTTTGGTTCGGGTCA
TACTGTTGGTTACAAACGTCTCTTACGAGGAGCAGTTGTTGAGGCACAGAAGATACGTGTAGTCATTACAGAATCAC
AGGCTTTGCCTTTGTTGACCAAGATTTCCCTTTATAAAACTCCTGGATTATCAAAAAAAGAAGTTGTTCAGGAACTA
GCATTTGCAGAAAAAAGCCTAGCTGTGGCAAAGGGAGAAAATGCCTATTTTACAGTTAAGCGCAGAGAATGTAGTGG
TCCTTTAGAAGCTAAGATTTCGATTCAACCGGGGACAGGTGTCCATGGTGTCGCCTATCAGGATGAGATTCAAGTCC
TTGCGTTTCAAACTGGTGAGACTGAAAAAAGTCTGACGCTACCAACCTTGTATTTCGCAGGAGATAAAACCTTGGAT
TTCTATCTGAACCTAACGGTGGATGGTCAGCTTGTGGATCAACTTCAAGTCCAAGTTTCATAA

MKKIKPHGPLPSQTQLAYLGDELAAFIHFGPNTFYDQEWGTGQEDPERFNPSQLDAREWVRVLKETGFKKLILVVKH
HDGFVLYPTAHTDYSVKVSPWRRGKGDLLLEVSQAATEFDMDMGVYLSPWDAHSPLYHVDREADYNAYYLAQLKEIL
SNPNYGNAGKFAEVWMDGARGEGAQKVNYEFEKWFETIRDLQGDCLIFSTEGTSIRWIGNERGYAGDPLWQKVNPDK
LGTEAELNYLQHGDPSGTIFSIGEADVSIRPGWFYHEDQDPKSLEELVEIYFHSVGRGTPLLLNIPPNQAGLFDAKD
IERLYEFATYRNELYKEDLALGAEVSGPALSADFACRHLTDGLETSSWASDADLPIQLELDLGSPKTFDVIELREDL
KLGQRIAAFHVQVEVDGVWQEFGSGHTVGYKRLLRGAVVEAQKIRVVITESQALPLLTKISLYKTPGLSKKEVVQEL
AFAEKSLAVAKGENAYFTVKRRECSGPLEAKISIQPGTGVHGVAYQDEIQVLAFQTGETEKSLTLPTLYFAGDKTLD
FYLNLTVDGQLVDQLQVQVSZ

ID208 - 4125.12
ATGCTTGAAAGACTGAAAAGAATACATTATATGTTTTGGATCAGTTTAATTTTTATGATTTTCCCCATCCTGTCTGT
AGTGACTGGGTGGCTTTCTGCCTGGCATTTATTGATTGATATTCTATTTGTAGTGGCATATTTGGGTGTTTTAACAA
CTAAGAGCCAGCGCCTATCTTGGCTATATTGGGGCCTCATGCTGACTTATGTAGTTGGGAATACTGCCTTTGTTGCT
GTTAATTATATCTGGTTTTTCTTTTTCCTATCCAATCTCTTAAGTTATCATTTCAGCGTACGTAGTTTAAAGTCTTT
ACATGTCTGGACTTTTCTTCTTGCTCAAGTCCTTGTTGTGGGGCAACTGTTGATTTTTCAGAGAATCGAAGTTGAGT
TTCTATTCTATCTACTTGTAATTCTTACTTTTGTCGATTTAATGACTTTTGGATTGGTTCGGATTCGTATTGTCGAG
GATTTGAAAGAAGCTCAGGTCAAGCAAAATGCTCAGATAAATCTATTGCTTGCTGAAAATGAACGTAGTCGTATCGG
TCAGGATTTGCATGATAGTCTGGGACATACCTTTGCTATGCTGAGTGTCAAGACAGATTTAGCCTTGCAGTTATTTC
AGATGGAGGCTTATCCACAGGTGGAAAAGGAATTAAAAGAAATTCACCAGATCAGCAAGGATCCATGA

MLERLKRIHYMFWISLIFMIFPILSVVTGWLSAWHLLIDILFVVAYLGVLTTKSQRLSWLYWGLMLTYVVGNTAFVA
VNYIWFFFFLSNLLSYHFSVRSLKSLHVWTFLLAQVLVVGQLLIFQRIEVEFLFYLLVILTFVDLMTFGLVRIRIVE
DLKEAQVKQNAQINLLLAENERSRIGQDLHDSLGHTFAMLSVKTDLALQLFQMEAYPQVEKELKEIHQISKDPZ

ID209 - 4126.3
ATGAATGATAAGTTAAAAATCTTCTTGTTGCTAGGAGTATTTTTTCTAGCCATAACCGGTTTCTATGTTCTATTGAT
ACGAAATGCAGGGCAGACAGATGCCTCGCAAATTGAAAAGGCGGCAGTTAGCCAAGGAGGAAAAGCAGTGAAAAAAA
CAGAAATTAGTAAAGACGCAGACTTGCACGAAATTTATCTAGCTGGAGGTTGTTTCTGGGGAGTGGAGGAATATTTC
TCACGTGTTCCCGGGGTGACGGATGCCGTTTCAGGCTATGCAAATGGTAGAGGAGAAACAACCAAGTACGAATTGAT
TAACCAAACAGGTCATGCAGAAACCGTCCATGTCACCTATGATGCCAAGCAAATTTCTCTCAAGGAAATCCTGCTTC
ACTATTTCCGCATTATCAATCCAACCAGCAAAAATAAACAAGGAAATGATGTGGGGACCCAGTACCGTACTGGTGTT
TATTACACAGATGACAAGGATTTGGAAGTGATTAACCAAGTCTTTGATGAGGTGGCTAAGAAATACGATCAACCTCT
AGCAGTTGAAAAGGAAAACTTGAAGAATTTTGTGGTGGCTGAGGATTACCATCAAGACTATCTCAAGAAAAATCCAA
ATGGCTACTGCCATATCAATGTTAATCAGGCGGCCTATCCTGTCATTGATGCCAGCAAATATCCAAAACCAAGTGAT
GAGGAATTGAAAAAGACCCTGTCACCTGAGGAGTATGCAGTTACCCAGGAAAATCAAACAGAACGAGCTTTCTCAAA
CCGTTACTGGGATAAATTTGAATCCGGTATCTATGTGGATATAGCAACTGGGGAACCTCTCTTTTCATCAAAAGACA
AATTTGAGTCTGGTTGTGGCTGGCCTAGTTTTACCCAACCCATCAGTCCAGATGTTGTCACCTACAAGGAAGATAAG
TCCTACAATATGACGCGTATGGAAGTGCGGAGCCGAGTAGGAGATTCTCACCTTGGGCATGTCTTTACGGATGGTCC
ACAGGACAAGGGCGGCTTACGTTACTGTATCAATAGCCTCTCTATCCGCTTTATTCCCAAAGACCAAATGGAAGAAA
AAGgcTACGCTTATTTACTAGATTATGTTGATTAA

MNDKLKIFLLLGVFFLAITGFYVLLIRNAGQTDASQIEKAAVSQGGKAVKKTEISKDADLHEIYLAGGCFWGVEEYF
SRVPGVTDAVSGYANGRGETTKYELINQTGHAETVHVTYDAKQISLKEILLHYFRIINPTSKNKQGNDVGTQYRTGV
YYTDDKDLEVINQVFDEVAKKYDQPLAVEKENLKNFVVAEDYHQDYLKKNPNGYCHINVNQAAYPVIDASKYPKPSD
EELKKTLSPEEYAVTQENQTERAFSNRYWDKFESGIYVDIATGEPLFSSKDKFESGCGWPSFTQPISPDVVTYKEDK
SYNMTRMEVRSRVGDSHLGHVFTDGPQDKGGLRYCINSLSIRFIPKDQMEEKGYAYLLDYVDZ

ID210 - 4127.1
ATGAAAAAGAAATGGATGTATTATGCTGCTTGTTCTTCTAATGAATCTGCCGATGACAGTTCATCTGATAAAGGAGA
CGGCGGTTCGCTAGTCGTTTATTCACCAAACTCAGAGGGCTTAATTGGAGCAACTATTCCTGCCTTTGAAGAAAAAT
ATGGTATCAAAGTAGAACTGATTCAAGCTGGTACTGGAGAACTTTTCAAAAAACTAGAGTCAGAAAAAGAAGTTCCT
GTAGCTGATGTTATCTTTGGTGGTTCTTATACACAATATACTACCCACGGAGAACTCTTTGAAAACTATACTTCAAA
AGAAAATGATAATGTTATCAAAGAATATCAAAACACAACTGGCTACTCTACTCCTTATACACTAGATGGTAGTGTTT
TAATCGTCAACCCTGATTTAACTAAAGGCATGAACATCGAAGGATATAACGATCTTTTCAAACCTGAACTAAAAGGA
AAAATCGCAACTGCTGACCCAGCAAACTCTTCTAGCGCCTTTGCTCAATTAACAAATATGCTACAAGCTCAAGGTGG
TTACAAAGATGATAAGGCTTGGTCTTATGTAAAAGATCTTTTCACACTTATTGATGGTAAAATCGGTTCAAGTTCAT
CTAGTGTCTATAAAGTAGTCGCTGATGGAGAAATGGCTGTTGGTCTCTCTTATGAAGATCCAGCAGTTAAACTCTTA

AATGACGGAGCTAACATTAAGGTAGTCTATCCAAAAGAAGGAACCGTCTTCCTACCTGCTAGTGCTGCTATCGTTAA
AAAATCTAAAAATATGGAAAATGCCAAGAAATTTATCGATTTTATTATCTCTCAAGAAGTACAAGATACACTTGGTA
CAACCACTACTAACCGTCCTGTTCGTAAAAATGCTAAAACAAGCGAAAACATGAAACCAATTGACAAAATCAAAACA
CTCACTGAAGATTATGATTATGTCATCAAGAATAAATCAGATATCGTTAAGAAATACAACGAAGTCTTTACAGATAT
CCAATCTAAACAGTAA

MKKKWMYYAACSSNESADDSSSDKGDGGSLVVYSPNSEGLIGATIPAFEEKYGIKVELIQAGTGELFKKLESEKEVP
VADVIFGGSYTQYTTHGELFENYTSKENDNVIKEYQNTTGYSTPYTLDGSVLIVNPDLTKGMNIEGYNDLFKPELKG
KIATADPANSSSAFAQLTNMLQAQGGYKDDKAWSYVKDLFTLIDGKIGSSSSSVYKVVADGEMAVGLSYEDPAVKLL
NDGANIKVVYPKEGTVFLPASAAIVKKSKNMENAKKFIDFIISQEVQDTLGTTTTNRPVRKNAKTSENMKPIDKIKT
LTEDYDYVIKNKSDIVKKYNEVFTDIQSKQZ

ID211 - 4127.2
ATGAGTGAGATCAAAATTATTAACGCCAAAAAAATCTACCACGATGTCCCTGTTATTGAGAATTTGAACATTACAAT
TCCAAAAGGAAGTCTCTTTACCCTTCTTGGAGCTTCAGGATGTGGAAAAACGACCCTTCTTCGTATGATTGCAGGTT
TCAACAGTATCGAAGGTGGAGAATTTTACTTCGATGATACAAAAATCAATAATATGGAACCCAGCAAACGCAATATC
GGGATGGTTTTCCAAAACTACGCTATTTTCCCACATTTGACTGTCCGAGACAACGTTGCTTTTGGTCTTATGCAAAA
GAAGGTTCCAAAAGAAGAATTGATTCAACAGACCAACAAGTATCTTGAACTCATGCAAATTGCTCAATATGCGGATC
GAAAGCCCGATAAACTCAGTGGTGGACAACAACAACGTGTCACCTTGGCATGCGCCTTAGCGGTTAATCCAAGTGTT
CTCCTCATGGACGAGCCACTTAGTAATCTGGAGGCCAAACTTCGCTTGGATATGCGTCAAGCCATCCGAGAAATCCA
ACACGAAGTGGGAATTACAACTGTTTATGTAACCCACGACCAAGAAGAAGCCATGGCTATTTCAGACCAAATTGCTG
TTATGAAAGATGGGGTGATCCAACAAATCGGCCGACCAAAAGAACTCTATCATAAACCAGCTAATGAGTTTGTGGCA
ACCTTTATCGGACGCACAAATATTATCCCTGCCAATCTTGAAAAACGGAGCGACGGCGCTTATATCGTCTTTTCAGA
TGGCTATGCCCTTCGAATGCCAGCTCTTGATCAGGTTGAGGAGCAAGCTATTCATGTAAGCATTCGTCCCGAAGAGT
TTATCAAAGATGAATCTGGAGATATTGAAGGAACTATTAGAGATAGCGTCTATCTTGGACTAAATACGGATTATTTC
ATTGAGACAGGTTTTGCCTCAAAAATTCAAGTTAGTGAAGAATCAACTTTTGAAGAAGATCTACAAAAAGGCAATCG
TATTCGTCTACGAATCAATACGCAAAAATTAAACATCTTTTCTGCAGATGGTTCCCAAAACCTGATAAAAGGAGTCA
ACCATGGAACGTAA

MSEIKIINAKKIYHDVPVIENLNITIPKGSLFTLLGASGCGKTTLLRMIAGFNSIEGGEFYFDDTKINNMEPSKRNI
GMVFQNYAIFPHLTVRDNVAFGLMQKKVPKEELIQQTNKYLELMQIAQYADRKPDKLSGGQQQRVTLACALAVNPSV
LLMDEPLSNLEAKLRLDMRQAIREIQHEVGITTVYVTHDQEEAMAISDQIAVMKDGVIQQIGRPKELYHKPANEFVA
TFIGRTNIIPANLEKRSDGAYIVFSDGYALRMPALDQVEEQAIHVSIRPEEFIKDESGDIEGTIRDSVYLGLNTDYF
IETGFASKIQVSEESTFEEDLQKGNRIRLRINTQKLNIFSADGSQNLIKGVNHGTZ

ID212 - 4136.1
ATGAAGAAAAAATTATTGGCAGGTGCCATCACACTATTATCAGTAGCAACTTTAGCAGCTTGTTCGAAAGGGTCAGA
AGGTGCAGACCTTATCAGCATGAAAGGGGATGTCATTACAGAACATCAATTTTATGAGCAAGTGAAAAGCAACCCTT
CAGCCCAACAAGTCTTGTTAAATATGACCATCCAAAAAGTTTTTGAAAAACAATATGGCTCAGAGCTTGATGATAAA
GAGGTTGATGATACTATTGCCGAAGAAAAAAAACAATATGGCGAAAACTACCAACGTGTCTTGTCACAAGCAGGTAT
GACTCTTGAAACACGTAAAGCTCAAATTCGTACAAGTAAATTAGTTGAGTTGGCAGTTAAGAAGGTAGCAGAAGCTG
AATTGACAGATGAAGCCTATAAGAAAGCCTTTGATGAGTACACTCCAGATGTAACGGCTCAAATCATCCGTCTTAAT
AATGAAGATAAGGCCAAAGAAGTTCTCGAAAAAGCCAAGGCAGAAGGTGCTGATTTTGCTCAATTAGCCAAAGATAA
TTCAACTGATGAAAAAACAAAAGAAAATGGTGGAGAAATTACCTTTGATTCTGCTTCAACAGAAGTACCTGAGCAAG
TCAAAAAAGCCGCTTTCGCTTTAGATGTGGATGGTGTTTCTGATGTGATTACAGCAACTGGCACACAAGCCTACAGT
AGCCAATATTACATTGTAAAACTCACTAAGAAAACAGAAAAATCATCTAATATTGATGACTACAAAGAAAAATTAAA
AACTGTTATCTTGACTCAAAAACAAAATGATTCAACATTTGTTCAAAGCATTATCGGAAAAGAATTGCAAGCAGCCA
ATATCAAGGTTAAGGACCAAGCCTTCCAAAATATCTTTACCCAATATATCGGTGGTGGAGATTCAAGCTCAAGCAGT
AGTACATCAAACGAATAG

MKKKLLAGAITLLSVATLAACSKGSEGADLISMKGDVITEHQFYEQVKSNPSAQQVLLNMTIQKVFEKQYGSELDDK
EVDDTIAEEKKQYGENYQRVLSQAGMTLETRKAQIRTSKLVELAVKKVAEAELTDEAYKKAFDEYTPDVTAQIIRLN
NEDKAKEVLEKAKAEGADFAQLAKDNSTDEKTKENGGEITFDSASTEVPEQVKKAAFALDVDGVSDVITATGTQAYS
SQYYIVKLTKKTEKSSNIDDYKEKLKTVILTQKQNDSTFVQSIIGKELQAANIKVKDQAFQNIFTQYIGGGDSSSSS
STSNEZ

ID213 - 4137.3
ATGAAAAAAAATATTAAACAATATGTAACCTTAGGTACTGTAGTGGTATTATCAGCATTTGTTGCTAACTCAGTTGC
AGCTCAGGAGACTGAAACTTCTGAAGTATCAACACCAAAGTTGGTGCAACCTGTTGCACCAACGACTCCGATTTCGG
AAGTACAACCTACATCGGATAACTCTTCGGAAGTTACTGTACAACCTCGAACAGTTGAAACTACTGTTAAGGATCCA
TCTTCTACAGCGGAAGAAACTCCTGTCTTAGAAAAAAATAATGTTACTTTAACAGGGGGCGGAGAAAATGTTACTAA
AGAGTTAAAGGATAAATTTACTAGCGGTGACTTTACTGTAGTGATTAAGTACAATCAGTCAAGTGAGAAAGGCTTAC
AAGCTCTGTTTGGAATATCTAATTCCAAACCCGGTCAACAAAATAGTTATGTAGATGTGTTCCTTAGAGACAATGGT
GAGTTGGGGATGGAAGCGCGTGATACTTCTTCCAATAAAAATAACCTAGTATCCAGACCTGCTTCAGTTTGGGGTAA
GTACAAACAAGAGGCTGTGACTAACACTGTTGCAGTAGTAGCAGATTCAGTCAAAAAAACATATTCTTTATACGCAA
ATGGTACAAAAGTAGTAGAAAAGAAAGTGGATAATTTCCTAAACATCAAGGATATTAAAGGTATTGATTACTATATG
CTTGGGGGAGTGAAACGTGCAGGAAAAACGGCGTTTGGTTTTAACGGAACACTAGAAAATATCAAATTCTTTAATAG
TGCATTGGATGAAGAAACTGTTAAAAAGATGACAACAAACGCTGTTACTGGACATTTAATTTATACGGCTAATGATA
CAACAGGTTCTAACTATTTCCGTATTCCAGTTCTGTATACTTTTAGCAATGGTCGGGTATTTTCAAGCATTGACGCT
CGTTACGGTGGAACTCATGATTTCTTGAATAAAATTAATATTGCTACAAGTTATAGTGATGATAATGGTAAGACATG
GACTAAACCAAAATTAACATTGGCATTCGATGATTTTGCGCCAGTACCATTAGAATGGCCTCGTGAAGTTGGTGGAC
GTGACTTACAAATCAGCGGTGGTGCAACCTATATTGACTCTGTTATTGTTGAAAAAAAGAACAAACAAGTACTCATG
TTTGCTGATGTGATGCCTGCTGGAGTAAGTTTTAGAGAAGCAACTAGAAAAGATTCAGGTTATAAACAAATTGATGG
TAATTATTACCTTAAATTAAGGAAACAAGGTGATACTGATTACAATTATACTATTCGTGAGAATGGTACTGTATACG
ACGATCGTACCAACAGACCAACTGAATTTTCAGTAGATAAAAATTTCGGTATTAAACAAAATGGTAATTATTTGACG
GTAGAGCGG

MKKNIKQYVTLGTVVVLSAFVANSVAAQETETSEVSTPKLVQPVAPTTPISEVQPTSDNSSEVTVQPRTVETTVKDP
SSTAEETPVLEKNNVTLTGGGENVTKELKDKFTSGDFTVVIKYNQSSEKGLQALFGISNSKPGQQNSYVDVFLRDNG
ELGMEARDTSSNKNNLVSRPASVWGKYKQEAVTNTVAVVADSVKKTYSLYANGTKVVEKKVDNFLNIKDIKGIDYYM
LGGVKRAGKTAFGFNGTLENIKFFNSALDEETVKKMTTNAVTGHLIYTANDTTGSNYFRIPVLYTFSNGRVFSSIDA
RYGGTHDFLNKINIATSYSDDNGKTWTKPKLTLAFDDFAPVPLEWPREVGGRDLQISGGATYIDSVIVEKKNKQVLM
FADVMPAGVSFREATRKDSGYKQIDGNYYLKLRKQGDTDYNYTIRENGTVYDDRTNRPTEFSVDKNFGIKQNGNYLT
VER

ID214 - 4185
ATGAAAAAATTTAGCCTATTACTAGCTATCCTACCATTTTTGGTTGCCTGTGAGAATCAAGCTACACCCAAAGAGAC
TAGCGCTCAAAAGACAATCGTCCTTGCTACAGCTGGCGACGTGCCACCATTTGACTACGAAGACAAGGGCAATCTGA
CAGGCTTTGATATCGAAGTTTTAAAGGCAGTAGATGAAAAACTCAGCGACTACGAGATTCAATTCCAAAGAACCGCC
TGGGAGAGCATCTTCCCAGGACTTGATTCTGGTCACTATCAGGCTGCGGCCAATAACTTGAGTTACACAAAAGAGCG
TGCTGAAAAATACCTTTACTCGCTTCCAATTTCCAACAATCCCCTCGTCCTTGTCAGCAACAAGAAAAATCCTTTGA
CTTCTCTTGACCAGATCGCTGGTAAAACAACACAAGAGGATACCGGAACTTCTAACGCTCAATTCATCAATAACTGG
AATCAGAAACACACTGATAATCCCGCTACAATTAATTTTTCTGGTGAGGATATTGGTAAACGAATCCTAGACCTTGC
TAACGGAGAGTTTGATTTCCTAGTTTTTGACAAGGTATCCGTTCAAAAGATTATCAAGGACCGTGGTTTAGACCTCT
CAGTCGTTGATTTACCTTCTGCAGATAGCCCCAGCAATTATATCATTTTCTCAAGCGACCAAAAAGAGTTTAAAGAG
CAATTTGATAAAGCGCTCAAAGAACTCTATCAAGACGGAACCCTTGAAAAACTCAGCAATACCTATCTAGGTGGTTC
TTACCTCCCAGATCAATCTCAGTTACAATAA

MKKFSLLLAILPFLVACENQATPKETSAQKTIVLATAGDVPPFDYEDKGNLTGFDIEVLKAVDEKLSDYEIQFQRTA
WESIFPGLDSGHYQAAANNLSYTKERAEKYLYSLPISNNPLVLVSNKKNPLTSLDQIAGKTTQEDTGTSNAQFINNW
NQKHTDNPATINFSGEDIGKRILDLANGEFDFLVFDKVSVQKIIKDRGLDLSVVDLPSADSPSNYIIFSSDQKEFKE
QFDKALKELYQDGTLEKLSNTYLGGSYLPDQSQLQZ

ID215 - 4211.1
ATGAAAAAAAATAGTTTATATATCATATCCTCACTCTTTTTTGCTTGTGTCTTATTTGTCTATGCTACGGCGACGAA
TTTTCAAAACAGTACCAGTGCTAGGCAGGTAAAAACGGAAACCTATACTAATACAGTAACAAATGTCCCTATTGACA
TACGCTATAATAGTGATAAGTATTTTATTAGCGGTTTTGCTTCAGAAGTATCAGTGGTCTTGACTGGTGCAAATCGC
CTATCGCTAGCTAGTGAAATGCAAGAAAGTACACGTAAATTCAAGGTTACTGCTGACCTAACAGATGCCGGTGTTGG
AACGATTGAAGTTCCTTTGAGCATTGAAGATTTACCCAATGGGCTGACCGCTGTGGCGACTCCGCAAAAAATTACAG
TCAAGATTGGTAAGAAGGCTCAGAAGGATAAGGTAAAGATTGTACCAGAGATTGACCCTAGTCAAATTGATAGTCGG
GTACAAATTGAAAATGTCATGGTGTCAGATAAAGAAGTGTCTATTACGAGTGACCAAGAGACATTGGATAGAATTGA
TAAGATTATCGCTGTTTTGCCAACTAGCGAACGTATAACAGGTAATTACAGTGGTTCAGTACCTTTGCAGGCAATCG
ACCGCAATGGTGTTGTCTTACCGGCAGTTATCACTCCGTTTGATACAATAATGAAGGTGACTACAAAACCAGTAGCA
CCAAGTTCAAGCACATCAAATTCAAGTACAAGCAGTTCATCGGAGACATCTTCGTCAACGAAAGCAACTAGTTCAAA
AACGAATTAA

MKKNSLYIISSLFFACVLFVYATATNFQNSTSARQVKTETYTNTVTNVPIDIRYNSDKYFISGFASEVSVVLTGANR
LSLASEMQESTRKFKVTADLTDAGVGTIEVPLSIEDLPNGLTAVATPQKITVKIGKKAQKDKVKIVPEIDPSQIDSR
VQIENVMVSDKEVSITSDQETLDRIDKIIAVLPTSERITGNYSGSVPLQAIDRNGVVLPAVITPFDTIMKVTTKPVA
PSSSTSNSSTSSSSETSSSTKATSSKTNZ

ID216 - 4127.3
ATGTTGATTGGCGAAGGGTATCGGACTTTCCCTGTCCTGATTTATACCCAATTTATTAGCGAGGTTGGAGGAAATTC
TGCTTTTGCAATTATGGCGATTATCATTGCCTTGGCAATTTTCCTTATCCAAAAACACATTGCAAACCGCTACAGTT
TCAGCATGAATCTGCTCCATCCAATTGAGCCTAAAAAAACTACAAAAGGAAAAATGGCTGCCATTTATGCAACAGTC
TACGGAATTATCTTTATCTCTGTTTTACCTCAAATCTACTTAATTTATACCTCTTTCCTAAAAACATCAGGTATGGT
ATCTGTTAAAGGTTATTCTCCAAACAGTTACAAGGTAGCTTTCCATCGTATGGGATCTGCTATTTTCAATACCATTC
GTATCCCTTTGATTGCCTTAGTTCTAGTTGTTCTATTTGCGACATTTATCTCCTACCTAGCCGTTAGAAAACGGAAT
TTGTTTACAAACTTAATTGACAGCCTCAGTATGGTACCTTATATTGTACCAGGAACCGTTCTAGGGATTGCCTTCAT
TTCTTCCTTCAATACTGGTCTATTTGGAAGTGGATTTCTTATGATTACAGGGACTGCTTTCATCTTGATTATGTCTC
TATCTGCCAGAAGATTACCTTATACTATTCGCTCATCTGTTGCTAGCTTACAACAAATAGCACCAAGTATTGAAGAA
GCTGCTGAAAGCTTAGGAAGTAGTCGTCTCAATACCTTTGCTAAGATTACAACTCCAATGATGCTATCTGGTATCAT
TTCTGGAGCCATCTTATCTTGA

MLIGEGYRTFPVLIYTQFISEVGGNSAFAIMAIIIALAIFLIQKHIANRYSFSMNLLHPIEPKKTTKGKMAAIYATV
YGIIFISVLPQIYLIYTSFLKTSGMVSVKGYSPNSYKVAFHRMGSAIFNTIRIPLIALVLVVLFATFISYLAVRKRN
LFTNLIDSLSMVPYIVPGTVLGIAFISSFNTGLFGSGFLMITGTAFILIMSLSARRLPYTIRSSVASLQQIAPSIEE
AAESLGSSRLNTFAKITTPMMLSGIISGAILSZ

Table 4

ID301
ATGAATAAGAAAAAAATGATTTTAACAAGTCTAGCCAGCGTCGCTATCTTAGGGGCTGGTTTTGTTACGTCTCAGCC
TACTTTTGTAAGAGCAGAAGAATCTCCACAAGTTGTCGAAAAATCTTCATTAGAGAAGAAATATGAGGAAGCAAAAG
CAAAAGCTGATACTGCCAAGAAAGATTACGAAACGGCTAAAAAGAAAGCAGAAGACGCTCAGAAAAAGTATGAAGAT
GATCAGAAGAGAACTGAGGAGAAAGCTCGAAAAGAAGCAGAAGCATCTCAAAAATTGAATGATGTGGCGCTTGTTGT
TCAAAATGCATATAAAGAGTACCGAGAAGTTCAAAATCAACGTAGTAAATATAAATCTGACGCTGAATATCAGAAAA
AATTAACAGAGGTCGACTCTAAAATAGAGAAGGCTAGGAAAGAGCAACAGGACTTGCAAAATAAATTTAATGAAGTA
AGAGCAGTTGTAGTTCCTGAACCAAATGCGTTGGCTGAGACTAAGAAAAAAGCAGAAGAAGCTAAAGCAGAAGAAAA
AGTAGCTAAGAGAAAATATGATTATGCAACTCTAAAGGTAGCACTAGCGAAGAAAGAAGTAGAGGCTAAGGAACTTG
AAATTGAAAAACTTCAATATGAAATTTCTACTTTGGAACAAGAAGTTGCTACTGCTCAACATCAAGTAGATAATTTG
AAAAAACTTCTTGCTGGTGCGGATCCTGATGATGGCACAGAAGTTATAGAAGCTAAATTAAAAAAAGGAGAAGCTGA
GCTAAACGCTAAACAAGCTGAGTTAGCAAAAAAACAAACAGAACTTGAAAAACTTCTTGACAGCCTTGATCCTGAAG
GTAAGACTCAGGATGAATTAGATAAAGAAGCAGAAGAAGCTGAGTTGGATAAAAAAGCTGATGAACTTCAAAATAAA
GTTGCTGATTTAGAAAAAGAAATTAGTAACCTTGAAATATTACTTGGAGGGGCTGATCCTGAAGATGATACTGCTGC
TCTTCAAAATAAATTAGCTGCTAAAAAAGCTGAGTTAGCAAAAAAACAAACAGAACTTGAAAAACTTCTTGACAGCC
TTGATCCTGAAGGTAAGACTCAGGATGAATTAGATAAAGAAGCAGAAGAAGCTGAGTTGGATAAAAAAGCTGATGAA
CTTCAAAATAAAGTTGCTGATTTAGAAAAAGAAATTAGTAACCTTGAAATATTACTTGGAGGGGCTGATTCTGAAGA
TGATACTGCTGCTCTTCAAAATAAATTAGCTACTAAAAAAGCTGAATTGGAAAAAACTCAAAAAGAATTAGATGCAG
CTCTTAATGAGTTAGGCCCTGATGGAGATGAAGAAGAAACTCCAGCGCCGGCTCCTCAACCAGAGCAACCAGCTCCT
GCACCAAAACCAGAGCAACCAGCTCCAGCTCCAAAACCAGAGCAACCAGCTCCTGCACCAAAACCAGAGCAACCAGC
TCCAGCTCCAAAACCAGAGCAACCAGCTCCAGCTCCAAAACCAGAGCAACCAGCTAAGCCGGAGAAACCAGCTGAAG
AGCCTACTCAACCAGAAAAACCAGCCACTCCAAAAACAGGCTGGAAACAAGAAAACGGTATGTGGTATTTCTACAAT
ACTGATGGTTCAATGGCAATAGGTTGGCTCCAAAACAACGGTTCATGGTACTACCTAAACGCTAACGGCGCTATGGC
AACAGGTTGGGTGAAAGATGGAGATACCTGGTACTATCTTGAAGCATCAGGTGCTATGAAAGCAAGCCAATGGTTCA
AAGTATCAGATAAATGGTACTATGTCAACAGCAATGGCGCTATGGCGACAGGCTGGCTCCAATACAATGGCTCATGG
TACTACCTCAACGCTAATGGTGATATGGCGACAGGATGGCTCCAATACAACGGTTCATGGTATTACCTCAACGCTAA
TGGTGATATGGCGACAGGATGGGCTAAAGTCAACGGTTCATGGTACTACCTAAACGCTAACGGTGCTATGGCTACAG
GTTGGGCTAAAGTCAACGGTTCATGGTACTACCTAAACGCTAACGGTTCAATGGCAACAGGTTGGGTGAAAGATGGA
GATACCTGGTACTATCTTGAAGCATCAGGTGCTATGAAAGCAAGCCAATGGTTCAAAGTATCAGATAAATGGTACTA
TGTCAATGGCTTAGGTGCCCTTGCAGTCAACACAACTGTAGATGGCTATAAAGTCAATGCCAATGGTGAATGGGTTT
AA

MNKKKMILTSLASVAILGAGFVTSQPTFVRAEESPQVVEKSSLEKKYEEAKAKADTAKKDYETAKKKAEDAQKKYED
DQKRTEEKARKEAEASQKLNDVALVVQNAYKEYREVQNQRSKYKSDAEYQKKLTEVDSKIEKARKEQQDLQNKFNEV
RAVVVPEPNALAETKKKAEEAKAEEKVAKRKYDYATLKVALAKKEVEAKELEIEKLQYEISTLEQEVATAQHQVDNL
KKLLAGADPDDGTEVIEAKLKKGEAELNAKQAELAKKQTELEKLLDSLDPEGKTQDELDKEAEEAELDKKADELQNK
VADLEKEISNLEILLGGADPEDDTAALQNKLAAKKAELAKKQTELEKLLDSLDPEGKTQDELDKEAEEAELDKKADE
LQNKVADLEKEISNLEILLGGADSEDDTAALQNKLATKKAELEKTQKELDAALNELGPDGDEEETPAPAPQPEQPAP
APKPEQPAPAPKPEQPAPAPKPEQPAPAPKPEQPAPAPKPEQPAKPEKPAEEPTQPEKPATPKTGWKQENGMWYFYN
TDGSMAIGWLQNNGSWYYLNANGAMATGWVKDGDTWYYLEASGAMKASQWFKVSDKWYYVNSNGAMATGWLQYNGSW
YYLNANGDMATGWLQYNGSWYYLNANGDMATGWAKVNGSWYYLNANGAMATGWAKVNGSWYYLNANGSMATGWVKDG
DTWYYLEASGAMKASQWFKVSDKWYYVNGLGALAVNTTVDGYKVNANGEWVZ

ID302
ATGTTTGCATCAAAAAGCGAAAGAAAAGTACATTATTCAATTCGTAAATTTAGTGTTGGAGTAGCTAGTGTAGTTGT
TGCCAGTCTTGTTATGGGAAGTGTGGTTCATGCGACAGAGAACGAGGGAGCTACCCAAGTACCCACTTCTTCTAATA
GGGCAAATGAAAGTCAGGCAGAACAAGGAGAACAACCTAAAAAACTCGATTCAGAACGAGATAAGGCAAGGAAAGAG
GTCGAGGAATATGTAAAAAAAATAGTGGGTGAGAGCTATGCAAAATCAACTAAAAAGCGACATACAATTACTGTAGC
TCTAGTTAACGAGTTGAACAACATTAAGAACGAGTATTTGAATAAAATAGTTGAATCAACCTCAGAAAGCCAACTAC
AGATACTGATGATGGAGAGTCGATCAAAAGTAGATGAAGCTGTGTCTAAGTTTGAAAAGGACTCATCTTCTTCGTCA
AGTTCAGACTCTTCCACTAAACCGGAAGCTTCAGATACAGCGAAGCCAAACAAGCCGACAGAACCAGGAGAAAAGGT
AGCAGAAGCTAAGAAGAAGGTTGAAGAAGCTGAGAAAAAAGCCAAGGATCAAAAAGAAGAAGATCGTCGTAACTACC
CAACCATTACTTACAAAACGCTTGAACTTGAAATTGCTGAGTCCGATGTGGAAGTTAAAAAAGCGGAGCTTGAACTA
GTAAAAGTGAAAGCTAACGAACCTCGAGACGAGCAAAAAATTAAGCAAGCAGAAGCGGAAGTTGAGAGTAAACAAGC
TGAGGCTACAAGGTTAAAAAAAATCAAGACAGATCGTGAAGAAGCAGAAGAAGAAGCTAAACGAAGAGCAGATGCTA
AAGAGCAAGGTAAACCAAAGGGGCGGGCAAAACGAGGAGTTCCTGGAGAGCTAGCAACACCTGATAAAAAAGAAAAT
GATGCGAAGTCTTCAGATTCTAGCGTAGGTGAAGAAACTCTTCCAAGCCCATCCCTGAAACCAGAAAAAAAGGTAGC
AGAAGCTGAGAAGAAGGTTGAAGAAGCTAAGAAAAAAGCCGAGGATCAAAAAGAAGAAGATCGCCGTAACTACCCAA

CCAATACTTACAAAACGCTTGAACTTGAAATTGCTGAGTCCGATGTGGAAGTTAAAAAAGCGGAGCTTGAACTAGTA
AAAGAGGAAGCTAAGGAACCTCGAAACGAGGAAAAAGTTAAGCAAGCAAAAGCGGAAGTTGAGAGTAAAAAAGCTGA
GGCTACAAGGTTAGAAAAAATCAAGACAGATCGTAAAAAAGCAGAAGAAGAAGCTAAACGAAAAGCAGCAGAAGAAG
ATAAAGTTAAAGAAAAACCAGCTGAACAACCACAACCAGCGCCGGCTCCAAAAGCAGAAAAACCAGCTCCAGCTCCA
AAACCAGAGAATCCAGCTGAACAACCAAAAGCAGAAAAACCAGCTGATCAACAAGCTGAAGAAGACTATGCTCGTAG
ATCAGAAGAAGAATATAATCGCTTGACTCAACAGCAACCGCCAAAAACTGAAAAACCAGCACAACCATCTACTCCAA
AAACAGGCTGGAAACAAGAAAACGGTATGTGGTACTTCTACAATACTGATGGTTCAATGGCGACAGGATGGCTCCAA
AACAATGGCTCATGGTACTACCTCAACAGCAATGGCGCTATGGCGACAGGATGGCTCCAAAACAATGGTTCATGGTA
CTATCTAAACGCTAATGGTTCAATGGCAACAGGATGGCTCCAAAACAATGGTTCATGGTACTACCTAAACGCTAATG
GTTCAATGGCGACAGGATGGCTCCAATACAATGGCTCATGGTACTACCTAAACGCTAATGGTTCAATGGCGACAGGA
TGGCTCCAATACAATGGCTCATGGTACTACCTAAACGCTAATGGTGATATGGCGACAGGTTGGGTGAAAGATGGAGA
TACCTGGTACTATCTTGAAGCATCAGGTGCTATGAAAGCAAGCCAATGGTTCAAAGTATCAGATAAATGGTACTATG
TCAATGGCTCAGGTGCCCTTGCAGTCAACACAACTGTAGATGGCTATGGAGTCAATGCCAATGGTGAATGGGTAAAC
TAA

MFASKSERKVHYSIRKFSVGVASVVVASLVMGSVVHATENEGATQVPTSSNRANESQAEQGEQPKKLDSERDKARKE
VEEYVKKIVGESYAKSTKKRHTITVALVNELNNIKNEYLNKIVESTSESQLQILMMESRSKVDEAVSKFEKDSSSSS
SSDSSTKPEASDTAKPNKPTEPGEKVAEAKKKVEEAEKKAKDQKEEDRRNYPTITYKTLELEIAESDVEVKKAELEL
VKVKANEPRDEQKIKQAEAEVESKQAEATRLKKIKTDREEAEEEAKRRADAKEQGKPKGRAKRGVPGELATPDKKEN
DAKSSDSSVGEETLPSPSLKPEKKVAEAEKKVEEAKKKAEDQKEEDRRNYPTNTYKTLELEIAESDVEVKKAELELV
KEEAKEPRNEEKVKQAKAEVESKKAEATRLEKIKTDRKKAEEEAKRKAAEEDKVKEKPAEQPQPAPAPKAEKPAPAP
KPENPAEQPKAEKPADQQAEEDYARRSEEEYNRLTQQQPPKTEKPAQPSTPKTGWKQENGMWYFYNTDGSMATGWLQ
NNGSWYYLNSNGAMATGWLQNNGSWYYLNANGSMATGWLQNNGSWYYLNANGSMATGWLQYNGSWYYLNANGSMATG
WLQYNGSWYYLNANGDMATGWVKDGDTWYYLEASGAMKASQWFKVSDKWYYVNGSGALAVNTTVDGYGVNANGEWVN
Z

ID303
ATGGTAAAAAGACGTATAAGGAGAGGGACGAGAGAACCTGAAAAAGTTGTTGTTCCTGAGCAATCATCTATTCCTTC
GTATCCTGTATCTGTTACATCTAACCAAGGAACAGATGTAGCAGTAGAACCAGCTAAAGCAGTTGCTCCAACAACAG
ACTGGAAACAAGAAAATGGTATGTGGTATTTTTATAATACTGATGGTTCCATGGCAACAGGTTGGGTACAAGTTAAT
AGTTCATGGTACTACCTCAACAGCAACGGTTCTATGAAAGTCAATCAATGGTTCCAAGTTGGTGGTAAATGGTATTA
TGTAAATACATCGGGTGAGTTAGCGGTCAATACAAGTATAGATGGCTATAGAGTCAATGATAATGGTGAATGGGTGC
GTTAA

MVKRRIRRGTREPEKVVVPEQSSIPSYPVSVTSNQGTDVAVEPAKAVAPTTDWKQENGMWYFYNTDGSMATGWVQVN
SSWYYLNSNGSMKVNQWFQVGGKWYYVNTSGELAVNTSIDGYRVNDNGEWVRZ

ID304
CTGAATACAAGTTTTGTTCATGCTGCTGATGGGATTCAATATGTCAGAGATGATACTAGAGATAAAGAAGAGGGAAT
AGAGTATGATGACGCTGACAATGGGGATATTATTGTAAAAGTAGCGACTAAACCTAAGGTAGTAACCAAGAAAATTT
CAAGTACGCGAATTCGTTATGAAAAAGATGAAACAAAAGACCGTAGTGAAAATCCTGTTACAATTGATGGAGAGGAT
GGCTATGTAACTACGACAAGGACCTACGATGTTAATCCAGAGACTGGTTATGTTACCGAACAGGTTACTGTTGATAG
AAAAGAAGCCACGGATACAGTTATCAAAGTTCCAGCTAAAAGCAAGGTTGAAGAAGTTCTTGTTCCATTTGCTACTA
AATATGAAGCAGACAATGACCTTTCTGCAGGACAGGAGCAAGAGATTACTCTAGGAAAGAATGGAAAAACAGTTACA
ACGATAACTTATAATGTAGATGGAAAGAGTGGACAAGTAACTGAGAGTACTTTAAGTCAAAAAAAAGACTCTCAAAC
AAGAGTTGTTAAAAAAAGAACCAAGCCCCAAGTTCTTGTCCAAGAAATTCCAATCGAAACAGAATATCTCGATGGCC
CAACTCTTGATAAAAGTCAAGAAGTAGAAGAAGTAGGAGAAATTGGTAAATTACTCTTACTACAATCTATACTGTAG

LNTSFVHAADGIQYVRDDTRDKEEGIEYDDADNGDIIVKVATKPKVVTKKISSTRIRYEKDETKDRSENPVTIDGED
GYVTTTRTYDVNPETGYVTEQVTVDRKEATDTVIKVPAKSKVEEVLVPFATKYEADNDLSAGQEQEITLGKNGKTVT
TITYNVDGKSGQVTESTLSQKKDSQTRVVKKRTKPQVLVQEIPIETEYLDGPTLDKSQEVEEVGEIGKLLLLQSILZ

ID305
ATGAAGCTTTTGAAAAAAATGATGCAAATCGCACTAGCCACATTTTTCTTCGGTTTGTTAGCGACAAATACAGTATT
TGCAGATGATTCTGAAGGATGGCAGTTTGTCCAAGAAAATGGTAGAACCTACTACAAAAAGGGGGATCTAAAAGAAA
CCTACTGGAGAGTGATAGATGGGAAGTACTATTATTTTGATCCTTTATCCGGAGAGATGGTTGTCGGCTGGCAATAT
ATACCTGCTCCACACAAGGGGGTTACGATTGGTCCTTCTCCAAGAATAGAGATTGCTCTTAGACCAGATTGGTTTTA
TTTTGGTCAAGATGGTGTATTACAAGAATTTGTTGGCAAGCAAGTTTTAGAAGCAAAAACTGCTACGAATACCAACA
AACATCATGGGGAAGAATATGATAGCCAAGCAGAGAAACGAGTCTATTATTTTGAAGATCAGCGTAGTTATCATACT

TTAAAAACTGGTTGGATTTATGAAGAGGGTCATTGGTATTATTTACAGAAGGATGGTGGCTTTGATTCGCGCATCAA
CAGATTGACGGTTGGAGAGCTAGCACGTGGTTGGGTTAAGGATTACCCTCTTACGTATGATGAAGAGAAGCTAAAAG
CAGCTCCATGGTACTATCTAAATCCAGCAACTGGCATTATGCAAACAGGTTGGCAATATCTAGGTAATAGATGGTAC
TACCTCCATTCGTCAGGAGCTATGGCAACTGGCTGGTATAAGGAAGGCTCAACTTGGTACTATCTAGATGCTGAAAA
TGGTGATATGAGAACTGGCTGGCAAAACCTTGGGAACAAATGGTACTATCTCCGTTCATCAGGAGCTATGGCAACTG
GTTGGTATCAGGAAAGTTCGACTTGGTACTATCTAAATGCAAGTAATGGAGATATGAAAACAGGCTGGTTCCAAGTC
AATGGTAACTGGTACTATGCCTATGATTCAGGTGCTTTAGCTGTTAATACCACAGTAGGTGGTTACTACTTAAACTA
TAATGGTGAATGGGTTAAGTAA

MKLLKKMMQIALATFFFGLLATNTVFADDSEGWQFVQENGRTYYKKGDLKETYWRVIDGKYYYFDPLSGEMVVGWQY
IPAPHKGVTIGPSPRIEIALRPDWFYFGQDGVLQEFVGKQVLEAKTATNTNKHHGEEYDSQAEKRVYYFEDQRSYHT
LKTGWIYEEGHWYYLQKDGGFDSRINRLTVGELARGWVKDYPLTYDEEKLKAAPWYYLNPATGIMQTGWQYLGNRWY
YLHSSGAMATGWYKEGSTWYYLDAENGDMRTGWQNLGNKWYYLRSSGAMATGWYQESSTWYYLNASNGDMKTGWFQV
NGNWYYAYDSGALAVNTTVGGYYLNYNGEWVKZ

ID306
TTGGCTGGTAGATATGGTTCTGCTGTTCAGTGTACAGAAGTGACTGCCTCAAACCTTTCAACAGTTAAAACTAAAGC
TACGGTTGTAGAAAAACCACTGAAAGATTTTAGAGCGTCTACGTCTGATCAGTCTGGTTGGGTGGAATCTAATGGTA
AATGGTATTTCTATGAGTCTGGTGATGTGAAGACAGGTTGGGTGAAAACAGATGGTAAATGGTACTATTTGAATGAC
TTAGGTGTCATGCAGACTGGATTTGTAAAATTTTCTGGTAGCTGGTATTACTTGAGCAATTCAGGTGCTATGTTTAC
AGGCTGGGGAACAGATGGTAGCAGATGGTTCTACTTTGACGGCTCAGGAGCTATGAAGACAGGCTGGTACAAGGAAA
ATGGCACTTGGTATTACCTTGACGAAGCAGGTATCATGAAGACAGGTTGGTTTAAAGTCGGACCACACTGGTACTAT
GCCTACGGTTCAGGAGCTTTGGCTGTGAGCACAACAACACCAGATGGTTACCGTGTAAATGGTAATGGTGAATGGGT
AAACTAG

LAGRYGSAVQCTEVTASNLSTVKTKATVVEKPLKDFRASTSDQSGWVESNGKWYFYESGDVKTGWVKTDGKWYYLND
LGVMQTGFVKFSGSWYYLSNSGAMFTGWGTDGSRWFYFDGSGAMKTGWYKENGTWYYLDEAGIMKTGWFKVGPHWYY
AYGSGALAVSTTTPDGYRVNGNGEWVNZ

ID307
ATGAAAATTTTGAAAAAAACTATGCAAGTTGGACTGACAGTATTTTTCTTTGGTTTGCTAGGGACCAGTACAGTATT
TGCAGATGATTCTGAAGGATGGCAGTTTGTCCAAGAAAACGGAAGAACCTACTACAAAAAGGGGGACCTCAAAGAAA
CCTACTGGCGAGTGATTGATGGTAAGTACTATTATTTTGATTCTCTATCTGGAGAGATGGTTGTCGGCTGGCAATAT
ATCCCGTTTCCATCTAAAGGTAGTACAATTGGTCCTTACCCAAATGGTATCAGATTAGAAGGTTTTCCAAAGTCAGA
GTGGTACTACTTCGATAAAAATGGAGTGCTACAAGAGTTTGTTGGTTGGAAAACATTAGAGATTAAAACTAAAGACA
GTGTTGGAAGAAAGTACGGGGAAAAACGTGAAGATTCAGAAGATAAAGAAGAGAAGCGTTATTATACGAACTATTAC
TTTAATCAAAATCATTCTTTAGAGACAGGTTGGCTTTATGATCAGTCTAACTGGTATTATCTAGCTAAGACGGAAAT
TAATGGAGAAAACTACCTTGGTGGTGAAAGACGTGCGGGGTGGATAAACGATGATTCGACTTGGTACTACCTAGATC
CAACAACTGGTATTATGCAAACAGGTTGGCAATATCTAGGTAATAAGTGGTACTACCTCCGTTCCTCAGGAGCAATG
GCCACTGGCTGGTATCAGGAAGGTACCACTTGGTATTATTTAGACCACCCAAATGGCGATATGAAAACAGGTTGGCA
AAACCTTGGGAACAAATGGTACTATCTCCGTTCATCAGGAGCTATGGCAACTGGTTGGTATCAAGATGGTTCAACTT
GGTACTACCTAAATGCAGGTAATGGAGACATGAAGACAGGTTGGTTCCAGGTCAATGGCAACTGGTACTATGCTTAT
AGCTCAGGTGCTTTGGCAGTGAATACGACCGTAGATGGCTATTCTGTCAACTATAATGGCGAATGGGTTCGGTAA

MKILKKTMQVGLTVFFFGLLGTSTVFADDSEGWQFVQENGRTYYKKGDLKETYWRVIDGKYYYFDSLSGEMVVGWQY
IPFPSKGSTIGPYPNGIRLEGFPKSEWYYFDKNGVLQEFVGWKTLEIKTKDSVGRKYGEKREDSEDKEEKRYYTNYY
FNQNHSLETGWLYDQSNWYYLAKTEINGENYLGGERRAGWINDDSTWYYLDPTTGIMQTGWQYLGNKWYYLRSSGAM
ATGWYQEGTTWYYLDHPNGDMKTGWQNLGNKWYYLRSSGAMATGWYQDGSTWYYLNAGNGDMKTGWFQVNGNWYYAY
SSGALAVNTTVDGYSVNYNGEWVRZ

ID308
ATGAAAAAGAAATTAACTAGTTTAGCACTTGTAGGCGCTTTTTTAGGTTTGTCATGGTATGGAATGTTCAGGCTCA
AGAAAGTTCAGGAAATAAAATCCACTTTATCAATGTTCAAGAAGGTGGCAGTGATGCGATTATTCTTGAAAGCAATG
GACATTTTGCCATGGTGGATACAGGAGAAGATTATGATTTCCCAGATGGAAGTGATTCTCGCTATCCATGGAGAGAA
GGAATTGAAACGTCTTATAAGCATGTTCTAACAGACCGTGTCTTTCGTCGTTTGAAGGAATTGGGTGTCCAAAAACT
TGATTTTATTTTGGTGACCCATACCCACAGTGATCATATTGGAAATGTTGATGAATTACTGTCTACCTATCCAGTTG
ACCGAGTCTATCTTAAGAAATATAGTGATAGTCGTATTACTAATTCTGAACGTCTATGGGATAATCTGTATGGCTAT
GATAAGGTTTTACAGACTGCTGCAGAAAAAGGTGTTTCAGTTATTCAAAATATCACACAAGGGGATGCTCATTTTCA
GTTTGGGGACATGGATATTCAGCTCTATAATTATGAAAATGAAACTGATTCATCGGGTGAATTAAAGAAAATTTGGG

ATGACAATTCCAATTCCTTGATTAGCGTGGTGAAAGTCAATGGCAAGAAAATTTACCTTGGGGGCGATTTAGATAAT
GTTCATGGAGCAGAAGACAAGTATGGTCCTCTCATTGGAAAAGTTGATTTGATGAAGTTTAATCATCACCATGATAC
CAACAAATCAAATACCAAGGATTTCATTAAAAATTTGAGTCCGAGTTTGATTGTTCAAACTTCGGATAGTCTACCTT
GGAAAAATGGTGTTGATAGTGAGTATGTTAATTGGCTCAAAGAACGAGGAATTGAGAGAATCAACGCAGCCAGCAAA
GACTATGATGCAACAGTTTTTGATATTCGAAAAGACGGTTTTGTCAATATTTCAACATCCTACAAGCCGATTCCAAG
TTTTCAAGCTGGTTGGCATAAGAGTGCATATGGGAACTGGTGGTATCAAGCGCCTGATTCTACAGGAGAGTATGCTG
TCGGTTGGAATGAAATCGAAGGTGAATGGTATTACTTTAACCAAACGGGTATCTTGTTACAGAATCAATGGAAAAAA
TGGAACAATCATTGGTTCTATTTGACAGACTCTGGTGCTTCTGCTAAAAATTGGAAGAAAATCGCTGGAATCTGGTA
TTATTTTAACAAAGAAAACCAGATGGAAATTGGTTGGATTCAAGATAAAGAGCAGTGGTATTATTTGGATGTTGATG
GTTCTATGAAGACAGGATGGCTTCAATATATGGGGCAATGGTATTACTTTGCTCCATCAGGGGAAATGAAAATGGGC
TGGGTAAAAGATAAAGAAACCTGGTACTATATGGATTCTACTGGTGTCATGAAGACAGGTGAGATAGAAGTTGCTGG
TCAACATTATTATCTGGAAGATTCAGGAGCTATGAAGCAAGGCTGGCATAAAAAGGCAAATGATTGGTATTTCTACA
AGACAGACGGTTCACGAGCTGTGGGTTGGATCAAGGACAAGGATAAATGGTACTTCTTGAAAGAAAATGGTCAATTA
CTTGTGAACGGTAAGACACCAGAAGGTTATACTGTGGATTCAAGTGGTGCCTGGTTAGTGGATGTTTCGATCGAGAA
ATCTGCTACAATTAAAACTACAAGTCATTCAGAAATAAAAGAATCCAAAGAAGTAGTGAAAAAGGATCTTGAAAATA
AAGAAACGAGTCAACATGAAAGTGTTACAAATTTTTCAACTAGTCAAGATTTGACATCCTCAACTTCACAAAGCTCT
GAAACGAGTGTAAACAAATCGGAATCAGAACAGTAG

MKKKLTSLALVGAFLGLSWYGNVQAQESSGNKIHFINVQEGGSDAIILESNGHFAMVDTGEDYDFPDGSDSRYPWRE
GIETSYKHVLTDRVFRRLKELGVQKLDFILVTHTHSDHIGNVDELLSTYPVDRVYLKKYSDSRITNSERLWDNLYGY
DKVLQTAAEKGVSVIQNITQGDAHFQFGDMDIQLYNYENETDSSGELKKIWDDNSNSLISVVKVNGKKIYLGGDLDN
VHGAEDKYGPLIGKVDLMKFNHHHDTNKSNTKDFIKNLSPSLIVQTSDSLPWKNGVDSEYVNWLKERGIERINAASK
DYDATVFDIRKDGFVNISTSYKPIPSFQAGWHKSAYGNWWYQAPDSTGEYAVGWNEIEGEWYYFNQTGILLQNQWKK
WNNHWFYLTDSGASAKNWKKIAGIWYYFNKENQMEIGWIQDKEQWYYLDVDGSMKTGWLQYMGQWYYFAPSGEMKMG
WVKDKETWYYMDSTGVMKTGEIEVAGQHYYLEDSGAMKQGWHKKANDWYFYKTDGSRAVGWIKDKDKWYFLKENGQL
LVNGKTPEGYTVDSSGAWLVDVSIEKSATIKTTSHSEIKESKEVVKKDLENKETSQHESVTNFSTSQDLTSSTSQSS
ETSVNKSESEQZ

ID309
ATGGAAATTAATGTGAGTAAATTAAGAACAGATTTGCCTCAAGTCGGCGTGCAACCATATAGGCAAGTACACGCACA
CTCAACTGGGAATCCGCATTCAACCGTACAGAATGAAGCGGATTATCACTGGCGGAAAGACCCAGAATTAGGTTTTT
TCTCGCACATTGTTGGGAACGGTTGCATCATGCAGGTAGGACCTGTTGATAATGGTGCCTGGGACGTTGGGGGCGGT
TGGAATGCTGAGACCTATGCAGCGGTTGAACTGATTGAAAGCCATTCAACCAAAGAAGAGTTCATGACGGACTACCG
CCTTTATATCGAACTCTTACGCAATCTAGCAGATGAAGCAGGTTTGCCGAAAACGCTTGATACAGGGAGTTTAGCTG
GAATTAAAACGCACGAGTATTGCACGAATAACCAACCAAACAACCACTCAGACCACGTTGACCCTTATCCATATCTT
GCTAAATGGGGCATTAGCCGTGAGCAGTTTAAGCATGATATTGAGAACGGCTTGACGATTGAAACAGGCTGGCAGAA
GAATGACACTGGCTACTGGTACGTACATTCAGACGGCTCTTATCCAAAAGACAAGTTTGAGAAAATCAATGGCACTT
GGTACTACTTTGACAGTTCAGGCTATATGCTTGCAGACCGCTGGAGGAAGCACACAGACGGCAACTGGTACTGGTTC
GACAACTCAGGCGAAATGGCTACAGGCTGGAAGAAAATCGCTGATAAGTGGTACTATTTCAACGAAGAAGGTGCCAT
GAAGACAGGCTGGGTCAAGTACAAGGACACTTGGTACTACTTAGACGCTAAAGAAGGCGCCATGGTATCAAATGCCT
TTATCCAGTCAGCGGACGGAACAGGCTGGTACTACCTCAAACCAGACGGAACACTGGCAGACAAGCCAGAATTCACA
GTAGAGCCAGATGGCTTGATTACAGTAAAATAA

MEINVSKLRTDLPQVGVQPYRQVHAHSTGNPHSTVQNEADYHWRKDPELGFFSHIVGNGCIMQVGPVDNGAWDVGGG
WNAETYAAVELIESHSTKEEFMTDYRLYIELLRNLADEAGLPKTLDTGSLAGIKTHEYCTNNQPNNHSDHVDPYPYL
AKWGISREQFKHDIENGLTIETGWQKNDTGYWYVHSDGSYPKDKFEKINGTWYYFDSSGYMLADRWRKHTDGNWYWF
DNSGEMATGWKKIADKWYYFNEEGAMKTGWVKYKDTWYYLDAKEGAMVSNAFIQSADGTGWYYLKPDGTLADKPEFT
VEPDGLITVKZ

ID310
ATGGCACAACAGGATTTACAATAATTGACTTAATTATCTTGATTGTTTATTTACTTGCGGTGTTGGTTGCAGGTAT
CTATTTCTCTAAAAAAGAGATGAAAGGAAAAGAGTTCTTTAAAGGAGATGGTTCGGTTCCTTGGTATGTTACTTCGG
TATCCATTTTTGCCACAATGCTCAGTCCGATTTCCTTCTTGGGACTCGCTGGTAGCTCTTATGCAGGTAGCTGGATT
TTATGGTTTGCTCAATTAGGGATGGTAGTAGCTATTCCACTGACAATTCGTTTTATCTTACCTATCTTTGCACGGAT
AGACATCGATACGGCATATGATTACTTGGATAAACGTTTTAATTCTAAAGCACTTCGTATTATTTCAGCACTCTTGT
TTATTATTTATCAATTGGGACGTATGTCTATCATTATGTACCTCCCATCAGCTGGTTTATCAGTATTGACAGGAATT
GACATCAATATTTTGATTATTTTGATGGGTGTAGTTGCAATTGTTTATTCTTATACTGGTGGTCTAAAATCCGTATT
ATGGACAGACTTTATTCAAGGTGTGATTCTGATTAGTGGTGTCGTTTTAGCTTTATTTGTACTGATTGCTAATATTA
AAGGTGGCTTTGGTGCAGTAGCAGAAACATTAGCAAACGGGAAATTCCTTGCTGCAAATGAAAAACTTTTCGATCCT
AACTTGCTTTCAAACTCCATCTTTTTAATTGTGATGGGTTCAGGCTTTACAATCTTGTCTTCCTATGCTTCATCTCA

AGATTTGGTTCAACGTTTTACTACAACACAAAATATTAAGAAACTTAATAAGATGTTGTTCACAAACGGTGTTTTGT
CACTTGCAACTGCAACAGTCTTTTACTTGATTGGTACAGGCTTGTACGTATTCTATCAAGTACAAAATGCAGATAGT
GCAGCTAGCAATATCCCTCAAGACCAAATCTTTATGTACTTTATTGCATACCAGTTACCAGTAGGTATCACAGGTTT
GATCTTGGCAGCGATTTATGCAGCATCTCAATCAACTATTTCAACAGGTTTGAACTCTGTTGCAACTTCATGGACAT
TGGATATTCAAGATGTCATTTCTAAAAATATGTCAGACAATCGTCGTACGAAAATTGCACAATTCGTATCTCTAGCA
GTAGGTTTATTCTCAATTGGTGTTTCCATTGTCATGGCTCACTCAGATATTAAATCTGCATACGAATGGTTCAATAG
TTTCATGGGACTTGTACTTGGTCTACTTGGTGGTGTATTTATTCTTGGATTTGTTTCTAAAAAAGCAAATAAACAAG
GTGCTTATGCAGCGCTGATTGTATCAACCATCGTCATGGTATTTATTAAATACTTCCTTCCTCCAACAGCTGTTAGC
TACTGGGCATATTCATTGATTTCAATCTCTGTATCAGTAGTTTCAGGTTATATTGTATCTGTTCTTACTGGAAATAA
AGTATCTGCACCTAAATATACAACGATTCATGATATTACAGAAATTAAAGCGGATTCAAGTTGGGAAGTTCGTCACT
AA

MGTTGFTIIDLIILIVYLLAVLVAGIYFSKKEMKGKEFFKGDGSVPWYVTSVSIFATMLSPISFLGLAGSSYAGSWI
LWFAQLGMVVAIPLTIRFILPIFARIDIDTAYDYLDKRFNSKALRIISALLFIIYQLGRMSIIMYLPSAGLSVLTGI
DINILIIILMGVVAIVYSYTGGLKSVLWTDFIQGVILISGVVLALFVLIANIKGGFGAVAETLANGKFLAANEKLFDP
NLLSNSIFLIVMGSGFTILSSYASSQDLVQRFTTTQNIKKLNKMLFTNGVLSLATATVFYLIGTGLYVFYQVQNADS
AASNIPQDQIFMYFIAYQLPVGITGLILAAIYAASQSTISTGLNSVATSWTLDIQDVISKNMSDNRRTKIAQFVSLA
VGLFSIGVSIVMAHSDIKSAYEWFNSFMGLVLGLLGGVFILGFVSKKANKQGAYAALIVSTIVMVFIKYFLPPTAVS
YWAYSLISISVSVVSGYIVSVLTGNKVSAPKYTTIHDITEIKADSSWEVRHZ

ID311
ATGAAAATTAATAAAAAATATCTAGCAGGTTCAGTGGCAGTCCTTGCCCTAAGTGTTTGTTCCTATGAGCTTGGTCG
TCACCAAGCTGGTCAGGATAAGAAAGAGTCTAATCGAGTTGCTTATATAGATGGTGATCAGGCTGGTCAAAAGGCAG
AAAACTTGACACCAGATGAAGTCAGTAAGAGGGAGGGGATCAACGCCGAACAAATCGTCATCAAGATTACGGATCAA
GGTTATGTGACCTCTCATGGAGACCATTATCATTACTATAATGGCAAGGTCCCTTATGATGCCATCATCAGTGAAGA
GCTCCTCATGAAAGATCCGAATTATCAGTTGAAGGATTCAGACATTGTCAATGAAATCAAGGGTGGTTATGTCATCA
AGGTAGACGGAAAATACTATGTTTACCTTAAGGATGCAGCTCATGCGGATAATATTCGGACAAAAGAAGAGATTAAA
CGTCAGAAGCAGGAACGCAGTCATAATCACGGGTCAGGAGCTAACGATCATGCAGTAGCTGCAGCCAGAGCCCAAGG
ACGCTATACAACGGATGATGGGTATATCTTCAATGCATCTGATATCATTGAGGACACGGGTGATGCTTATATCGTTC
CTCACGGCGACCATTACCATTACATTCCTAAGAATGAGTTATCAGCTAGCGAGTTAGCTGCTGCAGAAGCCTATTGG
AATGGGAAGCAGGGATCTCGTCCTTCTTCAAGTTCTAGTTATAATGCAAATCCAGCTCAACCAAGATTGTCAGAGAA
CCACAATCTGACTGTCACTCCAACTTATCATCAAAATCAAGGGGAAAACATTTCAAGCCTTTTACGTGAATTGTATG
CTAAACCCTTATCAGAACGCCATGTGGAATCTGATGGCCTTATTTTCGACCCAGCGCAAATCACAAGTCGAACCGCC
AGAGGTGTAGCTGTCCCTCATGGTAACCATTACCACTTTATCCCTTATGAACAAATGTCTGAATTGGAAAAACGAAT
TGCTCGTATTATTCCCCTTCGTTATCGTTCAAACCATTGGGTACCAGATTCAAGACCAGAACAACCAAGTCCACAAT
CGACTCCGGAACCTAGTCCAAGTCCGCAACCTGCACCAAATCCTCAACCAGCTCCAAGCAATCCAATTGATGAGAAA
TTGGTCAAAGAAGCTGTTCGAAAAGTAGGCGATGGTTATGTCTTTGAGGAGAATGGAGTTTCTCGTTATATCCCAGC
CAAGGATCTTTCAGCAGAAACAGCAGCAGGCATTGATAGCAAACTGGCCAAGCAGGAAAGTTTATCTCATAAGCTAG
GAGCTAAGAAAACTGACCTCCCATCTAGTGATCGAGAATTTTACAATAAGGCTTATGACTTACTAGCAAGAATTCAC
CAAGATTTACTTGATAATAAAGGTCGACAAGTTGATTTTGAGGCTTTGGATAACCTGTTGGAACGACTCAAGGATGT
CCCAAGTGATAAAGTCAAGTTAGTGGATGATATTCTTGCCTTCTTAGCTCCGATTCGTCATCCAGAACGTTTAGGAA
AACCAAATGCGCAAATTACCTACACTGATGATGAGATTCAAGTAGCCAAGTTGGCAGGCAAGTACACAACAGAAGAC
GGTTATATCTTTGATCCTCGTGATATAACCAGTGATGAGGGGGATGCCTATGTAACTCCACATATGACCCATAGCCA
CTGGATTAAAAAAGATAGTTTGTCTGAAGCTGAGAGAGCGGCAGCCCAGGCTTATGCTAAAGAGAAAGGTTTGACCC
CTCCTTCGACAGACCATCAGGATTCAGGAAATACTGAGGCAAAAGGAGCAGAAGCTATCTACAACCGCGTGAAAGCA
GCTAAGAAGGTGCCACTTGATCGTATGCCTTACAATCTTCAATATACTGTAGAAGTCAAAAACGGTAGTTTAATCAT
ACCTCATTATGACCATTACCATAACATCAAATTTGAGTGGTTTGACGAAGGCCTTTATGAGGCACCTAAGGGGTATA
CTCTTGAGGATCTTTTGGCGACTGTCAAGTACTATGTCGAACATCCAAACGAACGTCCGCATTCAGATAATGGTTTT
GGTAACGCTAGCGACCATGTTCAAAGAAACAAAAATGGTCAAGCTGATACCAATCAAACGGAAAAACCAAGCGAGGA
GAAACCTCAGACAGAAAAACCTGAGGAAGAAACCCCTCGAGAAGAGAAACCGCAAAGCGAGAAACCAGAGTCTCCAA
AACCAACAGAGGAACCAGAAGAATCACCAGAGGAATCAGAAGAACCTCAGGTCGAGACTGAAAAGGTTGAAGAAAAA
CTGAGAGAGGCTGAAGATTTACTTGGAAAAATCCAGGATCCAATTATCAAGTCCAATGCCAAAGAGACTCTCACAGG
ATTAAAAAATAATTTACTATTTGGCACCCAGGACAACAATACTATTATGGCAGAAGCTGAAAAACTATTGGCTTTAT
TAAAGGAGAGTAAGTAA

MKINKKYLAGSVAVLALSVCSYELGRHQAGQDKKESNRVAYIDGDQAGQKAENLTPDEVSKREGINAEQIVIKITDQ
GYVTSHGDHYHYYNGKVPYDAIISEELLMKDPNYQLKDSDIVNEIKGGYVIKVDGKYYVYLKDAAHADNIRTKEEIK
RQKQERSHNHGSGANDHAVAAARAQGRYTTDDGYIFNASDIIEDTGDAYIVPHGDHYHYIPKNELSASELAAAEAYW
NGKQGSRPSSSSSYNANPAQPRLSENHNLTVTPTYHQNQGENISSLLRELYAKPLSERHVESDGLIFDPAQITSRTA
RGVAVPHGNHYHFIPYEQMSELEKRIARIIPLRYRSNHWVPDSRPEQPSPQSTPEPSPSPQPAPNPQPAPSNPIDEK

LVKEAVRKVGDGYVFEENGVSRYIPAKDLSAETAAGIDSKLAKQESLSHKLGAKKTDLPSSDREFYNKAYDLLARIH
QDLLDNKGRQVDFEALDNLLERLKDVPSDKVKLVDDILAFLAPIRHPERLGKPNAQITYTDDEIQVAKLAGKYTTED
GYIFDPRDITSDEGDAYVTPHMTHSHWIKKDSLSEAERAAAQAYAKEKGLTPPSTDHQDSGNTEAKGAEAIYNRVKA
AKKVPLDRMPYNLQYTVEVKNGSLIIPHYDHYHNIKFEWFDEGLYEAPKGYTLEDLLATVKYYVEHPNERPHSDNGF
GNASDHVQRNKNGQADTNQTEKPSEEKPQTEKPEEETPREEKPQSEKPESPKPTEEPEESPEESEEPQVETEKVEEK
LREAEDLLGKIQDPIIKSNAKETLTGLKNNLLFGTQDNNTIMAEAEKLLALLKESKZ

ID312
ATGGAGGGATTGGTTAGAGTGCATTTATTGCCTGTATTTGGCGATTACAAGCTATCTAAACTTACTACGCCTATTCT
TCAACAGCAAGTAAACAAATGGGCTGACAAGGCAAATAAAGGCGAAAAAGGGGCATTTGCTAACTACTCTTTGCTCC
ATAACATGAATAAGCGTATTTTGAAATATGGCGTAGCTATCCAGGTAATACAATACAACCCAGCTAATGATGTCATC
GTTCCACGCAAACAGCAAAAAGAAAAGGCTGCTGTCAAATACTTAGACAACAAAGAATTAAAACAGTTTCTTGATTA
TTTAGATGCTCTGGATCAATCAAATTATGAGAACTTATTTGATGTTGTTCTGTATAAGACTTTATTGGCCACTGGTT
GCCGTATTAGTGAGGCTCTGGCTCTTGAATGGTCTGATATTGACCTAGAAAGCGGTGTTATCAGCATCAATAAGACA
CTAAACCGCTATCAGGAAATAAACTCACCTAAATCAAGCGCTGGTTATCGTGATATACCAATAGACAAAGCCACATT
ACTTTTACTGAAACAATACAAAAACCGTCAACAAATTCAGTCTTGGAAATTAGGCCGATCTGAAACAGTTGTATTCT
CTGTATTTACGGAGAAATATGCTTATGCTTGTAACTTACGCAAACGCCTAAATAAGCATTTTGATGCTGCTGGAGTA
ACTAACGTATCATTTCATGGTTTCCGCCATACACATACTACTATGATGCTCTATGCTCAGGTTAGCCCGAAAGATGT
TCAGTATAGATTAGGCCACTCTAATTTAATGATCACTGAAAATACTTACTGGCATACTAACCAAGAGAATGCAAAAA
AAGCCGTCTCAAATTATGAAACAGCTATCAACAATTTATAA

MEGLVRVHLLPVFGDYKLSKLTTPILQQQVNKWADKANKGEKGAFANYSLLHNMNKRILKYGVAIQVIQYNPANDVI
VPRKQQKEKAAVKYLDNKELKQFLDYLDALDQSNYENLFDVVLYKTLLATGCRISEALALEWSDIDLESGVISINKT
LNRYQEINSPKSSAGYRDIPIDKATLLLLKQYKNRQQIQSWKLGRSETVVFSVFTEKYAYACNLRKRLNKHFDAAGV
TNVSFHGFRHTHTTMMLYAQVSPKDVQYRLGHSNLMITENTYWHTNQENAKKAVSNYETAINNLZ

## Claims

1. A *Streptococcus pneumoniae* protein or polypeptide having a sequence selected from 
MKKKLTSLALVGAFLGLSWYGNVQAQESSGNKIHFINVQEGGSDAIILESNG
HFAMVDTGEDYDFPDGSDSRYPWREGIETSYKHVLTDRVFRRLKELGVQKL
DFILVTHTHSDHIGNVDELLSTYPVDRVYLKKYSDSRITNSERLWDNLYGYD
KVLQTAAEKGVSVIQNITQGDAHFQFGDMDIQLYNYENETDSSGELKKIWDD
NSNSLISVVKVNGKKIYLGGDLDNVHGAEDKYGPLIGKVDLMKFNHHHDTN
KSNTKDFIKNLSPSLIVQTSDSLPWKNGVDSEYVNWLKERGIERINAASKDYD
ATVFDIRKDGFVNISTSYKPIPSFQAGWHKSAYGNWWYQAPDSTGEYAVGW
NEIEGEWYYFNQTGILLQNQWKKWNNHWFYLTDSGASAKNWKKIAGIWYY
FNKENQMEIGWIQDKEQWYYLDVDGSMKTGWLQYMGQWYYFAPSGEMK
MGWVKDKETWYYMDSTGVMKTGEIEVAGQHYYLEDSGAMKQGWHKKAN
DWYFYKTDGSRAVGWIKDKDKWYFLKENGQLLVNGKTPEGYTVDSSGAWL
VDVSIEKSATIKTTSHSEIKESKEVVKKDLENKETSQHESVTNFSTSQDLTSSTS
QSSETSVNKSESEQ
 or any other of those shown in table 4.

2. A *Streptococcus pneumoniae* protein or polypeptide having a sequence selected from those shown in table 2.

3. A protein or polypeptide as claimed in claim 1 or claim 2 provided in substantially pure form.

4. A protein or polypeptide which is substantially identical to one defined in any one of claims 1 to 3.

5. A homologue or derivative of a protein or polypeptide as defined in any one of claims 1 to 4.

6. An antigenic and/or immunogenic fragment of a protein or polypeptide as defined in Tables 2-4.

7. A nucleic acid molecule comprising or consisting of a sequence which is:
(i) any of the DNA sequences set out in Table 1 or their RNA equivalents;
(ii) a sequence which is complementary to any of the sequences of (i);
(iii) a sequence which codes for the same protein or polypeptide, as those sequences of (i) or (ii);
(iv) a sequence which is substantially identical with any of those of (i), (ii) and (iii);
(v) a sequence which codes for a homologue, derivative or fragment of a protein as defined in Table 1.

8. A nucleic acid molecule comprising or consisting of a sequence which is:
(i) any of the DNA sequences set out in Table 4 or their RNA equivalents;
(ii) a sequence which is complementary to any of the sequences of (i);
(iii) a sequence which codes for the same protein or polypeptide, as those sequences of (i) or (ii);
(iv) a sequence which is substantially identical with any of those of (i), (ii) and (iii);
(v) a sequence which codes for a homologue, derivative or fragment of a protein as defined in Table 4.

9. The use of a protein or polypeptide having a sequence selected from those shown in Tables 2-4, or homologues, derivatives and/or fragments thereof, as an immunogen and/or antigen.

10. An immunogenic and/or antigenic composition comprising one or more proteins or polypeptides selected from those whose sequences are shown in Tables 2-4, or homologues or derivatives thereof, and/or fragments of any of these.

11. An immunogenic and/or antigenic composition as claimed in claim 10 which is a vaccine or is for use in a diagnostic assay.

12. A vaccine as claimed in claim 11 which comprises one or more additional components selected from excipients, diluents, adjuvants or the like.

13. A vaccine composition comprising one or more nucleic acid sequences as defined in Tables 1, 3 or 4.

14. A method for the detection/diagnosis of *S.pneumoniae* which comprises the step of bringing into contact a sample to be tested with at least one protein or polypeptide as defined in Tables 2-4, or homologue, derivative or fragment thereof.

15. An antibody capable of binding to a protein or polypeptide as defined in Tables 2-4, or for a homologue, derivative or fragment thereof.

16. An antibody as defined in claim 15 which is a monoclonal antibody.

17. A method for the detection/diagnosis of *S.pneumoniae* which comprises the step of bringing into contact a sample to be tested and at least one antibody as define din claim 15 or claim 16.

18. A method for the detection/diagnosis of *S.pneumoniae* which comprises the step of bringing into contact a sample to be tested with at least one nucleic acid sequence as defined in claim 7 or claim 8.

19. A method of determining whether a protein or polypeptide as defined in Tables 2-4 represents a potential anti-microbial target which comprises inactivating said protein or polypeptide and determining whether *S.pneumoniae* is still viable *in vitro* or *in vivo.*

20. The use of an agent capable of antagonising, inhibiting or otherwise interfering with the function or expression of a protein or polypeptide as defined in Tables 2-4 in the manufacture of a medicament for use in the treatment or prophylaxis of *S.pneumoniae* infection
